# EUROPEAN PATENT APPLICATION

(11) **EP 4 339 189 A1**
(43) Date of publication of application: **20.03.2024**
(21) Application number: 22806811.0
(22) Date of filing: 11.05.2022
(51) Int. Cl.: C07D 265/36, C07D 267/14, A61K 31/538, A61K 31/553, A61P 23/00, A61P 25/04, A61P 9/10, A61P 25/00, A61P 25/28, A61P 25/16, A61P 25/14, A61P 25/24, A61P 25/22, A61P 25/18, A61P 25/08, A61P 29/00

(54) **NMDA RECEPTOR ANTAGONIST AND USE THEREOF**

(30) Priority: 12.05.2021 CN 202110518639
(71) Applicant: Synphatec (Shanghai) Biopharmaceutical Technology Co., Ltd., Shanghai 201204 (CN)
(72) Inventor: ZHU, Jidong, Shanghai 200032 (CN); CHEN, Yelin, Shanghai 200032 (CN); GENG, Yang, Shanghai 200032 (CN); CAO, Hengyi, Shanghai 200032 (CN); LI, Shiyun, Shanghai 200032 (CN); LI, Ziwen, Shanghai 200032 (CN); WANG, Qiuyan, Shanghai 200032 (CN); SU, Tonghui, Shanghai 200032 (CN); FU, Chaoying, Shanghai 200032 (CN)
(74) Representative: Finetti, Claudia
(86) International application number: PCT/CN2022/092300
(87) International publication number: WO 2022/237849

(57) **Abstract**

The present invention relates to a NMDA receptor antagonist and use thereof. The NMDA receptor antagonist of the present invention is a compound of formula I below, or a pharmaceutically acceptable salt, an enantiomer, a diastereomer, a tautomer, a solvate, an isotopic substituent, a polymorphic substance, a prodrug or a metabolite thereof. In the formula, ring A, ring B, and R2 are as described herein. The present invention also provides a pharmaceutical composition comprising these compounds, and use of these compounds in the preparation of a drug for treating or preventing NMDA receptor-mediated diseases.

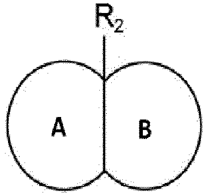

## Description

### Technical Field

The present disclosure relates to NMDA receptor antagonist and use thereof.

### Background Art

NMDA-type glutamate receptor (NMDAR) is a ligand-gated ion channel. This receptor can be activated in vivo by glutamate, the primary excitatory neurotransmitter in central nervous system. It mediates the conduction of intersynaptic excitatory signals. Upon opening its ion channels, NMDAR enhances the permeability of cations such as Ca²⁺, K⁺, and Na⁺, generating excitatory postsynaptic potentials that trigger various physiological and biochemical reactions. Normally, NMDAR is blocked by Mg²⁺ in a voltage-dependent manner during the resting state. Nerve cell membrane depolarization relieves the blocking effect of Mg²⁺ on NMDAR ion channels, and then bind to the corresponding ligand, so as to activate NMDAR. Thus, NMDAR activation is regulated by both the membrane potential and ligand interaction.

A fully functional NMDAR comprises four subunits. Most natural occurring NMDARs consist of two GluNls and two GluN2s (or GluN3s). The function of NMDAR is highly subtype dependent. GluN1 is the basic subtype that forms NMDAR, while GluN2 and GluN3 act as regulatory subtypes, diversifying NMDAR functions. NMDAR properties depend on GluN2 or GluN3 subtype. NMDARs with different GluN2 subtypes have great differences in ion permeability, Mg²⁺ blocking sensitivity, antagonist sensitivity, agonist affinity and channel kinetic properties, etc.

NMDAR has a complex molecular structure, with various subtypes exhibiting specificity in spatio-temporal distribution and pharmacological properties. Its number, composition and distribution show dynamic changes in different developmental periods and different brain regions. NMDARs participate in numerous physiological activities, providing a molecular basis for complex neural activities that ensure the normal functioning of neural networks. The integration, localization, recycling, and intrasynaptic/extrasynaptic distribution of NMDAR depend on neural activity regulation. The failure of its functional homeostasis is highly correlated with many brain diseases, including depression, schizophrenia, epilepsy, etc.

Depression is a serious mental disorder, mainly manifested as depressed mood, low selfesteem, and even suicidal tendencies. Currently, the main drugs for depression treatment are SSRI/SSNI (selective serotonin/norepinephrine reuptake inhibitors). However, these drugs are ineffective in nearly one-third of patients, and there are generally side effects such as slow onset and increased suicidal tendencies. This suggests that the monoamine system may not be the direct cause of depression. Early evidence indicates that NMDAR antagonists, including AP-7 and MK-801, exhibit antidepressant effects in mice. Recent studies found that intravenous injection of subanesthetic doses of NMDAR antagonist ketamine can rapidly relieve symptoms of depression within hours and maintain the effect for at least a week. Ketamine, used as a good anesthetic for over 50 years, provides reliable safety data for clinical use. Therefore, NMDAR is a potential target for rapid-acting antidepressant drugs.

Schizophrenia is a relatively common severe psychosis, affects about 1% of the population, typically appearing in late adolescence. It includes three major types of symptoms: positive, negative and cognitive dysfunction. In the 1970s, the hyperactivity of dopamine hypothesis emerged based on the effectiveness of dopamine D2 receptor antagonists in treating some symptoms of schizophrenia. However, the hypothesis of hyperactivity of dopamine cannot explain negative symptoms and cognitive disorders, and autopsy results of schizophrenic patients find that dopamine and corresponding receptors of striatum are not changed, so it is also believed that hyperactivity of dopamine is not the direct cause of schizophrenia. NMDAR antagonists, such as phencyclidine and ketamine, induce symptoms similar to schizophrenia in healthy individuals and exacerbate symptoms of schizophrenia patients. NMDAR antagonists can also induce similar symptoms of schizophrenia in rodent model animals, such as improving mobility and inhibiting social behavior in rats. Autopsy results of schizophrenic patients show varying changes in different subunits of NMDA in prefrontal cortex. Recently, the hypothesis of low NMDA receptor function causing schizophrenia has gained widespread attention, suggesting that drugs targeting NMDAR may be used in the treatment of schizophrenia.

Epilepsy is a chronic, recurrent, transient brain dysfunctional neurological disorder characterized by highly synchronized abnormal firing of neurons. The cause is currently unknown, with previous studies focusing on the inhibitory neurotransmitter GABA and related receptors. However, many recent studies have found that the activity and expression of NMDAR are altered in epilepsy patients. Clinical trials indicate that NMDAR antagonists have anti-epileptic effects, and commonly used anti-epileptic drugs also have an impact NMDAR function. Human genetic evidence suggests that mutations in NMDAR can cause certain types of epilepsy. NMDAR is present on both excitatory and inhibitory neurons, and changes in NMDAR activity on different neurons may produce diametrically opposed results in brain activity. In fact, changes in excitatory and inhibitory nerve conduction have been found in the brain of epilepsy patients, so targeted development of corresponding drugs for the changes in the activity of NMDAR in different neurons may provide new tools for the treatment of epilepsy.

In addition, due to NMDAR's critical role in synaptic plasticity and excitatory neurotoxicity, its functional abnormalities are also highly associated with multiple neurodegenerative diseases that cause cognitive impairment, such as Alzheimer's disease, Parkinson's disease, and Huntington's disease, etc. The NMDA receptor antibodies produced by human autoimmunity can interfere with the normal function of NMDA receptors, leading to schizophrenia symptoms and anti-NMDA receptor encephalitis, and NMDAR can be used as a potential target to develop corresponding agents for such diseases. NMDA receptor is also a target protein for anesthesia, sedation, and analgesia in both human and veterinary medicine, drawing long-term attention to its potential applications.

### Summary

A first aspect of the present disclosure provides a compound of Formula I, or a pharmaceutically acceptable salt thereof, or an enantiomer, a diastereomer, a tautomer, a solvate, an isotope substitute, a polymorph, a prodrug or a metabolite thereof: 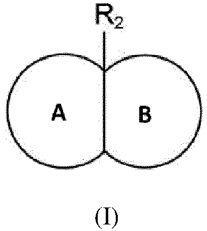 wherein:
ring A is a substituted or unsubstituted 4-10 membered heterocyclic group containing 1-3 heteroatoms selected from the group consisting of N, O and S;
ring B is a substituted or unsubstituted, saturated or unsaturated 3-10 membered carbon ring;
R₂ is a substituted or unsubstituted 3-8 membered cycloalkyl, substituted or unsubstituted 6-14 membered aryl, substituted or unsubstituted 5-10 membered heteroaryl, or substituted or unsubstituted 4-10 membered heterocyclic group.

A second aspect of the present disclosure provides a pharmaceutical composition comprising the compound according to any embodiment of the present disclosure, or a pharmaceutically acceptable salt thereof, or an enantiomer, a diastereomer, a tautomer, a solvate, an isotope substitute, a polymorph, a prodrug or a metabolite thereof and pharmaceutically acceptable carriers.

A third aspect of the present disclosure provides a use of the compound according to the present disclosure, or a pharmaceutically acceptable salt thereof, or an enantiomer, a diastereomer, a tautomer, a solvate, an isotope substitute, a polymorph, a prodrug or a metabolite thereof in the manufacture of drugs for treating or preventing NMDA receptor-mediated diseases, or in the manufacture of anesthetics or analgesics.

A fourth aspect of the present disclosure provides a method for treating or preventing NMDA receptor-mediated diseases, which comprises administering a therapeutically effective amount of the compound according to the present disclosure, or a pharmaceutically acceptable salt thereof, or an enantiomer, a diastereomer, a tautomer, a solvate, an isotope substitute, a polymorph, a prodrug or a metabolite thereof, or pharmaceutical composition thereof to the subject in need.

A detailed description of various aspects of the present disclosure and preferred embodiments are described in various parts of the present disclosure.

### Description of the Drawings

Fig.1 shows the results of whole-cell patch-clamp experiments for Compounds 1-16.
Fig.2 shows the results of whole-cell patch-clamp experiments for Compounds 17-28.

### Detailed Description

In the following description, certain specific details are stated in order to provide a comprehensive understanding of various embodiments. However, those skilled in the art should understand that the present disclosure may be implemented without these details. In other cases, well-known structures are not shown or described in detail to avoid unnecessary confusion of the described embodiment. Unless otherwise required, throughout the description and in the following claims, the words "comprising" and variations thereof, such as "including" and "containing", will be interpreted in an open, inclusive meaning, i.e. interpreted as "including but not limited to". Further, the headings provided herein are for convenience only and do not explain the scope or meaning of the disclosure claimed for protection.

References to "one embodiment" in the present specification mean that the particular features, structures or characteristics described in the embodiment are included in at least one embodiment. Thus, the phrase "in one embodiment" appearing in a plurality of places in the present specification does not necessarily all refer to the same embodiment. Further, the particular features, structures, or characteristics may be combined in any suitable manner in one or more embodiments. Unless otherwise expressly provided in context, the singular forms "one" and "the" include plural forms, as used in the present description and claims. It should also be noted that, unless otherwise expressly defined, the term "or" is generally used in the meaning including "and/or".

### I. Terms

Unless otherwise indicated, as used herein, the following terms have the following meanings:
"Amino" refers to -NH₂.
"Cyano" refers to -CN.
"Halogen" refers to fluorine, chlorine, bromine or iodine.
"Hydroxyl" refers to -OH.
"Imino" refers to =NH.
"Nitro" refers to -NO₂.
"Oxo" refers to =O.
"Thio" refers to =S.

In the present disclosure, as a group or a part of other groups, "alkyl" refers to a fully saturated linear or branched hydrocarbon chain group having 1-12 carbon atoms connected to the rest of the molecule by a single bond, usually expressed as C1-C12 alkyl. In the present disclosure, the alkyl group is preferably C1-C6 alkyl, more preferably C1-C4 alkyl or C1-C3 alkyl. Non-limiting examples of alkyl groups include methyl, ethyl, n-propyl, isopropyl, sec-propyl, n-butyl, isobutyl, sec-butyl, tert-butyl, n-pentyl, tert-pentyl, n-hexyl, n-heptyl, n-octyl, n-nonyl, n-decyl, n-undecyl and n-dodecyl. Unless otherwise specified in the present specification, the alkyl group may be optionally substituted.

In the present disclosure, as a group or a part of other groups, "alkylene" or "alkylidene" refers to a fully saturated linear or branched divalent hydrocarbon chain groups having 1-12 carbon atoms, usually expressed as C1-C12 alkylene. The alkylene group is preferably C1-C6 alkylene, more preferably C1-C4 alkylene. Non-limiting examples of C1-C12 alkylene include methylene, ethylene, propylene, n-butylene, vinylene, propenylidene, n-butenylidene, propynylene, n-butynelene and the like. Alkylidene is linked to the rest of the molecule by a single bond and to said group by a single bond. Alkylidene is connected to the rest of the molecule and the linking point of said group via one carbon or any two carbons in the chain. Unless otherwise specified in the present specification, alkylidene may be optionally substituted.

In the present disclosure, as a group or a part of other groups, "alkenyl" refers to a linear or branched hydrocarbon chain group having two to twelve carbon atoms and one or more carbon-carbon double bonds (-C=C-), usually expressed as C2-C12 alkenyl. The alkenyl group is preferably C2-C6 alkenyl, more preferably C2-C4 alkenyl. Non-limiting examples of alkenyl comprise vinyl, 1-propenyl, 2-propenyl(allyl), isopropenyl, 2-methyl-1-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, 7-octenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, 9-decenyl, 1-undeceneyl, 2-undeceneyl, 3-undeceneyl, 4-undeceneyl, 5-undeceneyl, 6-undeceneyl, 7-undeceneyl, 8-undeceneyl, 9-undeceneyl, 10-undeceneyl, 1-dodecaenyl, 2-dodecaenyl, 3-dodecaenyl, 4-dodecaenyl, 5-dodecaenyl, 6-dodecaenyl, 7-dodecaenyl, 8-dodecaenyl, 9-dodecaenyl, 10-dodecaenyl, 11-dodecaenyl. Unless otherwise specified in the present specification, alkenyl may be optionally substituted.

In the present disclosure, as a group or a part of other groups, "alkenylene" or "alkenylidene" refers to a linear or branched divalent hydrocarbon chain group having two to twelve carbon atoms and one or more carbon-carbon double bonds, usually expressed as C2-C12 alkenylene. The alkenylene group is preferably C2-C6 alkenylene, more preferably C2-C4 alkenylene. Non-limiting examples of C2-C12 alkenylene comprise vinylidene, propenylene, butenylene, etc. Alkenylidene is linked to the rest of the molecule by a single bond and to said group by a single bond. Alkenylidene is connected to the rest of the molecule and the linking point of said group via one or any two carbons in the chain. Unless otherwise specified in the present specification, alkenylidene may be optionally substituted.

In the present disclosure, as a group or a part of other groups, "alkynyl" refers to a linear or branched hydrocarbon chain having two to twelve carbon atoms and one or more carbon-carbon triple bonds (-C=C-), usually expressed as C2-C12 alkynyl. The alkynyl group is preferably C2-C6 alkynyl, more preferably C2-C4 alkynyl. Non-limiting examples comprise ethynyl, propynyl, butynyl, pentynyl, etc. Unless otherwise specified in the present specification, alkynyl may be optionally substituted.

In the present disclosure, as a group or a part of other groups, "alkynylene" or "alkynylidene" refers to a linear or branched divalent hydrocarbon chain having two to twelve carbon atoms and one or more carbon-carbon triple bonds, usually expressed as C2-C12 alkynylene. The alkynylene group is preferably C2-C6 alkynylene, more preferably C2-C4 alkynylene. Non-limiting examples of C2-C12 alkynylene comprise ethynylene, propynylene, etc. Alkynylene is linked to the rest of the molecule by a single bond and to said group by a single bond. Alkynylene is connected to the rest of the molecule and the linking point of said group via one or any two carbons in the chain. Unless otherwise specified in the present specification, alkynylene may be optionally substituted.

In the present disclosure, as a group or a part of other groups, "hydrocarbyloxy " refers to a group of OR, wherein R refers to an alkyl, alkenyl or alkynyl group containing one to twelve carbon atoms as defined herein. The hydrocarbyloxy is preferably alkoxy, preferably C1-C6 alkoxy, more preferably C1-C4 alkoxy. Unless otherwise specified in the present specification, hydrocarbyloxy may be optionally substituted.

In the present disclosure, as a group or a part of other groups, "alkamino" refers to a group of -NHRₐ or -NRₐRₐ, wherein Rₐ is each independently an alkyl, alkenyl or alkynyl group as defined herein. The alkamino group is preferably alkylamino, preferably alkylamino in which Rₐ is each independently C1-C6 alkyl, more preferably alkylamino in which Rₐ is each independently C1-C4 alkyl. Unless otherwise specified in the present specification, alkamino may be optionally substituted.

In the present disclosure, as a group or a part of other groups, "alkcarbonyl" or "acyl" refers to C(=O)R, wherein R refers to an alkyl, alkenyl or alkynyl group as defined herein. The alkcarbonyl group is preferably acyl, i.e., C1-C11 alkyl-C(O)-, preferably C1-C5 alkyl-C(O)-, more preferably C1-C3 alkyl-C(O)-. Unless otherwise specified in the present specification, alkcarbonyl may be optionally substituted.

In the present disclosure, as a group or a part of other groups, the term "cyclic hydrocarbon group" means a stable non-aromatic monocyclic or polycyclic hydrocarbon group (e.g., alkyl, alkenyl or alkynyl) composed only of carbon atoms and hydrogen atoms, which may include a fused ring system, a bridge ring system or a spiro ring system, having 3 to 15 carbon atoms, preferably having 3 to 10 carbon atoms, more preferably having 3 to 8 carbon atoms, such as 3, 4, 5, 6, 7 or 8 carbon atoms, and which are saturated or unsaturated and may be connected to the rest of the molecule by a single bond via any suitable carbon atom. Unless otherwise specified in the present specification, the carbon atom in the cyclic hydrocarbon group may be optionally oxidized. In some preferred embodiments, the cyclic hydrocarbon group is cycloalkyl, preferably C3-C8 cycloalkyl, as well as endocyclic group and spirocyclic group. The number of ring atoms of each endocyclic group and spirocyclic group can be 5-10. Examples of cyclic hydrocarbon group include but not limited to cyclopropyl, cyclobutyl, cyclopentyl, cyclopentenyl, cyclohexyl, cyclohexenyl, cyclohexadienyl, cycloheptyl, cyclooctyl, 1*H*-indenyl, 2,3-indanyl, 1,2,3,4-tetrahydro-naphthyl, 5,6,7,8-tetrahydro-naphthyl, 8,9-dihydro-7*H*-benzocyclohepten-6-yl, 6,7,8,9- tetrahydro-5*H-*benzocycloheptenyl, 5,6,7,8,9,10-hexahydro-benzocyclooctenyl, fluorenyl, dicyclo[2.2.1]heptyl, 7,7-dimethyl-dicyclo[2.2.1]heptyl, dicyclo[2.2.1]heptenyl, dicyclo[2.2.2]octyl, dicyclo[3.1.1]heptyl, dicyclo[3.2.1]octyl, dicyclo[2.2.2]octenyl, dicyclo[3.2.1]octenyl, adamantyl, octahydro-4,7-methylene-1*H*-indenyl and octahydro-2,5-methylene-pentaleno, etc.

The endocyclic group and spirocyclic group may comprise heteroatoms, including but not limited to O, N and S, and the number of heteroatoms may be 1-3. When it contains S, S can be oxygenated, such as di-oxygenated. The endocyclic group and spirocyclic group containing heteroatoms are referred to as bridged heterocyclic groups and spiroheterocyclic groups, respectively. In some embodiments, the number of their ring atoms is 6-10.

In the present disclosure, the term "cyclic hydrocarbon group-alkyl" refers to an alkyl group defined above that is substituted by a cyclic hydrocarbon group defined herein, preferably a cycloalkyl alkyl group

In the present disclosure, as a group or part of other groups, the term "heterocyclic group" refers to a stable 3-20 membered non-aromatic cyclic group composed of 2 to 14 carbon atoms (e.g., 2, 3, 4, 5, 6, 7, 8, 9, 10, 11, 12, 13 or 14 carbon atoms) and 1 to 6 heteroatoms selected from nitrogen, phosphorus, oxygen and sulfur. Unless otherwise specified in the specification, heterocyclic groups may be ring systems of single-ring, bicyclic ring, tricyclic ring or more rings, which may include a fused ring system, a bridge ring system, or a spiro ring system. The nitrogen, carbon or sulfur atoms in the heterocyclic groups can be optionally oxidized. Nitrogen atoms can optionally be quaternized and the heterocyclic groups can be partially or fully saturated. Heterocyclic groups can be connected to the rest of the molecule via carbon atoms or heteroatoms and through single bonds. In a heterocyclic group containing a fused ring, one or more rings may be aryl or heteroaryl as defined below, provided that the connecting point to the rest of the molecule is a non-aromatic ring atom. For the object of the present disclosure, the heterocyclic group is preferably a stable 4-12, 5-12 or 4-10 membered non-aromatic monocyclic, bicyclic, bridged ring or spiro ring group comprising 1 to 3 heteroatoms selected from nitrogen, oxygen and sulfur, more preferably a stable 5-10 membered non-aromatic monocyclic, bicyclic, bridged ring or spiro ring group comprising 1 to 3 heteroatoms selected from nitrogen, oxygen and sulfur. Examples of heterocyclic group in each embodiment of the present disclosure include but are not limited to pyrrolidinyl, morpholinyl, piperazinyl, homopiperazinyl, piperidinyl, thiomorpholinyl, 2,7-diaza-spiro[3.5]nonan-7-yl, 2-oxa-6-aza-spiro [3.3]heptan-6-yl, 2,5-diaza-bicyclo[2.2.1]heptan-2-yl, azetidinyl, oxetanyl, thietanyl, thiolanyl, pyranyl, tetrahydropyranyl, thiopyranyl, tetrahydrofuranyl, oxazinyl, dioxolanyl, imidazolinyl, imidazolidinyl, quinazinyl, thiazolidinyl, isothiazolidinyl, isoxazolidinyl, pyrazolindinyl, phthalimido, thiomorpholine-1,1-dioxide, dioxothiolanyl, dioxothietanyl, thiacyclohexanyl, dioxo-thiacyclohexanyl, thiomorpholinyl, 1,4-oxathianyl, etc.

In the present disclosure, as a group or part of other groups, the term "aryl" means a conjugated hydrocarbon cyclic system group having 6 to 18 carbon atoms, such as 6 to 14 carbon atoms (preferably 6 to 10 carbon atoms, such as 6, 7, 8, 9 or 10 carbon atoms). For the purposes of the present disclosure, the aryl group may be a ring system of single ring, bicyclic ring, tricyclic ring or more rings, and may also be fused with cycloalkyl or heterocyclic group as defined above, provided that the aryl group is connected to the rest of the molecule by a single bond through an atom on the aromatic ring. Examples of the aryl group in each embodiment of the present disclosure include, but are not limited to, phenyl, naphthyl, anthracenyl, phenanthrenyl, fluorenyl, 2,3-dihydro-1H-isoindolyl, 2-benzoxazolinonyl, 2H-1,4-benzoxazin-3(4H)-one-7-yl, etc. Unless otherwise specified in the present specification, the term "aryl" comprises optionally substituted aryl.

In the present disclosure, the term "aralkyl" refers to the alkyl group defined above that is substituted by the aryl group defined above.

In the present disclosure, as a group or part of other groups, the term "heteroaryl" refers to a 5-16 membered conjugated ring group, wherein the ring has 1 to 15 carbon atoms (preferably 1 to 10 carbon atoms, such as 1, 2, 3, 4, 5, 6, 7, 8, 9 or 10 carbon atoms) and 1 to 6 heteroatoms selected from nitrogen, oxygen and sulfur. Unless otherwise specified in this specification, the heteroaryl group may be a ring system of single-ring, bicyclic ring, tricyclic ring or more rings, and may also be fused to cycloalkyl or heterocyclic groups as defined above, provided that the heteroaryl group is connected to the rest of the molecule by a single bond through an atom on the aromatic ring. The nitrogen, carbon or sulfur atoms in the heteroaryl group can be optionally oxidized. Nitrogen atoms can optionally be quaternized. For the purposes of the present disclosure, the heteroaryl group is preferably a stable 5-12 membered aromatic group comprising 1 to 5 heteroatoms selected from nitrogen, oxygen and sulfur, more preferably a stable 5-10 membered aromatic group comprising 1 to 4 heteroatoms selected from nitrogen, oxygen and sulfur or a 5-6 membered aromatic group comprising 1 to 3 heteroatoms selected from nitrogen, oxygen and sulfur. Examples of the heteroaryl group in each embodiment of the present disclosure include, but are not limited to, thienyl, imidazolyl, pyrazolyl, thiazolyl, isothiazolyl, oxazolyl, oxadiazolyl, isoxazolyl, pyridyl, pyrimidinyl, pyrazinyl, pyridazinyl, benzimidazolyl, benzopyrazolyl, benzoindolyl, benzomorpholinyl, benzisodiazolyl, indoyl, furanyl, pyrrolyl, triazolyl, tetrazoly, triazinyl, indolizinyl, isoindolyl, indazolyl, isoindazolyl, purinyl, quinolyl, isoquinolyl, phthalazinyl, naphthyridinyl, quinoxalinyl, peteridinyl, carbazolyl, carbolinyl, phenanthridinyl, phenanthrolinyl, acridinyl, phenazinyl, isothiazolyl, benzothiazolyl, benzothienyl, oxatriazolyl, cinnolinyl, quinazolinyl, phenylthio, indolizinyl, o-phenanthrolinyl, isoxazolyl, phenoxazinyl, phenothiazinyl, 4,5,6,7-tetrahydrobenzo[b]thienyl, naphthopyridyl, [1,2,4]triazolo[4,3-b]pyridazine, [1,2,4]triazolo[4,3-a]pyrazine, [1,2,4]triazolo[4,3-c]pyrimidine, [1,2,4]triazolo[4,3-a]pyridine, imidazolo[1,2-a]pyridine, imidazolo[1,2-b]pyridazine, imidazolo[1,2-a]pyrazine, tetrahydroisoquinolinyl, decahydroisoquinolinyl, dihydroindolyl, octahydroindolyl and octahydroisoindolyl, etc.

In the present disclosure, the term "heteroaralkyl" refers to the alkyl group defined above that is substituted by the heteroaryl group defined above.

In the present disclosure, "optional" or "optionally" means that the event or situation subsequently described may or may not occur, and the expression includes both the occurrence and non-occurrence of the event or situation. For example, "optionally substituted aryl" means that the aryl group is substituted or unsubstituted, and the expression includes both substituted aryl and unsubstituted aryl. When substituted, the "optional" substituents described in the claims and specification of the present disclosure include, but are not limited to, one or more of alkyl, alkenyl, alkynyl, halogen, halogenated alkyl, halogenated alkenyl, halogenated alkynyl, hydroxyl substituted alkyl, alkoxy, hydroxyl substituted alkoxy, cyano, hydroxyl, amino, monoalkylamino, dialkylamino, nitro, optionally substituted aryl, optionally substituted heteroaryl, optionally substituted cyclic hydrocarbon group, and optionally substituted heterocyclic groups. These groups as substituents include alkyl, alkenyl, alkynyl, alkyl in halogenated alkyl groups, alkenyl groups in haloalkenyl groups, alkynyl in halogenated alkyl, alkenyl in haloalkenyl, alkynyl in haloalkynyl, alkoxy, alkyl in monoalkylamino, alkyl in dialkylamino, aryl, heteroaryl, cyclic hydrocarbon groups and heterocyclic groups, which may be optionally substituted one or more groups selected from alkyl, halogen, halogenated alkyl, alkoxy, hydroxyl, amino, monoalkyl amino, dialkyl amino, nitro, aryl, heteroaryl, cyclic hydrocarbon groups and heterocyclic groups. Herein, the number of substituents may be 1 or more, i.e., 1, 2, 3, 4, 5 or 6 or more, depending on the nature of the group to be substituted and the substituents. For example, when the substituent is halogen, according to the structure of the substituted group, the group may be substituted with 1-6 substituents, such as trifluoromethyl, pentafluoroethyl, etc. When the substituents are aryl, heteroaryl, heterocyclic groups, cycloalkyl, cyano, sulfonyl, etc., the number of substituents is usually 1.

As used herein, the terms "part", "structural part", "chemical part", "group", "chemical group" refer to a specific fragment or functional group in a molecule. Chemical parts are often considered as chemical entities embedded or attached to molecules.

Those skilled in the art should also understand that in the method described below, the functional group of intermediate compounds may need protecting by an appropriate protective group. Such functional groups include hydroxyl, amino, mercapto and carboxyl. Suitable protective groups for hydroxyl include trialkyl silyl or diaryl alkylsilyl (e.g., tert-butyldimethylsilyl, tert-butyldiphenylsilyl or trimethylsilyl), tetrahydropyranyl, benzyl and the like. Suitable protective groups for amino, amidinyl and guanidinyl include tert-butoxycarbonyl, benzyloxycarbonyl, etc. Suitable protective groups for mercapto include -C(O)-R" (where R" is alkyl, aryl or aralkyl), p-methoxybenzyl, triphenylmethyl, etc. Suitable protective groups for carboxyl include esters of alkyl, aryl, or aralkyl.

The protective group may be introduced and removed according to standard techniques known to those skilled in the art and described herein. The use of protective groups is described in Greene, T. W. and P. G. M. Wuts, Protective Groups in Organi Synthesis, (1999), 4th Ed., Wiley. The protective group may also be a polymer resin.

As used herein, "subject" may be humans, non-human primates, mammals, rats, mice, cattle, horses, pigs, sheep, goats, dogs, cats, etc. Subjects may be suspected of having or suffering from neurodegenerative conditions, including cerebrovascular disease, epilepsy, schizophrenia, Alzheimer's disease, Parkinson's disease and Huntington's disease, cerebral ischemia, infarction, stroke, traumatic brain injury, neuropathic pain, psychosis. Subjects may also be suspected of having or suffering from other neurological events or neurodegeneration caused by NMDA receptor activation. Subjects may also be suspected of having or having a brain tumor, such as glioma.

"Mammals" include humans; keeping animals, such as laboratory animals, household pets (e.g., cats, dogs, pigs, cattle, sheep, goats, horses, rabbits), zoo animals (e.g., tigers, monkeys, bears, etc.), and non-keeping animals, such as wild animals, etc.

"Pharmaceutically acceptable carriers, diluents, or excipients" include, but are not limited to, any adjuvants, carriers, excipients, flow aids, sweeteners, diluents, preservatives, dyes/colorants, flavor enhancers, surfactants, humectants, dispersants, suspending agent s, stabilizers, isotonic agents, solvents or emulsifiers that have been approved, for example, by the U.S. Food and Drug Administration (FDA) for use in humans or keeping animals.

In the present disclosure, the term "pharmaceutically acceptable salt" includes pharmaceutically acceptable acid addition salt and pharmaceutically acceptable base addition salt.

"Pharmaceutically acceptable acid-addition salts" are salts formed with inorganic or organic acids that retain the bioavailability of free bases without other side effects. Inorganic salts include but are not limited to hydrochloride, hydrobromate, sulfate, nitrate, phosphate, etc. Organic salts include, but are not limited to, formate, acetate, 2,2-dichloroacetate, trifluoroacetate, propionate, caproate, octanoate, caprate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, mesylate, benzenesulfonate, p-toluenesulfonate, alginate, ascorbate, salicylate, 4-aminosalicylate, naphthalene disulfonate, etc. These salts can be prepared by methods known in the art.

"Pharmaceutically acceptable base addition salt" refers to salts formed with inorganic or organic bases that maintain the bioavailability of free acids without other side effects. Salts derived from inorganic bases include but are not limited to sodium salts, potassium salts, lithium salts, ammonium salts, calcium salts, magnesium salts, iron salts, zinc salts, copper salts, manganese salts, aluminum salts, etc. Preferred inorganic salts are ammonium salt, sodium salt, potassium salt, calcium salt and magnesium salt. Salts derived from organic bases include, but are not limited to, the following salts: primary amines, secondary amines and tertiary amines, substituted amines, including natural substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methyl glucosamine, theobromine, purine, piperazine, piperidine, N-ethylpiperidine, polyamide resin, etc. Preferred organic bases include isopropylamine, diethylamine, ethanolamine, trimethylamine, dicyclohexylamine, choline and caffeine. These salts can be prepared by methods known in the art.

Example of prodrugs of compounds of the present disclosure may include simple esters of compounds containing carboxylic acids (e.g., esters obtained by condensation with C1-4 alcohols according to methods known in the art); esters of compounds containing hydroxyl groups (e.g., esters obtained by condensation with Cl-4 carboxylic acid, C3-6 diacid or their anhydrides such as succinic anhydride and fumaric anhydride according to methods known in the art); imines of compounds containing amino groups (e.g., imines obtained by condensation with Cl-4 aldehydes or ketones according to methods known in the art); carbamates of compounds containing amino groups, such as those esters described by Leu et al. (J. Med. Chem., 42:3623-3628 (1999)) and Greenwald et al (J. Med. Chem., 42:3657-3667 (1999)). Aldehyde or ketone acetals of compounds containing alcohols (e.g., those obtained by condensation with chloromethyl methyl ether or chloromethyl ethyl ether according to methods known in the art).

As used herein, the term "solvate" refers to an aggregate comprising one or more molecules of the compounds of the present disclosure and one or more solvent molecules. The solvent may be water, in this case the solvate may be hydrate. Alternatively, the solvent may be an organic solvent. Thus, the compounds of the present disclosure may exist as hydrates, including monohydrates, dihydrates, hemihydrates, sesquihydrates, trihydrates, tetrahydrates, etc., and as corresponding solvated forms. The compounds of the present disclosure may be true solvates, and in other cases, the compounds of the present disclosure may merely conserve indefinite water or a mixture of water plus some indefinite solvents.

"Pharmaceutical composition" means a preparation containing a compound of the present disclosure and a medium generally accepted in the art for delivering a bioactive compound to a mammal (e.g., human). This medium includes all its pharmaceutically acceptable carriers, diluents or excipients.

"Effective amount" means a therapeutic effective amount or a prophylactic effective amount. "Therapeutic effective amount" is the amount that effectively achieves the desired treatment outcome (e.g., reduced tumor size, increased lifespan, or increased life expectancy) at the necessary dose and for a necessary sustained period. The therapeutic effective amount of the compound may vary according to factors such as the subject's disease status, age, sex, and weight, and the ability of the compound to elicit the desired response in the subject. Administering regimens can be adjusted to provide optimal response to treatment. The therapeutic effective amount is also the amount in which any toxic or harmful effects of the compounds are exceeded by the beneficial effects of the treatment. "Prophylactic effective amount" is the amount that effectively achieves the desired prophylactic outcome (e.g., smaller tumors, increased lifespan, increased life expectancy, or prevention of progression of prostate cancer to castration-resistant forms) at the necessary dose and for a necessary sustained period. Typically, prophylactic doses are used in subjects before or in the early stages of the disease such that the prophylactic effective amount can be less than the therapeutically effective amount.

As used herein, "treatment" covers the treatment of a disease or condition of concern in mammals (preferably humans) suffering from the disease or condition of concern, and comprises:
(i) preventing the occurrence of a disease or condition in mammals, especially when such mammals are susceptible to the condition but have not yet been diagnosed with the condition;
(ii) inhibiting a disease or condition, i.e., inhibiting its progression;
(iii) alleviating the disease or condition, i.e., making the state of the disease or condition subside; or
(iv) alleviating symptoms caused by the disease or condition, i.e., relieving pain without addressing the underlying disease or condition.

As used herein, the terms "take", "apply", "administer" and the like refer to a method capable of delivering a compound or composition to the desired site for biological action. All well-known administration methods in the art may be used in the present disclosure. These methods include, but are not limited to, oral administration, transduodenum administration, parenteral injection (including intrapulmonary, intranasal, intrathecal, intravenous, subcutaneous, intraperitoneal, intramuscular, intraarterial injection or infusion), topical administration, and rectal administration. Those skilled in the art are familiar with the application techniques that may be used for the compounds and methods described herein, for example, those discussed in Goodman and Gilman, The Pharmacological Basis of Therapeutics, current ed.; Pergamon; and Remington's, Pharmaceutical Sciences (current edition), Mack Publishing Co., Easton, Pa. In a preferred embodiment, a compound of Formula I according to the present disclosure, a pharmaceutically acceptable salt, or an enantiomer, a diastereomer, a tautomer, a solvate, an isotope substitute, a polymorph, a prodrug or a metabolite thereof or a pharmaceutical composition comprising the compound of Formula I according to the present disclosure is orally administered.

As used herein, "in combination", "in combination with drug", "together with" or "in combination with therapy" refers to drug treatment obtained by mixing or combining more than one active ingredients, which includes fixed and unfixed combinations of active ingredients, or a combination of two or more different therapeutic methods. The term "fixed combination" refers to the simultaneous administration of at least one compound as described herein and at least one synergistic agent to a patient in the form of a single entity or a single dosage form. The term "unfixed combination" refers to the administration of at least one compound and at least one synergistic formulation described herein to patients in the form of separate entities concurrently, in combination, or sequentially at variable intervals. These are also applied to cocktail therapies, such as the administration of three or more active ingredients.

In the present disclosure, a "stereoisomer" refers to compounds composed of the same atoms, bonded by the same bonds, but with different three-dimensional structures. The present disclosure covers various stereoisomers and mixtures thereof.

When the compounds of the present disclosure contain alkene double bonds, unless otherwise stated, the compounds of the present disclosure are intended to comprise E- and Z- geometric isomers.

A "tautomer" is an isomer formed by the transfer of protons from one atom of a molecule to another atom of the same molecule. All tautomeric forms of the compound according to the present disclosure will also be included within the scope of the present disclosure.

The compounds of the present disclosure or pharmaceutically acceptable salts thereof may comprise one or more chiral carbon atoms, and may thus produce enantiomers, diastereomers and other stereoisomeric forms. Each chiral carbon atom can be defined as (R)- or (S)- stereochemistry. The present disclosure is intended to include all possible isomers, and their racemates and optically pure forms. The preparation of compounds of the present disclosure may select racemates, diastereomers or enantiomers as raw materials or intermediates. Optically active isomers can be prepared using chiral synthons or chiral reagents, or resolved using conventional techniques such as crystallization and chiral chromatography, etc.

Conventional techniques for the preparation/separation of individual isomers include chiral synthesis from suitable optically pure precursors, or resolving racemates (or racemates of salts or derivatives) using, for example, chiral HPLC. Reference is made to, for example, Gerald Gübitz and Martin G. Schmid (Eds.), Chiral Separations, Methods and Protocols, Methods in Molecular Biology, Vol. 243, 2004; A.M. Stalcup, Chiral Separations, Annu. Rev. Anal. Chem. 3:341-63, 2010; Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; Heller, Acc. Chem. Res. 1990, 23, 128.

The present disclosure further comprises all suitable isotopic variants of compounds of the present disclosure, pharmaceutically acceptable salts or isomers thereof. Isotopic variants of compounds of the present disclosure, pharmaceutically acceptable salts thereof are defined as those in which at least one atom is replaced by atoms having the same atomic number, but atomic mass is different from atomic mass often found in nature. Isotopes that may be incorporated into compounds of the present disclosure, pharmaceutically acceptable salts thereof, include but are not limited to, isotopes of H, C, N and O, such as ²H, ³H, ¹¹C, ¹³C, ¹⁴C, ¹⁵N, ¹⁷O, ¹⁸O, ³⁵S, ¹⁸F, ³⁶Cl and ¹²⁵I. The isotopic variant of the compound of the present disclosure, a pharmaceutically acceptable salt thereof may be prepared by adopting appropriate isotopic variants of suitable reagents through conventional techniques.

### II. Compound

The present disclosure provides a compound of Formula I, a pharmaceutically acceptable salt thereof, or an enantiomer, a diastereomer, a tautomer, a solvate, an isotope substitute, a polymorph, a prodrug or a metabolite thereof: 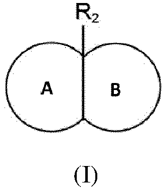 wherein:
ring A is a substituted or unsubstituted 4-10 membered heterocyclic group containing 1-3 heteroatoms selected from the group consisting of N, O and S;
ring B is a substituted or unsubstituted, saturated or unsaturated 3-10 membered carbon ring;
R₂ is a substituted or unsubstituted 3-8 membered cycloalkyl, substituted or unsubstituted 6-14 membered aryl, substituted or unsubstituted 5-10 membered heteroaryl, or substituted or unsubstituted 4-10 membered heterocyclic group.

Preferably, in Formula I, ring A contains 1 or 2 heteroatoms selected from the group consisting of N and O. More preferably, the ring atom number of ring A is 5-8. In some preferred embodiments, ring A is a morpholine ring.

Preferably, ring A is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl. In some embodiments, ring A is optionally substituted by 1-3 C1-C4 alkyl.

Preferably, in Formula I, ring B is a 5-8 membered saturated carbon ring. More preferably, ring B is a 6 membered saturated carbon ring.

Preferably, ring B is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl. In some embodiments, ring B is optionally substituted by 1-3 C1-C4 alkyl.

It should be understood that, in the present disclosure, when the ring atom number of ring A and ring B is mentioned, the ring atom number includes 2 carbon atoms shared by both ring A and ring B. In addition, two hydrogen atoms on the same carbon atom of ring A and ring B can be substituted at the same time.

Preferably, in Formula I, R₂ is a 3-8-membered cycloalkyl or 6-14-membered aryl, more preferably phenyl or naphthyl. Preferably, R₂ is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein said Rₐ and R_{b} are each independently selected from the group consisting of H, C1-C4 alkyl, halogenated C1-C4 alkyl and hydroxyl-substituted C1-C4 alkyl. More preferably, R₂ is optionally substituted by 1-3 substituents selected from the group consisting of C1-C4 alkoxy, halogen, C1-C4 alkyl, halogenated C1-C4 alkyl and halogenated C1-C4 alkoxy. More preferably, R₂ is optionally substituted by 1-3 substituents selected from the group consisting of F, Cl, C1-C3 alkyl, C1-C3 alkoxy, fluorinated or chlorinated C1-C3 alkyl, fluorinated or chlorinated C1-C3 alkoxy. In some embodiments, when the 6-14 membered aryl especially phenyl is substituted, the substituent does not include 4-Cl or 3-CF₃.

In some embodiments, in Formula I, R₂ is 5-10 membered heteroaryl, more preferably thienyl or furanyl. Said 5-10 membered heteroaryl is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl. In some embodiments, R₂ is optionally substituted by 1-3 substituents selected from the group consisting of C1-C4 alkoxy, halogen, C1-C4 alkyl, halogenated C1-C4 alkyl and halogenated C1-C4 alkoxy. In some embodiments, the 5-10 membered heteroaryl is optionally substituted by 1-3 substituents selected from the group consisting of F, Cl, C1-C3 alkyl, C1-C3 alkoxy, fluorinated or chlorinated C1-C3 alkyl, fluorinated or chlorinated C1-C3 alkoxy. In some embodiments, the 5-10 membered heteroaryl is optionally substituted by 1-2 substituents selected from the group consisting of halogen and C1-C3 alkyl. In some embodiments, the 5-10 membered heteroaryl is optionally substituted with 1, 2 or 3 Cl.

Preferably, in Formula I, when R₂ (preferably the 6-14 membered aryl, more preferably phenyl) is substituted, the substituent is 1 or 2 substituents selected from the group consisting of: F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position.

In a preferred embodiment, the compound of Formula I has the structure represented by the following Formula II: 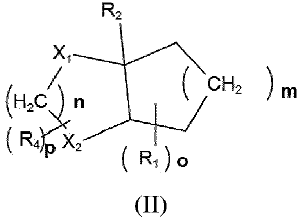 wherein:
X₁ is NH or O;
X₂ is NH or O;
each R₁ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
R₂ is as defined according to each embodiment of Formula I;
each R₄ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
n and m are each independently 1, 2 or 3;
o and p are each independently 0, 1, 2 or 3.

Preferably, in Formula II, X₁ is NH. Preferably, in Formula II, X₂ is O. Therefore, in a preferred embodiment, in Formula II, X₁ is NH, X₂ is O.

Preferably, in Formula II, R₁ is selected from the group consisting of hydroxyl, halogen, C1-C4 alkyl, C1-C4 alkoxy, C2-C4 alkenyl and C2-C4 alkynyl. More preferably, R₁ is C1-C4 alkyl. Preferably, o is 0, 1 or 2. In some embodiments, o is 0. In some embodiments, o is 1, R1 is C1-C4 alkyl.

Preferably, in Formula II, R₂ is 3-8 membered cycloalkyl or 6-14 membered aryl, more preferably phenyl or naphthyl. Preferably, R₂ is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl. Preferably, R₂ is optionally substituted by 1-3 substituents selected from the group consisting of halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl and C2-C4 alkynyl. More preferably, R₂ is optionally substituted by 1-3 substituents selected from the group consisting of C1-C4 alkoxy, halogen, C1-C4 alkyl, halogenated C1-C4 alkyl and halogenated C1-C4 alkoxy. More preferably, R₂ is optionally substituted by 1-3 substituents selected from the group consisting of F, Cl, C1-C3 alkyl, C1-C3 alkoxy, fluorinated or chlorinated C1-C3 alkyl, fluorinated or chlorinated C1-C3 alkoxy. In some embodiments, when the 6-14 membered aryl especially phenyl is substituted, the substituent does not include 4-Cl or 3-CF₃.

In some embodiments, in Formula II, R₂ is 5-10 membered heteroaryl, more preferably thienyl or furanyl. The 5-10 membered heteroaryl is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl. In some embodiments, R₂ is optionally substituted by 1-3 substituents selected from the group consisting of C1-C4 alkoxy, halogen, C1-C4 alkyl, halogenated C1-C4 alkyl and halogenated C1-C4 alkoxy. In some embodiments, the 5-10 membered heteroaryl is optionally substituted by 1-3 substituents selected from the group consisting of F, Cl, C1-C3 alkyl, C1-C3 alkoxy, fluorinated or chlorinated C1-C3 alkyl, fluorinated or chlorinated C1-C3 alkoxy. In some embodiments, the 5-10 membered heteroaryl is optionally substituted with 1-2 substituents selected from the group consisting of halogen and C1-C3 alkyl.

Preferably, in Formula II, when R₂ (preferably the 6-14 membered aryl, more preferably phenyl) is substituted, the substituent is 1 or 2 substituents selected from the group consisting of: F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position.

Preferably, in Formula II, each R₄ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. More preferably, each R₄ is independently C1-C4 alkyl. More preferably, each R₄ is independently methyl, ethyl. Preferably, p is 0, 1 or 2.

It should be understood in the present disclosure, when X₁ and X₂ are NH, it may be substituted with R₄ to form, for example, NR₄, at this time R₄ is preferably, for example, C1-C4 alkyl, halogenated C1-C4 alkyl, hydroxyl-substituted C1-C4 alkyl, etc.

In a preferred embodiment, the compound of Formula I has the structure represented by the following Formula III: 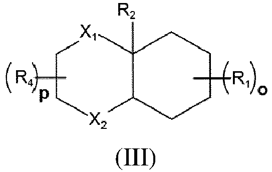 wherein:
X₁ is NH or O;
X₂ is NH or O;
each R₁ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
R₂ is as defined according to each embodiment of Formula I and II;
each R₄ is as defined according to Formula II;
o and p are each independently 0, 1, 2 or 3.

Preferably, in Formula III, X₁ is NH. Preferably, in Formula II, X₂ is O. Therefore, in a preferred embodiment, in Formula III, X₁ is NH, X₂ is O.

Preferably, in Formula III, each R₁ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. More preferably, each R₁ is independently C1-C4 alkyl. Preferably, o is 0, 1 or 2. In some embodiments, o is 0. In some embodiments, o is 1, R₁ is C1-C4 alkyl.

Preferably, in Formula III, R₂ is 3-8 membered cycloalkyl or 6-14 membered aryl, more preferably phenyl or naphthyl. Preferably, R₂ is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl. Preferably, R₂ is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl and C2-C4 alkynyl. More preferably, R₂ is optionally substituted by 1-3 substituents selected from the group consisting of C1-C4 alkoxy, halogen, C1-C4 alkyl, halogenated C1-C4 alkyl and halogenated C1-C4 alkoxy. More preferably, R₂ is optionally substituted by 1-3 substituents selected from the group consisting of F, Cl, C1-C3 alkyl, C1-C3 alkoxy, fluorinated or chlorinated C1-C3 alkyl, fluorinated or chlorinated C1-C3 alkoxy. In some embodiments, when the 6-14 membered aryl especially phenyl is substituted, the substituent does not include 4-Cl or 3-CF₃.

In some embodiments, in Formula III, R₂ is 5-10 membered heteroaryl, more preferably thienyl or furanyl. The 5-10 membered heteroaryl is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl. In some embodiments, R₂ is optionally substituted by 1-3 substituents selected from the group consisting of C1-C4 alkoxy, halogen, C1-C4 alkyl, halogenated C1-C4 alkyl and halogenated C1-C4 alkoxy. In some embodiments, the 5-10 membered heteroaryl is optionally substituted by 1-3 substituents selected from the group consisting of F, Cl, C1-C3 alkyl, C1-C3 alkoxy, fluorinated or chlorinated C1-C3 alkyl, fluorinated or chlorinated C1-C3 alkoxy. In some embodiments, the 5-10 membered heteroaryl is optionally substituted with 1-2 substituents selected from the group consisting of halogen and C1-C3 alkyl.

Preferably, in Formula III, when R₂ (preferably the 6-14 membered aryl, more preferably phenyl) is substituted, the substituent is 1 or 2 substituents selected from the group consisting of: F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position.

Preferably, in Formula III, each R₄ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. More preferably, each R₄ is independently C1-C4 alkyl. More preferably, each R₄ is independently methyl, ethyl. Preferably, p is 0, 1 or 2. In some embodiments, p is 0. In some embodiments, p is 1 or 2, each R₄ is independently C1-C4 alkyl, such as methyl or ethyl.

In a preferred embodiment, the compound of Formula I has the structure represented by the following Formula IV: 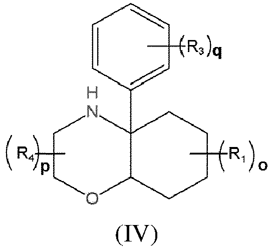 wherein:
R₁ is as defined according to each embodiment of Formula II and III;
each R₃ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
each R₄ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
o, p and q are each independently 0, 1, 2 or 3.

Preferably, in Formula IV, each R₁ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. More preferably, each R₁ is independently C1-C4 alkyl. Preferably, o is 0, 1 or 2. In some embodiments, o is 0. In some embodiments, o is 1, R₁ is C1-C4 alkyl.

Preferably, in Formula IV, each R₃ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. Preferably, each R₃ is independently selected from the group consisting of C1-C4 alkoxy, halogen, C1-C4 alkyl, halogenated C1-C4 alkyl and halogenated C1-C4 alkoxy. q is 0, 1 or 2. In some embodiments, when q is not 0, R₃ does not include 4-Cl or 3-CF₃. In some embodiments, when q is 1 or 2, R₃ is 1 or 2 substituents selected from the group consisting of: F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position.

Preferably, in Formula IV, each R₄ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. More preferably, each R₄ is independently C1-C4 alkyl. More preferably, each R₄ is independently methyl, ethyl. Preferably, p is 0, 1 or 2. In some embodiments, p is 0. In some embodiments, p is 1 or 2, each R₄ is independently C1-C4 alkyl, such as methyl or ethyl.

Preferably, in Formula IV, o is 0, i.e., R₁ is not present, or o is 1, R₁ is methyl, such as 6-methyl; q is 0, i.e., R₃ is not present, or q is 1 or 2, each R₃ is independently selected from the group consisting of halogen (such as F, Cl), C1-C4 alkyl, C1-C3 alkoxy, halogenated C1-C3 alkyl and halogenated C1-C3 alkoxy, preferably 1 or 2 substituents selected from the group consisting of F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position; p is 0, i.e., R₄ is not present.

Further preferably, in Formula IV, o is 0, i.e., R₁ is not present, or o is 1, R₁ is methyl, such as 6-methyl; q is 0, i.e., R₃ is not present, or q is 1 or 2, R₃ is selected from the group consisting of halogen (such as F, Cl) and C1-C3 alkyl, preferably 1 or 2 substituents selected from the group consisting of F in ortho position, F in meta position, F in para position, Cl in ortho position, Cl in meta position, C1-C4 alkyl in ortho position, C1-C4 alkyl in meta position and C1-C4 alkyl in para position; p is 0, i.e., R₄ is not present.

Further preferably, in Formula IV, p and q are both 0, i.e., R₃ and R₄ is not present; o is 0, i.e., R₁ is not present, or o is 1, and R₁ is methyl.

In some embodiments, the present disclosure provides the compound of Formula IIIa and IIIb or a pharmaceutically acceptable salt thereof, or an enantiomer, a diastereomer, a tautomer, a solvate, an isotope substitute, a polymorph, a prodrug or a metabolite thereof: 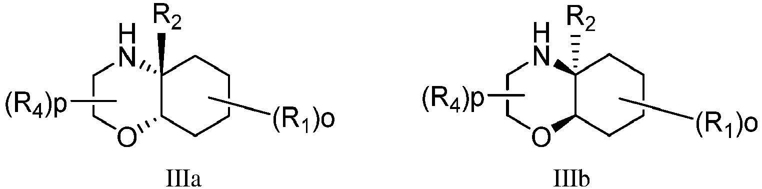 wherein:
each R₁ is as defined according to each embodiment of Formula II, III or IV;
R₂ is as defined according to each embodiment of Formula I, II or III;
each R₄ is as defined according to each embodiment of Formula II, III or IV;
o and p are as defined according to each embodiment of Formula II and III.

Preferably, in Formula IIIa and IIIb, each R₁ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. More preferably, each R₁ is independently C1-C4 alkyl. Preferably, o is 0, 1 or 2. In some embodiments, o is 0. In some embodiments, o is 1, R₁ is C1-C4 alkyl.

Preferably, in Formula IIIa and IIIb, R₂ is 3-8 membered cycloalkyl or 6-14 membered aryl, more preferably phenyl or naphthyl. Preferably, R₂ is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl. Preferably, R₂ is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl and C2-C4 alkynyl. More preferably, R₂ is optionally substituted by 1-3 substituents selected from the group consisting of C1-C4 alkoxy, halogen, C1-C4 alkyl, halogenated C1-C4 alkyl and halogenated C1-C4 alkoxy. More preferably, R₂ is optionally substituted by 1-3 substituents selected from the group consisting of F, Cl, C1-C3 alkyl, C1-C3 alkoxy, fluorinated or chlorinated C1-C3 alkyl, fluorinated or chlorinated C1-C3 alkoxy. In some embodiments, when the 6-14 membered aryl especially phenyl is substituted, the substituent does not include 4-Cl or 3-CF₃.

In some embodiments, in Formula IIIa and IIIb, R₂ is 5-10 membered heteroaryl, more preferably thienyl or furanyl. The 5-10 membered heteroaryl may be optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl. In some embodiments, R₂ is optionally substituted by 1-3 substituents selected from the group consisting of C1-C4 alkoxy, halogen, C1-C4 alkyl, halogenated C1-C4 alkyl and halogenated C1-C4 alkoxy. In some embodiments, the 5-10 membered heteroaryl is optionally substituted by 1-3 substituents selected from the group consisting of F, Cl, C1-C3 alkyl, C1-C3 alkoxy, fluorinated or chlorinated C1-C3 alkyl, fluorinated or chlorinated C1-C3 alkoxy. In some embodiments, the 5-10 membered heteroaryl is optionally substituted with 1-2 substituents selected from the group consisting of halogen and C1-C3 alkyl.

Preferably, in Formula IIIa and IIIb, when R₂ (preferably the 6-14 membered aryl, more preferably phenyl) is substituted, the substituent is 1 or 2 substituents selected from the group consisting of: F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position.

Preferably, in Formula IIIa and IIIb, each R₄ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. More preferably, each R₄ is independently C1-C4 alkyl. More preferably, each R₄ is independently methyl, ethyl. Preferably, p is 0, 1 or 2. In some embodiments, p is 0. In some embodiments, p is 1 or 2, each R₄ is independently C1-C4 alkyl, such as methyl or ethyl.

In some embodiments, the present disclosure provides the compound of Formula IVa and IVb or a pharmaceutically acceptable salt thereof, or an enantiomer, a diastereomer, a tautomer, a solvate, an isotope substitute, a polymorph, a prodrug or a metabolite thereof: 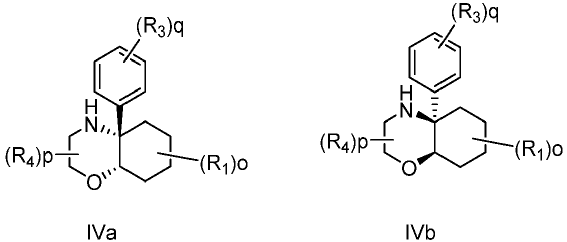 wherein:
each R₁ is as defined according to each embodiment of Formula II, III or IV;
each R₃ is as defined according to each embodiment of Formula IV;
each R₄ is as defined according to each embodiment of Formula II, III or IV;
o, p and q are as defined according to each embodiment of Formula II, III or IV.

Preferably, in Formula IVa and IVb, each R₁ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. More preferably, each R₁ is independently C1-C4 alkyl. Preferably, o is 0, 1 or 2. In some embodiments, o is 0. In some embodiments, o is 1, R₁ is C1-C4 alkyl.

Preferably, in Formula IVa and IVb, each R₃ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. Preferably, each R₃ is independently selected from the group consisting of C1-C4 alkoxy, halogen, C1-C4 alkyl, halogenated C1-C4 alkyl and halogenated C1-C4 alkoxy. q is 0, 1 or 2. In some embodiments, when q is not 0, R₃ does not include 4-Cl or 3-CF₃. In some embodiments, when q is 1 or 2, R₃ is 1 or 2 substituents selected from the group consisting of: F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position.

Preferably, in Formula IVa and IVb, each R₄ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl. More preferably, each R₄ is independently C1-C4 alkyl. More preferably, each R₄ is independently methyl, ethyl. Preferably, p is 0, 1 or 2. In some embodiments, p is 0. In some embodiments, p is 1 or 2, each R₄ is independently C1-C4 alkyl, such as methyl or ethyl.

Preferably, in Formula IVa and IVb, o is 0, i.e., R₁ is not present, or o is 1, R₁ is methyl, such as 6-methyl; q is 0, i.e., R₃ is not present, or q is 1 or 2, each R₃ is independently selected from the group consisting of halogen (such as F, Cl), C1-C4 alkyl, C1-C3 alkoxy, halogenated C1-C3 alkyl and halogenated C1-C3 alkoxy, preferably 1 or 2 substituents selected from the group consisting of F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position; p is 0, i.e., R₄ is not present.

Further preferably, in Formula IVa and IVb, o is 0, i.e., R₁ is not present, or o is 1, R₁ is methyl, such as 6-methyl; q is 0, i.e., R₃ is not present, or q is 1 or 2, R₃ is selected from the group consisting of halogen (such as F, Cl) and C1-C3 alkyl, preferably 1 or 2 substituents selected from the group consisting of F in ortho position, F in meta position, F in para position, Cl in ortho position, Cl in meta position, C1-C4 alkyl in ortho position, C1-C4 alkyl in meta position and C1-C4 alkyl in para position; p is 0, i.e., R₄ is not present.

Further preferably, in Formula IVa and IVb, p and q are both 0, i.e., R₃ and R₄ is not present; o is 0, i.e., R₁ is not present, or o is 1, and R₁ is methyl.

Preferably, the compound of Formula I according to the present disclosure comprises the following compounds, or a pharmaceutically acceptable salt thereof, or an enantiomer, a diastereomer, a tautomer, a solvate, an isotope substitute, a polymorph, a prodrug or a metabolite thereof:
4*a*-phenyloctahydro-2*H*-benzo[*b*] [1,4]oxazine;
4a-(1-methoxyphenyl)octahydro-2*H*-benzo[*b*][1,4]oxazine;
4*a*-(3-fluorophenyl)octahydro-2*H*-benzo[*b*][1,4]oxazine;
4a-(4-fluorophenyl)octahydro-2*H*-benzo[*b*][1,4]oxazine;
4*a*-(2,3-difluorophenyl)octahydro-2H-benzo[b] [1,4]oxazine;
4*a*-(2-fluorophenyl)octahydro-2H-benzo[b] [1,4]oxazine;
6-methyl-4*a*-phenyloctahydro-2H-benzo[b][1,4]oxazine;
6-ethyl-4*a*-phenyloctahydro-2*H*-benzo[*b*][1,4]oxazine;
4*a*-(3-chlorophenyl)octahydro-2*H*-benzo[b][1,4]oxazine;
4*a*-(3-methylphenyl)octahydro-2*H*-benzo[*b*][1,4]oxazine;
4*a*-(2-chlorophenyl)octahydro-2*H*-benzo[*b*][1,4]oxazine;
4*a*-(2-methylphenyl)octahydro-2*H*-benzo[*b*][1,4]oxazine;
4*a*-(4-chlorophenyl)octahydro-2*H*-benzo[*b*][1,4]oxazine;
4*a*-(4-methylphenyl)octahydro-2*H*-benzo[*b*][1,4]oxazine;
4*a*-(3-(trifluoromethyl)phenyl)octahydro-2*H*-benzo[*b*][1,4]oxazine;
8-methyl-4*a*-phenyloctahydro-2*H*-benzo[*b*][1,4]oxazine;
5-methyl-4*a*-phenyloctahydro-2*H*-benzo[*b*][1,4]oxazine;
7-methyl-4*a*-phenyloctahydro-2*H*-benzo[*b*][1,4]oxazine;
4-methyl-4*a*-phenyloctahydro-2H-benzo[b][1,4]oxazine;
3,3-dimethyl-5*a*-phenyldecahydrobenzo[*b*] [1,4]olanzapine;
4*a*-(3-methoxyphenyl)octahydro-2*H*-benzo[*b*][1,4]oxazine;
4*a*-(3-(trifluoromethoxy)phenyl)octahydro-2*H*-benzo[*b*][1,4]oxazine;
4*a*-(4-(trifluoromethyl)phenyl)octahydro-2*H-*benzo[*b*][1,4]oxazine;
4*a*-(2,6-dimethylphenyl)octahydro-2*H*-benzo[*b*] [1,4]oxazine;
4*a*-(4-(tertbutyl)phenyl)octahydro-2*H*-benzo[*b*] [1,4]oxazine;
4*a*-(2,3-dichlorophenyl)octahydro-2*H*-benzo[*b*] [1,4]oxazine;
4*a*-(2-isopropylphenyl)octahydro-2*H*-benzo[*b*] [1,4]oxazine;
*4*a-(2,5-dimethylphenyl)octahydro-2*H*-benzo[*b*] [1,4]oxazine;
*4*a-(2-chloro-3-fluorophenyl)octahydro-2*H*-benzo[*b*] [1,4]oxazine;
4*a-*(3,4-difluorophenyl)octahydro-2*H*-benzo[*b*] [1,4]oxazine;
6,6-dimethyl-4*a*-phenyloctahydro-2*H*-benzo[*b*][1,4]oxazine;
4*a*-(2-chloro-5-fluorophenyl)octahydro-2*H*-benzo[*b*] [1,4]oxazine;
4*α*-(3-ethoxyphenyl)octahydro-2*H*-benzo[*b*][1,4] oxazine;
4*α*-(2-chloro-4-methoxyphenyl)octahydro-2*H*-benzo[*b*][1,4]oxazine;
(4*a*R,8aS)-4*α*-(2-thiophenyl)octahydro-2*H*-benzo[*b*][1,4]oxazine;
4*a*-(2-chloro-6-fluorophenyl)octahydro-2*H*-benzo[*b*][1,4]oxazine;
(4aS,8aS)-4*α*-(2-chloro-3-thiophenyl)octahydro-2*H*-benzo[*b*][1,4]oxazine;
(4aS,8aR)-4*α*-(2-thiophenyl)octahydro-2*H*-benzo[*b*][1,4]oxazine;
(4aR,8aS)-4*α*-(3-methyl-2-thiophenyl)octahydro-2*H*-benzo[*b*][1,4]oxazine;
(4aR,8aR)-4*α*-(4-methyl-3-thiophenyl)octahydro-2*H*-benzo[*b*|[1,4[oxazine;
(4aR,8aR)-4*α*-(3-chlorophenyl)octahydro-2*H*-benzo[*b*][1,4]oxazine;
(4aS,8aS)-4*α*-(3-chlorophenyl)octahydro-2*H*-benzo[*b*][1,4]oxazine;
(4aR,8aR)-4*α*-(2-chlorophenyl)octahydro-2*H*-benzo[*b*][1,4]oxazine; and
(4aS,8aS)-4*α*-(2-chlorophenyl)octahydro-2*H*-benzo[*b*][1,4]oxazine.

In a preferred embodiment, the pharmaceutically acceptable salt of the compound of Formula I according to the present disclosure comprises:
4*a*-phenyloctahydro-2*H*-benzo[*b*][1,4]oxazine hydrochloride;
4*a*-(2-methoxyphenyl)octahydro-2*H*-benzo[*b*][1,4]oxazine hydrochloride;
4*a*-(3-fluorophenyl)octahydro-2*H*-benzo[*b*][1,4]oxazine hydrochloride;
4*a*-(4-fluorophenyl)octahydro-2*H*-benzo[*b*][1,4]oxazine formate;
4*a*-(2,3-difluorophenyl)octahydro-2*H*-benzo[*b*] [1,4]oxazine formate;
4*a*-(2-fluorophenyl)octahydro-2*H*-benzo[*b*] [1,4]oxazine formate;
6-methyl-4*a*-phenyloctahydro-2*H*-benzo[*b*][1,4]oxazine formate;
6-ethyl-4*a*-phenyloctahydro-2*H*-benzo[*b*][1,4]oxazine formate;
4*a*-(3-chlorophenyl)octahydro-2*H*-benzo[*b*][1,4]oxazine formate;
4*a*-(3-methylphenyl)octahydro-2*H*-benzo[*b*][1,4]oxazine formate;
4*a*-(2-chlorophenyl)octahydro-2*H*-benzo[*b*][1,4]oxazine formate;
4*a*-(2-methylphenyl)octahydro-2*H*-benzo[*b*][1,4]oxazine formate;
4*a*-(4-chlorophenyl)octahydro-2*H*-benzo[*b*][1,4]oxazine formate;
4*a*-(4-methylphenyl)octahydro-2*H*-benzo[*b*][1,4]oxazine formate;
4*a*-(3-(trifluoromethyl)phenyl)octahydro-2*H*-benzo[*b*][1,4]oxazine hydrochloride;
8-methyl-4*a*-phenyloctahydro-2*H*-benzo[*b*][1,4]oxazine formate;
5-methyl-4*a*-phenyloctahydro-2*H*-benzo[*b*][1,4]oxazine formate;
7-methyl-4*a*-phenyloctahydro-2*H*-benzo[*b*][1,4]oxazine formate;
4-methyl-4*a*-phenyloctahydro-2H-benzo[b] [1,4]oxazine hydrochloride;
3,3-dimethyl-5*a*-phenyldecahydrobenzo[*b*] [1,4]olanzapine hydrochloride;
4*a*-(3-methoxyphenyl)octahydro-2*H*-benzo[*b*][1,4]oxazine hydrochloride;
4*a*-(3-(trifluoromethoxy)phenyl)octahydro-2*H*-benzo[*b*][1,4]oxazine hydrochloride;
4*a*-(4-(trifluoromethyl)phenyl)octahydro-2*H*-benzo[*b*][1,4]oxazine hydrochloride;
4*a*-(2,6-dimethylphenyl)octahydro-2*H*-benzo[*b*] [1,4]oxazine hydrochloride;
4*a*-(4-(tertbutyl)phenyl)octahydro-2*H*-benzo[*b*] [1,4]oxazine hydrochloride;
4*a*-(2,3-dichlorophenyl)octahydro-2*H*-benzo[*b*] [1,4]oxazine hydrochloride;
4*a*-(2-isopropylphenyl)octahydro-2*H*-benzo[*b*] [1,4]oxazine hydrochloride;
4*a*-(2,5-dimethylphenyl)octahydro-2*H*-benzo[*b*] [1,4]oxazine hydrochloride;
4*a*-(2-chloro-3-fluorophenyl)octahydro-2*H*-benzo[*b*] [1,4]oxazine hydrochloride;
4*a*-(3,4-difluorophenyl)octahydro-2*H*-benzo[*b*] [1,4]oxazine hydrochloride;
6,6-dimethyl-4*a*-phenyloctahydro-2*H*-benzo[*b*][1,4]oxazine hydrochloride;
4*a*-(2-chloro-5-fluorophenyl)octahydro-2*H*-benzo[*b*] [1,4]oxazine hydrochloride;
4*α*-(3-ethoxyphenyl)octahydro-2*H*-benzo[*b*][1,4]oxazine hydrochloride;
4*α*-(2-chloro-4-methoxyphenyl)octahydro-2*H*-benzo[*b*][1,4]oxazine hydrochloride;
(4aR,8aS)-4*α*-(2-thiophenyl)octahydro-2*H*-benzo[*b*][1,4]oxazine hydrochloride;
4*a*-(2-chloro-6-fluorophenyl)octahydro-2*H*-benzo[*b*] [1,4]oxazine hydrochloride;
(4aS,8aS)-4*α*-(2-chloro-3-thiophenyl)octahydro-2*H*-benzo[*b*][1,4]oxazine hydrochloride;
(4aS,8aR)-4*α*-(2-thiophenyl)octahydro-2*H*-benzo[*b*][1,4]oxazine hydrochloride;
(4aR,8aS)-4*α*-(3-methyl-2-thiophenyl)octahydro-2*H*-benzo[*b*][1,4]oxazine hydrochloride;
(4aR,8aR)-4*α*-(4-methyl-3-thiophenyl)octahydro-2*H*-benzo[*b*][1,4]oxazine hydrochloride;
(4aR,8aR)-4*α-*(3-chlorophenyl)octahydro-2*H*-benzo[*b*][1,4]oxazine hydrochloride;
(4aS,8aS)-4*α*-(3-chlorophenyl)octahydro-2*H*-benzo[*b*][1,4]oxazine hydrochloride;
(4aR,8aR)-4*α*-(2-chlorophenyl)octahydro-2*H*-benzo[*b*][1,4]oxazine hydrochloride; and
(4aS,8aS)-4*α*-(2-chlorophenyl)octahydro-2*H*-benzo[*b*][1,4]oxazine hydrochloride.

### III. Preparation of Compound

Compound of Formula I according to the present disclosure may be prepared by the following explementary reaction process, wherein R₁, R₂, R₄, m, n, o and p in the formula are as described in any embodiment of the present disclosure, X is halogen:

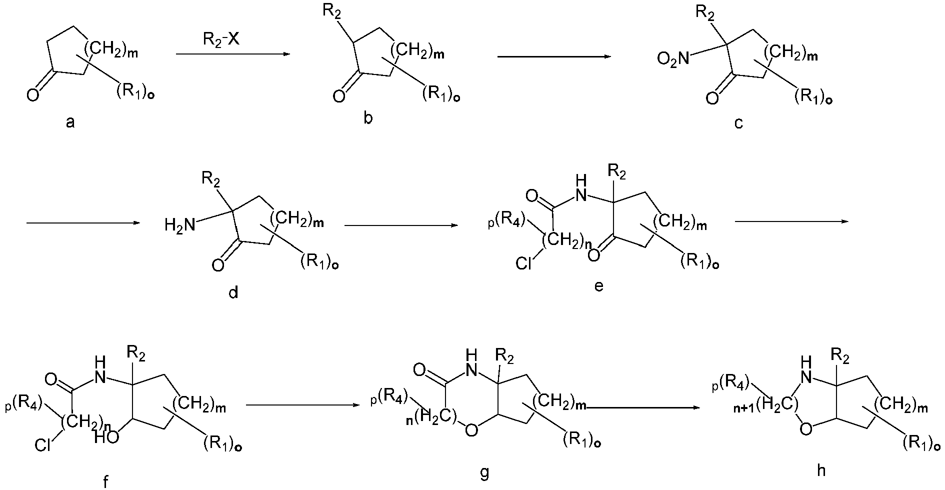

Step 1: Intermediate b is obtained by coupling reaction between raw material a and halide under the action of a palladium catalyst, a ligand and a base with the reaction condition of reflux at 70°C~80°C. In step 1, the solvent is selected from polar aprotic solvents, preferably high boiling point solvents such as 1,4-dioxane, toluene, xylene etc. The base is preferably inorganic bases such as cesium carbonate, potassium carbonate, potassium phosphate, etc., or organic bases such as tert-butoxide sodium, tert-butoxide potassium, etc. Palladium catalysts are preferably Pd(OAc)₂, Pd₂(dba)₃, Pd(dba)₂, Pd(PPh₃)₄, Pd(PPh₃)₂Cl₂. Ligands are preferred organic phosphine ligands, more preferably BINAP, XPhos, MePhos, XantPhos, P(t-Bu)₃, etc.

Step 2: α-nitrocyclone intermediate c was obtained from Intermediate b by nitration reaction with the reaction condition of reflux at 80°C~90°C. The reaction can be carried out in the presence of a copper-based catalyst, a nitrating agent and a solvent. Preferred the copper-based catalyst includes copper oxide, copper iodide, copper acetate or copper bromide. The nitrating agent is preferably ammonium cerium nitrate. The solvent is preferably polar solvent, more preferably acetonitrile, 1,2-dichloroethane, dichloromethane, etc.

Step 3: α-aminocyclone intermediate d was obtained by reduction reaction of Intermediate c with the reaction condition of reflux at 70°C~80°C. The reaction can be carried out in the presence of a reducing agent and a proton donor. The reducing agent is preferably zinc powder, iron powder. The solvent is preferably alcohol solvent, etc., more preferably methanol, ethanol, etc. The proton donor is preferably an organic acid, etc., more preferably formic acid, acetic acid, etc.

Step 3: Intermediate e is obtained by reacting Intermediate d with acid chloride. The reaction can be carried out at room temperature. The reaction usually takes place in the presence of a solvent and a base. The solvent is preferably dichloromethane, tetrahydrofuran. The base is preferably triethylamine, pyridine, DIPEA.

Step 4: Intermediate f is obtained from Intermediate e by reduction reaction. The reaction can be carried out at room temperature. The reaction usually takes place in the presence of a reducing agent and a solvent. Preferably sodium borohydride is used as the reducing agent. Preferably, proton solvents such as methanol, ethanol, etc. are used as the solvent.

Step 5: Intermediate g is obtained from Intermediate f through intramolecular ring formation. The reaction can be carried out at room temperature. The reaction usually takes place in the presence of a base and a solvent. The base is preferably sodium hydride, potassium carbonate, sodium tert-butoxide, etc. The solvent is preferably polar solvents and the like, and more preferably tetrahydrofuran, N,N-dimethylformamide, etc.

Step 6: The target compound h is obtained from Intermediate g by reduction reaction with the reaction condition of reflux at 60°C~80°C. The reaction is carried out in the presence of a reducing agent and a solvent. The reducing agent is preferably a solution of borane tetrahydrofuran and a solution of borane dimethyl sulfide. The solvent is preferably a polar solvent and the like, more preferably tetrahydrofuran, 1,4-dioxane, etc.

### IV. Use, treating method and pharmaceutical composition

The compound of Formula I according to the present disclosure is a NMDA receptor antagonist. Therefore, the compound of Formula I according to the present disclosure, or a pharmaceutically acceptable salt thereof, or an enantiomer, a diastereomer, a tautomer, a solvate, an isotope substitute, a polymorph, a prodrug or a metabolite thereof can be used to regulate the activity of NMDA receptors.

In the present disclosure, the modulation of NMDA receptor activity is employed for the treatment and/or prevention of NMDA receptor-mediated diseases. Thus, in some embodiments, the present disclosure provides a use of the compound of Formula I of the present disclosure, or a pharmaceutically acceptable salt thereof, or an enantiomer, a diastereomer, a tautomer, a solvate, an isotope substitute, a polymorph, a prodrug or a metabolite thereof in the preparation of drugs for the treatment or prevention of NMDA receptor-mediated diseases. In some other embodiments, the present disclosure provides the compound of Formula I of the present disclosure, or a pharmaceutically acceptable salt thereof, or an enantiomer, a diastereomer, a tautomer, a solvate, an isotope substitute, a polymorph, a prodrug or a metabolite thereof, for the treatment or prevention of NMDA receptor-mediated diseases.

In specific embodiments, the disclosure encompasses a method for treating or preventing NMDA receptor-mediated diseases in a subject. This method involves administering a therapeutically effective or a prophylactic effective amount of the compound of Formula I of the present disclosure, or a pharmaceutically acceptable salt thereof, or an enantiomer, a diastereomer, a tautomer, a solvate, an isotope substitute, a polymorph, a prodrug or a metabolite thereof, or a pharmaceutic composition comprising a therapeutically effective amount or a prophylactic effective amount of the compound of Formula I of the present disclosure, or a pharmaceutically acceptable salt thereof, or an enantiomer, a diastereomer, a tautomer, a solvate, an isotope substitute, a polymorph, a prodrug or a metabolite thereof, to the subject. Administration routes include oral, transduodenum, parenteral injection (including intrapulmonary, intranasal, intrathecal, intravenous, subcutaneous, intraperitoneal, intramuscular, intraarterial injection or infusion), topical, and rectal. Those skilled in the art are familiar with the application techniques that may be used for the compounds and methods described herein, for example, those discussed in Goodman and Gilman, The Pharmacological Basis of Therapeutics, current ed.; Pergamon; and Remington's, Pharmaceutical Sciences (current edition), Mack Publishing Co., Easton, Pa. In a preferred embodiment, a compound of Formula I according to the present disclosure, or a pharmaceutically acceptable salt thereof, or an enantiomer, a diastereomer, a tautomer, a solvate, an isotope substitute, a polymorph, a prodrug or a metabolite thereof or a pharmaceutical composition thereof is orally administered.

The present disclosure also provides a pharmaceutical composition comprising a therapeutically effective amount of the compound of Formula I of the present disclosure, or a pharmaceutically acceptable salt thereof, or an enantiomer, a diastereomer, a tautomer, a solvate, an isotope substitute, a polymorph, a prodrug or a metabolite thereof, and pharmaceutically acceptable carriers or excipients. The pharmaceutical composition of the present disclosure may be used to regulate NMDA receptor activity, thus can be used to treat and/or prevent NMDA receptor-mediated diseases.

Suitable pharmaceutical compositions may be formulated according to the methods known in the art and the mode of administration and dosage thereof determined by a skilled practitioner. With respect to parenteral administration, the compound may be dissolved in sterile water or normal saline or in a pharmaceutically acceptable medium for administering water-insoluble compounds (such as those for vitamin K). With respect to enteral administration, the compound may be administered in tablet, capsule form or dissolved in liquid form. Tablets or capsules can be enteric-coated or presented in the form of a formulation for sustained release. A variety of suitable formulations are known, including polymer or protein particles, ointments, pastes, gels, hydrogels, or solutions encapsulating compounds to be released, which may be applied to compounds superficially or topically. Continuous-release patches or implants may be used to provide release over an extended period of time. Formulations for parenteral administration may, for example, contain excipients such as polyalkylene glycol (e.g., polyethylene glycol), oils derived from plant or hydrogenated naphthalene. Biocompatible, biodegradable lactide polymers, lactide/glycolide copolymers, or polyoxyethylene-polyoxypropylene copolymers may be used to control the release of said compounds. Other potentially useful parenteral delivery systems for regulating compounds include ethylene-vinyl acetate copolymer particles, osmotic pumps, implantable infusion systems, and liposomes. The preparation for inhalation may contain excipients, such as lactose, or may be aqueous solutions containing, for example, polyoxyethylene-9-lauryl ether, glycocholate and deoxycholate, or may be an oily solution for administration in the form of nasal drops, or in gel form.

It should be noted that dose values can vary depending on the precise imaging protocol. With respect to any particular subject, the specific administration regimen may be adjusted over time according to individual needs and the professional judgment of the person administering or supervising the administration of the composition. The dosage ranges described herein are exemplary only and do not limit the dose ranges available to medical practitioners. The amount of active compound in the composition may vary according to factors such as the subject's disease status, age, sex and weight. Administration regimens can be adjusted to provide optimal imaging results. For example, a single large pill may be administered, several divided doses may be administered over time, or the dose may be reduced or increased proportionally according to the imaging results. The preparation of parenteral compositions in the form of unit doses that are easy to administer and provide dose uniformity may be advantageous.

In the present disclosure, diseases associated with NMDA receptor activity include, but are not limited to cerebral ischemia, traumatic brain injury, infarction, stroke, Alzheimer's disease, Parkinson's disease, Huntington's disease, depression, anxiety, bipolar disorder, schizophrenia, autism, epilepsy, anti-NMDA receptor encephalitis, neuropathic pain and other neurological events or neurodegeneration caused by NMDA receptor activation. In some embodiments, the neuropathic pain includes peripheral diabetic neuropathy, postherpetic neuralgia, complex focal pain syndrome, peripheral neuropathy, chemotherapy-induced neuropathic pain, cancerous neuropathic pain, neuropathic back pain, HIV neuropathic pain, trigeminal neuralgia and central post-stroke pain. In particular, the NMDA receptor activity-related disorders described herein or NMDA receptor-mediated disorders are depression, schizophrenia, or epilepsy.

In some embodiments, the compound of Formula I of the present disclosure, or a pharmaceutically acceptable salt thereof, or an enantiomer, a diastereomer, a tautomer, a solvate, an isotope substitute, a polymorph, a prodrug or a metabolite thereof, or the pharmaceutical composition thereof can be also used for anesthesia and analgesia of the subject. Thus, in some embodiments, the present disclosure provides an anesthetic or analgesic comprising the compound of Formula I of the present disclosure, or a pharmaceutically acceptable salt thereof, or an enantiomer, a diastereomer, a tautomer, a solvate, an isotope substitute, a polymorph, a prodrug or a metabolite thereof as an active ingredient for anesthesia or analgesia, and a safe carrier or excipient suitable for administration to human or animal bodies. In some other embodiments, the present disclosure also provides a use of the compound of Formula I of the present disclosure, or a pharmaceutically acceptable salt thereof, or an enantiomer, a diastereomer, a tautomer, a solvate, an isotope substitute, a polymorph, a prodrug or a metabolite thereof in the preparation of anesthetics or analgesics. In some other embodiments, the present disclosure also provides a method of anesthesia or analgesia, which comprises administering an effective amount of the compound of Formula I of the present disclosure, or a pharmaceutically acceptable salt thereof, or an enantiomer, a diastereomer, a tautomer, a solvate, an isotope substitute, a polymorph, a prodrug or a metabolite thereof or a pharmaceutical composition thereof to an individual or subject in need.

The present disclosure is further explained below in conjunction with specific Examples. It should be understood that these Examples are intended only to illustrate the present disclosure and are not intended to limit the scope of the present disclosure. In the following Examples, unless otherwise stated, materials, reagents and methods are conventional materials, reagents and methods in the art. The materials and reagents are commercially available.

The meanings of the English abbreviations involved in the chemical equation and in the text of the present disclosure are described in the following table.

| | | | |
|---|---|---|---|
| Ti(OEt)₄ | tetraethyl titanate | DMF | N,N-dimethylformamide |
| LiBH₄ | lithium borohydride | Na₂CO₃ | sodium carbonate |
| TFA | trifluoroacetic acid | EtOH | ethanol |
| LDA | lithium diisopropylamide | CBr₄ | tetrabromomethane |
| Pd(AmPhos) ₂Cl₂ | dichlorobis [di-tert-butyl(p-dimethylaminophenyl)phosphino]pall adium(II) | Ph₃P | triphenylphosphine |
| Cs₂CO₃ | cesium carbonate | NBS | N-bromosuccinimide |
| DMAc or DMA | N,N-dimethylacetamide | BPO | benzoyl Peroxide |
| THF | tetrahydrofuran | TMP | 2,2,6,6-Tetramethylpiperidine |
| DCM | dichloromethane | PBr₃ | phosphorus tribromide |
| Ms₂O | methanesulfonic anhydride | CCl₄ | tetrachloromethane |
| n-BuLi | n-butyl lithium | N₂H₄ | hydrazine |
| Dibal-H | diisobutylaluminum hydride | H₂SO₄ | sulfuric acid |
| Dioxane | 1,4-dioxane | POCl₃ | phosporus oxychoride |
| PPA | polyphosphoric acid | Pd(OAc)₂ | palladium acetate |
| NaBH₄ | sodium borohydride | EtONa | sodium ethoxide |
| MeOH | methanol | Pd₂(dba)₃ | tris(dibenzylideneacetone)dipalladium |
| Et₃N or TEA | triethylamine | XantPhos | 4,5-bis(diphenylphosphino)-9,9-dimethylxanthene |
| DIPEA | N,N-diisopropylethanamine | CH₃CN | acetonitrile |
| HCl | hydrochloride | Boc₂O | di-tert-butyl dicarbonate |
| PMB-Br | 4-methoxybenzyl bromide | K₂CO₃ | potassium carbonate |
| SEMCl | 2-(trimethylsilyl)ethoxymethyl chloride | DIAD | diisopropyl azodicarboxylate |
| t-BuXphos-Pd- G3 | methanesulfonato(2-ditertbutylphosphino-2',4',6'-triisopropyl-1,1'-biphenyl)(2'-amino-1,1'-biphenyl-2-yl)palladium (II) | Ruphos-Pd G3 | methanesulfonato(2-dicyclohexylphosphino-2',6'-diisopropoxy-1,1'-biphenyl)(2'-amino-1,1'-biphenyl-2-yl)palladium(II) |
| Cyclohexane | cyclohexane | Toluene | toluene |
| i-PrMgBr | isopropylmagnesium bromide | LiCl | lithium chloride |
| NaH | Sodium hydride | CH₃ONa | Sodium methoxide |
| NCS | N-chlorosuccinimide | LiOH | lithium hydroxide |
| NaBH(OAc)₃ | sodium triacetoxyborohydride | Pddppf(Cl)₂ | [1,1'-bis(diphenylphosphino)ferrocene]dichloro palladium (II) |
| Cul | copper iodide | MeI | methyl iodide |
| LiHMDS | lithium bis(trimethylsilyl)amide | bromo(phe nyl) magnesium | bromo(phenyl) magnesium |
| n-heptane | n-heptane | t-BuOK | potassium tert-butoxide |
| B₂(Pin)₂ | pinacol borate | KOPiv | potassium pivalate |
| xylene | xylene | Ti(iPrO)₄ | titanium tetraisopropanolate |
| HTMP | 2,2,6,6-tetramethylpiperidine | TBSOTf | trifluoromethanesulfonic acid tert-butyldimethylsilyl ester |
| D^{t}BuAD | di-tert-butyl azodicarboxylate | KOAc | potassium acetate |
| DMSO | dimethyl sulfoxide | DMAP | 4-dimethylaminopyridine |
| tert-BuOH | tert-butanol | acetone | acetone |
| 1-butanol/n-BuOH | n-butanol | NMP | N-methylpyrrolidone |
| Ac₂O | acetic anhydride | AcOH | acetic acid |
| Lawesson's Reagent | Lawesson's Reagent | NIS | N-iodosuccinimide |
| BnBr | benzyl bromide | N-xantphos | 4,6-bis(diphenylphosphino)phenoxazine) |
| Ruphos | 2-dicyclohexylphosphino-2',6'-di-iso-propoxy-1,1'-biphenyl | TsOH | p-toluenesulfonic acid |
| Cu(OAc)₂ | copper acetate | Microwave | microwave |
| RT | room temperature | CAN | ammonium ceric nitrate |
| DCE | 1,2-dichloroethane | Chloroacet yl chloride | chloracetyl chloride |
| BH₃/Me₂S | borane/dimethyl sulfide | (PPh₃)₄Pd | tetrakis(triphenylphosphine)palladium |
| K₃Fe(CN)₆ | potassium ferricyanide | MsNH₂ | methylsulfonylamide |
| (DHQD)₂PH AL | hydroquinidine 1,4-phthalazinediyl ether | (DHQ)₂PH AL | hydroquinine 1,4-phthalazinediyl ether |
| TfOH | trifluoromethanesulfonic acid | i-PrOH | isopropanol |
| S,S Jacobsen cat. | (S,S)-(+)-N,N'-bis(3,5-ditertbutylsalicylidene)-1,2-cyclohexanediaminomanganese(III) chloride | R,R Jacobsen cat. | (R,R)-(-)-N,N'-bis(3,5-ditertbutylsalicylidene)- 1,2-cyclohexanediaminomanganese(III) chloride |
| 4-PPNO | 4-phenylpyridine 1-oxide | NaClO | sodium hypochlorite |
| LiAlH₄ | lithium aluminum hydride | i-Bu₂AlN₃ | diisobutyl aluminum azide |
| Na₂HPO₄ | dibasic sodium phosphate | DME | glycol dimethyl ether |
| DMP | Dess-Martin periodinane | EA | Ethyl acetate |

### Synthesis of Compound 1

### 4a-phenyloctahydro-2H-benzo[b][1,4]oxazine hydrochloride(Compound 1)

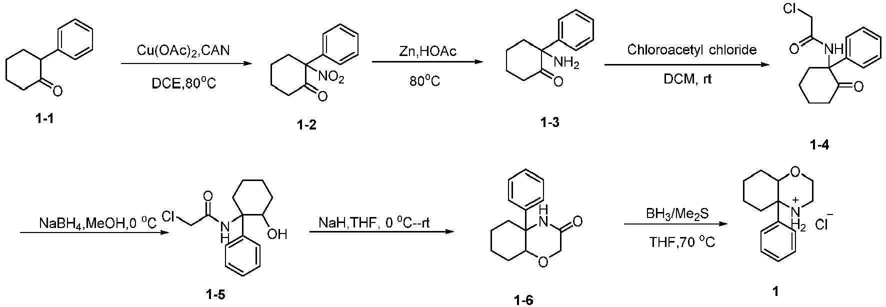

### Step 1: Synthesis of 2-nitro-2-phenylcyclohexanone (1-2)

**1-1** (2 g, 11.48 mmol) was dissolved in DCE (20 mL). Copper acetate (0.42 g, 2.30 mmol) and cerium ammonium nitrate (15.7 g, 2.50 mmol) were added and heated at 80°C for 12 hours. The reaction liquid was filtered, washed with DCM, dried by a rotary evaporator, then separated through silica gel column chromatography to obtain 1.2 g yellow oil of 2-nitro-2-phenylcyclohexanone (1-2), yield 46.4%.

### Step 2: Synthesis of 2-amino-2 phenylcyclohexanone (1-3)

**1-2** (1.17 g, 5.34 mmol) was dissolved in acetic acid (20 mL) under nitrogen protection. Zinc powder (1.7 g, 26.68 mmol) was added and heated at 80°C for 12 hours. After being cooled, the reaction liquid was adjusted to pH>10 with a 2 M solution of sodium hydroxide, extracted with ethyl acetate. Then the organic phase was dried with anhydrous sodium sulfate, separated and purified by column chromatography after concentration to obtain a colorless oil of 2-amino-2phenylcyclohexanone (**1-3**) 470 mg, yield 46.5%. LCMS: m/z= 190.05 (M+H)⁺.

### Step 3: Synthesis of 2-chloro-N-(2-oxo-l-phenylhexyl)acetamide (1-4)

Under nitrogen protection, **1-3** (210 mg, 1.11 mmol) was dissolved in anhydrous DCM (5 mL) and added with anhydrous triethylamine (0.2 mL, 1.22 mmol). Chloroacetyl chloride (88 µL, 1.10 mmol) was added dropwise at 0°C and stirred at room temperature for 1 hour. The reaction liquid was concentrated directly, separated and purified by column chromatography to obtain a white solid of 2-chloro-N-(2-oxo-1-phenylhexyl)acetamide (**1-4**) 226 mg, yield 76.6 %. LCMS: m/z= 266.05 (M+H)⁺.

### Step 4: Synthesis of 2-chloro-N-(2-hydroxyl-1-phenylhexyl)acetamide (1-5)

**1-4** (226 mg, 0.85 mmol) was put in a 25 mL round bottom flask. After 5 mL methanol was added for dissolving, sodium borohydride (32 mg, 0.85 mmol) was slowly added at zero °C. The reaction liquid was concentrated after stirring for 30 mins, separated and purified by column chromatography to obtain a white solid of 2-chloro-N-(2-hydroxyl-1-phenylhexyl)acetamide (**1-5**) 188 mg, yield 83 %, LCMS: m/z=268.05(M+H)⁺

### Step 5: Synthesis of 4a-phenylhexahydro-2H-benzo[b][1,4]oxazine-3(4H)-one (1-6)

1-5 (94 mg, 0.35 mmol) was put in a 25 mL round bottom flask. After 3 mL THF was added for dissolving, 60% sodium hydride (17 mg, 0.42 mmol) was added under an ice bath. After the mixture was reacted for 10 hours, it was quenched with saturated saline and extracted with ethyl acetate. The organic phase was dried by a rotatory evaporator, separated and purified by column chromatography to obtain a white solid of 4*a*-phenylhexahydro-2*H*-benzo[*b*][1,4]oxazine-3(4*H*)-one(**1-6**) 67 mg, yield 83%. LCMS: m/z= 232 .05(M+H)⁺.

### Step 6: Synthesis of 4a-phenyloctahydro-2H-benzo[b][1,4]oxazine hydrochloride (Compound 1)

Under nitrogen protection, **1-6** (17 mg, 0.07 mmol) was dissolved in ultra-dry THF (1 mL). A solution of borane-methyl sulfide in THF (0.37 mL, 0.74 mmol, 2.0 M) was added, refluxed at 70°C for 12 hours, and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product of Compound 1.

The crude product of Compound **1** was separated and purified by HPLC. The purified product was concentrated, followed by adding 1 mL diluted hydrochloric acid, then lyophilized to obtain a white solid of 4*a*-phenyloctahydro-2*H*-benzo[*b*][1,4]oxazine hydrochloride 8 mg, yield 50 %. LCMS: m/z= 218.10 (M+H)⁺.

¹H NMR (400 MHz, CDC13) δ 10.40 (s, 1H), 9.77 (s, 1H), 8.09 (d, J = 7.9 Hz, 2H), 7.46 (t, J = 7.7 Hz, 2H), 7.37 (t, J = 7.3 Hz, 1H), 4.42 (t, J = 11.6 Hz, 1H), 4.24 (dd, J = 12.0, 4.5 Hz, 1H), 4.06 (dd, J = 12.4, 3.0 Hz, 1H), 3.00 (dd, J = 38.9, 11.8 Hz, 2H), 2.84 (d, J = 11.7 Hz, 1H), 2.29 (td, J = 13.2, 2.5 Hz, 1H), 2.15 - 1.94 (m, 2H), 1.74 (d, J = 11.5 Hz, 1H), 1.64 - 1.50 (m, 2H), 1.02 (q, J = 13.9 Hz, 1H).

### Synthesis of Compound 2

### Synthesis of 4a-(2-methoxy)octahydro-2H-benzo[b][1,4]oxazine hydrochloride (Compound 2)

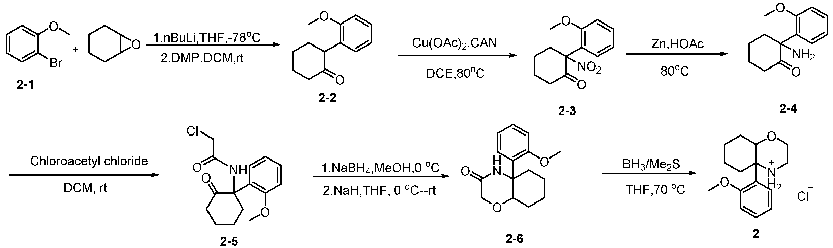

### Step 1: Synthesis of 2-(2-methoxyphenyl)cyclohexanone (2-2)

O-bromoanisole (5 g, 26.73 mmol) was dissolved in 40 mL ultra-dry THF, and n-butyllithium (12 mL, 30.00 mmol) was added dropwise at -78°C and continued to stirred for 3 hours at this temperature. Then, cyclohexene oxide (3.5 mL, 34.75 mmol), boron trifluoride ethyl ether (4.3 mL, 34.75 mmol) were added dropwise in sequence. After the completion of the reaction, the temperature was raised to 0 °C and the reaction was quenched by adding 20 mL saturated ammonium chloride. The reaction liquid was extracted with ethyl acetate. The organic phase was combined. The organic layer was dried with anhydrous sodium sulfate and concentrated.

After concentration, it was dissolved in 60 mL DCM and cooled with ice bath. Dess-martin periodinane (14.7 g, 35.00 mmol) was added in batches and stirred at room temperature for 3 hours. The reaction was quenched by adding 20 mL saturated sodium sulfite solution, then neutralized with saturated sodium bicarbonate, and extracted with dichloromethane. The organic phase was combined and washed with saturated sodium chloride solution, dried with anhydrous sodium sulfate, filtered, concentrated by rotatory evaporation, separated and purified with silica column chromatography to obtain a colorless oil of 2-(2-methoxyphenyl)cyclohexanone (**2-2**)2.09 g, yield 38.3 %. LCMS: m/z= 205.05 (M+H)⁺.

### Step 2: Synthesis of 2-(2-methoxyphenyl)-2-nitrocyclohexanone (2-3)

**2-2** (2.09 g, 10.23 mmol) was dissolved in DCE (40 mL). Copper acetate (0.93 g, 5.12 mmol) and cerium ammonium nitrate (11.2 g, 10.50 mmol) were added and heated at 80°C for 12 hours. The reaction liquid was filtered, washed with DCM, dried by a rotary evaporator, then separated through silica gel column chromatography to obtain 0.3 g yellow oil of 2-nitro-2-phenylcyclohexanone(**2-3**), yield 12 %.

### Step 3: Synthesis of 2-amino-2phenylcyclohexanone (2-4)

**2-3** (0.3 g, 1.20 mmol) was dissolved in acetic acid (5 mL) under nitrogen protection. Zinc powder (0.4 g, 6.00 mmol) was added and heated at 80°C for 12 hours. After being cooled, the reaction liquid was adjusted to pH>10 with a 2 M solution of sodium hydroxide, extracted with ethyl acetate. Then the organic phase was dried with anhydrous sodium sulfate, separated and purified by silica gel column after concentration to obtain a colorless oil of 2-amino-2 phenylcyclohexanone (**2-4**) 180 mg, yield 68%. LCMS: m/z= 220.05 (M+H)⁺.

### Step 4: Synthesis of 2-chloro-N-(2-oxo-1-phenylhexyl)acetamide (2-5)

Under nitrogen protection, **2-4** (180 mg, 0.82 mmol) was dissolved in ultra-dry DCM (2 mL) and added with ultra-dry triethylamine (0.2 mL, 0.84 mmol). Chloroacetyl chloride (65 µL, 0.82 mmol) was added dropwise at 0°C and stirred at room temperature for 1 hour until the raw material disappeared by analysis of TLC. The reaction liquid was concentrated directly, separated and purified by silica gel column to obtain a white solid of 2-chloro-N-(2-oxo-1-phenylhexyl)acetamide (**2-5**)160 mg, yield 66 %. LCMS: m/z= 296.05 (M+H)⁺.

### Step 5: Synthesis of 4a-(2-methoxyphenyl)hexahydro-2H-benzo[b][1,4]oxazine-3(4H)-one (2-6)

**2-5** (160 mg, 0.54 mmol) was put in a 25 mL round bottom flask. After 5 mL methanol was added for dissolving, sodium borohydride (22 mg, 0.54 mmol) was slowly added at 0 °C. The reaction liquid was dried by a rotary evaporator after stirring for 30 mins. After 3 mL THF was added for dissolving, 60% sodium hydride (26 mg, 0.65 mmol) was added under ice bath and reacted for 10 hours. The reaction liquid was quenched with saturated saline, extracted with ethyl acetate. The organic phase was concentrated, separated and purified by silica gel column chromatography to obtain a white solid of 4*a*-(2-methoxyphenyl)hexahydro-2*H-*benzo[*b*][1,4]oxazine-3(4*H*)-one(**2-6**)80 mg, yield 57 %. LCMS: m/z= 262.05 (M+H)⁺.

### Step 6: Synthesis of 4a-(2-methoxyphenyl)octahydro-2H-benzo[b][1,4]oxazine hydrochloride (Compound 2)

Under nitrogen protection, **2-6** (79 mg, 0.30 mmol) was dissolved in anhydrous THF (3 mL). A solution of borane-methyl sulfide in THF (1.5 mL, 3.0 mmol, 2.0 M) was added, refluxed at 70°C for 12 hours, and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product of Compound 2.

The crude product of Compound **2** was separated and purified by HPLC. The purified product was concentrated, followed by adding 1 mL diluted hydrochloric acid, then lyophilized to obtain a white solid of 4*a*-(2-methoxyphenyl)octahydro-2*H*-benzo[*b*][1,4]oxazine hydrochloride 50 mg, yield 67 %. LCMS: m/z= 248.10 (M+H)⁺.

¹H NMR (400 MHz, chloroform-d) δ 11.10 (s, 1H), 8.18 - 7.98 (m, 1H), 7.94 (dd, *J* = 8.2, 1.6 Hz, 1H), 7.40 - 7.33 (m, 1H), 7.02 - 6.97 (m, 2H), 4.55 - 4.38 (m, 2H), 3.98 (s, 3H), 3.93 - 3.87 (m, 1H), 3.23 (d, *J* = 13.1 Hz, 1H), 3.00 - 2.91 (m, 1H), 2.85 (d, *J* = 12.4 Hz, 1H), 2.28 - 2.08 (m, 2H), 2.07 - 2.00 (m, 2H), 1.75 (d, *J* = 14.2 Hz, 1H), 1.61 - 1.48 (m, 2H), 1.04 - 0.85 (m, 1H).

### Synthesis of Compound 3

### Synthesis of 4a-(3-fluorophenyl)octahydro-2H-benzo[b][1,4]oxazine hydrochloride (Compound 3)

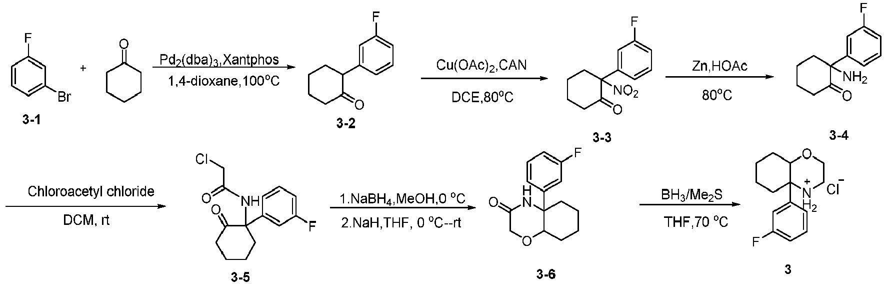

### Step 1: Synthesis of 2-(3-fluorophenyl)cyclohexanone (3-2)

Under nitrogen protection, Pd₂(dba)₃ (262 mg, 0.30 mmol), Xantphos (331 mg, 0.57 mmol) and cesium carbonate (20.5 g, 63.00 mmol) were dissolved in ultra-dry 1,4-dioxane (30 mL), followed by adding 3-bromofluorobenzene (5 g, 28.60 mmol) and cyclohexanone (5.6 g, 57.10 mmol), heated at 100°C for 20 hours, and extracted with EA and water after being cooled. The organic layer was dried with anhydrous sodium sulfate, separated and purified by silica gel column chromatography after concentration to obtain 3.05 g yellow oil of 2-(3-fluorophenyl)cyclohexanone (**3-2**), yield 55 %. LCMS: m/z= 193.05 (M+H)⁺.

### Step 2: Synthesis of 2-(3-fluorophenyl)-2-nitrocyclohexanone (3-3)

**3-2** (3.05 g, 15.90 mmol) was dissolved in DCE (30 mL). Copper acetate (1.44 g, 7.90 mmol) and cerium ammonium nitrate (21.7 g, 39.70 mmol) were added and heated at 80°C for 12 hours. The reaction liquid was filtered, washed with EA, separated and purified by silica gel column chromatography after concentration to obtain 1.18 g yellow oil of 2-(3-fluorophenyl)-2-nitrocyclohexanone (**3-3**), yield 31 %.

### Step 3: Synthesis of 2-(3-fluorophenyl)-2-aminocyclohexanone (3-4)

Under nitrogen protection, **3-3** (1.18 g, 4.97 mmol) was dissolved in acetic acid (10 mL). Zinc powder (1.62 g, 25.00 mmol) was added and heated at 80°C for 12 hours. After being cooled, the reaction liquid was adjusted to pH>10 with a 2 M solution of sodium hydroxide, extracted with ethyl acetate. Then the organic phase was dried with anhydrous sodium sulfate, concentrated, separated and purified by silica gel column chromatography, to obtain a colorless oil of 2-(3-fluorophenyl)-2-aminocyclohexanone (**3-4**) 510 mg, yield 50 %. LCMS: m/z= 208.00 (M+H)⁺.

### Step 4: Synthesis of 2-chloro-N-(1-(3-fluorophenyl)-2-oxocyclohexyl)acetamide (3-5)

Under nitrogen protection, **3-4** (510 mg, 2.46 mmol) was dissolved in anhydrous DCM (10 mL) and added with anhydrous triethylamine (0.4 mL, 2.71 mmol). Chloroacetyl chloride (196 µL, 2.46 mmol) was added dropwise at 0°C, stirred at room temperature for 1 hour until the raw material disappeared by analysis of TLC. The reaction liquid was concentrated directly, separated and purified by silica gel column chromatography to obtain a white solid of 2-chloro-N-(1-(3-fluorophenyl)-2-oxocyclohexyl)acetamide (**3-5**) 510 mg, yield 73 %. LCMS: m/z= 283.95 (M+H)⁺.

### Step 5: Synthesis of 4a-(3-fluorophenyl)hexahydro-2H-benzo[b][1,4]oxazine-3(4H)-one (3-6)

**3-5** (510 mg, 1.80 mmol) was put in a 25 mL round bottom flask. After 5 mL methanol was added for dissolving, sodium borohydride (68 mg, 1.80 mmol) was slowly added under ice bath. The reaction liquid was dried by a rotary evaporator after stirring for 30 mins. After 5 mL THF was added for dissolving, 60% sodium hydride (65 mg, 2.20 mmol) was added under ice bath, and reacted for 10 hours. The reaction liquid was quenched with saturated saline, extracted with ethyl acetate. The organic phase was concentrated, separated and purified by silica gel column chromatography to obtain a white solid of 4*a*-(3-fluorophenyl)hexahydro-2*H-*benzo[*b*][1,4]oxazine-3(4*H*)-one (*3-6*)430 mg, yield 96 %. LCMS: m/z= 250.05 (M+H)⁺.

### Step 6: Synthesis of 4a-(3-fluorophenyl)octahydro-2H-benzo[b][1,4]oxazine hydrochloride(Compound 3)

Under nitrogen protection, **3-6** (430 mg, 1.72 mmol) was dissolved in ultra-dry THF (5 mL). A solution of borane-methyl sulfide in THF (8.6 mL, 17.20 mmol, 2.0 M) was added, refluxed at 70°C for 12 hours, and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product of 4*a*-(3-fluorophenyl)octahydro-2*H*-benzo[*b*][1,4] oxazine.

The crude product was separated and purified by HPLC. The purified product was concentrated, followed by adding 1 mL diluted hydrochloric acid, then lyophilized to obtain a white solid of 4*a*-(3-fluorophenyl)octahydro-2*H*-benzo[*b*][1,4]oxazine hydrochloride(Compound **3**) 330 mg, yield 71 %. LCMS: m/z= 236.10 (M+H)⁺.

¹H NMR (400 MHz, chloroform-d) δ 10.46 (s, 1H), 9.84 (s, 1H), 8.06 - 7.77 (m, 2H), 7.54 - 7.39 (m, 1H), 7.09 (t, *J* = 7.6 Hz, 1H), 4.39 (t, *J* = 11.5 Hz, 1H), 4.29 - 4.15 (m, 1H), 4.07 (d, *J* = 11.7 Hz, 1H), 3.13 - 2.88 (m, 2H), 2.78 (d, *J* = 11.7 Hz, 1H), 2.27 (t, *J* = 12.2 Hz, 1H), 1.99 (s, 2H), 1.80 - 1.70 (m, 1H), 1.65 - 1.49 (m, 2H), 1.11 - 0.91 (m, 1H). ¹⁹F NMR (376 MHz, chloroform-*d*) δ -112.45.

### Synthesis of Compound 4

### Synthesis of 4a-(4-fluorophenyl)octahydro-2H-benzo[b][1,4]oxazine formate (Compound 4)

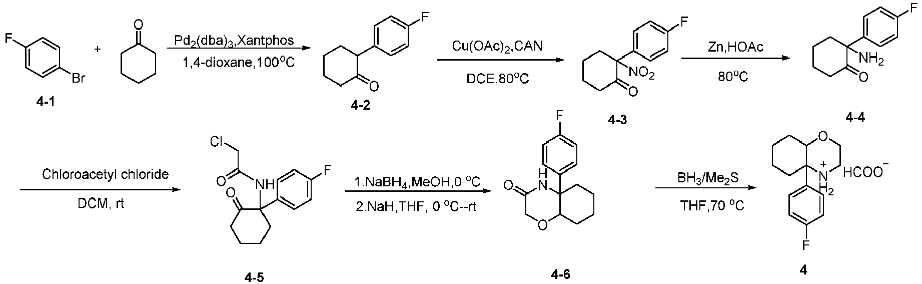

### Step 1: Synthesis of 2-(4-fluorophenyl)cyclohexanone (4-2)

Under nitrogen protection, Pd₂(dba)₃ (262 mg, 0.30 mmol), Xantphos (331 mg, 0.57 mmol) and cesium carbonate (20.5 g, 63.00 mmol) were dissolved in ultra-dry 1,4-dioxane (30 mL), added with 3-bromofluorobenzene (5 g, 28.60 mmol) and cyclohexanone (5.6 g, 57.10 mmol), and heated at 100°C for 20 hours. The reaction liquid was extracted with EA and water after being cooled. The organic layer was dried with anhydrous sodium sulfate, concentrated, separated and purified by silica gel column chromatography to obtain 2.58 g yellow oil of 2-(4-fluorophenyl)cyclohexanone (**4-2**), yield 47 %. LCMS: m/z= 193.05 (M+H)⁺.

### Step 2: Synthesis of 2-(4-fluorophenyl)-2-nitrocyclohexanone (4-3)

**4-2** (2.58 g, 13.42 mmol) was dissolved in DCE (25 mL), added with copper acetate (1.22 g, 6.71 mmol) and cerium ammonium nitrate (18.4 g, 33.60 mmol), and heated at 80°C for 12 hours. The reaction liquid was filtered, washed with EA, concentrated, separated and purified by silica gel column chromatography to obtain 0.79 g yellow oil of 2-(4-fluorophenyl)-2-nitrocyclohexanone (**4-3**), yield 25 %.

### Step 3: Synthesis of 2-(4-fluorophenyl)-2-aminocyclohexanone (4-4)

Under nitrogen protection, **4-3** (0.79 g, 3.33 mmol) was dissolved in acetic acid (6 mL), added with zinc powder (1.3 g, 20.00 mmol), and heated at 80°C for 12 hours. After being cooled, the reaction liquid was adjusted to pH>10 with a 2 M solution of sodium hydroxide, extracted with ethyl acetate. Then the organic phase was dried with anhydrous sodium sulfate, concentrated, separated and purified by silica gel column chromatography, to obtain a colorless oil of 2-(4-fluorophenyl)-2-aminocyclohexanone (**4-4**) 440 mg, yield 64 %. LCMS: m/z= 208.05 (M+H)⁺.

### Step 4: Synthesis of 2-chloro-N-(1-(4-fluorophenyl)-2-oxocyclohexyl)acetamide (4-5)

Under nitrogen protection, **4-4** (440 mg, 2.12 mmol) was dissolved in ultra-dry DCM (5 mL), added with ultra-dry triethylamine (0.33 mL, 2.33 mmol). Chloroacetyl chloride (170 µL, 2.12 mmol) was added dropwise at 0°C and stirred at room temperature for 1 hour. The reaction liquid was concentrated directly, separated and purified by silica gel column chromatography to obtain a white solid of 2-chloro-N-(1-(4-fluorophenyl)-2-oxocyclohexyl)acetamide (**4-5**) 340 mg, yield 56 %. LCMS: m/z= 284.00 (M+H)⁺.

### Step 5: Synthesis of 4a-(4-fluorophenyl)hexahydro-2H-benzo[b][1,4]oxazine-3(4H)-one (4-6)

**4-5** (340 mg, 1.20 mmol) was put in a 25 mL round bottom flask. After 5 mL methanol was added for dissolving, sodium borohydride (45 mg, 1.20 mmol) was added slowly at 0°C. The reaction liquid was dried by a rotary evaporator after stirring for 30 mins. After 5 mL THF was added for dissolving, 60% sodium hydride (60 mg, 1.40 mmol) was added under ice bath and reacted for 10 hours. The reaction liquid was quenched with saturated saline, extracted with ethyl acetate. The organic phase was concentrated, separated and purified by silica gel column chromatography to obtain a white solid of 4*a*-(4-fluorophenyl)hexahydro-2*H-*benzo[*b*][1,4]oxazine-3(4*H*)-one(**4-6**) 200 mg, yield 66 %. LCMS: m/z= 250.05 (M+H)⁺.

### Step 6: Synthesis of 4a-(4-fluorophenyl)octahydro-2H-benzo[b][1,4]oxazine formate (Compound 4)

Under nitrogen protection, **4-6**(100 mg, 0.40 mmol) was dissolved in ultra-dry THF (3 mL), added with a solution of borane-methyl sulfide in THF (2 mL, 4.00 mmol, 2.0 M), refluxed at 70°C for 12 hours, and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product containing 4*a*-(4-fluorophenyl)octahydro-2*H*-benzo[*b*][1,4]oxazine.

The crude product was separated and purified by HPLC. The purified product was concentrated, followed by adding a small amount of formic acid, then lyophilized, to obtain a white solid of 4*a*-(4-fluorophenyl)octahydro-2*H*-benzo[*b*][1,4]oxazine formate (Compound **4**) 54 mg, yield 48 %. LCMS: m/z= 236.05 (M+H)⁺.

¹H NMR (400 MHz, chloroform-d) δ 8.67 (s, 2H), 8.42 (s, 1H), 7.92 (s, 2H), 7.10 (t, *J* = 8.6 Hz, 2H), 4.16 - 3.93 (m, 3H), 2.85 (d, *J* = 5.4 Hz, 2H), 2.52 (d, *J* = 13.9 Hz, 1H), 2.00 - 1.81 (m, 3H), 1.71 (d, *J* = 13.0 Hz, 1H), 1.59 - 1.43 (m, 2H), 1.01 (t, *J* = 13.4 Hz, 1H).

### Synthesis of Compound 5

### Synthesis of 4a-(2,3-difluorophenyl)octahydro-2H-benzo[b] [1,4]oxazine formate (Compound 5)

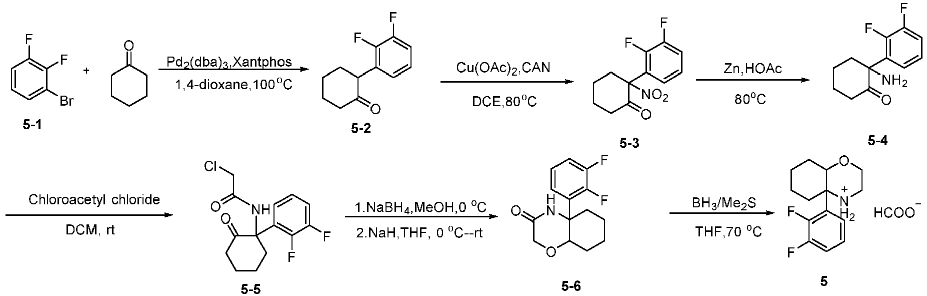

### Step 1: Synthesis of 2-(2,3-difluorophenyl)cyclohexanone (5-2)

Under nitrogen protection, Pd₂(dba)₃(237 mg, 0.26 mmol), Xantphos (301 mg, 0.52 mmol) and cesium carbonate (18.6 g, 57.00 mmol) were dissolved in ultra-dry 1,4-dioxane (30 mL), added with 3-bromofluorobenzene (5 g, 28.60 mmol) and cyclohexanone (5.6 g, 57.10 mmol) and heated at 100°C for 20 hours. The reaction liquid was extracted with EA and water after being cooled. The organic layer was dried with anhydrous sodium sulfate, concentrated, separated and purified by silica gel column chromatography to obtain 2.34 g yellow oil of 2-(2, 3-difluorophenyl)cyclohexanone (**5-2**), yield 43 %. LCMS: m/z= 211.00 (M+H)⁺.

### Step 2: Synthesis of 2-(2,3-difluorophenyl)-2-nitrocyclohexanone (5-3)

**5-2** (2.34 g, 11.13 mmol) was dissolved in DCE (25 mL), added with copper acetate (1.01 g, 5.57 mmol) and cerium ammonium nitrate (15.3 g, 27.80 mmol), and heated at 80°C for 12 hours, The reaction liquid was filtered, washed with EA, concentrated, separated and purified by silica gel column chromatography to obtain 0.75 g yellow oil of 2-(2,3-difluorophenyl)-2-nitrocyclohexanone(**5-3**), yield 26 %.

### Step 3: Synthesis of 2-(2,3-difluorophenyl)-2-aminocyclohexanone (5-4)

Under nitrogen protection, **5-3** (0.75 g, 2.94 mmol) was dissolved in acetic acid (6 mL), added with zinc powder (1.2 g, 17.60 mmol) and heated at 80°C for 12 hours. After being cooled, the reaction liquid was adjusted to pH> 10 with a 2 M solution of sodium hydroxide and extracted with ethyl acetate. Then the organic phase was dried with anhydrous sodium sulfate, concentrated, separated and purified by silica gel column chromatography, to obtain a colorless oil of 2-(2,3-difluorophenyl)-2-aminocyclohexanone (**5-4**) 440 mg, yield 66 %. LCMS: m/z= 226 .10 (M+H)⁺.

### Step 4: Synthesis of 2-chloro-N-(1-(2,3-difluorophenyl)-2-oxocyclohexyl)acetamide (5-5)

Under nitrogen protection, **5-4**(440 mg, 1.95 mmol) was dissolved in ultra-dry DCM (5 mL), added with ultra-dry triethylamine (0.33 mL, 2.15 mmol). Chloroacetyl chloride (155 µL, 1.95 mmol) was added dropwise at 0°C, stirred at room temperature for 1 hour until the raw material disappeared by analysis of TLC. The reaction liquid was concentrated directly, separated and purified by silica gel column chromatography to obtain a white solid of 2-chloro-N-(1-(2,3-difluorophenyl)-2-oxocyclohexyl)acetamide (**5-5**) 340 mg, yield 59 %. LCMS: m/z= 302.05 (M+H)⁺.

### Step 5: Synthesis of 4a-(2,3-difluorophenyl)hexahydro-2H-benzo[b][1,4]oxazine-3(4H)-one (5-6)

**5-5** (340 mg, 1.13 mmol) was put in a 25 mL round bottom flask. After 5 mL methanol was added for dissolving, sodium borohydride (43 mg, 1.13 mmol) was added slowly at 0°C. The reaction liquid was dried by a rotary evaporator after stirring for 30 mins. After 5 mL THF was added for dissolving, 60% sodium hydride (54 mg, 1.40 mmol) was added under ice bath and reacted for 10 hours. The reaction liquid was quenched with saturated saline, extracted with ethyl acetate. The organic phase was concentrated, separated and purified by silica gel column chromatography to obtain a white solid of 4*a*-(2,3-difluorophenyl)hexahydro-2*H-*benzo[*b*][1,4]oxazine-3(4*H*)-one (**5-6**)180 mg, yield 60 %. LCMS: m/z= 268.05 (M+H)⁺.

### Step 6: Synthesis of 4a-(2,3-difluorophenyl)octahydro-2H-benzo[b][1,4]oxazine formate (Compound 5)

Under nitrogen protection, **5-6** (180 mg, 0.67 mmol) was dissolved in ultra-dry THF (3 mL), added with a solution of borane-methyl sulfide in THF (3.4 mL, 6.80 mmol, 2.0 M), refluxed at 70°C for 12 hours, and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product of Compound **5.**

The crude product of Compound **5** was separated and purified by HPLC. The purified product was concentrated, followed by adding a small amount of formic acid, then lyophilized to obtain a white solid of 4*a*-(2,3-difluorophenyl)octahydro-2*H*-benzo[*b*][1,4]oxazine formate 145 mg, yield 55 %. LCMS: m/z= 236.10 (M+H)⁺.

¹H NMR (400 MHz, chloroform-*d*) δ 7.85 - 7.76 (m, 1H), 7.10 - 6.98 (m, 2H), 3.94 - 3.79 (m, 2H), 3.61 (dd, *J* = 12.7, 4.5 Hz, 1H), 2.72 (td, *J* = 11.8, 3.9 Hz, 1H), 2.63 - 2.54 (m, 2H), 2.29 (s, 1H), 2.00 (qd, *J* = 12.8, 4.6 Hz, 1H), 1.91 - 1.83 (m, 1H), 1.79 - 1.71 (m, 1H), 1.57 - 1.39 (m, 3H), 0.97 (qt, *J* = 12.4, 3.1 Hz, 1H). ¹⁹F NMR (376 MHz, chloroform-d) δ -136.94 , -137.78 - - 138.10 (m).

### Synthesis of Compound 6

### Synthesis of 4a-(2-fluorophenyl)octahydro-2H-benzo[b][1,4]oxazine formate (Compound 6)

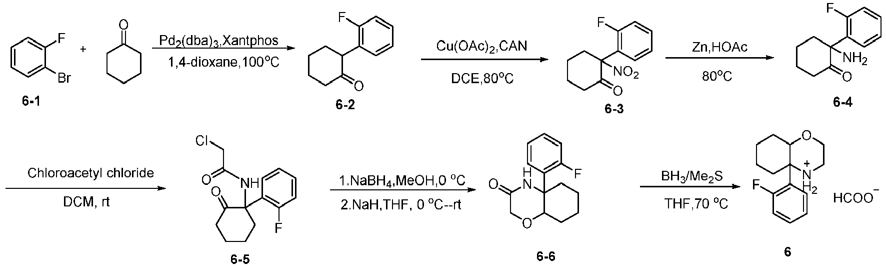

### Step 1: Synthesis of 2-fluorophenylcyclohexanone (6-2)

Under nitrogen protection, Pd₂(dba)₃(262 mg, 0.30 mmol), Xantphos (331 mg, 0.57 mmol) and cesium carbonate (20.5 g, 63.00 mmol) were dissolved in ultra-dry 1,4-dioxane (30 mL), added with 2-bromofluorobenzene (5 g, 28.60 mmol) and cyclohexanone (5.6 g, 57.10 mmol) and heated at 100°C for 20 hours. The reaction liquid was extracted with EA and water after being cooled. The organic layer was dried with anhydrous sodium sulfate, concentrated, separated and purified by silica gel column chromatography to obtain 1.21 g yellow oil of 2-fluorophenylcyclohexanone (**6-2**), yield 22 %. LCMS: m/z= 193.05 (M+H)⁺.

### Step 2: Synthesis of 2-(2-fluorophenyl)-2-nitrocyclohexanone (6-3)

**6-2** (1.21 g, 6.30 mmol) was dissolved in DCE (10 mL), added with copper acetate (0.6 g, 3.15 mmol) and cerium ammonium nitrate (8.6 g, 15.80 mmol), and heated at 80°C for 12 hours. The reaction liquid was filtered, washed with EA, concentrated, separated and purified by silica gel column chromatography to obtain 0.52 g yellow oil of 2-(2-fluorophenyl)-2-nitrocyclohexanone (**6-3**), yield 35 %.

### Step 3: Synthesis of 2-(2-fluorophenyl)-2-aminocyclohexanone (6-4)

Under nitrogen protection, **6-3** (0.52 g, 2.20 mmol) was dissolved in acetic acid (5 mL), added with zinc powder (0.86 g, 13.20 mmol), and heated at 80°C for 12 hours. After being cooled, the reaction liquid was adjusted to pH>10 with a 2 M solution of sodium hydroxide, extracted with ethyl acetate. Then the organic phase was dried with anhydrous sodium sulfate, concentrated, separated and purified by silica gel column chromatography to obtain a colorless oil of 2-(2-fluorophenyl)-2-aminocyclohexanone (**6-4**) 300 mg, yield 66 %. LCMS: m/z= 208.05 (M+H)⁺.

### Step 4: Synthesis of 2-chloro-N-(1-(2-fluorophenyl)-2-oxocyclohexyl)acetamide (6-5)

Under nitrogen protection, **6-4** (300 mg, 1.45 mmol) was dissolved in ultra-dry DCM (5 mL), added with ultra-dry triethylamine (0.3 mL, 1.60 mmol). Chloroacetyl chloride (115 µL, 1.45 mmol) was added dropwise at 0°C and stirred at room temperature for 1 hour. The reaction liquid was concentrated directly, separated and purified by silica gel column chromatography to obtain a white solid of 2-chloro-N-(1-(2-fluorophenyl)-2-oxocyclohexyl)acetamide (**6-5**)300 mg, yield 61 %. LCMS: m/z= 284.00 (M+H)⁺.

### Step 5: Synthesis of 4a-(2-fluorophenyl)hexahydro-2H-benzo[b][1,4]oxazine-3(4H)-one (6-6)

**6-5** (250 mg, 0.88 mmol) was put in a 25 mL round bottom flask. After 5 mL methanol was added for dissolving, sodium borohydride (33 mg, 0.88 mmol) was added slowly at 0°C. The reaction liquid was dried by a rotary evaporator after stirring for 30 mins. After 5 mL THF was added for dissolving, 60% sodium hydride (42 mg, 1.06 mmol) was added under ice bath and reacted for 10 hours. The reaction liquid was quenched with saturated saline, extracted with ethyl acetate. The organic phase was concentrated, separated and purified by silica gel column chromatography to obtain a white solid of4*a*-(2-fluorophenyl)hexahydro-2*H-*benzo[*b*][1,4]oxazine-3(4*H*)-one(**6-6**) 155 mg, yield 71 %. LCMS: m/z= 250.05 (M+H)⁺.

### Step 6: Synthesis of 4a-(2-fluorophenyl)octahydro-2H-benzo[b][1,4]oxazine formate (Compound 6)

Under nitrogen protection, **6-6** (155 mg, 0.62 mmol) was dissolved in ultra-dry THF (3 mL), added with a solution of borane-methyl sulfide in THF (3.1 mL, 6.20 mmol, 2.0 M), refluxed at 70°C for 12 hours, and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product of Compound **6.**

The crude product was separated and purified by HPLC. The purified product was concentrated, followed by adding a small amount of formic acid, then lyophilized, to obtain a white solid of 4*a*-(2-fluorophenyl)octahydro-2*H*-benzo[*b*][1,4]oxazine formate 104 mg, yield 71 %.

LCMS: m/z= 236.10 (M+H)⁺.

¹H NMR (400 MHz, chloroform-*d*) δ 7.92 (td, *J* = 8.1, 1.6 Hz, 1H), 7.30 - 7.22 (m, 2H), 7.13 - 7.02 (m, 2H), 6.73 (s, 1H), 4.39 - 4.21 (m, 2H), 3.78 (dd, *J* = 12.4, 4.8 Hz, 1H), 2.81 - 2.70 (m, 1H), 2.25 - 2.06 (m, 2H), 1.91 - 1.81 (m, 1H), 1.64 - 1.41 (m, 3H), 1.04 - 0.88 (m, 1H).

### Synthesis of Compound 7

### Synthesis of 6-methyl-4a-phenyloctahydro-2H-benzo[b][1,4]oxazine formate (Compound 7)

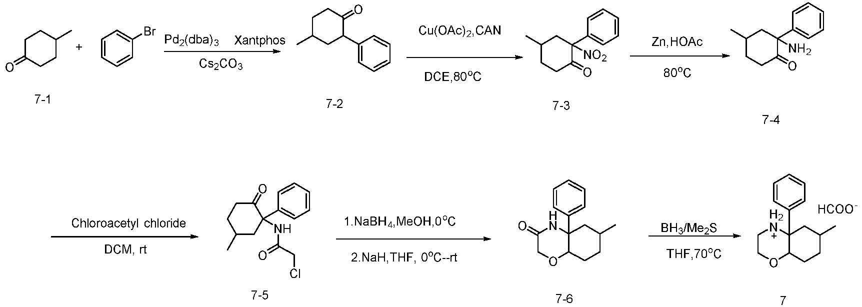

### Step 1: Synthesis of 4-methyl-2-phenylcyclohexanone (7-2):

Under nitrogen protection, Pd₂(dba)₃ (293 mg, 0.32 mmol), Xantphos (367 mg, 0.64 mmol), cesium carbonate (22.77 g, 70.07 mmol) were dissolved in 30 mL ultra-dry dioxane, added with bromobenzene (5 g, 31.85 mmol), 4-methyl cyclohexanone (7.13 g, 63.7 mmol, **7-1**) and stirred at 80°C for 20 hours. After being cooled to room temperature, the reaction liquid was diluted with EA, filtered with diatomite, extracted with water, dried with anhydrous sodium sulfate, and separated by flash silica chromatography to obtain 4.58 g light yellow liquid of 4-methyl-2-phenylcyclohexanone (**7-2**), yield 76.3%. LCMS: m/z=189.05(M+H)⁺.

### Step 2: Synthesis of 4-methyl-2-nitro-2-phenylcyclohexanone (7-3):

Under nitrogen protection, cerium ammonium nitrate (31.3 g, 57.12 mmol), copper acetate (3.45 g, 19.04 mmol), **7-2**(3.58 g, 19.04 mmol) were dissolved in 1,2-dichloroethane (60 ml) and stirred at 80°C for 12 hours. After being cooled to room temperature, the reaction liquid was diluted with DCM, filtered with diatomite, washed with DCM. After concentration, the filtrate was separated by flash silica chromatography to obtain 1.8 g yellow liquid of 4-methyl-2-nitro-2-phenylcyclohexanone (**7-3**), yield 41.1%.

### Step 3: Synthesis of 2-amino-4-methyl-2-phenylcyclohexanone (7-4):

Under nitrogen protection, **7-3** (1.12 g, 4.80 mmol) was dissolved in 20 ml methanol and added with 10 mL icy acetic acid. 1.56 g zinc powder was added slowly and stirred at 80°C for 12 hours, the reaction liquid was adjusted to pH>10 with a 2 M solution of sodium hydroxide, extracted with EA, dried with anhydrous sodium sulfate, separated and purified by flash silica gel column chromatography to obtain 515 mg light yellow liquid of 2-amino-4-methyl-2-phenylcyclohexanone (**7-4**), yield 52.8%. LCMS: m/z=204.05(M+H)⁺.

### Step 4: Synthesis of 2-chloro-N-(5-methyl-2-oxo-1-phenylhexyl)acetamide (7-5):

Under nitrogen protection, **7-4** (515 mg, 2.54 mmol) was dissolved in 12 mL ultra-dry DCM, added with triethylamine (513 mg, 5.08 mmol), cooled to 0°C, added dropwise chloroacetyl chloride (300 mg, 2.66 mmol) and stirred at room temperature for 1 hour. The reaction liquid was extracted with EA. The organic phase was dried with anhydrous sodium sulfate, separated and purified by flash silica gel column chromatography after concentration to obtain 402 mg light yellow solid of 2-chloro-N-(5-methyl-2-oxo-1-phenylhexyl)acetamide (**7-5**), yield 56.8%. LCMS: m/z=280.05(M+H)⁺.

### Step 5: Synthesis of 6-methyl-4a-phenylhexahydro-2H-benzo[b][1,4]oxazine-3(4H)-one(7-6):

Under nitrogen protection, **7-5** (200 mg, 0.72 mmol) was dissolved in 5 mL ultra-dry methanol, cooled to 0°C, added with sodium borohydride (27 mg, 0.72 mmol), warmed to room temperature and stirred for 1 hour with rotary evaporating solvents under reduced pressure, added with 5 mL ultra-dry THF for dissolving, cooled to 0°C, added with 60% sodium hydride (35 mg, 0.86 mmol), warmed to room temperature and stirred for 12 hours. The reaction was quenched by adding 1 N HCl and extracted with DCM. The organic phase was dried with anhydrous sodium sulfate, separated and purified by flash silica gel column chromatography after concentration to obtain 105 mg white solid of 6-methyl-4*a*-phenylhexahydro-2*H*-benzo[*b*][1,4]oxazine-3(4*H*)-one(**7-6**), yield 60%. LCMS: m/z=246.05(M+H)⁺.

### Step 6: Synthesis of 6-methyl-4a-phenyloctahydro-2H-benzo[b][1,4]oxazine formate (Compound 7):

Under nitrogen protection, **7-6** (108 mg, 0.44 mmol) was dissolved in 4 mL ultra-dry THF, added with 2.2 mL borane/dimethyl sulfide and stirred at 70°C for 12 hours. After being cooled to room temperature, the reaction was quenched by adding MeOH dropwise. 1 N HCl was added and stirred for 1 hour. The reaction liquid was adjusted to pH of 8-9 with saturated NaHCO₃ solution and extracted with EA. The organic phase was dried with anhydrous sodium sulfate, then dried by a rotary evaporator under reduced pressure to obtain a crude product of 6-methyl-4*a-*phenyloctahydro-2*H*-benzo[*b*][1,4]oxazine.

The crude product was dissolved in a small amount of MeOH, separated and purified by HPLC, then lyophilized to obtain 58 mg white solid of 6-methyl-4*a*-phenyloctahydro-2*H-*benzo[b][1,4]oxazine formate (Compound 7), yield 57.4%. LCMS: m/z=232.05(M+H)⁺.

¹H NMR (400 MHz, chloroform-d) δ 8.48 (s, 1H), 7.93 (d, J = 6.3 Hz, 2H), 7.39 (dt, J = 29.6, 7.2 Hz, 3H), 4.17 - 3.94 (m, 3H), 2.98 - 2.78 (m, 2H), 2.53 (d, J = 12.7 Hz, 1H), 2.11 - 1.87 (m, 2H), 1.70 (t, J = 11.9 Hz, 2H), 1.23 (dq, J = 16.4, 5.8, 4.5 Hz, 2H), 0.87 (d, J = 5.8 Hz, 3H).

### Synthesis of Compound 8

### Synthesis of 6-ethyl-4a-phenyloctahydro-2H-benzo[b][1,4]oxazine formate (Compound 8)

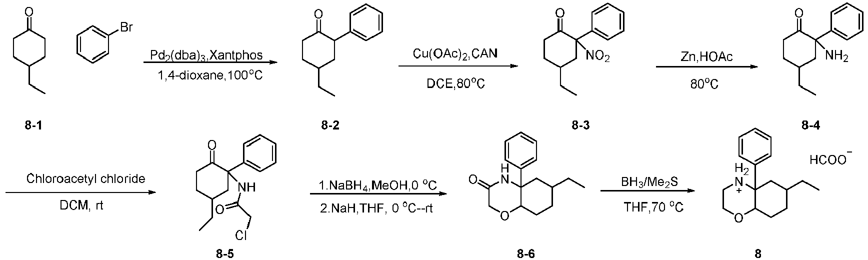

### Step 1: Synthesis of 4-ethyl-2-phenylcyclohexanone (8-2):

Under nitrogen protection, Pd₂(dba)₃(293 mg, 0.32 mmol), Xantphos (367 mg, 0.64 mmol), cesium carbonate (22.77 g, 70.07 mmol) were dissolved in ultra-dry dioxane 30 mL, added with bromobenzene (5 g, 31.85 mmol), 4-ethyl cyclohexanone (8.03 g, 31.85 mmol, **8-1**) and stirred at 80°C for 20 hours. After being cooled to room temperature, the reaction liquid was diluted with EA, filtered with diatomite, extracted with water, dried with anhydrous sodium sulfate, separated by combi-flash silica chromatography to obtain 3.56 g light yellow liquid of 4-ethyl-2-phenylcyclohexanone (**8-2**), yield 55.2%. LCMS: m/z=203.05(M+H)⁺.

### Step 2: Synthesis of 4-ethyl-2-nitro-2-phenylcyclohexanone (8-3):

Under nitrogen protection, cerium ammonium nitrate (28.96 g, 58.26 mmol), copper acetate (3.19 g, 17.62 mmol), **8-2** (3.56 g, 17.62 mmol) were dissolved in 1,2-dichloroethane (60 mL) and stirred at 80°C for 12 hours. After being cooled to room temperature, the reaction liquid was diluted with DCM, filtered with diatomite, washed with DCM, filtered to remove residue, separated by combi-flash silica chromatography to obtain 1.4 g yellow liquid of 4-ethyl-2-nitro-2-phenylcyclohexanone (**8-3**), yield 32.6%.

### Step 3: Synthesis of 2-amino-4-ethyl-2-phenylcyclohexanone (8-4):

Under nitrogen protection, **8-3** (873 mg, 3.75 mmol) was dissolved in 16 mL methanol, added with 8 mL icy acetic acid. Zinc powder (1.22 g, 18.75 mmol) was added slowly and stirred at 80°C for 12 hours. The reaction liquid was adjusted to pH of 10 with a 2 M NaOH, extracted with EA, dried with anhydrous sodium sulfate, separated and purified by flash silica gel column chromatography after concentration to obtain 377 mg yellow oil of 2-amino-4-ethyl-2-phenylcyclohexanone (**8-4**), yield 46.4%. LCMS: m/z=218.05(M+H)⁺.

### Step 4: Synthesis of 2-chloro-N-(5-ethyl-2-oxo-l-phenylhexyl)acetamide (8-5):

Under nitrogen protection, **8-4** (377 mg, 1.74 mmol) was dissolved in 6 mL ultra-dry DCM, added with triethylamine (210 mg, 2.08 mmol) and cooled to 0°C. Chloroacetyl chloride (197 mg, 1.74 mmol) was added dropwise, stirred at room temperature for 1 hour. The reaction liquid was extracted with EA. The organic phase was dried with anhydrous sodium sulfate, separated and purified by flash silica gel column chromatography after concentration to obtain 394 mg light yellow solid of 2-chloro-N-(5-ethyl-2-oxo-1-phenylhexyl)acetamide (**8-5**), yield 77.4%. LCMS: m/z=294.05(M+H)⁺.

### Step 5: Synthesis of 6-ethyl-4a-phenylhexahydro-2H-benzo[b] [1,4]oxazine-3(4H)-one (8-6):

Under nitrogen protection, **8-5** (190 mg, 0.65 mmol) was dissolved in 4 mL ultra-dry methanol, cooled to 0°C, added with sodium borohydride (25 mg, 0.65 mmol), warmed to room temperature and stirred for 1 hour with rotary evaporating solvents under reduced pressure, added with 4 mL ultra-dry THF for dissolving, cooled to 0°C, added with 60% sodium hydride (31 mg, 0.78 mmol), warmed to room temperature and stirred for 12 hours. The reaction was quenched by adding 1 N HCl, extracted with DCM. The organic phase was dried with anhydrous sodium sulfate, separated and purified by flash silica gel column chromatography after concentration to obtain 132 mg white solid of 6-ethyl-4*a*-phenylhexahydro-2*H*-benzo[*b*][1,4]oxazine-3(4*H*)-one(**8-6**), yield 78.5%. LCMS: m/z=260.05(M+H)⁺.

### Step 6: Synthesis of 6-ethyl-4a-phenyloctahydro-2H-benzo[b][1,4]oxazine formate (Compound 8):

Under nitrogen protection, **8-6** (132 mg, 0.51 mmol) was dissolved in 6 mL ultra-dry THF, added with borane/dimethyl sulfide, stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution and extracted with EA. The organic phase was dried with anhydrous sodium sulfate, then dried with a rotary evaporator under reduced pressure. The product was dissolved with a small amount of MeOH, separated and purified by HPLC, then lyophilized to obtain 16 mg colorless viscous liquid of 6-ethyl-4*a*-phenyloctahydro-2*H*-benzo[*b*][1,4]oxazine formate (Compound **8**), yield 12.9%. LCMS: m/z=246.10(M+H)⁺.

¹H NMR (400 MHz, chloroform-d) δ 7.86 (d, J = 7.3 Hz, 2H), 7.40 (t, J = 7.9 Hz, 2H), 7.31 (t, J = 7.3 Hz, 1H), 4.00 (d, J = 5.9 Hz, 2H), 3.87 (dd, J = 12.2, 4.4 Hz, 1H), 2.88 - 2.71 (m, 2H), 2.49 (d, J = 13.1 Hz, 1H), 2.04 - 1.85 (m, 2H), 1.75 (d, J = 13.1 Hz, 1H), 1.48 (t, J = 12.6 Hz, 1H), 1.19 (ddt, J = 20.8, 13.1, 7.4 Hz, 3H), 0.80 (t, J = 7.4 Hz, 3H).

### Synthesis of Compound 9

### Synthesis of 4a-(3-chlorophenyl)octahydro-2H-benzo[b][1,4]oxazine formate (Compound 9)

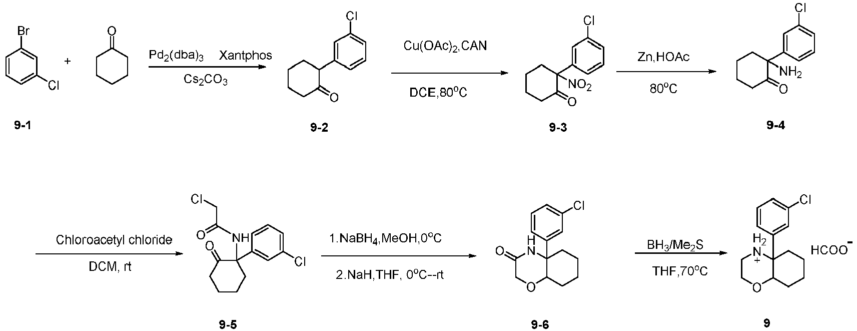

### Step 1: Synthesis of 2-(3-chlorophenyl)cyclohexanone (9-2)

Under nitrogen protection, Pd₂(dba)₃ (238 mg, 0.26 mmol), Xantphos (301 mg, 0.52 mmol) and cesium carbonate (18.72 g, 57.46 mmol) were dissolved in ultra-dry 1,4-dioxane (25 mL), added with m-chlorobromobenzene (5 g, 26.12 mmol, **9-1**) and cyclohexanone (5.13 g, 52.23 mmol), heated at 100°C for 20 hours. The reaction liquid was extracted with EA and water after being cooled. The organic layer was dried with anhydrous sodium sulfate, concentrated, then separated by silica gel column chromatography to obtain 3.4 g yellow clear liquid of 2-(3-chlorophenyl)cyclohexanone (**9-2**), yield 62.4%. LCMS: m/z= 209.00 (M+H)⁺.

### Step 2: Synthesis of 2-(3-chlorophenyl)-2-nitrocyclohexanone(9-3)

**9-2** (3.4 g, 16.29 mmol) was dissolved in DCE (55 mL), added with copper acetate (2.96 g, 16.29 mmol) and cerium ammonium nitrate (26.8 g, 48.87 mmol), and heated at 80°C for 12 hours. The reaction liquid was filtered, washed with DCM, dried with a rotary evaporator, then separated by silica gel column chromatography to obtain 1.8 g yellow solid of 2-(3-chlorophenyl)-2-nitrocyclohexanone (**9-3**), yield 43.6%. LCMS: m/z = 207.00(M-NO₂)⁺.

### Step 3: Synthesis of 2-amino-2-(3-chlorophenyl)cyclohexanone (9-4)

Under nitrogen protection, **9-3** (900 mg, 3.55 mmol) was dissolved in methanol (15 mL), added with icy acetic acid (7.5 mL). Zinc powder (1.15 g, 17.75 mmol) was added slowly and heated at 80°C for 12 hours. The reaction liquid was neutralized by adding dropwise saturated sodium bicarbonate solution after being cooled, adjusted to pH> 10 with a 2 M NaOH. The organic phase was dried with anhydrous sodium sulfate, separated by silica gel column chromatography to obtain 468 mg yellow liquid of 2-amino-2-(3-chlorophenyl)cyclohexanone (**9-4**), yield 58.9%. LCMS: m/z= 224.00 (M+H)⁺.

### Step 4: Synthesis of 2-chloro-N-(1-(3-chlorophenyl)-2-oxocyclohexyl)acetamide (9-5)

Under nitrogen protection, **9-4** (468 mg, 2.09 mmol) was dissolved in ultra-dry DCM (10 mL), added with ultra-dry triethylamine (423 mg, 4.18 mmol). Chloroacetyl chloride (248 mg, 2.20 mmol) was added dropwise at 0°C and stirred at room temperature for 1 hour. Water and EA were added for extraction. The organic layer was washed with saturated ammonium chloride solution, dried with anhydrous sodium sulfate, separated by flash silica gel column chromatography after concentration to obtain 366 mg white solid of 2-chloro-N-(1-(3-chlorophenyl)-2-oxocyclohexyl)acetamide (**9-5**), yield 58.4%. LCMS: m/z= 300.00 (M+H)⁺.

### Step 5: Synthesis of 4a-(3-chlorophenyl)hexahydro-2H-benzo[b][1,4]oxazine-3(4H)-one (9-6)

**9-5** (150 mg, 0.50 mmol) was dissolved in ultra-dry methanol (1.5 mL) at 0°C, added with sodium borohydride (19 mg, 0.50 mmol), stirred at room temperature for 1 hour, dissolved in ultra-dry THF (1.5 mL) after rotary evaporating solvents, added with 60% sodium hydride (24 mg, 0.60 mmol), stirred at room temperature for 12 hours. The reaction was quenched with 1M hydrochloric acid and extracted with DCM. The organic layer was dried with anhydrous sodium sulfate, separated by silica gel column chromatography after concentration to obtain 62mg white solid of 4*a*-(3-chlorophenyl)hexahydro-2*H*-benzo[*b*][1,4]oxazine-3(4*H*)-one(**9-6**), yield 46.6%. LCMS: m/z= 266.05 (M+H)⁺.

### Step 6: Synthesis of 4a-(3-chlorophenyl)octahydro-2H-benzo[b][1,4]oxazine formate (Compound 9)

Under nitrogen protection, **9-6** (62 mg, 0.23 mmol) was dissolved in ultra-dry THF (1 mL), added with a solution of borane-methyl sulfide in THF (0.58 mL, 1.17 mmol, 2.0 M), heated at 70°C for 12 hours. The reaction was quenched by adding dropwise a small amount of methanol after being cooled, added with 2 M hydrochloric acid and stirred at room temperature for 1 hour. After the reaction liquid was neutralized with saturated sodium bicarbonate, EA was added for extraction. The organic layer was dried with anhydrous sodium sulfate, separated by HPLC after concentration to obtain 31 mg yellow viscous liquid of 4*a*-(3-chlorophenyl)octahydro-2*H-*benzo[*b*][1,4]oxazine formate (Compound **9**), yield 45.8%. LCMS: m/z= 252.05 (M+H)⁺.

¹H NMR (400 MHz, chloroform-d) δ 8.43 (br, 1H), 7.91 (s, 1H), 7.76 (s, 1H), 7.52 - 7.14 (m, 4H), 4.02 (s, 2H), 3.95 - 3.81 (m, 1H), 2.81 (s, 2H), 2.44 (d, *J* = 8.9 Hz, 1H), 1.98 - 1.63 (m, 4H), 1.61 - 1.37 (m, 2H), 1.10 - 0.90 (m, 1H).

### Synthesis of Compound 10

### Synthesis of 4a-(3-methylphenyl)octahydro-2H-benzo[b][1,4]oxazine formate (Compound 10)

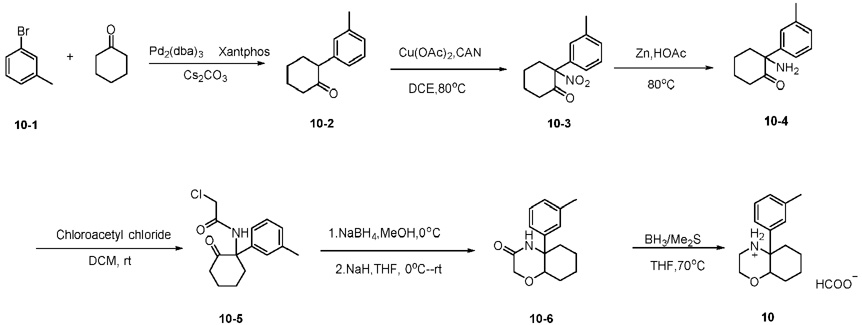

### Step 1: Synthesis of 2-(3-methylphenyl)cyclohexanone (10-2)

Under nitrogen protection, Pd₂(dba)₃ (266 mg, 0.29 mmol), Xantphos (336 mg, 0.58 mmol) and cesium carbonate (21 g, 64.31 mmol) were dissolved in ultra-dry 1,4-dioxane (30 mL), added with 3-bromotoluene (5 g, 29.23 mmol, **10-1**) and cyclohexanone (5.74 g, 58.46 mmol), heated at 100°C for 20 hours. The reaction liquid was extracted with EA and water after being cooled. The organic layer was dried with anhydrous sodium sulfate, separated by silica gel column chromatography after concentration to obtain 3 g yellow clear liquid of 2-(3-methylphenyl)cyclohexanone (**10-2**), yield 54.5%. LCMS: m/z= 189.05 (M+H)⁺.

### Step 2: Synthesis of 2-nitro-2-(3-methylphenyl)cyclohexanone (10-3)

**10-2** (3 g, 15.93 mmol) was dissolved in DCE (55 mL), added with copper acetate (2.89 g, 15.93 mmol) and cerium ammonium nitrate (26.2 g, 47.79 mmol), and heated at 80°C for 12 hours. The reaction liquid was filtered, washed with DCM, separated by silica gel column chromatography after concentration to obtain 1.56 g yellow solid of 2-nitro-2-(3-methylphenyl)cyclohexanone (**10-3**) crude product, yield 42.0%. LCMS: m/z = 187.00 (M-NO₂)⁺.

### Step 3: Synthesis of 2-amino-2-(3-methylphenyl)cyclohexanone (10-4)

Under nitrogen protection, **10-3** (800 mg, 3.43 mmol) was dissolved in methanol (15 mL) and added with icy acetic acid (7.5 mL). Zinc powder (1.11 g, 17.15 mmol) was added slowly and heated at 80°C for 12 hours. The reaction liquid was neutralized by adding dropwise saturated sodium bicarbonate solution after being cooled and adjusted to pH> 10 with a 2 M NaOH. The organic phase was dried with anhydrous sodium sulfate, separated by silica gel column chromatography after concentration to obtain 289 mg white solid of 2-amino-2-(3-methylphenyl)cyclohexanone (**10-4**), yield 41.5%. LCMS: m/z= 204.05 (M+H)⁺.

### Step 4: Synthesis of 2-chloro-N-(2-oxo-1-(3-methylphenyl)hexyl)acetamide (10-5)

Under nitrogen protection, **10-4** (289 mg, 1.42 mmol) was dissolved in ultra-dry DCM (7 mL), added with ultra-dry triethylamine (287 mg, 2.84 mmol). Chloroacetyl chloride (168 mg, 1.49 mmol) was added dropwise at 0°C and stirred at room temperature for 1 hour. Water and EA were added for extraction. The organic layer was washed with saturated ammonium chloride solution, dried with anhydrous sodium sulfate, separated by flash silica gel column chromatography after concentration to obtain 208 mg white solid of 2-chloro-N-(2-oxo-1-(3-methylphenyl)hexyl)acetamide (**10-5**), yield 52.4%. LCMS: m/z= 280.05 (M+H)⁺.

### Step 5: Synthesis of 4a-(3-methylphenyl)hexahydro-2H-benzo[b][1,4]oxazine-3(4H)-one (10-6)

**10-5** (208 mg, 0.74 mmol) was dissolved in ultra-dry methanol (2 mL) at 0°C, added with sodium borohydride (28 mg, 0.74 mmol), stirred at room temperature for 1 hour, dissolved in ultra-dry THF (2 mL) after rotary evaporating solvents, added with 60% sodium hydride (36 mg, 0.89 mmol) and stirred at room temperature for 12 hours. The reaction was quenched by adding dropwise methanol and extracted with water and EA. The organic layer was dried with anhydrous sodium sulfate, separated by silica gel column chromatography after concentration to obtain 101mg white solid of 4*a*-(3-methylphenyl)hexahydro-2*H*-benzo[*b*][1,4]oxazine-3(4*H*)-one (**10-6**), yield : 55.5%. LCMS: m/z= 246.05 (M+H)⁺.

### Step 6: Synthesis of 4a-(3-methylphenyl)octahydro-2H-benzo[b][1,4]oxazine formate (Compound 10)

Under nitrogen protection, **10-6** (101 mg, 0.41 mmol) was dissolved in ultra-dry THF (1 mL), added with a solution of borane-methyl sulfide in THF (1.03 mL, 2.06 mmol, 2.0 M) and heated at 70°C for 12 hours. The reaction was quenched by adding dropwise a small amount of methanol after being cooled, added with 2 M hydrochloric acid and stirred at room temperature for 1 hour. After the reaction liquid was neutralized with saturated sodium bicarbonate, EA was added for extraction. The organic layer was dried with anhydrous sodium sulfate, separated by HPLC after concentration to obtain 76 mg yellow viscous liquid of 4*a*-(3-methylphenyl)octahydro-2*H-*benzo[b][1,4]oxazine formate (Compound **10**), yield 66.8%. LCMS: m/z= 232.10 (M+H)⁺.

¹H NMR (400 MHz, chloroform-d) δ 9.33 (br, 2H), 8.47 (s, 1H), 7.70 (s, 2H), 7.33 - 7.22 (m, 1H), 7.13 (d, *J* = 7.4 Hz, 1H), 4.11 (s, 1H), 4.01 (t, *J* = 10.2 Hz, 2H), 2.87 (s, 2H), 2.63 - 2.45 (m, 1H), 2.36 (s, 3H), 2.03 - 1.82 (m, 3H), 1.79 - 1.60 (m, 1H), 1.50 (d, *J* = 11.3 Hz, 2H), 1.10 - 0.90 (m, 1H).

### Synthesis of Compound 11

### Synthesis of 4a-(2-chlorophenyl)octahydro-2H-benzo[b][1,4]oxazine formate (Compound 11)

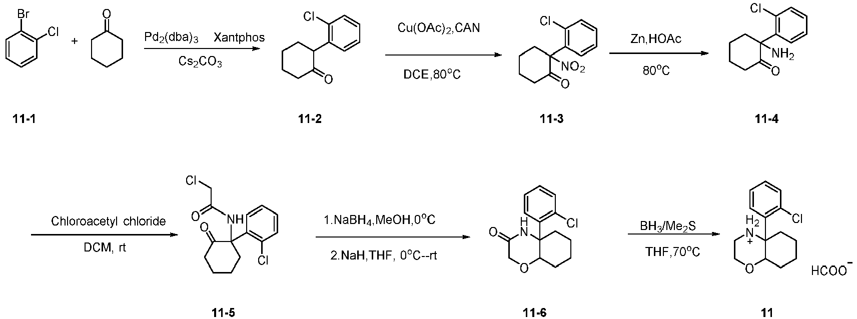

### Step 1: Synthesis of 2-(2-chlorophenyl)cyclohexanone (11-2)

Under nitrogen protection, Pd₂(dba)₃ (238 mg, 0.26 mmol), Xantphos (301 mg, 0.52 mmol) and cesium carbonate (18.72 g, 57.46 mmol) were dissolved in ultra-dry 1,4-dioxane (25 mL), added with 2-bromochlorobenzene (5 g, 26.12 mmol, **11-1**) and cyclohexanone (5.13 g, 52.23 mmol), and heated at 100°C for 20 hours. The reaction liquid was extracted with EA and water after being cooled. The organic layer was dried with anhydrous sodium sulfate, separated by silica gel column chromatography after concentration to obtain 2.07 g white solid of 2-(2-chlorophenyl)cyclohexanone (**11-2**), yield 38.0%. LCMS: m/z = 209.00 (M+H)⁺.

### Step 2: Synthesis of 2-(2-chlorophenyl)-2-nitrocyclohexanone (11-3)

**11-2** (1.87 g, 8.96 mmol) was dissolved in 30 mL DCE, added with copper acetate (1.63 g, 8.96 mmol) and cerium ammonium nitrate (12.3 g, 22.40 mmol), and heated at 80°C for 12 hours. The reaction liquid was filtered, washed with DCM, dried with a rotary evaporator, then separated by silica gel column chromatography to obtain 835 mg yellow solid of 2-(2-chlorophenyl)-2-nitrocyclohexanone(**11-3**) crude product, which was directly used for the next step. LCMS: m/z = 207.00(M-NO₂)⁺.

### Step 3: Synthesis of 2-amino-2-(2-chlorophenyl)cyclohexanone (11-4)

Under nitrogen protection, **11-3** (835 mg, 3.29 mmol) was dissolved in 14 mL methanol and added with 7 mL icy acetic acid. Zinc powder (1.07 g, 16.46 mmol) was added slowly and heated at 80°C for 12 hours. The reaction liquid was neutralized by adding dropwise saturated sodium bicarbonate solution after being cooled and adjusted to pH> 10 with a 2 M NaOH. The organic phase was dried with anhydrous sodium sulfate, separated by silica gel column chromatography to obtain 545 mg yellow liquid of 2-amino-2-(2-chlorophenyl)cyclohexanone (**11-4**), yield 74.0%. LCMS: m/z = 224.00 (M+H)⁺.

### Step 4: Synthesis of 2-chloro-N-(1-(2-chlorophenyl)-2-oxocyclohexyl)acetamide (11-5)

Under nitrogen protection, **11-4** (545 mg, 2.44 mmol) was dissolved in ultra-dry DCM (10 mL), added with ultra-dry triethylamine (494 mg, 4.88 mmol). Chloroacetyl chloride (289 mg, 2.56 mmol) was added dropwise at 0°C and stirred at room temperature for 1 hour. Water and EA were added for extraction. The organic layer was washed with saturated ammonium chloride solution, dried with anhydrous sodium sulfate, separated by flash silica gel column chromatography after concentration to obtain 393 mg white solid of 2-chloro-N-(1-(2-chlorophenyl)-2-oxocyclohexyl)acetamide (**11-5**), yield 53.7%. LCMS: m/z = 299.95 (M+H)⁺.

### Step 5: Synthesis of 4a-(2-chlorophenyl)hexahydro-2H-benzo[b][1,4]oxazine-3(4H)-one (11-6)

**11-5** (393 mg, 1.31 mmol) was dissolved in ultra-dry methanol (4 mL) at 0°C, added with sodium borohydride (50 mg, 1.31 mmol) and stirred at room temperature for 1 hour, dissolved in ultra-dry THF (4 mL) after rotary evaporating solvents, added with 60% sodium hydride (63 mg, 1.57 mmol) and stirred at room temperature for 12 hours. The reaction was quenched by adding dropwise methanol. Water and EA were added for extraction. The organic layer was dried with anhydrous sodium sulfate, separated by silica gel column chromatography after concentration to obtain 274 mg white solid of 4*a*-(2-chlorophenyl)hexahydro-2*H*-benzo[*b*][1,4]oxazine-3(4*H*)-one (**11-6**), yield 78.7%. LCMS: m/z= 266.00 (M+H)⁺.

### Step 6: Synthesis of 4a-(2-chlorophenyl)octahydro-2H-benzo[b][1,4]oxazine formate (Compound 11)

Under nitrogen protection, **11-6** (274 mg, 1.03 mmol) was dissolved in ultra-dry THF (5 mL), added with borane/dimethyl sulfide solution (2.58 mL, 5.15 mmol, 2.0 M THF solution) and heated at 70°C for 12 hours. The reaction was quenched by adding dropwise a small amount of methanol after being cooled, added with 2 M hydrochloric acid and stirred at room temperature for 1 hour. After the reaction liquid was neutralized with saturated sodium bicarbonate, EA was added for extraction. The organic layer was dried with anhydrous sodium sulfate, separated by HPLC after concentration to obtain 161 mg clear liquid of 4*a*-(2-chlorophenyl)octahydro-2*H-*benzo[*b*][1,4]oxazine formate (Compound **11**), yield 52.4%. LCMS: m/z = 252.00 (M+H)⁺.

¹H NMR (400 MHz, chloroform-d) δ 8.14 (dd, *J* = 7.8, 1.7 Hz, 1H), 7.38 (dd, *J* = 7.7, 1.6 Hz, 1H), 7.21 (dtd, *J* = 19.5, 7.4, 1.7 Hz, 2H), 6.07 (br, 1H), 4.01 - 3.84 (m, 2H), 3.78 (dd, *J* = 12.9, 4.5 Hz, 1H), 3.21 - 3.11 (m, 1H), 2.70 - 2.62 (m, 2H), 2.11 (qd, *J* = 12.8, 4.9 Hz, 1H), 1.97 - 1.85 (m, 1H), 1.80 - 1.66 (m, 1H), 1.56 - 1.33 (m, 3H), 1.00 - 0.76 (m, 1H).

### Synthesis of Compound 12

### Synthesis of 4a-(2-methylphenyl)octahydro-2H-benzo[b][1,4]oxazine formate (Compound 12)

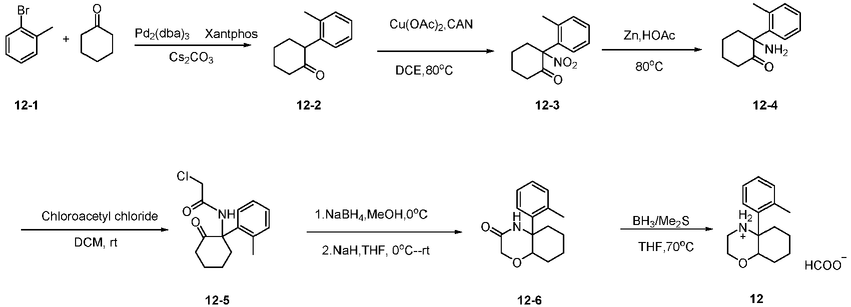

### Step 1: Synthesis of 2-(2-methylphenyl)cyclohexanone (12-2)

Under nitrogen protection, Pd₂(dba)₃ (266 mg, 0.29 mmol), Xantphos (336 mg, 0.58 mmol) and cesium carbonate (21 g, 64.31 mmol) were dissolved in ultra-dry 1,4-dioxane (30 mL), added with 2-bromotoluene (5 g, 29.23 mmol, **12-1**) and cyclohexanone (5.74 g, 58.46 mmol), heated at 100°C for 20 hours. The reaction liquid was extracted with EA and water after being cooled. The organic layer was dried with anhydrous sodium sulfate, separated by flash silica gel chromatography after concentration to obtain 1.72 g clear liquid of 2-(2-methylphenyl)cyclohexanone **(12-2)**, yield 31.3%. LCMS: m/z= 189.05 (M+H)⁺.

### Step 2: Synthesis of 2-nitro-2-(2-methylphenyl)cyclohexanone (12-3)

**12-2**(1.72 g, 9.14 mmol) was dissolved in DCE (30 mL), added with copper acetate (1.66 g, 9.14mmol) and cerium ammonium nitrate (12.5 g, 22.85 mmol), and heated at 80°C for 12 hours. The reaction liquid was filtered, washed with DCM, separated by silica gel column chromatography after concentration to obtain 864 mg yellow solid of 2-nitro-2-(2-methylphenyl)cyclohexanone (**12-3**) crude product, yield 40.5%. LCMS: m/z = 187.05 (M-NO₂)⁺.

### Step 3: Synthesis of 2-amino-2-(2-methylphenyl)cyclohexanone (12-4)

Under nitrogen protection, **12-3** (864 mg, 3.70 mmol) was dissolved in 16 mL methanol, added with 8 mL icy acetic acid. Zinc powder (1.2 g, 18.50 mmol) was added slowly and heated at 80°C for 12 hours. The reaction liquid was neutralized by adding dropwise saturated sodium bicarbonate solution after being cooled and adjusted to pH> 10 with a 2 M NaOH. The organic phase was dried with anhydrous sodium sulfate, separated by silica gel chromatography to obtain 537mg yellow liquid of 2-amino-2-(2-methylphenyl)cyclohexanone **(12-4)**, yield 71.4%. LCMS: m/z= 204.05 (M+H)⁺.

### Step 4: Synthesis of 2-chloro-N-(2-oxo-1-(2-methylphenyl)hexyl)acetamide (12-5)

Under nitrogen protection, **12-4** (537 mg, 2.64 mmol) was dissolved in ultra-dry DCM (10 mL), added with ultra-dry triethylamine (534 mg, 5.28 mmol). Chloroacetyl chloride (313 mg, 2.77 mmol) was added dropwise at 0°C, stirred at room temperature for 1 hour. Water and EA were added for extraction. The organic layer was washed with saturated ammonium chloride solution, dried with anhydrous sodium sulfate, separated by flash silica gel chromatography after concentration to obtain 408 mg 2-chloro-N-(2-oxo-1-(2-methylphenyl)hexyl)acetamide (**12-5**), yield : 55.2%. LCMS: m/z= 280.05 (M+H)⁺.

### Step 5: Synthesis of 4a-(2-methylphenyl)hexahydro-2H-benzo[b][1,4]oxazine-3(4H)-one (12-6)

**12-5** (408 mg, 1.46 mmol) was dissolved in ultra-dry methanol (5 mL) at 0°C, added with sodium borohydride (55 mg, 1.46 mmol) and stirred at room temperature for 1 hour, dissolved in ultra-dry THF (5 mL) after rotary evaporating solvents, added with 60% sodium hydride (70 mg, 1.75 mmol) and stirred at room temperature for 12 hours. The reaction was quenched by adding dropwise methanol and extracted with water and EA. The organic layer was dried with anhydrous sodium sulfate, separated by silica gel chromatography after concentration to obtain 217 mg white solid of 4*a*-(2-methylphenyl)hexahydro-2*H*-benzo[*b*][1,4]oxazine-3(4*H*)-one (**12-6**), yield 60.6%. LCMS: m/z= 246.05 (M+H)⁺.

### Step 6: Synthesis of 4a-(2-methylphenyl)octahydro-2H-benzo[b][1,4]oxazine formate (Compound 12)

Under nitrogen protection, **12-6** (217 mg, 0.88 mmol) was dissolved in ultra-dry THF (4 mL), added with a solution of borane-methyl sulfide in THF (2.21 mL, 4.42 mmol, 2.0 M) and heated at 70°C for 12 hours. The reaction was quenched by adding dropwise a small amount of methanol after being cooled, added with 2 M hydrochloric acid and stirred at room temperature for 1 hour. After the reaction liquid was neutralized with saturated sodium bicarbonate, EA was added for extraction. The organic layer was dried with anhydrous sodium sulfate, separated by HPLC after concentration to obtain 87.3 mg white solid of 4*a*-(2-methylphenyl)octahydro-2*H-*benzo[b][1,4]oxazine formate (Compound **12**), yield 35.6%. LCMS: m/z= 232.10 (M+H)⁺.

¹H NMR (400 MHz, chloroform-d) δ 8.38 (s, 1H), 8.25 (br, 1H), 8.21 (d, *J* = 7.1 Hz, 1H), 7.23 - 7.13 (m, 3H), 3.98 (s, 2H), 3.88 (dd, *J* = 12.5, 4.0 Hz, 1H), 2.95 - 2.77 (m, 3H), 2.56 (s, 3H), 2.15 (qd, *J* = 12.7, 4.8 Hz, 1H), 1.91 (d, *J* = 12.3 Hz, 1H), 1.79 - 1.59 (m, 2H), 1.57 - 1.41 (m, 2H), 0.96 (q, *J* = 14.8, 14.1 Hz, 1H).

### Synthesis of Compound 13

### Synthesis of 4a-(4-chlorophenyl)octahydro-2H-benzo[b][1,4]oxazine formate (Compound 13)

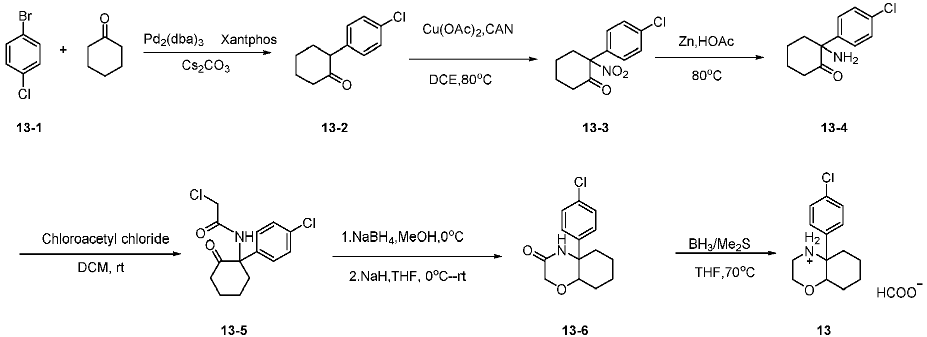

### Step 1: Synthesis of 2-(4-chlorophenyl)cyclohexanone (13-2)

Under nitrogen protection, Pd₂(dba)₃ (238 mg, 0.26 mmol), Xantphos (301 mg, 0.52 mmol) and cesium carbonate (18.72 g, 57.46 mmol) were dissolved in ultra-dry 1,4-dioxane (25 mL), added with 1-bromo-4-chlorobenzene (5 g, 26.12 mmol, **13-1**) and cyclohexanone (5.13 g, 52.23 mmol), heated at 100°C for 20 hours and extracted with EA and water after being cooled. The organic layer was dried with anhydrous sodium sulfate, separated by silica gel chromatography after concentration to obtain 2.96 g light yellow solid of 2-(4-chlorophenyl)cyclohexanone (**13-2**), yield 54.3%. LCMS: m/z= 209.00(M+H)⁺.

### Step 2: Synthesis of 2-(4-chlorophenyl)-2-nitrocyclohexanone (13-3)

**13-2** (2.96 g, 14.18 mmol) was dissolved in DCE (50 mL), added with copper acetate (2.58 g, 14.18 mmol) and cerium ammonium nitrate (23.3 g, 42.54 mmol), and heated at 80°C for 12 hours. The reaction liquid was filtered, washed with DCM, separated by silica gel column chromatography after concentration to obtain 1.41 g yellow solid of 2-(4-chlorophenyl)-2-nitrocyclohexanone (**13-3**), yield 39.2%. LCMS: m/z = 207.00(M-NO₂)⁺.

### Step 3: Synthesis of 2-amino-2-(4-chlorophenyl)cyclohexanone (13-4)

Under nitrogen protection, **13-3** (800 mg, 3.15 mmol) was dissolved in methanol (15 mL), added with icy acetic acid (7.5 mL). Zinc powder (1.02 g, 15.77 mmol) was added slowly and heated at 80°C for 12 hours. The reaction liquid was neutralized by adding dropwise saturated sodium bicarbonate solution after being cooled, adjusted to pH> 10 with a 2 M NaOH. The organic phase was dried with anhydrous sodium sulfate, separated by silica gel chromatography to obtain 477 mg yellow liquid of 2-amino-2-(4-chlorophenyl)cyclohexanone **(13-4)**, yield 67.7%. LCMS: m/z= 224.00 (M+H)⁺.

### Step 4: Synthesis of 2-chloro-N-(1-(4-chlorophenyl)-2-oxocyclohexyl)acetamide (13-5)

Under nitrogen protection, **13-4** (477 mg, 2.13 mmol) was dissolved in ultra-dry DCM (10 mL), added with ultra-dry triethylamine (431 mg, 4.26 mmol). Chloroacetyl chloride (253 mg, 2.24 mmol) was added dropwise at 0°C and stirred at room temperature for 1 hour. Water and EA were added for extraction. The organic layer was washed with saturated ammonium chloride solution, dried with anhydrous sodium sulfate, separated by flash silica gel chromatography after concentration to obtain 366 mg light yellow solid of 2-chloro-N-(1-(4-chlorophenyl)-2-oxocyclohexyl)acetamide **(13-5)**, yield 57.3%. LCMS: m/z= 300.00 (M+H)⁺.

### Step 5: Synthesis of 4a-(4-chlorophenyl)hexahydro-2H-benzo[b][1,4]oxazine-3(4H)-one (13-6)

**13-5** (366 mg, 1.22 mmol) was dissolved in ultra-dry methanol (5 mL) at 0°C, added with sodium borohydride (46 mg, 1.22 mmol) and stirred at room temperature for 1 hour, dissolved in ultra-dry THF (5 mL) after rotary evaporating solvents, added with 60% sodium hydride (58 mg, 1.46 mmol) and stirred at room temperature for 12 hours. The reaction was quenched by adding dropwise methanol and extracted with water and EA. The organic layer was dried with anhydrous sodium sulfate, separated by flash silica gel chromatography after concentration to obtain 144 mg clear liquid 4*a*-(4-chlorophenyl)hexahydro-2*H*-benzo[*b*][1,4]oxazine-3(4*H*)-one **(13-6)**, yield 44.4%. LCMS: m/z= 266.05(M+H)⁺.

### Step 6: Synthesis of 4a-(4-chlorophenyl)octahydro-2H-benzo[b][1,4]oxazine formate (Compound 13)

Under nitrogen protection, **13-6** (144 mg, 0.54 mmol) was dissolved in ultra-dry THF (2.5 mL), added with a solution of borane-methyl sulfide in THF (2.5 mL, 5.00 mmol, 2.0 M), heated at 70°C for 12 hours. The reaction was quenched by adding dropwise a small amount of methanol after being cooled, added with 2 M hydrochloric acid and stirred at room temperature for 1 hour. After the reaction liquid was neutralized with saturated sodium bicarbonate, EA was added for extraction. The organic layer was dried with anhydrous sodium sulfate, separated by HPLC after concentration, then lyophilized to obtain 56.8 mg white solid of 4*a*-(4-chlorophenyl)octahydro-2*H-*benzo[b][1,4]oxazine formate (Compound **13**), yield 35.4%. LCMS: m/z= 252.05 (M+H)⁺.

¹H NMR (400 MHz, chloroform-*d*) δ 8.82 (br, 2H), 8.44 (s, 1H), 7.86 (d, *J* = 8.0 Hz, 2H), 7.37 (d, *J* = 8.7 Hz, 2H), 4.19 - 3.87 (m, 3H), 2.96 - 2.73 (m, 2H), 2.49 (d, *J* = 12.8 Hz, 1H), 1.99 - 1.79 (m, 3H), 1.76 - 1.64 (m, 1H), 1.62 - 1.38 (m, 2H), 1.09 - 0.89 (m, 1H).

### Synthesis of Compound 14

### Synthesis of 4a-(4-methylphenyl)octahydro-2H-benzo[b][1,4]oxazine formate (Compound 14)

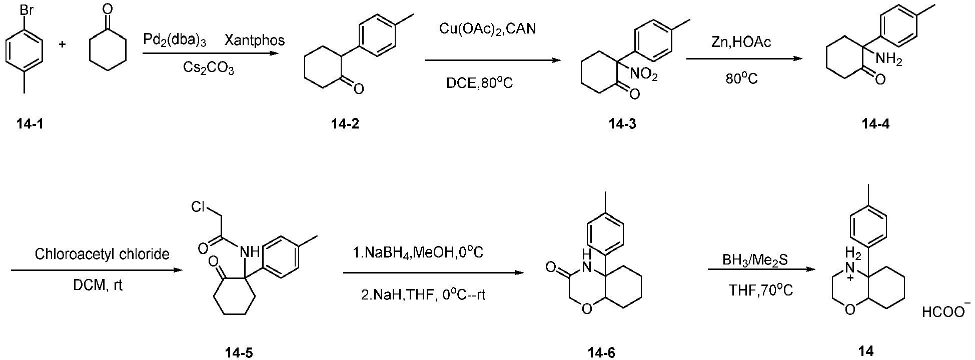

### Step 1: Synthesis of 2-(4-methylphenyl)cyclohexanone (14-2)

Under nitrogen protection, Pd₂(dba)₃ (266 mg, 0.29 mmol), Xantphos (336 mg, 0.58 mmol) and cesium carbonate (21 g, 64.31 mmol) were dissolved in ultra-dry 1,4-dioxane (30 mL), added with 4-bromotoluene (5 g, 29.23 mmol, **14-1)** and cyclohexanone (5.74 g, 58.46 mmol) and heated at 100°C for 20 hours. The reaction liquid was extracted with EA and water after being cooled. The organic layer was dried with anhydrous sodium sulfate, separated by silica gel column chromatography after concentration to obtain 3.15 g yellow clear liquid of 2-(4-methylphenyl)cyclohexanone **(14-2)**, yield 57.2%. LCMS: m/z= 189.05(M+H)⁺.

¹H NMR (400 MHz, chloroform-d) δ 7.14 (d, *J* = 7.9 Hz, 2H), 7.02 (d, *J* = 8.0 Hz, 2H), 3.56 (dd, *J* = 12.1, 5.4 Hz, 1H), 2.57 - 2.38 (m, 2H), 2.32 (s, 3H), 2.28 - 2.08 (m, 2H), 2.07 - 1.93 (m, 2H), 1.90 - 1.69 (m, 2H).

### Step 2: Synthesis of 2-nitro-2-(4-methylphenyl)cyclohexanone (14-3)

**14-2** (3.15 g, 16.73 mmol) was dissolved in DCE (50 mL), added with copper acetate (3.04 g, 16.73 mmol) and cerium ammonium nitrate (27.5 g, 50.19 mmol), and heated at 80°C for 12 hours. The reaction liquid was filtered, washed with DCM, separated by silica gel column chromatography after concentration to obtain 1.6 g yellow solid of 2-nitro-2-(4-methylphenyl)cyclohexanone (**14-3**), yield 41.0%. LCMS: m/z = 187.05 (M-NO₂)⁺.

### Step 3: Synthesis of 2-amino-2-(4-methylphenyl)cyclohexanone (14-4)

Under nitrogen protection, **14-3** (800 mg, 3.43 mmol) was dissolved in methanol (15 mL), added with icy acetic acid (7.5 mL). Zinc powder (1.11 g, 17.15 mmol) was added slowly and heated at 80°C for 12 hours. The reaction liquid was neutralized by adding dropwise saturated sodium bicarbonate solution after being cooled, adjusted to pH> 10 with a 2 M NaOH. The organic phase was dried with anhydrous sodium sulfate, separated by flash silica gel chromatography to obtain 495 mg yellow solid of 2-amino-2-(4-methylphenyl)cyclohexanone **(14-4)**, yield 71.0%. LCMS: m/z= 204.05 (M+H)⁺.

### Step 4: Synthesis of 2-chloro-N-(2-oxo-1-(4-methylphenyl)hexyl)acetamide (14-5)

Under nitrogen protection, **14-4** (495 mg, 2.43 mmol) was dissolved in ultra-dry DCM (10 mL), added with ultra-dry triethylamine (492 mg, 4.86 mmol). Chloroacetyl chloride (288 mg, 2.55 mmol) was added dropwise at 0°C and stirred at room temperature for 1 hour. Water and EA were added for extraction. The organic layer was washed with saturated ammonium chloride solution, dried with anhydrous sodium sulfate, separated by flash silica gel chromatography after concentration to obtain 481 mg white solid of 2-chloro-N-(2-oxo-1-(4-methylphenyl)hexyl)acetamide **(14-5)**, yield 70.7%. LCMS: m/z= 280.05 (M+H)⁺.

### Step 5: Synthesis of 4a-(4-methylphenyl)hexahydro-2H-benzo[b][1,4]oxazine-3(4H)-one (14-6)

**14-5** (481 mg, 1.72 mmol) was dissolved in ultra-dry methanol (5 mL) at 0°C, added with sodium borohydride (65 mg, 1.72 mmol) and stirred at room temperature for 1 hour, dissolved in ultra-dry THF (5 mL) after rotary evaporating solvents, added with 60% sodium hydride (82 mg, 2.06 mmol) and stirred at room temperature for 12 hours. The reaction was quenched by adding dropwise methanol and extracted with water and EA. The organic layer was dried with anhydrous sodium sulfate, separated by silica gel chromatography after concentration to obtain 244 mg white solid of 4*a*-(4-methylphenyl)hexahydro-2*H*-benzo[*b*][1,4]oxazine-3(4*H*)-one **(14-6)**, yield 57.8%. LCMS: m/z= 246.05 (M+H)⁺.

### Step 6: Synthesis of 4a-(4-methylphenyl)octahydro-2H-benzo[b][1,4]oxazine formate (Compound 14)

Under nitrogen protection, **14-6** (244 mg, 0.99 mmol) was dissolved in ultra-dry THF (5 mL), added with a solution of borane-methyl sulfide in THF (2.49 mL, 4.97 mmol, 2.0 M) and heated at 70°C for 12 hours. The reaction was quenched by adding dropwise a small amount of methanol after being cooled, added with 2 M hydrochloric acid and stirred at room temperature for 1 hour. After the reaction liquid was neutralized with saturated sodium bicarbonate, EA was added for extraction. The organic layer was dried with anhydrous sodium sulfate, separated by HPLC after concentration, then lyophilized to obtain 139 mg white solid of 4*a*-(4-methylphenyl)octahydro-2*H-*benzo[b][1,4]oxazine formate (Compound **14**), yield 50.7%. LCMS: m/z=232.15 (M+H)⁺.

¹H NMR (400 MHz, chloroform-*d*) δ 9.67 (br, 2H), 8.47 (s, 1H), 7.78 (d, *J* = 6.9 Hz, 2H), 7.18 (d, *J* = 7.8 Hz, 2H), 4.21 - 3.89 (m, 3H), 2.85 (s, 2H), 2.65 - 2.42 (m, 1H), 2.32 (s, 3H), 2.06 - 1.81 (m, 3H), 1.76 - 1.58 (m, 1H), 1.55 - 1.39 (m, 2H), 1.12 - 0.88 (m, 1H).

### Synthesis of Compound 15

### Synthesis of 4a-(3-(trifluoromethyl)phenyl)octahydro-2H-benzo[b][1,4]oxazine hydrochloride (Compound 15)

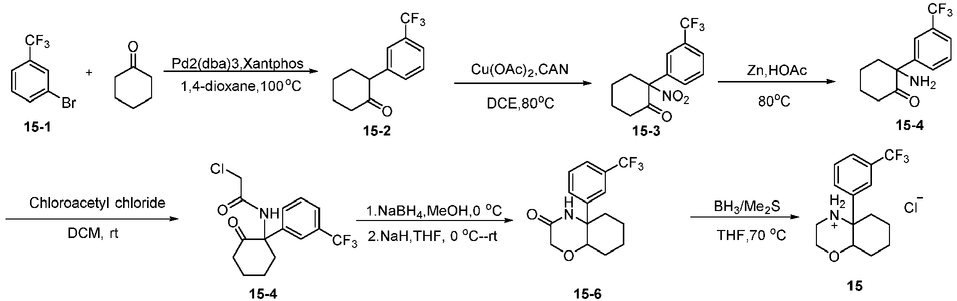

### Step 1: Synthesis of 2-(3-(trifluoromethyl)phenyl)hexyl-1-one(15-2)

Under nitrogen protection, Pd₂(dba)₃ (204 mg, 0.23 mmol), Xantphos (254 mg, 0.44 mmol) and cesium carbonate (16 g, 48.90 mmol) were dissolved in ultra-dry 1,4-dioxane (30 mL), added with m-trifluoromethyl bromobenzene (5 g, 28.60 mmol) and cyclohexanone (5.6 g, 57.10 mmol) and heated at 100°C for 20 hours. The reaction liquid was extracted with EA and water after being cooled. The organic layer was dried with anhydrous sodium sulfate, concentrated, separated and purified by silica gel chromatography to obtain 1.48 g yellow oil of 2-(3-(trifluoromethyl)phenyl)cyclohexanone **(15-2)**, yield 27 %. LCMS: m/z= 243.00 (M+H)⁺.

### Step 2: Synthesis of 2-(3-(trifluoromethyl)phenyl)-2-nitrocyclohexanone(15-3)

**15-2** (1.48 g, 6.11 mmol) was dissolved in DCE (10 mL), added with copper acetate (0.56 g, 3.05 mmol) and cerium ammonium nitrate (8.4 g, 15.30 mmol), and heated at 80°C for 12 hours. The reaction liquid was filtered, washed with EA, concentrated, separated and purified by silica gel chromatography to obtain 0.36 g yellow oil of 2-(3-(trifluoromethyl)phenyl)-2-nitrocyclohexanone **(15-3)**, yield 20 %.

### Step 3: Synthesis of 2-(3-(trifluoromethyl)phenyl)-2-aminocyclohexanone (15-4)

Under nitrogen protection, **3-3** (1.36 g, 4.73 mmol) was dissolved in acetic acid (10 mL), added with zinc powder (1.54 g, 24 mmol), and heated at 80°C for 12 hours. After being cooled, the reaction liquid was adjusted to pH> 10 with a 2 M solution of sodium hydroxide, extracted with ethyl acetate. Then the organic phase was dried with anhydrous sodium sulfate, concentrated, separated and purified by silica gel chromatography to obtain a colorless oil of 2-(3-(trifluoromethyl)phenyl)-2-aminocyclohexanone **(15-4)** 430 mg, yield 35 %. LCMS: m/z= 258.05 (M+H)⁺.

### Step 4: Synthesis of 2-chloro-N-(1-(3-(trifluoromethyl)phenyl)-2-oxocyclohexyl) acetamide (15-5)

Under nitrogen protection, **15-4** (430 mg, 1.67 mmol) was dissolved in ultra-dry DCM (5 mL), added with ultra-dry triethylamine (0.3 mL, 1.80 mmol). Chloroacetyl chloride (133 µL, 2.46 mmol) was added dropwise at 0°C and stirred at room temperature for 1 hour until the raw material disappeared by TLC analysis. The reaction liquid was concentrated directly, separated and purified by silica gel chromatography to obtain a white solid of 2-chloro-N-(1-(3-(trifluoromethyl)phenyl)-2-oxocyclohexyl)acetamide (**15-5**) 273 mg, yield 49 %. LCMS: m/z= 334.00 (M+H)⁺.

### Step 5: Synthesis of 4a-(3-(trifluoromethyl)phenyl)hexahydro-2H-benzo[b][1,4]oxazine-3(4H)-one (15-6)

**15-5** (273 mg, 0.82 mmol) was put in a 25 mL round bottom flask and added with 3 mL methanol for dissolving. Sodium borohydride (31 mg, 0.82 mmol) was added slowly at 0°C. The reaction liquid was dried by a rotary evaporator after stirring for 30 mins. After 3 mL THF was added for dissolving, 60%sodium hydride (40 mg, 0.98 mmol) was added under ice bath and reacted for 10 hours. The reaction liquid was quenched with saturated saline, extracted with ethyl acetate, separated and purified by silica gel chromatography to obtain a white solid of 4*a*-(3-(trifluoromethyl)phenyl)hexahydro-2*H*-benzo[*b*][1,4]oxazine-3(4*H*)-one (**15-6**) 204 mg, yield 83 %. LCMS: m/z= 300.05 (M+H)⁺.

### Step 6: Synthesis of 4a-(3-(trifluoromethyl)phenyl)octahydro-2H-benzo[b][1,4]oxazine hydrochloride (Compound 15)

Under nitrogen protection, **15-6** (204 mg, 0.68 mmol) was dissolved in ultra-dry THF (5 mL), added with a solution of borane-methyl sulfide in THF (1.7 mL, 3.40 mmol, 2.0 M) and refluxed at 70°C for 12 hours. The reaction was quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate, separated and purified by HPLC after concentration, added with 1 mL diluted hydrochloric acid after concentration and lyophilized to obtain a white solid of 4*a*-(3-(trifluoromethyl)phenyl)octahydro-2*H-*benzo[b][1,4]oxazine hydrochloride (Compound **15**) 150 mg, yield 68 %. LCMS: m/z= 286.05 (M+H)⁺.

¹H NMR (400 MHz, chloroform-d) δ 10.56 (s, 1H), 10.04 (s, 1H), 8.45 (s, 1H), 8.30 (d, *J* = 7.3 Hz, 1H), 7.69 - 7.59 (m, 2H), 4.39 (t, *J* = 11.6 Hz, 1H), 4.24 (dd, *J* = 12.0, 4.6 Hz, 1H), 4.09 (dd, *J* = 12.5, 3.2 Hz, 1H), 3.09 - 2.99 (m, 1H), 2.95 - 2.77 (m, 2H), 2.37 - 2.24 (m, 1H), 2.07 - 1.89 (m, 2H), 1.81 - 1.71 (m, 1H), 1.65 - 1.52 (m, 2H), 1.04 - 0.87 (m, 1H).

### Synthesis of Compound 16

### Synthesis of 8-methyl-4a-phenyloctahydro-2H-benzo[b][1,4]oxazine formate (Compound 16)

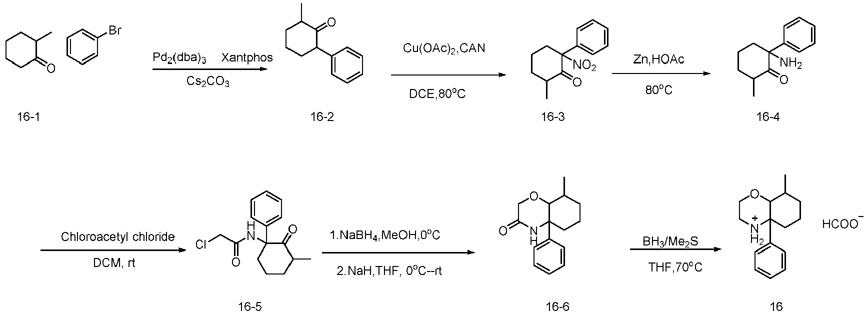

### Step 1: Synthesis of 2-methyl-6-phenylcyclohexanone (16-2):

Under nitrogen protection, Pd₂(dba)₃ (293 mg, 0.32 mmol), Xantphos (367 mg, 0.64 mmol), cesium carbonate (22.77 g, 70.07 mmol) were dissolved in 30 mL ultra-dry dioxane, added with bromobenzene (5 g, 31.85 mmol), **16-1** (7.13 g, 63.70 mmol) and stirred at 80°C for 20 hours. After the reaction liquid was cooled to room temperature, it was filtered with diatomite, extracted with EA, dried with anhydrous sodium sulfate, separated by flash silica gel chromatography to obtain 2.14 g light yellow liquid of 2-methyl-6-phenylcyclohexanone **(16-2)**, yield 35.8%. LCMS: m/z=189.00(M+H)⁺.

### Step 2: Synthesis of 6-methyl-2-nitro-2-phenylcyclohexanone (16-3):

Under nitrogen protection, cerium ammonium nitrate (18.69 g, 34.10 mmol), copper acetate (2.06 g, 11.36 mmol), **16-2** (2.14 g, 11.36 mmol) were dissolved in 1,2-dichloroethane (40 mL) and stirred at 80°C for 12 hours. After being cooled to room temperature, the reaction liquid was diluted with DCM, filtered with diatomite, washed with DCM and filtered to remove residue, separated by flash silica chromatography to obtain 1.21 g yellow liquid of 6-methyl-2-nitro-2-phenylcyclohexanone **(16-3),** yield 45.8%.

### Step 3: Synthesis of 6-methyl-2-amino-2-phenylcyclohexanone (16-4):

Under nitrogen protection, **16-3** (556 mg, 2.39 mmol) was dissolved in 10 mL methanol and added with 5 mL icy acetic acid. 775 mg zinc powder was added slowly and stirred at 80°C for 12 hours. The reaction liquid was adjusted to pH of 10 by adding 2 M NaOH, extracted with EA, dried with anhydrous sodium sulfate, separated and purified by flash silica gel column chromatography to obtain 296 mg light yellow liquid of 6-methyl-2-amino-2-phenylcyclohexanone **(16-4)**, yield 61%. LCMS: m/z=204.05(M+H)⁺.

### Step 4: Synthesis of 2-chloro-N-(3-methyl-2-oxo-1-phenylhexyl)acetamide (16-5):

Under nitrogen protection, **16-4** (296 mg, 1.48 mmol) was dissolved in 8 mL ultra-dry DCM, added with triethylamine (179 mg, 1.78 mmol), cooled to 0°C, added dropwise with chloroacetyl chloride (167 mg, 1.48 mmol), stirred at room temperature for 1 hour, extracted with EA. The organic phase was dried with anhydrous sodium sulfate, separated and purified by flash silica gel column chromatography after concentration to obtain 276 mg light yellow solid of 2-chloro-N-(3-methyl-2-oxo-1-phenylhexyl)acetamide **(16-5)**, yield 67%. LCMS: m/z=280.05(M+H)⁺.

### Step 5: Synthesis of 8-methyl-4a-phenylhexahydro-2H-benzo[b][1,4]oxazine-3(4H)-one (16-6):

Under nitrogen protection, **16-5** (276 mg, 0.99 mmol) was dissolved in 7 mL ultra-dry methanol, cooled to 0°C, added with sodium borohydride (37 mg, 0.99 mmol), warmed to room temperature and stirred for 1 hour with rotary evaporating solvents under reduced pressure, added with 7 mL ultra-dry THF for dissolving, cooled to 0°C, added with 60% sodium hydride (48 mg, 1.19 mmol), warmed to room temperature and stirred for 12 hours. The reaction was quenched by adding 1 N HCl, extracted with DCM. The organic phase was dried with anhydrous sodium sulfate, separated and purified by combi-flash silica gel column chromatography after concentration to obtain a white solid of 8-methyl-4*a*-phenylhexahydro-2*H*-benzo[*b*][1,4]oxazine-3(4*H*)-one **(16-6)**, yield 71.5%. LCMS: m/z=246.05(M+H)⁺.

### Step 6: Synthesis of 8-methyl-4a-phenyloctahydro-2H-benzo[b][1,4]oxazine formate (16):

Under nitrogen protection, **16-6** (170 mg, 0.69 mmol) was dissolved in 7 mL ultra-dry THF, added with borane/dimethyl sulfide, and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved with a small amount of MeOH, separated and purified by HPLC, then lyophilized to obtain 136 mg white powder of 8-methyl-4*a-*phenyloctahydro-2*H*-benzo[*b*][1,4]oxazine formate (**16**), yield 85.5%. LCMS: m/z=232.10(M+H)⁺.

¹H NMR (400 MHz, chloroform-d) δ 9.43 (s, 2H), 8.48 (s, 1H), 7.93 (d, J = 7.7 Hz, 2H), 7.46 - 7.30 (m, 3H), 4.13 - 4.01 (m, 2H), 3.63 (d, J = 11.2 Hz, 1H), 2.93 - 2.80 (m, 2H), 2.60 (dq, J = 13.5, 2.8 Hz, 1H), 2.18 (tdd, J = 11.2, 6.4, 4.6 Hz, 1H), 1.98 (td, J = 13.3, 3.4 Hz, 1H), 1.68 (dtd, J = 12.8, 4.0, 2.0 Hz, 1H), 1.50 (dt, J = 13.6, 3.4 Hz, 1H), 1.21 (tdd, J = 13.1, 11.4, 3.9 Hz, 1H), 1.13 - 0.98 (m, 4H).

### Synthesis of Compound 17

### Synthesis of 5-methyl-4a-phenyloctahydro-2H-benzo[b][1,4]oxazine formate (Compound 17)

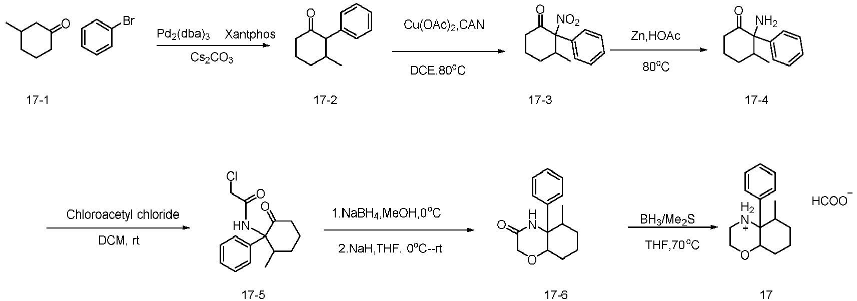

### Step 1: Synthesis of 3-methyl-2-phenylcyclohexanone(17-2):

Under nitrogen protection, Pd₂(dba)₃ (293 mg, 0.32 mmol), Xantphos (367 mg, 0.64 mmol), cesium carbonate (22.77 g, 70.07 mmol) were dissolved in ultra-dry dioxane 30 mL, added with bromobenzene (5 g, 31.85 mmol), **17-1** (7.13 g, 63.70 mmol) and stirred at 80°C for 20 hours. After being cooled to room temperature, the reaction liquid was diluted with EA, filtered with diatomite, extracted with water, dried with anhydrous sodium sulfate, separated by flash silica chromatography to obtain 2.42 g light yellow liquid of 3-methyl-2-phenylcyclohexanone (**17-2**), yield 40.5%. LCMS: m/z=189.05(M+H)⁺.

### Step 2: Synthesis of 3-methyl-2-nitro-2-phenylcyclohexanone (17-3):

Under nitrogen protection, cerium ammonium nitrate (21.16 g, 38.61 mmol), copper acetate (2.33 g, 12.87 mmol), **17-2** (2.42 g, 12.87 mmol) was dissolved in 1,2-dichloroethane (50 mL) and stirred at 80°C for 12 hours. After being cooled to room temperature, the reaction liquid was diluted with DCM, filtered with diatomite, washed with DCM and filtered to remove residue, separated by flash silica chromatography to obtain 911 mg light yellow liquid of 3-methyl-2-nitro-2-phenylcyclohexanone (**17-3**), yield 30.5%.

### Step 3: Synthesis of 3-methyl-2-amino-2-phenylcyclohexanone(17-4):

Under nitrogen protection, **17-3** (471 mg, 2.02 mmol) was dissolved in 10 mL methanol and added with 5 mL icy acetic acid. 656 mg zinc powder was added slowly and stirred at 80°C for 12 hours. The reaction liquid was adjusted to pH of 10 by adding 2 M NaOH, extracted with EA, dried with anhydrous sodium sulfate, separated and purified by flash silica gel column chromatography to obtain 223 mg yellow liquid of 3-methyl-2-amino-2-phenylcyclohexanone (**17-4**), yield 55.0%. LCMS: m/z=204.05(M+H)⁺.

### Step 4: Synthesis of 2-chloro-N-(2-methyl-6-oxo-1-phenylhexyl)acetamide (17-5):

Under nitrogen protection, **17-4** (210 mg, 1.03 mmol) was dissolved in 5 mL ultra-dry DCM, added with triethylamine (125 mg, 1.24 mmol), cooled to 0°C, added dropwise with chloroacetyl chloride (117 mg, 1.03 mmol), stirred at room temperature for 1 hour, diluted with DCM, stirred with silica gel, separated and purified by flash silica chromatography to obtain 177 mg white solid of 2-chloro-N-(2-methyl-6-oxo-1-phenylhexyl)acetamide (**17-5**), yield 61.7%. LCMS: m/z=280.05(M+H)⁺.

### Step 5: Synthesis of 5-methyl-4a-phenylhexahydro-2H-benzo[b][1,4]oxazine-3(4H)-one (17-6):

Under nitrogen protection, **17-5** (177 mg, 0.63 mmol) was dissolved in 5 mL ultra-dry methanol, cooled to 0°C, added with sodium borohydride (24 mg, 0.63 mmol), warmed to room temperature and stirred for 1 hour with rotary evaporating solvents under reduced pressure, added with 5 mL ultra-dry THF for dissolving, cooled to 0°C, added with 60% sodium hydride (31 mg, 0.76 mmol), warmed to room temperature and stirred for 12 hours. The reaction was quenched by adding 1 N HCl and extracted with DCM. The organic phase was dried with anhydrous sodium sulfate, separated and purified by flash silica gel column chromatography after concentration to obtain 100 mg white solid of 5-methyl-4*a*-phenylhexahydro-2*H*-benzo[*b*][1,4]oxazine-3(4*H*)-one (**17-6**), yield 64.9%. LCMS: m/z=246.05(M+H)⁺.

### Step 6: Synthesis of 5-methyl-4a-phenyloctahydro-2H-benzo[b][1,4]oxazine formate (17):

Under nitrogen protection, **17-6** (100 mg, 0.41 mmol) was dissolved in 3 mL ultra-dry THF, added with borane/dimethyl sulfide, and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then lyophilized to obtain 40 mg white powder of 5-methyl-4*a-*phenyloctahydro-2*H*-benzo[*b*][1,4]oxazine formate (**17**), yield 42.6%. LCMS: m/z=232.10(M+H)⁺.

¹H NMR (400 MHz, chloroform-d) δ 8.99 (s, 2H), 7.94 (d, J = 7.2 Hz, 2H), 7.39 (dt, J = 28.5, 7.3 Hz, 3H), 4.28 (dd, J = 12.9, 4.1 Hz, 1H), 4.15 (dt, J = 12.2, 8.0 Hz, 1H), 4.03 (d, J = 11.3 Hz, 1H), 2.89 (d, J = 5.7 Hz, 2H), 2.39 (d, J = 13.5 Hz, 1H), 2.20 (td, J = 12.9, 5.7 Hz, 2H), 2.06 (s, 1H), 1.71 (dd, J = 13.2, 3.6 Hz, 1H), 1.35 - 1.26 (m, 2H), 1.15 (d, J = 7.3 Hz, 3H).

### Synthesis of Compound 18

### Synthesis of 7-methyl-4a-phenyloctahydro-2H-benzo[b][1,4]oxazine formate (Compound 18)

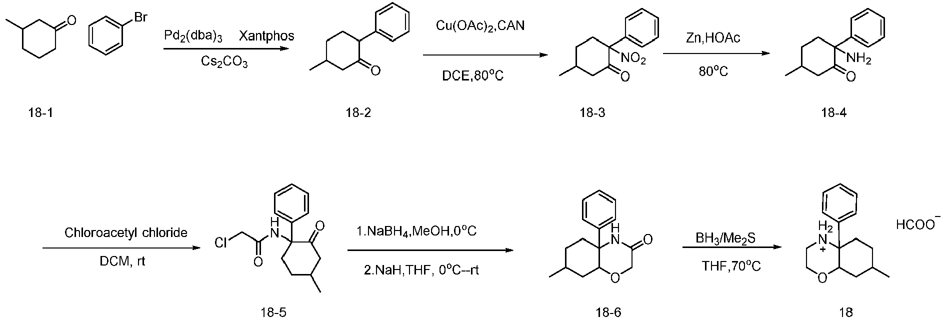

### Step 1: Synthesis of 5-methyl-6-phenylcyclohexanone (18-2):

Under nitrogen protection, Pd₂(dba)₃ (293 mg, 0.32 mmol), Xantphos (367 mg, 0.635 mmol), cesium carbonate (22.77 g, 70.07 mmol) were dissolved in ultra-dry dioxane 30 mL, added with bromobenzene (5 g, 31.85 mmol), **18-1**(7.13 g, 63.70 mmol) and stirred at 80°C for 20 hours. After being cooled to room temperature, the reaction liquid was diluted with EA, filtered with diatomite, extracted with water, dried with anhydrous sodium sulfate, separated by flash silica chromatography to obtain 2.42 g light yellow liquid of 5-methyl-6-phenylcyclohexanone (**18-2**), yield 40.5%. LCMS: m/z=189.05(M+H)⁺.

### Step 2: Synthesis of 5-methyl-2-nitro-2-phenylcyclohexanone (18-3):

Under nitrogen protection, cerium ammonium nitrate (21.16 g, 38.61 mmol), copper acetate (2.33 g, 12.87 mmol), **18-2** (2.42 g, 12.87 mmol) were dissolved in 1,2-dichloroethane (50 mL) and stirred at 80°C for 12 hours. After being cooled to room temperature, the reaction liquid was diluted with DCM, filtered with diatomite, washed with DCM, separated by silica gel chromatography to obtain 911 mg light yellow liquid of 5-methyl-2-nitro-2-phenylcyclohexanone (**18-3**), yield 30.5%.

### Step 3: Synthesis of 5-methyl-2-amino-2-phenylcyclohexanone (18-4):

Under nitrogen protection, **18-3** (440 mg, 1.89 mmol) was dissolved in 8 mL methanol and added with 4 mL icy acetic acid. 614 mg zinc powder was added slowly and stirred at 80°C for 12 hours. The reaction liquid was adjusted to pH of 10 by adding 2 M NaOH, extracted with EA, dried with anhydrous sodium sulfate, separated and purified by flash silica gel column chromatography to obtain 229 mg yellow liquid of 5-methyl-2-amino-2-phenylcyclohexanone (**18-4**), yield 59.8%. LCMS: m/z=204.05(M+H)⁺.

### Step 4: Synthesis of 2-chloro-N-(4-methyl-2-oxo-1-phenylhexyl)acetamide (18-5):

Under nitrogen protection, **18-4** (210 mg, 1.03 mmol) was dissolved in 5 mL ultra-dry DCM, added with triethylamine (125 mg, 1.24 mmol), cooled to 0°C, added dropwise with chloroacetyl chloride (117 mg, 1.03 mmol), stirred at room temperature for 1 hour, diluted with DCM, separated and purified by flash silica chromatography after concentration to obtain 224 mg light yellow liquid of 2-chloro-N-(4-methyl-2-oxo-1-phenylhexyl)acetamide **(18-5)**, yield 78%. LCMS: m/z=280.05(M+H)⁺.

### Step 5: Synthesis of 7-methyl-4a-phenylhexahydro-2H-benzo[b][1,4]oxazine-3(4H)-one (18-6):

Under nitrogen protection, **18-5** (224 mg, 0.80 mmol) was dissolved in 5 mL ultra-dry methanol, cooled to 0°C, added with sodium borohydride (30 mg, 0.80 mmol), warmed to room temperature and stirred for 1 hour with rotary evaporating solvents under reduced pressure, added with 5 mL ultra-dry THF for dissolving, cooled to 0°C, added with 60% sodium hydride (38 mg, 0.96 mmol), warmed to room temperature and stirred for 12 hours. The reaction was quenched by adding 1 N HCl and extracted with DCM. The organic phase was dried with anhydrous sodium sulfate, separated and purified by silica gel chromatography after concentration to obtain 111 mg white solid of 7-methyl-4*a*-phenylhexahydro-2*H*-benzo[*b*][1,4]oxazine-3(4*H*)-one (**18-6**), yield 56.6%. LCMS: m/z=246.05(M+H)⁺.

### Step 6: Synthesis of 7-methyl-4a-phenyloctahydro-2H-benzo[b][1,4]oxazine formate (18):

Under nitrogen protection, **18-6** (111 mg, 0.45 mmol) was dissolved in 3 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then lyophilized to obtain 73 mg colorless viscous liquid of 7-methyl-4*a*-phenyloctahydro-2*H*-benzo[*b*][1,4]oxazine formate (**18**), yield 70.2%. LCMS: m/z=232.10(M+H)⁺.

¹H NMR (400 MHz, chloroform-d) δ 8.46 (d, J = 20.4 Hz, 4H), 7.92 (d, J = 7.2 Hz, 2H), 7.39 (dt, J = 27.0, 7.1 Hz, 3H), 4.19 - 3.96 (m, 3H), 2.99 - 2.81 (m, 2H), 2.65 - 2.53 (m, 1H), 2.06 - 1.89 (m, 2H), 1.82 - 1.64 (m, 2H), 1.52 (d, J = 12.6 Hz, 1H), 0.84 (d, J = 6.2 Hz, 3H).

### Synthesis of Compound 19

### Synthesis of 4-methyl-4a-phenyloctahydro-2H-benzo[b][1,4]oxazine hydrochloride (19)

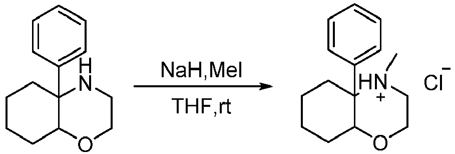

Compound 1 (30 mg, 0.14 mmol) was put in a 25 mL round bottom flask, added with 2 mL THF for dissolving, added with 60% sodium hydride (11 mg, 0.28 mmol), methyl iodide (17 µL, 0.28 mmol), stirred at room temperature for 2 hours, analyzed by LCMS, dried by a rotary evaporator, separated and purified by HPLC to obtain 23 mg white powder of 4-methyl-4a-phenyloctahydro-2*H*-benzo[*b*][1,4]oxazine hydrochloride (Compound **19**). yield 62 %. LCMS: m/z= 232.10 (M+H)⁺.

¹H NMR (400 MHz, chloroform-d) δ 12.56 (s, 1H), 7.95 (s, 2H), 7.48 (d, *J* = 5.6 Hz, 3H), 4.66 (t, *J* = 10.0 Hz, 1H), 4.49 - 4.39 (m, 1H), 4.09 (d, *J* = 12.3 Hz, 1H), 3.04 (s, 2H), 2.78 (d, *J* = 12.5 Hz, 1H), 2.41 (d, *J* = 26.4 Hz, 4H), 1.89 (d, *J* = 9.8 Hz, 1H), 1.77 - 1.50 (m, 4H), 1.22 - 1.06 (m, 1H).

### Synthesis of Compound 20

### Synthesis of 3,3-dimethyl-5a-phenyldecahydrobenzo[b] [1,4]olanzapine hydrochloride (Compound 20)

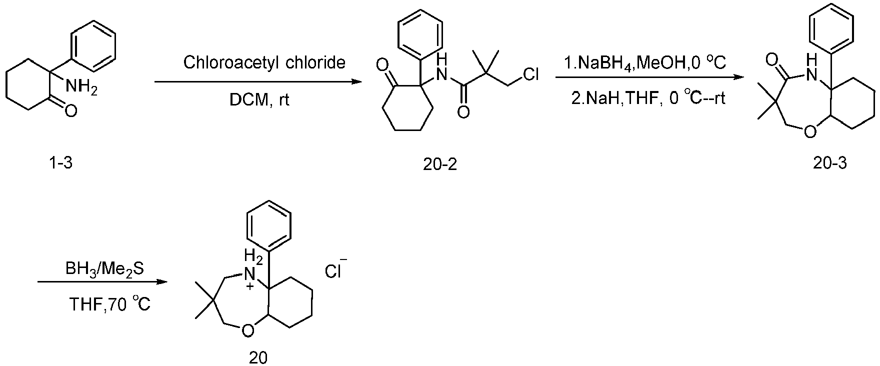

### Step 1: Synthesis of 3-chloro-2,2-dimethyl-N-(2-oxo-1-phenylhexyl)propionamide (20-2)

Under nitrogen protection, **1-3** (101 mg, 0.53 mmol) was dissolved in ultra-dry DCM (2 mL) and added with ultra-dry triethylamine (81 µL, 0.58 mmol). Chloroacetyl chloride (69 µL, 0.53 mmol) was added dropwise at 0°C and stirred at room temperature for 1 hour, until the raw material disappeared by TLC analysis. The reaction liquid was concentrated directly, separated and purified by silica gel chromatography to obtain white solid of 3-chloro-2,2-dimethyl-N-(2-oxo-1-phenylhexyl)propionamide **(20-2)** 154 mg, yield 94 %. LCMS: m/z= 308.10 (M+H)⁺.

### Step 2: Synthesis of 3,3-dimethyl-5a-phenyloctahydrobenzo[b][1,4]oxazepin-4(5H)-one (20-3)

**20-2** (103 mg, 0.34 mmol) was put in a 25 mL round bottom flask. After 2 mL methanol was added for dissolving, sodium borohydride (13 mg, 0.34 mmol) was added slowly at 0°C. The reaction liquid was dried by a rotary evaporator after stirring for 30 mins. After 3 mL THF was added for dissolving, 60% sodium hydride (16 mg, 0.40 mmol) was added under ice bath and reacted for 10 hours. The reaction liquid was quenched with saturated saline, extracted with ethyl acetate. The organic phase was concentrated, separated and purified by silica gel chromatography to obtain a white solid of 3,3-dimethyl-5*a*-phenyloctahydrobenzo[*b*][1,4]oxazepin-4(5*H*)-one (**20-3**) 58 mg, yield 63 %. LCMS: m/z= 274.15 (M+H)⁺.

### Step 6: Synthesis of 3,3-dimethyl-5a-phenyldecahydrobenzo[b][1,4]olanzapine hydrochloride (Compound 20)

Under nitrogen protection, **20-3** (58 mg, 0.21 mmol) was dissolved in ultra-dry THF (2 mL), added with a solution of borane-methyl sulfide in THF (1 mL, 2.00 mmol, 2.0 M), refluxed at 70°C for 12 hours, and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate, separated and purified by HPLC after concentration, added with 1 mL diluted hydrochloric acid after concentration, lyophilized to obtain a white solid of 3,3-dimethyl-5*a*-phenyldecahydrobenzo[*b*] [1,4]olanzapine hydrochloride (Compound **20**) 40 mg, yield 63 %. LCMS: m/z= 260.10 (M+H)⁺. ¹H NMR (400 MHz, chloroform-*d*) δ 11.64 (s, 1H), 8.04 (d, *J* = 7.1 Hz, 2H), 7.54 - 7.45 (m, 3H), 4.85 - 4.74 (m, 1H), 4.54 (s, 1H), 4.18 - 4.09 (m, 1H), 3.86 - 3.72 (m, 2H), 3.55 (t, *J* = 7.3 Hz, 1H), 2.41 (d, *J* = 12.9 Hz, 1H), 2.06 - 1.87 (m, 2H), 1.68 - 1.53 (m, 3H), 1.44 (s, 4H), 1.23 - 1.07 (m, 1H), 0.51 (s, 3H).

### Synthesis of Compound 21

### Synthesis of 4a-(3-methoxyphenyl)octahydro-2H-benzo[b][1,4]oxazine hydrochloride (Compound 21)

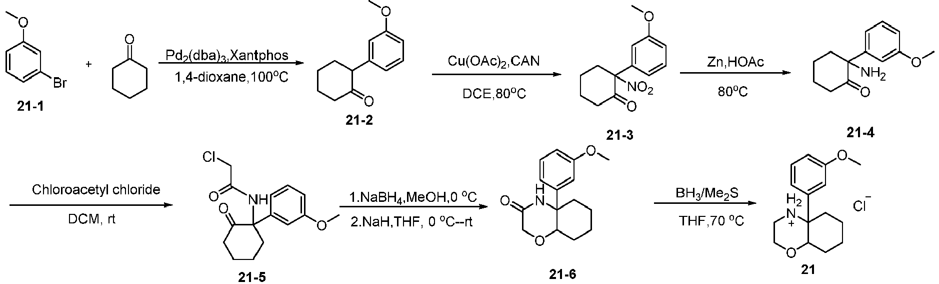

### Step 1: Synthesis of 2-(3-methoxyphenyl)cyclohexanone (21-2)

Under nitrogen protection, Pd₂(dba)₃ (245 mg, 0.27 mmol), Xantphos (301 mg, 0.54 mmol) and cesium carbonate (19 g, 58.80 mmol) were dissolved in ultra-dry 1,4-dioxane (30 mL), added with m-methoxybromobenzene (5 g, 26.73 mmol) and cyclohexanone (5.25 g, 53.46 mmol), heated at 100°C for 20 hours. The reaction liquid was extracted with EA and water after being cooled. The organic layer was dried with anhydrous sodium sulfate, separated by flash silica gel chromatography after concentration to obtain 2.55 g yellow clear liquid of 2-(3-methoxyphenyl)cyclohexanone **(21-2)**, yield 46.7 %. LCMS: m/z= 205.05 (M+H)⁺.

### Step 2: Synthesis of 2-(3-methoxyphenyl)-2-nitrocyclohexanone (21-3)

**2-2** (2.55 g, 12.48 mmol) was dissolved in DCE (25 mL), added with copper acetate (1.13 g, 6.24 mmol) and cerium ammonium nitrate (17 g, 31.20 mmol), and heated at 80°C for 12 hours. The reaction liquid was filtered, washed with DCM, dried with a rotary evaporator, then separated by silica gel chromatography to obtain 1.4 g yellow oil of 2-(3-methoxyphenyl)-2-nitrocyclohexanone **(21-3)**, yield 45 %.

### Step 3: Synthesis of 2-(3-methoxyphenyl)-2-aminocyclohexanone (21-4)

Under nitrogen protection, **21-3** (1.4 g, 5.62 mmol) was dissolved in acetic acid (12 mL), added with zinc powder (2.2 g, 33.70 mmol), and heated at 80°C for 12 hours. After being cooled, the reaction liquid was adjusted to pH> 10 with a 2 M solution of sodium hydroxide, extracted with ethyl acetate. Then the organic phase was dried with anhydrous sodium sulfate, concentrated, separated and purified by silica gel chromatography to obtain a colorless oil of 2-(3-methoxyphenyl)-2-aminocyclohexanone **(21-4)** 654 mg, yield 53 %. LCMS: m/z= 220.05 (M+H)⁺.

### Step 4: Synthesis of 2-chloro-N-(3-oxo-1-phenylhexyl)acetamide (21-5)

Under nitrogen protection, **21-4** (654 mg, 2.98 mmol) was dissolved in ultra-dry DCM (10 mL), added with ultra-dry triethylamine (0.5 mL, 3.30 mmol). Chloroacetyl chloride (237 µL, 2.98 mmol) was added dropwise at 0°C and stirred at room temperature for 1 hour. The reaction liquid was concentrated directly, separated and purified by silica gel chromatography to obtain a white solid of 2-chloro-N-(3-oxo-1-phenylhexyl)acetamide **(21-5)** 758 mg, yield 86 %. LCMS: m/z= 296 .1(M+H)⁺.

### Step 5: Synthesis of 4a-(3-methoxyphenyl)hexahydro-2H-benzo[b][1,4]oxazine-3(4H)-one (21-6)

**21-5** (693 mg, 2.34 mmol) was put in a 25 mL round bottom flask. After 5 mL methanol was added for dissolving, sodium borohydride (89 mg, 2.34 mmol) was added slowly at 0°C. The reaction liquid was dried by a rotary evaporator after stirring for 30 mins. After 3 mL THF was added for dissolving, 60% sodium hydride (113 mg, 2.82 mmol) was added under ice bath and reacted for 10 hours. The reaction liquid was quenched with saturated saline, extracted with ethyl acetate. The organic phase was concentrated, separated and purified by silica gel chromatography to obtain a white solid of 4*a*-(3-methoxyphenyl)hexahydro-2*H*-benzo[*b*][1,4]oxazine-3(4*H*)-one (**21-6**) 448 mg, yield 73 %. LCMS: m/z= 262.2 (M+H)⁺.

### Step 6: Synthesis of 4a-(3-methoxyphenyl)octahydro-2H-benzo[b][1,4]oxazine hydrochloride (Compound 21)

Under nitrogen protection, **21-6** (448 mg, 1.71 mmol) was dissolved in ultra-dry THF (5 mL), added with a solution of borane-methyl sulfide in THF (4.3 mL, 8.60 mmol, 2.0 M), refluxed at 70°C for 12 hours and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate, separated and purified by HPLC after concentration, added with 1 mL diluted hydrochloric acid after concentration, lyophilized to obtain a white solid of 4*a*-(3-methoxyphenyl)octahydro-2*H*-benzo[*b*][1,4]oxazine hydrochloride (Compound **21**) 360 mg, yield 74 %.

LCMS: m/z= 248 .2(M+H)⁺.

¹H NMR (400 MHz, chloroform-d) δ 10.37 (s, 1H), 9.69 (s, 1H), 7.65 (d, *J* = 7.2 Hz, 2H), 7.36 (t, *J* = 8.0 Hz, 1H), 6.92 (dd, *J* = 8.3, 2.2 Hz, 1H), 4.46 - 4.35 (m, 1H), 4.22 (dd, *J* = 12.2, 4.4 Hz, 1H), 4.05 (dd, *J* = 12.6, 3.4 Hz, 1H), 3.88 (s, 3H), 3.11 - 2.91 (m, 2H), 2.85 - 2.74 (m, 1H), 2.25 (td, *J* = 13.4, 3.0 Hz, 1H), 2.10 (qd, *J* = 12.6, 4.5 Hz, 1H), 2.02 - 1.93 (m, 1H), 1.80 - 1.69 (m, 1H), 1.58 (dp, *J* = 14.0, 4.3 Hz, 2H), 1.04 (dtd, *J* = 15.3, 11.8, 11.4, 3.8 Hz, 1H).

### Synthesis of Compound 22

### Synthesis of 4a-(3-(trifluoromethoxy)phenyl)octahydro-2H-benzo[b][1,4]oxazine hydrochloride (Compound 22)

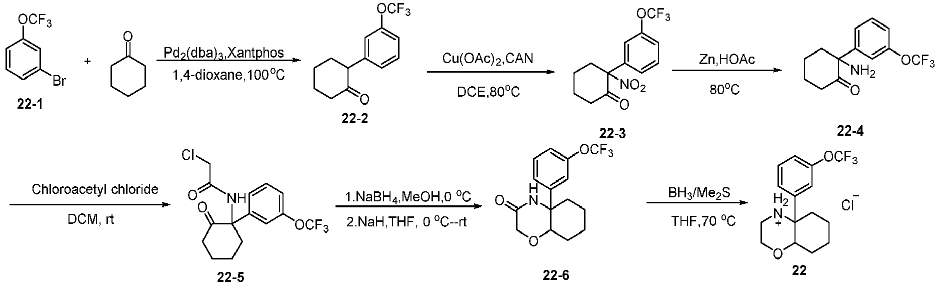

### Step 1: Synthesis of 2-(3-(trifluoromethoxy)phenyl)hexyl-1-one (22-2)

Under nitrogen protection, Pd₂(dba)₃ (245 mg, 0.27 mmol), Xantphos (301 mg, 0.54 mmol) and cesium carbonate (19 g, 58.80 mmol) were dissolved in ultra-dry 1,4-dioxane (30 mL), added with m-trifluoromethoxybromobenzene (7.2 g, 26.73 mmol) and cyclohexanone (5.25 g, 53.46 mmol), heated at 100°C for 20 hours. The reaction liquid was extracted with EA and water after being cooled. The organic layer was dried with anhydrous sodium sulfate, separated by silica gel column chromatography after concentration to obtain 4.6 g yellow clear liquid of 2-(3-(trifluoromethoxy)phenyl)cyclohexanone **(21-2)**, yield 46.7 %.

LCMS: m/z= 259.1 (M+H)⁺.

### Step 2: Synthesis of 2-nitro-2-(3-(trifluoromethoxy)phenyl)hexyl-1-one (22-3)

**22-2** (4.6 g, 17.80 mmol) was dissolved in DCE (40 mL), added with copper acetate (1.62 g, 8.90 mmol) and cerium ammonium nitrate (24.4g, 44.50 mmol), and heated at 80°C for 12 hours. The reaction liquid was filtered, washed with DCM, dried with a rotary evaporator, then separated by silica gel column chromatography to obtain 2.58 g yellow oil of 2-nitro-2-(3-(trifluoromethoxy)phenyl)hexyl-1-one **(21-3)**, yield 48 %.

### Step 3: Synthesis of 2-amino-2-(3-(trifluoromethoxy)phenyl)hexyl-1-one (22-4)

Under nitrogen protection, **22-3** (2.58 g, 8.51 mmol) was dissolved in acetic acid (20 mL), added with zinc powder (3.3 g, 51.00 mmol), and heated at 80°C for 12 hours. After being cooled, the reaction liquid was adjusted to pH> 10 with a 2 M solution of sodium hydroxide, extracted with ethyl acetate. Then the organic phase was dried with anhydrous sodium sulfate, concentrated, separated and purified by silica gel chromatography to obtain a colorless oil of 2-amino-2-(3-(trifluoromethoxy)phenyl)hexyl-1-one **(22-4)** 0.86 g, yield 37 %. LCMS: m/z= 274.1 (M+H)⁺.

### Step 4: Synthesis of 2-chloro-N-(2-oxo-1-(3-(trifluoromethoxy)phenyl)hexyl)acetamide (22-5)

Under nitrogen protection, **22-4** (860 mg, 3.15 mmol) was dissolved in ultra-dry DCM (10 mL), added with ultra-dry triethylamine (0.5 mL, 3.50 mmol). Chloroacetyl chloride (250 µL, 3.15 mmol) was added dropwise at 0°C and stirred at room temperature for 1 hour. The reaction liquid was concentrated directly, separated and purified by silica gel chromatography to obtain a white solid of 2-chloro-N-(2-oxo-1-(3-(trifluoromethoxy)phenyl)hexyl)acetamide **(22-5)** 562 mg, yield 51 %. LCMS: m/z= 350.1 (M+H)⁺.

### Step 5: Synthesis of 4a-(3-(trifluoromethoxy)phenyl)hexahydro-2H-benzo[b][1,4]oxazine-3(4H)-one (22-6)

**22-5** (562 mg, 1.61 mmol) was put in a 25 mL round bottom flask. After 5 mL methanol was added for dissolving, sodium borohydride (61 mg, 1.61 mmol) was added slowly at 0°C. The reaction liquid was dried by a rotary evaporator after stirring for 30 mins. After 5 mL THF was added for dissolving, 60% sodium hydride (77 mg, 1.93 mmol) was added under ice bath and reacted for 10 hours. The reaction liquid was quenched with saturated saline, extracted with ethyl acetate. The organic phase was concentrated, separated and purified by silica gel chromatography to obtain a white solid of 4*a*-(3-(trifluoromethoxy)phenyl)hexahydro-2*H*-benzo[*b*][1,4]oxazine-3(4*H*)-one **(22-6)** 385 mg, yield 76 %. LCMS: m/z= 316.1 (M+H)⁺.

### Step 6: Synthesis of 4a-(3-(trifluoromethoxy)phenyl)octahydro-2H-benzo[b][1,4]oxazine hydrochloride (Compound 22)

Under nitrogen protection, **22-6** (335 mg, 1.06 mmol) was dissolved in ultra-dry THF (5 mL), added with a solution of borane-methyl sulfide in THF (2.7 mL, 5.3 mmol, 2.0 M), refluxed at 70°C for 12 hours, and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate, was separated and purified by HPLC after concentration, added with 1 mL diluted hydrochloric acid after concentration, lyophilized to obtain a white solid of 4*a*-(3-(trifluoromethoxy)phenyl)octahydro-2*H*-benzo[*b*][1,4]oxazine hydrochloride (Compound **22**) 220 mg, yield 61 %.

LCMS: m/z= 302.1 (M+H)⁺.

¹H NMR (400 MHz, chloroform-d) δ 10.54 (s, 1H), 10.00 (s, 1H), 8.13 - 7.96 (m, 2H), 7.54 (t, *J* = 8.0 Hz, 1H), 7.27 (s, 1H), 4.40 (td, *J* = 12.5, 2.4 Hz, 1H), 4.22 (dd, *J* = 11.7, 4.8 Hz, 1H), 4.08 (dd, *J* = 12.7, 3.5 Hz, 1H), 3.07 (d, *J* = 12.7 Hz, 1H), 3.01 - 2.89 (m, 1H), 2.82 (d, *J* = 13.2 Hz, 1H), 2.30 (td, *J* = 13.4, 3.0 Hz, 1H), 2.06 - 1.88 (m, 2H), 1.80 - 1.71 (m, 1H), 1.66 - 1.50 (m, 2H), 1.09 - 0.93 (m, 1H).

### Synthesis of Compound 23

### Synthesis of 4a-(4-(trifluoromethyl)phenyl)octahydro-2H-benzo[b][1,4]oxazine hydrochloride (Compound 23)

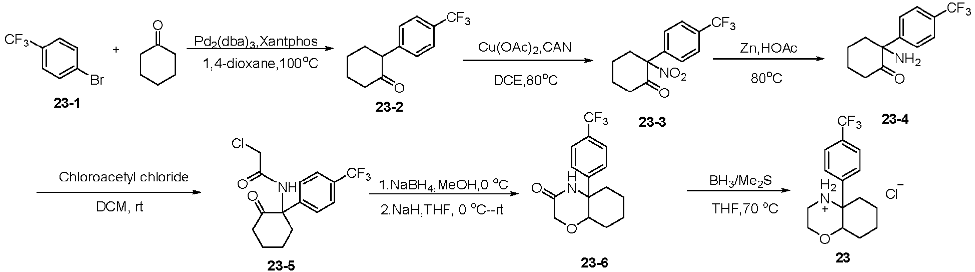

### Step 1: Synthesis of 2-(4-(trifluoromethyl)phenyl)hexyl-1-one (23-2)

Under nitrogen protection, Pd₂(dba)₃ (204 mg, 0.23 mmol), Xantphos (254 mg, 0.44 mmol) and cesium carbonate (16 g, 48.90 mmol) were dissolved in ultra-dry 1,4-dioxane (30 mL), added with 3-bromofluorobenzene (5 g, 28.60 mmol) and cyclohexanone (5.6 g, 57.01 mmol), heated at 100°C for 20 hours. The reaction liquid was extracted with EA and water after being cooled. The organic layer was dried with anhydrous sodium sulfate, separated and purified by silica gel chromatography after concentration to obtain 1.48 g yellow oil of 2-(4-(trifluoromethyl)phenyl)hexyl-1-one **(23-2)**, yield 50.7 %. LCMS: m/z= 243.00 (M+H)⁺.

### Step 2: Synthesis of 2-(4-(trifluoromethyl)phenyl)-2-nitrocyclohexanone (23-3)

**23-2** (2 g, 8.26 mmol) was dissolved in DCE (20 mL), added with copper acetate (0.75 g, 4.13 mmol) and cerium ammonium nitrate (9 g, 16.50 mmol), and heated at 80°C for 12 hours. The reaction liquid was filtered and washed with EA. After concentration, the filtrate was separated and purified by silica gel chromatography to obtain 1.053 g yellow oil of 2-(4-(trifluoromethyl)phenyl)-2-nitrocyclohexanone **(23-3)**, yield 44.4 %.

### Step 3: Synthesis of 2-(4-(trifluoromethyl)phenyl)-2-aminocyclohexanone (23-4)

Under nitrogen protection, **23-3** (1.08 g, 3.76 mmol) was dissolved in acetic acid (10 mL), added with zinc powder (1.22 g, 18.80 mmol), and heated at 80°C for 12 hours. After being cooled, the reaction liquid was adjusted to pH> 10 with a 2 M solution of sodium hydroxide, extracted with ethyl acetate. Then the organic phase was dried with anhydrous sodium sulfate, separated and purified by silica gel chromatography after concentration to obtain a colorless oil of 2-(4-(trifluoromethyl)phenyl)-2-aminocyclohexanone **(23-4)** 307 mg, yield 31.7 %. LCMS: m/z= 258.05 (M+H)⁺.

### Step 4: Synthesis of 2-chloro-N-(1-(4-(trifluoromethyl)phenyl)-2-oxocyclohexyl)acetamide (23-5)

Under nitrogen protection, **23-4**(302 mg, 1.17 mmol) was dissolved in ultra-dry DCM (5 mL) and added with ultra-dry triethylamine (0.2 mL, 1.30 mmol). Chloroacetyl chloride (93 µL, 1.17 mmol) was added dropwise at 0°C and stirred at room temperature for 1 hour. The reaction liquid was concentrated directly, then separated and purified by silica gel chromatography to obtain a white solid of 2-chloro-N-(1-(4-(trifluoromethyl)phenyl)-2-oxocyclohexyl)acetamide **(23-5)** 178 mg, yield 46 %. LCMS: m/z= 334.1 (M+H)⁺.

### Step 5: Synthesis of 4a-(4-(trifluoromethyl)phenyl)hexahydro-2H-benzo[b][1,4]oxazine-3(4H)-one (23-6)

**23-5** (147 mg, 0.95 mmol) was put in a 25 mL round bottom flask, added with 3 mL methanol for dissolving, sodium borohydride was slowly added under ice bath (17 mg, 0.95 mmol). The reaction liquid was dried by a rotary evaporator after stirring for 30 mins. After 3 mL THF was added for dissolving, 60% sodium hydride (21 mg, 1.14 mmol) was added under ice bath and reacted for 10 hours. The reaction liquid was quenched with saturated saline, extracted with ethyl acetate, then the organic phase was concentrated, separated and purified by silica gel chromatography to obtain a white solid of 4*a*-(4-(trifluoromethyl)phenyl)hexahydro-2*H-*benzo[*b*][1,4]oxazine-3(4*H*)-one (**23-6**) 54 mg, yield 23 %. LCMS: m/z= 300.05 (M+H)⁺.

### Step 6: Synthesis of 4a-(4-(trifluoromethyl)phenyl)octahydro-2H-benzo[b][1,4]oxazine hydrochloride (Compound 23)

Under nitrogen protection, **23-6** (54 mg, 0.18 mmol) was dissolved in ultra-dry THF (5 mL), added with a solution of borane-methyl sulfide in THF (0.9 mL, 1.8 mmol, 2.0 M), refluxed at 70°C for 12 hours, and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate, separated and purified by HPLC after concentration, added with 1 mL diluted hydrochloric acid after concentration, lyophilized to obtain a white solid of 4*a*-(4-(trifluoromethyl)phenyl)octahydro-2*H*-benzo[*b*][1,4]oxazine hydrochloride(Compound **23**) 35 mg, yield 68 %.

LCMS: m/z= 286.05 (M+H)⁺.

¹H NMR (400 MHz, chloroform-d) δ 10.53 (s, 1H), 9.99 (s, 1H), 8.26 (d, *J =* 8.3 Hz, 2H), 7.72 (d, *J* = 8.3 Hz, 2H), 4.38 (td, *J* = 12.6, 2.5 Hz, 1H), 4.23 (dd, *J* = 11.3, 5.4 Hz, 1H), 4.07 (dd, *J* = 12.7, 3.6 Hz, 1H), 3.06 (d, *J* = 12.8 Hz, 1H), 2.94 - 2.77 (m, 2H), 2.31 (td, *J* = 13.4, 3.1 Hz, 1H), 2.06 - 1.95 (m, 2H), 1.81 - 1.69 (m, 1H), 1.66 - 1.49 (m, 2H), 1.04 - 0.87 (m, 1H).

### Synthesis of Compound 24

### Synthesis of 4a-(2,6-dimethylphenyl)octahydro-2H-benzo[b][1,4]oxazine hydrochloride (Compound 24)

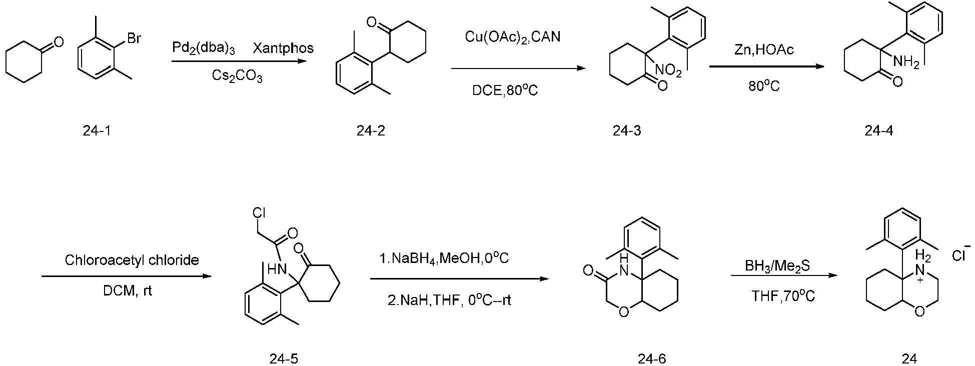

### Step 1: Synthesis of 2-(2,6-dimethylphenyl)cyclohexanone (24-2):

Under nitrogen protection, Pd₂(dba)₃ (247 mg, 0.27 mmol), Xantphos (312 mg, 0.54 mmol), cesium carbonate (19.3 g, 59.40 mmol) were dissolved in ultra-dry dioxane 30 mL, added with **24-1** (5 g, 27 mmol), cyclohexanone (5.3 g, 54 mmol), stirred at 80°C for 20 hours. After the reaction liquid was cooled to room temperature, it was filtered with diatomite, and extracted with EA. The organic phase was dried with anhydrous sodium sulfate, separated by flash silica gel chromatography to obtain 2.59 g yellow liquid of 2-(2,6-dimethylphenyl)cyclohexanone **(24-2)**, yield 47.5%. LCMS: m/z=203.05(M+H)⁺.

### Step 2: Synthesis of 2-(2,6-dimethylphenyl)-2-nitrocyclohexanone (24-3):

Under nitrogen protection, cerium ammonium nitrate (21 g, 38.40 mmol), copper acetate (2.32 g, 12.80 mmol), **24-2** (2.59 g, 12.80 mmol) were dissolved in 1,2-dichloroethane (40 mL), stirred at 80°C for 12 hours, diluted with DCM after being cooled to room temperature. The reaction liquid was filtered with diatomite, washed with DCM, separated and purified by flash silica chromatography after concentration to obtain 1.307 g light yellow liquid of 2-(2,6-dimethylphenyl)-2-nitrohexyl-1-one **(24-3)**, yield 41.4%.

### Step 3: Synthesis of 2-(2,6-dimethylphenyl)-2-aminocyclohexanone (24-4):

Under nitrogen protection, **24-3** (1 g, 4.05 mmol) was dissolved in 16 mL methanol, added with 8 mL icy acetic acid. 1.32 g zinc powder was added slowly and stirred at 80°C for 12 hours. The reaction liquid was adjusted to pH of 10 by adding 2 M NaOH, extracted with EA. The organic phase was dried with anhydrous sodium sulfate, separated and purified by flash silica gel column chromatography to obtain 583 mg yellow oil of 2-(2,6-dimethylphenyl)-2-aminocyclohexanone **(24-4)**, yield 66.4%. LCMS: m/z=218.25(M+H)⁺.

### Step 4: Synthesis of 2-chloro-N-(1-(2,6-dimethylphenyl)-2-oxocyclohexyl)acetamide (24-5):

Under nitrogen protection, **24-4** (500 mg, 2.30 mmol) was dissolved in 8 mL ultra-dry DCM, added with triethylamine (279 mg, 2.76 mmol), cooled to 0°C, added dropwise with chloroacetyl chloride (260 mg, 2.3 mmol), stirred at room temperature for 1 hour, and extracted with EA. The organic phase was dried with anhydrous sodium sulfate, separated and purified by flash silica gel column chromatography after concentration to obtain 468 mg white solid of 2-chloro-N-(1-(2,6-dimethylphenyl)-2-oxocyclohexyl)acetamide **(24-5)**, yield 69.5%. LCMS: m/z=294.10(M+H)⁺.

### Step 5: Synthesis of 4a-(2,6-dimethylphenyl)hexahydro-2H-benzo[b][1,4]oxazine-3(4H)-one (24-6):

Under nitrogen protection, **24-5** (389 mg, 1.33 mmol) was dissolved in 8 mL ultra-dry methanol, cooled to 0°C, added with sodium borohydride (50 mg, 1.33 mmol), warmed to room temperature and stirred for 1 hour with rotary evaporating solvents under reduced pressure, added with 8 ml ultra-dry THF for dissolving, cooled to 0°C, added with 60% sodium hydride (64 mg, 1.60 mmol), warmed to room temperature and stirred for 12 hours. The reaction was quenched by adding 1 N HCl, extracted with DCM. The organic phase was dried with anhydrous sodium sulfate, separated and purified by flash silica gel column chromatography after concentration to obtain 271 mg white solid of Compound 4*a*-(2,6-dimethylphenyl)hexahydro-2*H*-benzo[*b*] [1,4]oxazine-3(4*H*)-one **(24-6)**, yield 78.5%. LCMS: m/z=260.20(M+H)⁺.

### Step 6: Synthesis of 4a-(2,6-dimethylphenyl)octahydro-2H-benzo[b] [1,4]oxazine hydrochloride (24):

Under nitrogen protection, **24-6** (200 mg, 0.77 mmol) was dissolved in 7 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then lyophilized to obtain 30 mg white powder of 4*a*-(2,6-dimethylphenyl)octahydro-2*H*-benzo[*b*][1,4]oxazine hydrochloride **(24)**, yield 15.1%. LCMS: m/z=246.20(M+H)⁺.

¹H NMR (400 MHz, chloroform-d) δ 10.29 (s, 1H), 9.42 (s, 1H), 7.67 (s, 2H), 7.00 (s, 1H), 4.47 - 4.33 (m, 1H), 4.21 (dd, J = 12.3, 4.3 Hz, 1H), 4.04 (dd, J = 12.5, 3.4 Hz, 1H), 3.07 - 2.70 (m, 3H), 2.36 (s, 6H), 2.15 (dtd, J = 53.8, 12.9, 3.6 Hz, 2H), 1.97 (dd, J = 14.0, 3.6 Hz, 1H), 1.73 (d, J = 13.2 Hz, 1H), 1.57 (td, J = 11.7, 11.1, 5.1 Hz, 2H), 1.11- 0.94 (m, 1H).

### Synthesis of Compound 25

### Synthesis of 4a-(4-(tertbutyl)phenyl)octahydro-2H-benzo[b][1,4]oxazine hydrochloride (Compound 25)

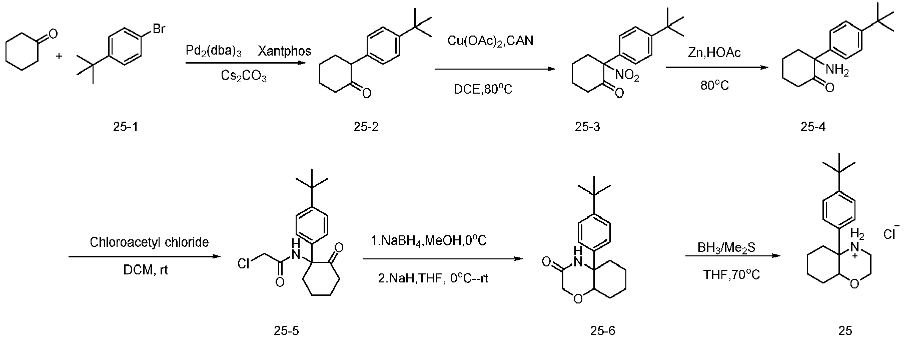

### Step 1: Synthesis of 2-(4-(tertbutyl)phenyl)cyclohexanone (25-2):

Under nitrogen protection, Pd₂(dba)₃ (215 mg, 0.23 mmol), Xantphos (272 mg, 0.46 mmol), cesium carbonate (16.8 g, 51.70 mmol) were dissolved in ultra-dry dioxane 30 mL, added with **25-1** (5 g, 23.50 mmol), cyclohexanone (4.6 g, 47.00 mmol) and stirred at 85°C for 20 hours. After the reaction liquid was cooled to room temperature, it was filtered with diatomite and extracted with EA. The organic phase was dried with anhydrous sodium sulfate, separated and purified by flash silica gel column chromatography to obtain 3.04 g yellow and white solid of 2-(4-(tertbutyl)phenyl)cyclohexanone **(25-2)**, yield 57.5%. LCMS: m/z=231.10(M+H)⁺.

### Step 2: Synthesis of 2-(4-(tertbutyl)phenyl)-2-nitrocyclohexanone (25-3):

Under nitrogen protection, cerium ammonium nitrate (21.7 g, 39.60 mmol), copper acetate (2.4 g, 13.20 mmol), **25-2** (3.04 g, 13.20 mmol) was dissolved in 1,2-dichloroethane (40 mL), stirred at 80°C for 12 hours, diluted with DCM after being cooled to room temperature. The reaction liquid was filtered with diatomite, washed with DCM, separated and purified by flash silica chromatography after concentration to obtain 1.2 g yellow and white solid of 2-(4-(tertbutyl)phenyl)-2-nitrocyclohexanone **(25-3)**, yield 33%.

### Step 3: Synthesis of 2-(4-(tertbutyl)phenyl)-2-aminocyclohexanone (25-4):

Under nitrogen protection, **25-3** (1 g, 3.64 mmol) was dissolved in 12 mL methanol, added with 6 mL icy acetic acid. 1.18 g zinc powder was added slowly and stirred at 80°C for 12 hours. The reaction liquid was adjusted to pH of 10 by adding 2 M NaOH and extracted with EA. The organic phase was dried with anhydrous sodium sulfate, separated and purified by flash silica gel column chromatography to obtain 633 mg light yellow solid of 2-(4-(tertbutyl)phenyl)-2-aminocyclohexanone **(25-4)**, yield 71%. LCMS: m/z=246.50(M+H)⁺.

### Step 4: Synthesis of N-(1-(4-(tertbutyl)phenyl)-2-oxocyclohexyl)-2-chloroacetamide (25-5):

Under nitrogen protection, **25-4** (596 mg, 2.43 mmol) was dissolved in 8 mL ultra-dry DCM, added with triethylamine (295 mg, 2.92 mmol), cooled to 0°C, added dropwise with chloroacetyl chloride (274 mg, 2.43 mmol), stirred at room temperature for 1 hour and extracted with EA. The organic phase was dried with anhydrous sodium sulfate, separated and purified by flash silica gel column chromatography after concentration to obtain 594 mg light yellow oily compound of N-(1-(4-(tertbutyl)phenyl)-2-oxocyclohexyl)-2-chloroacetamide **(25-5)**, yield 76.1%. LCMS: m/z=322.10(M+H)⁺.

### Step 5: Synthesis of 4a-(4-(tertbutyl)phenyl)hexahydro-2H-benzo[b][1,4]oxazine-3(4H)-one (25-6):

Under nitrogen protection, **25-5** (500 mg, 1.55 mmol) was dissolved in 8 mL ultra-dry methanol, cooled to 0°C, added with sodium borohydride (59 mg, 1.55 mmol), warmed to room temperature, stirred for 1 hour with rotary evaporating solvents under reduced pressure, added with 8 mL ultra-dry THF for dissolving, cooled to 0°C, added with 60 % sodium hydride (74 mg, 1.86 mmol), warmed to room temperature and stirred for 12 hours. The reaction was quenched by adding 1 N HCl, extracted with DCM. The organic phase was dried with anhydrous sodium sulfate, separated and purified by flash silica gel column chromatography after concentration to obtain 261 mg white solid of Compound 4*a*-(4-(tertbutyl)phenyl)hexahydro-2*H*-benzo[*b*][1,4]oxazine-3(4*H*)-one **(25-6)**, yield 58.8%. LCMS: m/z=288.20(M+H)⁺.

### Step 6: Synthesis of 4a-(4-(tertbutyl)phenyl)octahydro-2H-benzo[b][1,4]oxazine hydrochloride (25):

Under nitrogen protection, **25-6** (200 mg, 0.7 mmol) was dissolved in 7 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, and extracted with EA. The organic phase was dried with anhydrous sodium sulfate, then separated and purified by flash silica chromatography and dried with a rotary evaporator under reduced pressure. The product was dissolved in a small amount of MeOH, separated and purified by HPLC, then lyophilized to obtain 33 mg white solid of 4*a*-(4-(tertbutyl)phenyl)octahydro-2*H*-benzo[*b*] [1,4]oxazine hydrochloride **(25)**, yield 15.7%. LCMS: m/z=274.20(M+H)⁺.

¹H NMR (400 MHz, chloroform-d) δ 7.90 (d, *J* = 8.3 Hz, 2H), 7.37 (d, *J* = 8.7 Hz, 2H), 4.11 (dd, *J* = 12.6, 3.8 Hz, 1H), 3.88 (td, *J* = 12.7, 2.8 Hz, 1H), 3.77 (d, *J* = 11.8 Hz, 1H), 3.64 - 3.54 (m, 1H), 3.31 (dq, *J* = 13.9, 2.9 Hz, 1H), 3.09 - 2.93 (m, 1H), 2.82 (dt, *J* = 14.2, 2.7 Hz, 1H), 1.83 (td, *J* = 10.0, 8.4, 3.8 Hz, 2H), 1.72 - 1.52 (m, 3H), 1.49 - 1.40 (m, 1H), 1.33 (s, 9H).

### Synthesis of Compound 26

### Synthesis of 4a-(2,3-dichlorophenyl)octahydro-2H-benzo[b][1,4]oxazine hydrochloride (Compound 26)

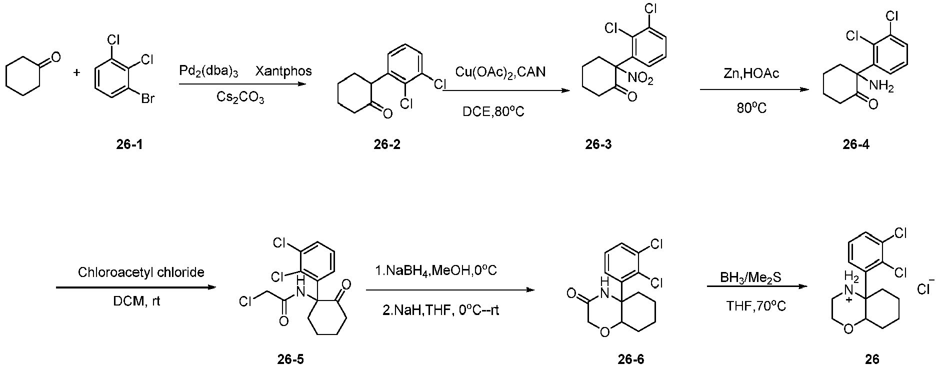

### Step 1: Synthesis of 2-(2,3-dichlorophenyl)cyclohexanone (26-2):

Under nitrogen protection, Pd₂(dba)₃ (201 mg, 0.22 mmol), Xantphos (255 mg, 0.44 mmol), cesium carbonate (15.8 g, 59.40 mmol) were dissolved in ultra-dry dioxane 30 mL, added with **26-1** (5 g, 22.10 mmol), cyclohexanone (5.3 g, 44.20 mmol) and stirred at 85°C for 20 hours. After the reaction liquid was cooled to room temperature, it was filtered with diatomite, extracted with EA. The organic phase was dried with anhydrous sodium sulfate, separated by flash silica gel chromatography to obtain 2.28 g yellow liquid of 2-(2,3-dichlorophenyl)cyclohexanone (**26-2**), yield 42.6%. LCMS: m/z=242.95(M+H)⁺.

### Step 2: Synthesis of 2-(2,3-dichlorophenyl)-2-nitrocyclohexanone (26-3):

Under nitrogen protection, cerium ammonium nitrate (15.43 g, 28.14 mmol), copper acetate (1.7 g, 9.38 mmol), **26-2** (2.28 g, 9.38 mmol) was dissolved in 1,2-dichloroethane (30 mL), stirred at 80°C for 12 hours, diluted with DCM after being cooled to room temperature. The reaction liquid was filtered with diatomite, washed with DCM, separated and purified by flash silica chromatography after concentration to obtain 959 mg yellow oil of 2-(2,3-dichlorophenyl)-2-nitrocyclohexanone **(26-3)**, yield 35.5%.

### Step 3: Synthesis of 2-(2,3-dichlorophenyl)-2-aminocyclohexanone (26-4):

Under nitrogen protection, **26-3** (900 mg, 3.12 mmol) was dissolved in 14 mL methanol and added with 7 mL icy acetic acid. 1.02 g zinc powder was added slowly and stirred at 80°C for 12 hours. The reaction liquid was adjusted to pH of 10 by adding 2 M NaOH, extracted with EA. The organic phase was dried with anhydrous sodium sulfate, separated and purified by flash silica gel column chromatography to obtain 344 mg yellow liquid of 2-(2,3-dichlorophenyl)-2-aminocyclohexanone **(26-4)**, yield 42.8%. LCMS: m/z=258.00(M+H)⁺.

### Step 4: Synthesis of 2-chloro-N-(1-(2,3-dichlorophenyl)-2-oxocyclohexyl)acetamide (26-5):

Under nitrogen protection, **26-4** (326 mg, 1.26 mmol) was dissolved in 5 mL ultra-dry DCM, added with triethylamine (154 mg, 1.52 mmol), cooled to 0°C, added dropwise with chloroacetyl chloride (142 mg, 1.26 mmol), stirred at room temperature for 1 hour, and extracted with EA. The organic phase was dried with anhydrous sodium sulfate, separated and purified by flash silica gel column chromatography after concentration to obtain 337 mg light yellow oily compound of 2-chloro-N-(1-(2,3-dichlorophenyl)-2-oxocyclohexyl)acetamide **(26-5)**, yield 80.2%. LCMS: m/z=335.10(M+H)⁺.

### Step 5: Synthesis of 4a-(2,3-dichlorophenyl)hexahydro-2H-benzo[b][1,4]oxazine-3(4H)-one (26-6):

Under nitrogen protection, **26-5** (300 mg, 0.90 mmol) was dissolved in 5 mL ultra-dry methanol, cooled to 0°C, added with sodium borohydride (34 mg, 0.90 mmol), warmed to room temperature, stirred for 1 hour with rotary evaporating solvents under reduced pressure, added with 5 mL ultra-dry THF for dissolving, cooled to 0°C, added with 60% sodium hydride (44 mg, 1.1 mmol), warmed to room temperature and stirred for 12 hours. The reaction was quenched by adding 1 N HCl, extracted with DCM. The organic phase was dried with anhydrous sodium sulfate, separated and purified by flash silica gel column chromatography after concentration to obtain 159 mg white solid of Compound 4*a*-(2,3-dichlorophenyl)hexahydro-2*H*-benzo[*b*][1,4]oxazine-3(4*H*)-one **(26-6)**, yield 58.9%. LCMS: m/z=300.10(M+H)⁺.

### Step 6: Synthesis of 4a-(2,3-dichlorophenyl)octahydro-2H-benzo[b][1,4]oxazine hydrochloride (26):

Under nitrogen protection, **26-6** (140 mg, 0.50 mmol) was dissolved in 5 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then lyophilized to obtain 31 mg white solid of 4*a*-(2,3-dichlorophenyl)octahydro-2*H*-benzo[*b*][1,4]oxazine hydrochloride **(26)**, yield 21.7%. LCMS: m/z=286.10(M+H)⁺.

¹H NMR (400 MHz, chloroform-d) δ 11.57 (t, *J* = 11.8 Hz, 1H), 8.35 (d, *J* = 12.0 Hz, 1H), 8.22 (dd, *J* = 8.2, 1.5 Hz, 1H), 7.57 (dd, *J* = 8.0, 1.4 Hz, 1H), 7.34 - 7.27 (m, 1H), 4.67 - 4.53 (m, 2H), 4.01 (dd, *J* = 12.5, 3.7 Hz, 1H), 3.59 - 3.40 (m, 2H), 2.97 - 2.84 (m, 1H), 2.31 (td, *J* = 13.7, 2.9 Hz, 1H), 2.11 (ddt, *J* = 15.7, 11.3, 5.5 Hz, 2H), 1.81 - 1.72 (m, 1H), 1.63 (dp, *J* = 12.8, 4.3 Hz, 2H), 0.88 (qt, *J* = 14.0, 2.9 Hz, 1H).

### Synthesis of Compound 27

### Synthesis of 4a-(2-isopropylphenyl)octahydro-2H-benzo[b][1,4]oxazine hydrochloride (Compound 27)

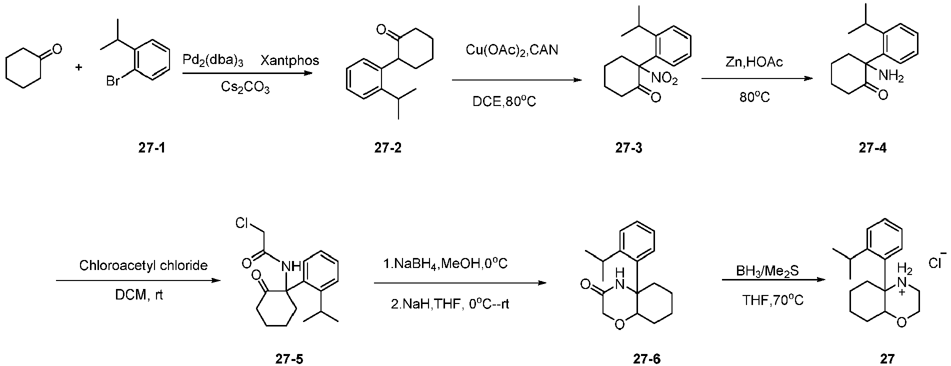

### Step 1: Synthesis of 2-(2-isopropylphenyl)cyclohexanone (27-2):

Under nitrogen protection, Pd₂(dba)₃ (229 mg, 0.25 mmol), Xantphos (294 mg, 0.5 mmol), cesium carbonate (17.9 g, 55.20 mmol) were dissolved in ultra-dry dioxane 30 mL, added with **27-1** (5 g, 25.10 mmol), cyclohexanone (4.9 g, 50.20 mmol) and stirred at 85°C for 20 hours. After the reaction liquid was cooled to room temperature, it was filtered with diatomite, extracted with EA. The organic phase was dried with anhydrous sodium sulfate, separated by flash silica gel chromatography to obtain 2.36 g light yellow liquid of 2-(2-isopropylphenyl)cyclohexanone (**27-**2), yield 43.8%. LCMS: m/z=217.10(M+H)⁺.

### Step 2: Synthesis of 2-(2-isopropylphenyl)-2-nitrocyclohexanone (27-3):

Under nitrogen protection, cerium ammonium nitrate (18 g, 32.80 mmol), copper acetate (1.98 g, 10.93 mmol), **27-2** (2.364 g, 10.93 mmol) were dissolved in 1,2-dichloroethane (35 mL), stirred at 80°C for 12 hours, and diluted with DCM after being cooled to room temperature. The reaction liquid was filtered with diatomite, washed with DCM, separated and purified by silica gel chromatography after concentration to obtain 721 mg brown yellow liquid of 2-(4-isopropylphenyl)-2-nitrohexyl-1-one **(27-3)**, yield 25.4%.

### Step 3: Synthesis of 2-(2-isopropylphenyl)-2-aminocyclohexanone (27-4):

Under nitrogen protection, **27-3** (700 mg, 2.68 mmol) was dissolved in 10 mL methanol and added with 5 mL icy acetic acid. 872 mg zinc powder was added slowly and stirred at 80°C for 12 hours. The reaction liquid was adjusted to pH of 10 by adding 2 M NaOH, extracted with EA. The organic phase was dried with anhydrous sodium sulfate, separated and purified by flash silica gel column chromatography to obtain 269 mg yellow oily compound of 2-(2-isopropylphenyl)-2-aminocyclohexanone **(27-4)**, yield 43.4%. LCMS: m/z=232.45(M+H)⁺.

### Step 4: Synthesis of 2-chloro-N-(1-(2-isopropylphenyl)-2-oxocyclohexyl)acetamide (27-5):

Under nitrogen protection, **27-4** (230 mg, 1.00 mmol) was dissolved in 5 mL ultra-dry DCM, added with triethylamine (121 mg, 1.00 mmol), cooled to 0°C, added dropwise with chloroacetyl chloride (113 mg, 1.00 mmol), stirred at room temperature for 1 hour and extracted with EA. The organic phase was dried with anhydrous sodium sulfate, separated and purified by flash silica gel column chromatography after concentration to obtain 224 mg light yellow oily compound of 2-chloro-N-(1-(2-isopropylphenyl)-2-oxocyclohexyl)acetamide **(27-5)**, yield 72.9%. LCMS: m/z=308.1(M+H)⁺.

### Step 5: Synthesis of 4a-(2-isopropylphenyl)hexahydro-2H-benzo[b][1,4]oxazine-3(4H)-one (27-6):

Under nitrogen protection, **27-5** (200 mg, 0.65 mmol) was dissolved in 5 mL ultra-dry methanol, cooled to 0°C, added with sodium borohydride (25 mg, 0.65 mmol), warmed to room temperature and stirred for 1 hour with rotary evaporating solvents under reduced pressure, added with 5 mL ultra-dry THF for dissolving, cooled to 0°C, added with 60% sodium hydride (32 mg, 0.78 mmol), heated to room temperature and stirred for 12 hours. The reaction was quenched by adding 1 N HCl and extracted with DCM. The organic phase was dried with anhydrous sodium sulfate, separated and purified by flash silica gel column chromatography after concentration to obtain 80 mg white solid of 4*a*-(2-isopropylphenyl)hexahydro-2*H*-benzo[*b*][1,4]oxazine-3(4*H*)-one **(27-6)**, yield 45.2%. LCMS: m/z=274.2(M+H)⁺.

### Step 6: Synthesis of 4a-(2-isopropylphenyl)octahydro-2H-benzo[b][1,4]oxazine hydrochloride (27):

Under nitrogen protection, **27-6** (80 mg, 0.30 mmol) was dissolved in 5 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then lyophilized to obtain 20 mg white powder of 4*a*-(2-isopropylphenyl)octahydro-2*H*-benzo[*b*][1,4]oxazine hydrochloride **(27)**, yield 26%. LCMS: m/z=260.2(M+H)⁺.

¹H NMR (400 MHz, chloroform-d) δ 10.91 (s, 1H), 8.52 (s, 1H), 8.23 (d, *J* = 8.2 Hz, 1H), 7.50 (d, *J* = 7.9 Hz, 1H), 7.36 (t, *J* = 7.5 Hz, 1H), 7.19 (t, *J* = 7.3 Hz, 1H), 4.60 (t, *J* = 12.1 Hz, 1H), 4.46 (dd, *J* = 12.4, 4.2 Hz, 1H), 4.01 (dd, *J* = 12.3, 3.2 Hz, 1H), 3.58 - 3.45 (m, 1H), 3.33 (d, *J* = 12.4 Hz, 1H), 3.04 (q, *J* = 10.2 Hz, 1H), 2.87 (d, *J=* 13.0 Hz, 1H), 2.36 (t, *J* = 12.5 Hz, 1H), 2.20 (qd, *J* = 12.6, 4.8 Hz, 1H), 1.99 (d, *J* = 12.8 Hz, 1H), 1.71 (d, *J* = 10.9 Hz, 1H), 1.59 (dt, *J* = 8.5, 3.9 Hz, 2H), 1.41 (d, *J* = 5.9 Hz, 3H), 1.28 (d, *J* = 6.1 Hz, 3H), 0.94 (q, *J* = 13.9 Hz, 1H).

### Synthesis of Compound 28

### Synthesis of 4a-(2,5-dimethylphenyl)octahydro-2H-benzo[b][1,4]oxazine hydrochloride (Compound 28)

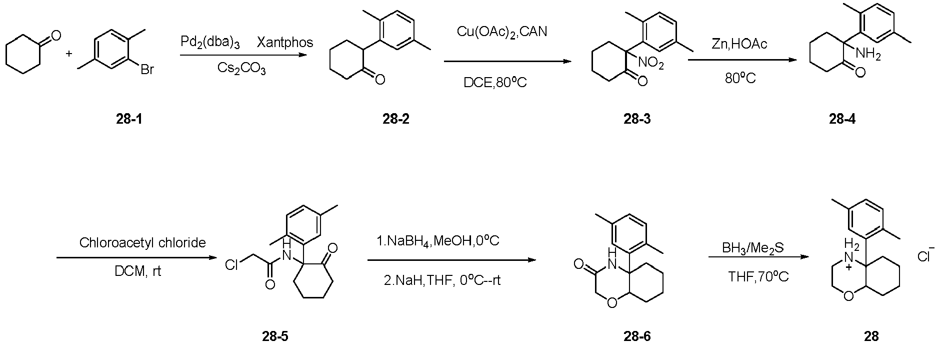

### Step 1: Synthesis of 2-(2,5-dimethylphenyl)cyclohexanone (28-2):

Under nitrogen protection, Pd₂(dba)₃ (247 mg, 0.27 mmol), Xantphos (312 mg, 0.54 mmol), cesium carbonate (19.3 g, 59.40 mmol) were dissolved in ultra-dry dioxane 30 mL, added with **28-1** (5 g, 27.00 mmol), cyclohexanone (5.3 g, 54.00 mmol) and stirred at 80°C for 20 hours. After the reaction liquid was cooled to room temperature, it was filtered with diatomite, extracted with EA, dried with anhydrous sodium sulfate, separated by flash silica gel chromatography to obtain 2.19 g yellow liquid of 2-(2,5-dimethylphenyl)cyclohexanone **(28-2)**, yield 40.3%. LCMS: m/z=203.05(M+H)⁺.

### Step 2: Synthesis of 2-(2,5-dimethylphenyl)-2-nitrocyclohexanone (28-3):

Under nitrogen protection, cerium ammonium nitrate (17.87 g, 32.60 mmol), copper acetate (1.97 g, 10.87 mmol), **28-2** (2.19 g, 10.87 mmol) was dissolved in 1,2-dichloroethane (35 mL), stirred at 80°C for 12 hours, diluted with DCM after being cooled to room temperature. The reaction liquid was filtered with diatomite, washed with DCM, then separated and purified by flash silica chromatography after concentration to obtain 639 mg yellow oil of 2-(2,5-dimethylphenyl)-2-nitrocyclohexanone **(28-3)**, yield 23.8%.

### Step 3: Synthesis of 2-(2,5-dimethylphenyl)-2-aminocyclohexanone (28-4):

Under nitrogen protection, **28-4(600** mg, 2.43 mmol) was dissolved in 10 mL methanol and added with 5 mL icy acetic acid. 790 mg zinc powder was added slowly and stirred at 80°C for 12 hours. The reaction liquid was adjusted to pH of 10 by adding 2 M NaOH and extracted with EA. The organic phase was dried with anhydrous sodium sulfate, separated and purified by flash silica gel column chromatography to obtain 281 mg yellow liquid of 2-(2,5-dimethylphenyl)-2-aminocyclohexanone **(28-4)**, yield 53.3%. LCMS: m/z=218.10(M+H)⁺.

### Step 4: Synthesis of 2-chloro-N-(1-(2,5-dimethylphenyl)-2-oxocyclohexyl)acetamide (28-5):

Under nitrogen protection, **28-4** (267 mg, 1.23 mmol) was dissolved in 5 mL ultra-dry DCM, added with triethylamine (150 mg, 1.48 mmol), cooled to 0°C, added dropwise with chloroacetyl chloride (139 mg, 1.23 mmol), stirred at room temperature for 1 hour and extracted with EA. The organic phase was dried with anhydrous sodium sulfate, separated and purified by flash silica gel column chromatography after concentration to obtain 242 mg yellow and white solid of 2-chloro-N-(1-(2,5-dimethylphenyl)-2-oxocyclohexyl)acetamide **(28-5)**, yield 67.2%. LCMS: m/z=294.1(M+H)⁺.

### Step 5: Synthesis of 4a-(2,5-dimethylphenyl)hexahydro-2H-benzo[b][1,4]oxazine-3(4H)-one (28-6):

Under nitrogen protection, **28-5** (220 mg, 0.75 mmol) was dissolved in 5 mL ultra-dry methanol, cooled to 0°C, added with sodium borohydride (28 mg, 0.75 mmol), warmed to room temperature and stirred for 1 hour with rotary evaporating solvents under reduced pressure. 5 mL ultra-dry THF was added for dissolving, cooled to 0°C, added with 60% sodium hydride (36 mg, 0.9 mmol), warmed to room temperature and stirred for 12 hours. The reaction was quenched by adding 1 N HCl and extracted with DCM. The organic phase was dried with anhydrous sodium sulfate, separated and purified by flash silica gel column chromatography after concentration to obtain 74 mg white solid of Compound 4*a*-(2,5-dimethylphenyl)hexahydro-2*H-*benzo[*b*][1,4]oxazine-3(4*H*)-one **(28-6)**, yield 38.1%. LCMS: m/z=260.2(M+H)⁺.

### Step 6: Synthesis of 4a-(2,5-dimethylphenyl)octahydro-2H-benzo[b] [1,4]oxazine hydrochloride (28):

Under nitrogen protection, **28-6** (73 mg, 0.28 mmol) was dissolved in 3 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution and extracted with EA. The organic phase was dried with anhydrous sodium sulfate, then dried with a rotary evaporator under reduced pressure. The product was dissolved in a small amount of MeOH, separated and purified by HPLC, then lyophilized to obtain 29 mg white powder of 4*a*-(2,5-dimethylphenyl)octahydro-2H-benzo[b][1,4]oxazine hydrochloride **(28)**, yield 42.6%. LCMS: m/z=246.2(M+H)⁺.

¹H NMR (400 MHz, chloroform-d) δ 10.43 (s, 1H), 8.71 (s, 1H), 8.11 (s, 1H), 7.15 - 7.06 (m, 2H), 4.49 (t, *J* = 12.1 Hz, 1H), 4.28 (dd, *J* = 12.3, 4.0 Hz, 1H), 4.05 (d, *J* = 12.3 Hz, 1H), 3.21 (d, *J* = 12.5 Hz, 1H), 3.04 (t, *J* = 14.0 Hz, 2H), 2.69 (s, 3H), 2.33 (s, 3H), 2.15 (t, *J* = 12.5 Hz, 2H), 1.97 (d, *J* = 11.8 Hz, 1H), 1.71 (d, *J* = 10.1 Hz, 1H), 1.58 (d, *J* = 13.7 Hz, 2H), 1.00 (q, *J* = 13.4 Hz, 1H).

### Synthesis of Compound 29

### Synthesis of 4a-(2-chloro-3-fluorophenyl)octahydro-2H-benzo[b][1,4]oxazine hydrochloride (Compound 29)

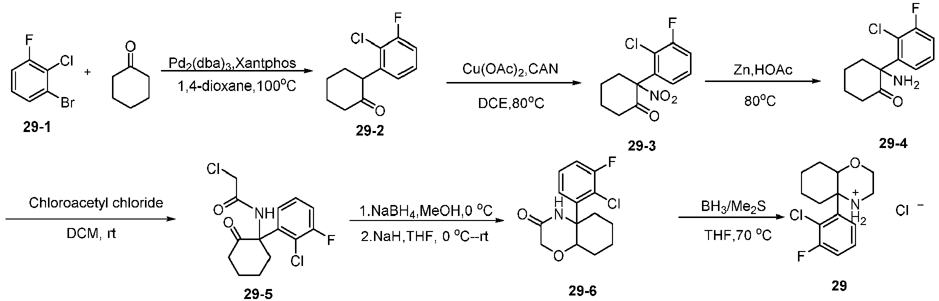

### Step 1: Synthesis of 2-(2-chloro-3-fluorophenyl)cyclohexanone (29-2)

Under nitrogen protection, Pd₂(dba)₃ (349 mg, 0.38 mmol), Xantphos (419 mg, 0.76 mmol) and cesium carbonate (27 g, 84.04 mmol) were dissolved in ultra-dry 1,4-dioxane (50 mL), added with 2-chloro-3-fluorobromobenzene (8 g, 38.20 mmol) and cyclohexanone (7.5 g, 76.40 mmol) and heated at 100°C for 20 hours. The reaction liquid was extracted with EA and water after being cooled. The organic layer was dried with anhydrous sodium sulfate, concentrated, separated and purified by silica gel column chromatography to obtain 5.32 g yellow oil of 2-(2-chloro-3-fluorophenyl)cyclohexanone **(29-2)**, yield 61.4 %.

### Step 2: Synthesis of 2-(2-chloro-3-fluorophenyl)-2-nitrocyclohexanone (29-3)

**29-2** (5.32 g, 23.5 mmol) was dissolved in DCE (40 mL), added with copper acetate (2.13 g, 11.7 mmol) and cerium ammonium nitrate (32 g, 58.8 mmol), and heated at 80°C for 12 hours. The reaction liquid was filtered, washed with EA, concentrated, separated and purified by silica gel column chromatography to obtain 2.46 g yellow oil of 2-(2-chloro-3-fluorophenyl)-2-nitrocyclohexanone **(29-3)**, yield 38.6 %.

### Step 3: Synthesis of 2-(2-chloro-3-fluorophenyl)-2-aminocyclohexanone (29-4)

Under nitrogen protection, **29-3** (2.46 g, 9.05 mmol) was dissolved in acetic acid (20 mL), added with zinc powder (3.53 g, 54.3 mmol) and heated at 80°C for 12 hours. After being cooled, the reaction liquid was adjusted to pH> 10 with a 2 M solution of sodium hydroxide, extracted with ethyl acetate. Then the organic phase was dried with anhydrous sodium sulfate, concentrated, separated and purified by silica gel column chromatography to obtain a colorless oil of 2-(2-chloro-3-fluorophenyl)-2-aminocyclohexanone **(29-4)** 809 mg, yield 37 %.

### Step 4: Synthesis of 2-chloro-N-(1-(2-chloro-3-fluorophenyl)-2-oxocyclohexyl)acetamide (29-5)

Under nitrogen protection, **29-4** (440 mg, 1.95 mmol) was dissolved in ultra-dry DCM (10 mL), added with ultra-dry triethylamine (0.5 mL, 3.7 mmol). Chloroacetyl chloride (266 µL, 3.35 mmol) was added dropwise at 0°C and stirred at room temperature for 1 hour until the raw material disappeared by analysis of TLC. The reaction liquid was concentrated directly, separated and purified by silica gel column chromatography to obtain a white solid of 2-chloro-N-(1-(2-chloro-3-fluorophenyl)-2-oxocyclohexyl)acetamide **(29-5)** 875 mg, yield 82.1 %. LCMS: m/z= 302.05 (M+H)⁺.

### Step 5: Synthesis of 4a-(2-chloro-3-fluorophenyl)hexahydro-2H-benzo[b][1,4]oxazine-3(4H)-one (29-6)

**29-5** (800 mg, 2.51 mmol) was put in a 25 mL round bottom flask. After 5 mL methanol was added for dissolving, sodium borohydride (95 mg, 2.51 mmol) was added slowly at 0°C. The reaction liquid was dried by a rotary evaporator after stirring for 30 mins. After 5 mL THF was added for dissolving, 60% sodium hydride (120 mg, 3.01 mmol) was added under ice bath and reacted for 10 hours. The reaction liquid was quenched with saturated saline, extracted with ethyl acetate. The organic phase was concentrated, separated and purified by silica gel column chromatography to obtain a white solid of 4*a*-(2-chloro-3-fluorophenyl)hexahydro-2*H-*benzo[b][1,4]oxazine-3(4H)-one **(29-6)** 460 mg, yield 64.6 %. LCMS: m/z= 284.1 (M+H)⁺.

### Step 6: Synthesis of 4a-(2-chloro-3-fluorophenyl)octahydro-2H-benzo[b][1,4]oxazine hydrochloride (Compound 29)

Under nitrogen protection, **29-6** (460 mg, 1.62 mmol) was dissolved in ultra-dry THF (10 mL), added with a solution of borane-methyl sulfide in THF (4 mL, 8 mmol, 2.0 M) and refluxed at 70°C for 12 hours. The reaction was quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product of Compound **29**.

The crude product of Compound **29** was separated and purified by HPLC. The purified product was concentrated, added with a small amount of hydrochloric acid and lyophilized to obtain a white solid of 4*a*-(2-chloro-3-fluorophenyl)octahydro-2*H*-benzo[*b*][1,4]oxazine hydrochloride 360 mg, yield 82.4 %. LCMS: m/z= 270.1 (M+H)⁺.

¹H NMR (400 MHz, chloroform-d) δ 11.60 (s, 1H), 8.44 - 8.18 (m, 1H), 8.09 (d, J = 8.2 Hz, 1H), 7.40 - 7.30 (m, 1H), 7.30 - 7.19 (m, 1H), 4.68 - 4.47 (m, 2H), 4.08 - 3.96 (m, 1H), 3.51 (dd, J = 34.6, 13.4 Hz, 2H), 3.00 - 2.83 (m, 1H), 2.33 (t, J = 13.4 Hz, 1H), 2.21 - 2.02 (m, 2H), 1.85 - 1.72 (m, 1H), 1.71 - 1.54 (m, 2H), 0.99 - 0.81 (m, 1H).

### Synthesis of Compound 30

### Synthesis of 4a-(3,4-difluorophenyl)octahydro-2H-benzo[b][1,4]oxazine hydrochloride (Compound 30)

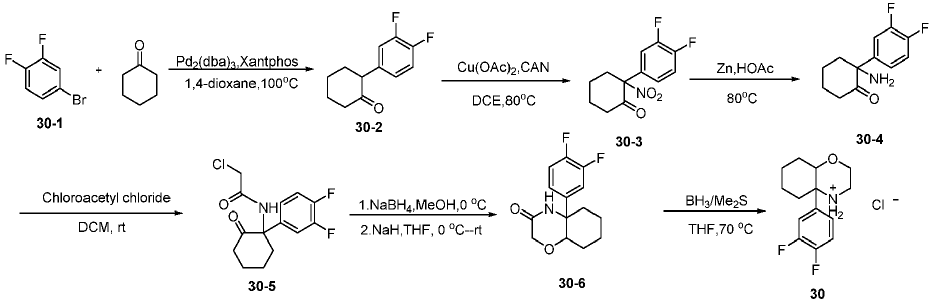

### Step 1: Synthesis of 2-(3,4-difluorophenyl)cyclohexanone (30-2)

Under nitrogen protection, Pd₂(dba)₃ (237 mg, 0.26 mmol), Xantphos (300 mg, 0.52 mmol) and cesium carbonate (18.6 g, 57 mmol) were dissolved in ultra-dry 1,4-dioxane (30 mL), added with 3,4-difluorobromobenzene (5 g, 25.9 mmol) and cyclohexanone (5.1 g, 51.8 mmol), and heated at 100°C for 20 hours. The reaction liquid was extracted with EA and water after being cooled. The organic layer was dried with anhydrous sodium sulfate, concentrated, separated and purified by silica gel column chromatography to obtain 2.1 g yellow oil of 2-(3,4-difluorophenyl)cyclohexanone **(30-2)**, yield 38.6 %.

### Step 2: Synthesis of 2-(3,4-difluorophenyl)-2-nitrocyclohexanone (30-3)

**30-2** (2.1 g, 10 mmol) was dissolved in DCE (30 mL), added with copper acetate (1 g, 5 mmol) and cerium ammonium nitrate (13.7 g, 25 mmol), and heated at 80°C for 12 hours. The reaction liquid was filtered, washed with EA, concentrated, separated and purified by silica gel column chromatography to obtain 0.96 g yellow oil of 2-(3,4-difluorophenyl)-2-nitrocyclohexanone **(30-3)**, yield 37.8 %.

### Step 3: Synthesis of 2-(3,4-difluorophenyl)-2-aminocyclohexanone (30-4)

Under nitrogen protection, **30-3** (0.96 g, 3.78 mmol) was dissolved in acetic acid (10 mL), added with zinc powder (1.47 g, 22.7 mmol), and heated at 80°C for 12 hours. After being cooled, the reaction liquid was adjusted to pH> 10 with a 2 M solution of sodium hydroxide and extracted with ethyl acetate. Then the organic phase was dried with anhydrous sodium sulfate, concentrated, separated and purified by silica gel column chromatography to obtain a colorless oil of 2-(3,4-difluorophenyl)-2-aminocyclohexanone **(30-4)** 354 mg, yield 41.6 %. LCMS: m/z= 226.1 (M+H)⁺

### Step 4: Synthesis of 2-chloro-N-(1-(3,4-difluorophenyl)-2-oxocyclohexyl)acetamide (30-5)

Under nitrogen protection, **30-4** (354 mg, 1.57 mmol) was dissolved in ultra-dry DCM (10 mL), added with ultra-dry triethylamine (0.3 mL, 1.73 mmol). Chloroacetyl chloride (125 µL, 1.57 mmol) was added dropwise at 0°C and stirred at room temperature for 1 hour until the raw material disappeared by analysis of TLC. The reaction liquid was concentrated directly, separated and purified by silica gel column chromatography to obtain a white solid of 2-chloro-N-(1-(3,4-difluorophenyl)-2-oxocyclohexyl)acetamide **(30-5)** 218 mg, yield 46 %. LCMS: m/z= 302.1 (M+H)⁺.

### Step 5: Synthesis of 4a-(3-4-difluorophenyl)hexahydro-2H-benzo[b][1,4]oxazine-3(4H)-one (30-6)

**30-5** (218 mg, 0.72 mmol) was put in a 25 mL round bottom flask. After 5 mL methanol was added for dissolving, sodium borohydride (27 mg, 0.72 mmol) was added slowly at 0°C. The reaction liquid was dried by a rotary evaporator after stirring for 30 mins. After 5 mL THF was added for dissolving, 60% sodium hydride (35 mg, 0.86 mmol) was added under ice bath and reacted for 10 hours. The reaction liquid was quenched with saturated saline, extracted with ethyl acetate, separated and purified by silica gel column chromatography to obtain a white solid of 4*a-*(3,4-difluorophenyl)hexahydro-2*H*-benzo[*b*] [1,4]oxazine-3(4*H*)- one **(30-6)** 88 mg, yield 48.3 %. LCMS: m/z= 268.1 (M+H)⁺.

### Step 6: Synthesis of 4a-(3,4-difluorophenyl)octahydro-2H-benzo[b][1,4]oxazine hydrochloride (Compound 30)

Under nitrogen protection, **30-6** (88 mg, 0.33 mmol) was dissolved in ultra-dry THF (5 mL), added with a solution of borane-methyl sulfide in THF (1.6 mL, 3.3 mmol, 2.0 M), refluxed at 70°C for 12 hours and quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product of Compound **30**.

The crude product of Compound **30** was separated and purified by HPLC. The purified product was concentrated, then added with a small amount of hydrochloric acid, and lyophilized to obtain a white solid of 4*a*-(3,4-difluorophenyl)octahydro-2*H*-benzo[*b*][1,4]oxazine hydrochloride 42 mg, yield 50 %. LCMS: m/z= 254.1 (M+H)⁺.

¹H NMR (400 MHz, chloroform-d) δ 10.45 (s, 1H), 9.88 (s, 1H), 8.06 (dd, J = 12.2, 7.6 Hz, 1H), 7.86 (s, 1H), 7.34 - 7.19 (m, 1H), 4.38 (s, 1H), 4.19 (d, J = 8.1 Hz, 1H), 4.09 (d, J = 10.7 Hz, 1H), 3.01 (d, J = 46.4 Hz, 2H), 2.83 - 2.70 (m, 1H), 2.28 (t, J = 11.3 Hz, 1H), 2.07 - 1.86 (m, 2H), 1.81 - 1.70 (m, 1H), 1.68 - 1.50 (m, 2H), 1.10 - 0.92 (m, 1H).

### Synthesis of Compound 31

### Synthesis of 6,6-dimethyl-4a-phenyloctahydro-2H-benzo[b][1,4]oxazine hydrochloride (Compound 31)

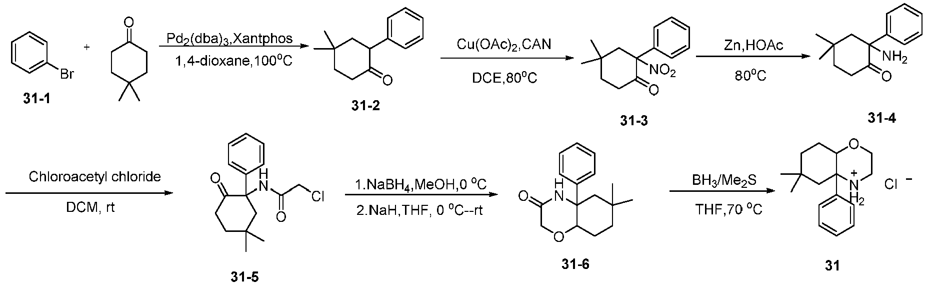

### Step 1: Synthesis of 4,4-dimethyl-2-phenylcyclohexanone (31-2)

Under nitrogen protection, Pd₂(dba)₃ (292 mg, 0.32 mmol), Xantphos (370 mg, 0.64 mmol) and cesium carbonate (22.8 g, 70.2 mmol) were dissolved in ultra-dry 1,4-dioxane (30 mL), added with bromobenzene (5 g, 31.9 mmol) and 4,4-dimethylcyclohexanone (8.04 g, 63.7 mmol), and heated at 100°C for 20 hours. The reaction liquid was extracted with EA and water after being cooled. The organic layer was dried with anhydrous sodium sulfate, concentrated, separated and purified by silica gel column chromatography to obtain 3.64 g yellow oil of 4,4-dimethyl-2-phenylcyclohexanone (**31-2**), yield 56.4 %.

### Step 2: Synthesis of 4,4-dimethyl-2-nitro-2-phenylcyclohexanone(31-3)

**31-2** (3.64 g, 18 mmol) was dissolved in DCE (40 mL), added with copper acetate (1.63 g, 9 mmol) and cerium ammonium nitrate (24.7 g, 45 mmol), and heated at 80°C for 12 hours. The reaction liquid was filtered, washed with EA, concentrated, separated and purified by silica gel column chromatography to obtain 1.65 g yellow oil of 4,4-dimethyl-2-nitro-2-phenylhexyl-1-one **(31-3)**, yield 37 %.

### Step 3: Synthesis of 4,4-dimethyl-2-amino-2-phenylcyclohexanone (31-4)

Under nitrogen protection, **31-4** (1.65 g, 6.67 mmol) was dissolved in acetic acid (15 mL), added with zinc powder (2.1 g, 33.4 mmol), and heated at 80°C for 12 hours. After being cooled, the reaction liquid was adjusted to pH> 10 with a 2 M solution of sodium hydroxide and extracted with ethyl acetate. Then the organic phase was dried with anhydrous sodium sulfate, concentrated, separated and purified by silica gel column chromatography to obtain a colorless oil of 4,4-dimethyl-2-amino-2-phenylcyclohexanone **(31-4)** 221 mg, yield 15.2 %. LCMS: m/z= 218.1 (M+H)⁺

### Step 4: Synthesis of 2-chloro-N-(5,5-dimethyl-2-oxo-1-phenylhexyl)acetamide (31-5)

Under nitrogen protection, **31-4** (221 mg, 1.02 mmol) was dissolved in ultra-dry DCM (5 mL), added with ultra-dry triethylamine (0.2 mL, 1.12 mmol). Chloroacetyl chloride (81 µL, 1.02 mmol) was added dropwise at 0°C and stirred at room temperature for 1 hour until the raw material disappeared by analysis of TLC. The reaction liquid was concentrated directly, separated and purified by silica gel column chromatography to obtain a white solid of 2-chloro-N-(5,5-dimethyl-2-oxo-1-phenylhexyl)acetamide **(31-5)** 168 mg, yield 56 %. LCMS: m/z= 294.1 (M+H)⁺.

### Step 5: Synthesis of 6,6-dimethyl-4a-phenylhexahydro-2H-benzo[b][1,4]oxazine-3(4H)-one (31-6)

**31-5** (168 mg, 0.57 mmol) was put in a 25 mL round bottom flask. After 3 mL methanol was added for dissolving, sodium borohydride (43 mg, 1.14 mmol) was added slowly at 0°C. The reaction liquid was dried by a rotary evaporator after stirring for 30 mins. After 5 mL THF was added for dissolving, 60% sodium hydride (46 mg, 1.14 mmol) was added under ice bath and reacted for 10 hours. The reaction liquid was quenched with saturated saline, extracted with ethyl acetate. The organic phase was concentrated, separated and purified by silica gel column chromatography to obtain a white solid of 6,6-dimethyl-4*a*-phenylhexahydro-2*H-*benzo[b][1,4]oxazine-3(4H)-one **(31-6)** 113 mg, yield 76.3 %. LCMS: m/z= 260.1 (M+H)⁺.

### Step 6: Synthesis of 6,6-dimethyl-4a-phenyloctahydro-2H-benzo[b][1,4]oxazine hydrochloride (Compound 31)

Under nitrogen protection, **31-6** (113 mg, 0.44 mmol) was dissolved in ultra-dry THF (5 mL), added with a solution of borane-methyl sulfide in THF (2.2 mL, 4.4 mmol, 2.0 M) and refluxed at 70°C for 12 hours. The reaction was quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product of Compound **31**.

The crude product of Compound **31** was separated and purified by HPLC. The purified product was concentrated, then added with a small amount of hydrochloric acid, and lyophilized to obtain a white solid of 6,6-dimethyl-4*a*-phenyloctahydro-2*H*-benzo[*b*][1,4]oxazine hydrochloride (Compound **31**) 44 mg, yield 40.7 %. LCMS: m/z= 246.2 (M+H)⁺.

¹H NMR (400 MHz, chloroform-d) δ 10.22 (s, 1H), 9.64 (s, 1H), 8.10 (s, 2H), 7.50 (s, 1H), 7.36 (d, J = 16.6 Hz, 2H), 4.38 (t, J = 12.2 Hz, 1H), 4.16 (dd, J = 12.6, 3.8 Hz, 1H), 3.99 (dd, J = 12.6, 3.2 Hz, 1H), 3.02 - 2.88 (m, 1H), 2.88 - 2.75 (m, 1H), 2.66 - 2.56 (m, 1H), 2.29 - 2.17 (m, 2H), 1.94 - 1.86 (m, 1H), 1.58 (td, J = 13.4, 3.9 Hz, 1H), 1.49 - 1.39 (m, 1H), 0.94 (s, 3H), 0.24 (s, 3H).

### Synthesis of Compound 32

### Synthesis of 4a-(2-chloro-5-fluorophenyl)octahydro-2H-benzo[b][1,4]oxazine hydrochloride (Compound 32)

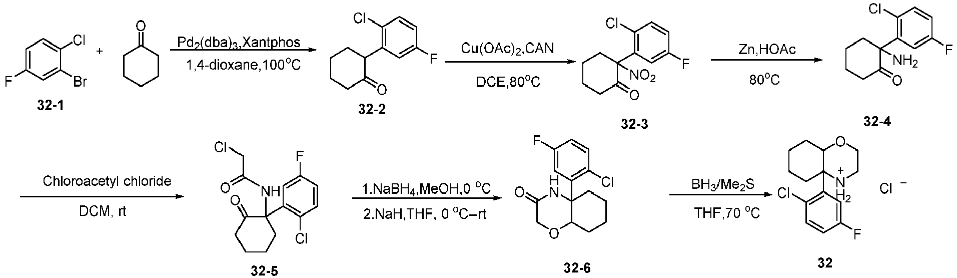

### Step 1: Synthesis of 2-(2-chloro-5-fluorophenyl)cyclohexanone (32-2)

Under nitrogen protection, Pd₂(dba)₃ (349 mg, 0.38 mmol), Xantphos (419 mg, 0.76 mmol) and cesium carbonate (27 g, 84.04 mmol) were dissolved in ultra-dry 1,4-dioxane (50 mL), added with 2-chloro-5-fluorobromobenzene (8 g, 38.20 mmol) and cyclohexanone (7.5 g, 76.40 mmol) and heated at 100°C for 20 hours. The reaction liquid was extracted with EA and water after being cooled. The organic layer was dried with anhydrous sodium sulfate, concentrated, separated and purified by silica gel column chromatography to obtain 5.58 g yellow oil of 2-(2-chloro-5-fluorophenyl)cyclohexanone **(32-2)**, yield 64.4%. LCMS: m/z= 227.1 (M+H)⁺.

### Step 2: Synthesis of 2-(2-chloro-5-fluorophenyl)-2-nitrocyclohexanone (32-3)

**32-2** (5.31 g, 23.43 mmol) was dissolved in DCE (50 mL), added with copper acetate (2.1 g, 11.71 mmol) and cerium ammonium nitrate (32 g, 58.6 mmol), and heated at 80°C for 12 hours, The reaction liquid was filtered, washed with EA, concentrated, separated and purified by silica gel column chromatography to obtain 2.7 g yellow oil of 2-(2-chloro-5-fluorophenyl)-2-nitrocyclohexanone **(32-3)**, yield 42 %.

### Step 3: Synthesis of 2-(2-chloro-5-fluorophenyl)-2-aminocyclohexanone (32-4)

Under nitrogen protection, **32-3** (2.7 g, 9.94 mmol) was dissolved in acetic acid (30 mL), added with zinc powder (3.23 g, 49.7 mmol), and heated at 80°C for 12 hours. After being cooled, the reaction liquid was adjusted to pH> 10 with a 2 M solution of sodium hydroxide and extracted with ethyl acetate. Then the organic phase was dried with anhydrous sodium sulfate, concentrated, separated and purified by silica gel column chromatography to obtain a colorless oil of 2-(2-chloro-5-fluorophenyl)-2-aminocyclohexanone **(32-4)** 658 mg, yield 27.4 %. LCMS: m/z= 242.1 (M+H)⁺.

### Step 4: Synthesis of 2-chloro-N-(1-(2-chloro-5-fluorophenyl)-2-oxocyclohexyl)acetamide (32-5)

Under nitrogen protection, **32-4** (431 mg, 1.78 mmol) was dissolved in ultra-dry DCM (10 mL), added with ultra-dry triethylamine (0.5 mL, 3.57 mmol). Chloroacetyl chloride (284 µL, 3.57 mmol) was added dropwise at 0 °C and stirred at room temperature for 1 hour until the raw material disappeared by analysis of TLC. The reaction liquid was concentrated directly, separated and purified by silica gel column chromatography to obtain a white solid of 2-chloro-N-(1-(2-chloro-5-fluorophenyl)-2-oxocyclohexyl)acetamide **(32-5)** 363 mg, yield 64.1 %. LCMS: m/z= 318.0 (M+H)⁺.

### Step 5: Synthesis of 4a-(2-chloro-5-fluorophenyl)hexahydro-2H-benzo[b][1,4]oxazine-3(4H)-one (32-6)

**32-5** (363 mg, 1.14mmol) was put in a 25 mL round bottom flask. After 5 mL methanol was added for dissolving, sodium borohydride (86 mg, 2.28 mmol) was added slowly at 0°C. The reaction liquid was dried by a rotary evaporator after stirring for 30 mins. After 5 mL THF was added for dissolving, 60 %sodium hydride (91 mg, 2.28 mmol) was added under ice bath and reacted for 10 hours. The reaction liquid was quenched with saturated saline, extracted with ethyl acetate. The organic phase was concentrated, separated and purified by silica gel column chromatography to obtain a white solid of 4*a*-(2-chloro-5-fluorophenyl)hexahydro-2*H-*benzo[*b*][1,4]oxazine-3(4*H*)-one **(32-6)**176 mg, yield 54.5 %. LCMS: m/z= 284.0 (M+H)⁺.

### Step 6: Synthesis of 4a-(2-chloro-5-fluorophenyl)octahydro-2H-benzo[b][1,4]oxazine hydrochloride (Compound 32)

Under nitrogen protection, **32-6** (176 mg, 0.62 mmol) was dissolved in ultra-dry THF (5 mL), added with a solution of borane-methyl sulfide in THF (1.55 mL, 3.1 mmol, 2.0 M), and refluxed at 70°C for 12 hours. The reaction was quenched by adding dropwise a small amount of methanol after being cooled. 2 M hydrochloric acid was added and stirred for 30 minutes at room temperature. After the reaction liquid was neutralized with a 2 M solution of sodium hydroxide, it was extracted with EA. The organic layer was dried with anhydrous sodium sulfate to obtain a crude product of Compound **32**.

The crude product of Compound **32** was separated and purified by HPLC. The purified product was concentrated, then added with a small amount of hydrochloric acid, and lyophilized to obtain a white solid of 4*a*-(2-chloro-5-fluorophenyl)octahydro-2*H*-benzo[*b*][1,4]oxazine hydrochloride 360 mg, yield 82.4 %. LCMS: m/z= 270.1 (M+H)⁺.

¹H NMR (400 MHz, chloroform-d) δ 11.53 (s, 1H), 8.31 (d, J = 7.7 Hz, 1H), 8.03 (dd, J = 11.0, 2.8 Hz, 2H), 7.43 (dd, J = 8.8, 5.6 Hz, 1H), 7.07 (ddd, J = 9.1, 7.0, 2.9 Hz, 1H), 4.61 - 4.44 (m, 2H), 3.99 (dd, J = 12.4, 3.0 Hz, 1H), 3.47 (dd, J = 28.5, 13.0 Hz, 2H), 2.87 (q, J = 10.3 Hz, 1H), 2.31 - 2.21 (m, 1H), 2.12 - 2.01 (m, 2H), 1.75 (d, J = 12.1 Hz, 1H), 1.68 - 1.52 (m, 2H), 0.89 (q, J = 14.1 Hz, 1H).19F NMR (376 MHz, Chloroform-d) δ -112.59 (dt, J = 11.5, 6.2 Hz).

### Synthesis of Compound 33

### Synthesis of 4α-(3-ethoxyphenyl)octahydro-2H-benzo[b][1,4]oxazine hydrochloride (Compound 33)

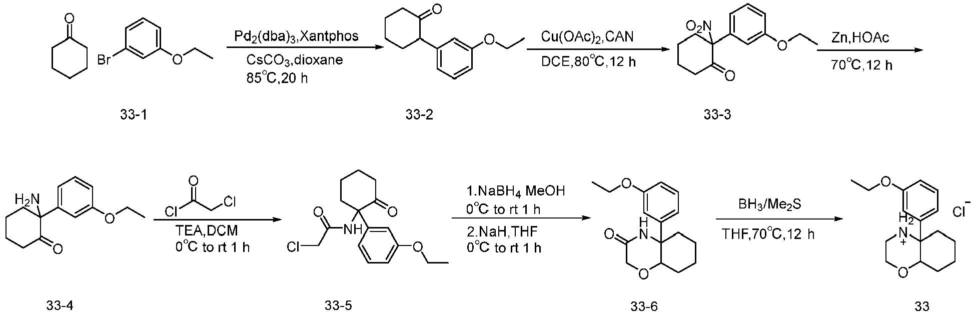

### Step 1: Synthesis of 2-(3-ethoxyphenyl)-1-cyclohexanone (33-2)

Under nitrogen protection, Pd₂(dba)₃ (229 mg, 0.25 mmol), Xantphos (289 mg, 0.50 mmol), cesium carbonate (17.8 g, 54.80 mmol) were dissolved in ultra-dry dioxane 30 mL, added with **33-1** (5 g, 24.90 mmol), cyclohexanone (4.9 g, 49.80 mmol) and stirred at 85°C for 20 hours. After the reaction liquid was cooled to room temperature, it was filtered with diatomite, extracted with EA, dried with anhydrous sodium sulfate, and separated by flash silica gel chromatography to obtain 2.79 g light yellow liquid of 2-(3-ethoxyphenyl)-1-cyclohexanone **(33-2)**, yield 51.4%. LCMS: m/z=219.1(M+H)⁺.

### Step 2: Synthesis of 2-(3-ethoxyphenyl)-2-nitrocyclohexanone (33-3)

Under nitrogen protection, cerium ammonium nitrate (21.0 g, 38.34 mmol), copper acetate (2.3 g, 12.78 mmol), **33-2** (2.79 g, 12.78 mmol) were dissolved in 1,2-dichloroethane (40 mL), stirred at 80°C for 12 hours and diluted with DCM after being cooled to room temperature. The reaction liquid was filtered with diatomite, washed with DCM, separated and purified by flash silica chromatography after concentration to obtain 1.03 g light yellow solid of 2-(3-ethoxyphenyl)-2-nitrocyclohexanone **(33-3)**, yield 30.7%.

### Step 3: Synthesis of 2-(3-ethoxyphenyl)-2-aminocyclohexanone (33-4)

Under nitrogen protection, **33-3** (1.0 g, 3.80 mmol) was dissolved in 10 mL methanol and added with 10 mL icy acetic acid. 1.24 g zinc powder was added slowly and stirred at 80°C for 12 hours. The reaction liquid was adjusted to pH of 10 by adding 2 M NaOH, extracted with EA. The organic phase was dried with anhydrous sodium sulfate, separated and purified by flash silica gel column chromatography to obtain 603 mg yellow liquid of 2-(3-ethoxyphenyl)-2-aminocyclohexanone **(33-4)**, yield 68.1%. LCMS: m/z=234.1 (M+H)⁺.

### Step 4: Synthesis of 2-chloro-N-(1-(3-ethoxyphenyl)-2-oxocyclohexyl)acetamide (33-5)

Under nitrogen protection, **33-4** (600 mg, 2.57 mmol) was dissolved in 10 mL ultra-dry DCM, added with triethylamine (312 mg, 3.09 mmol), cooled to 0°C, added dropwise with chloroacetyl chloride (290 mg, 2.57 mmol), stirred at room temperature for 1 hour, and extracted with EA. The organic phase was dried with anhydrous sodium sulfate, separated and purified by flash silica gel column chromatography after concentration to obtain 533 mg colorless solid of 2-chloro-N-(1-(3-ethoxyphenyl)-2-oxocyclohexyl)acetamide **(33-5)**, yield 67.1%. LCMS: m/z=310.1(M+H)⁺.

### Step 5: Synthesis of 4α-(3-ethoxyphenyl)hexahydro-2H-benzo[b][1,4]oxazin-3(4H)-one (33-6)

Under nitrogen protection, **33-5** (500 mg, 1.62 mmol) was dissolved in 8 mL ultra-dry methanol, cooled to 0°C, added with sodium borohydride (61 mg, 1.62 mmol), warmed to room temperature and stirred for 1 hour with rotary evaporating solvents under reduced pressure, added with 10 mL ultra-dry THF for dissolving, cooled to 0°C, added with 60% sodium hydride (78 mg, 1.94 mmol), warmed to room temperature and stirred for 12 hours. The reaction was quenched by adding 1 N HCl and extracted with DCM. The organic phase was dried with anhydrous sodium sulfate, separated and purified by flash silica gel column chromatography after concentration to obtain 410 mg white solid of 4*α*-(3-ethoxyphenyl)hexahydro-2*H*-benzo[*b*][1,4]oxazin-3(4*H*)-one **(33-6)**, yield 92.1%. LCMS: m/z=276.1(M+H)⁺.

### Step 6: Synthesis of 4α-(3-ethoxyphenyl)octahydro-2H-benzo[b][1,4]oxazine hydrochloride (33)

Under nitrogen protection, **33-6** (374 mg, 1.36 mmol) was dissolved in 12 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution and extracted with EA. The organic phase was dried with anhydrous sodium sulfate, then dried with a rotary evaporator under reduced pressure. The product was dissolved in a small amount of MeOH, separated and purified by HPLC, then added with 1 N HCl and lyophilized to obtain 142 mg white powder of 4*α*-(3-ethoxyphenyl)octahydro-2*H*-benzo[*b*][1,4]oxazine hydrochloride (33), yield 35.2%. LCMS: m/z=262.2(M+H)⁺.

¹H NMR (400 MHz, chloroform-d) δ 10.38 (s, 1H), 9.74 (s, 1H), 7.63 (d, *J =* 8.1 Hz, 2H), 7.34 (t, *J* = 8.1 Hz, 1H), 6.90 (d, *J* = 8.1 Hz, 1H), 4.42 (td, *J* = 11.9, 4.2 Hz, 1H), 4.26 - 4.00 (m, 4H), 3.00 (s, 2H), 2.79 (d, *J* = 12.8 Hz, 1H), 2.24 (t, *J* = 12.3 Hz, 1H), 2.10 (ddt, *J* = 16.4, 12.4, 6.3 Hz, 1H), 1.97 (d, *J* = 11.2 Hz, 1H), 1.73 (d, *J* = 11.1 Hz, 1H), 1.56 (d, *J* = 13.5 Hz, 2H), 1.43 (t, *J* = 6.9 Hz, 3H), 1.04 (q, *J* = 13.7 Hz, 1H).

### Synthesis of Compound 34

### Synthesis of 4α-(2-chloro-4-methoxyphenyl)octahydro-2H-benzo[b][1,4]oxazine hydrochloride (Compound 34)

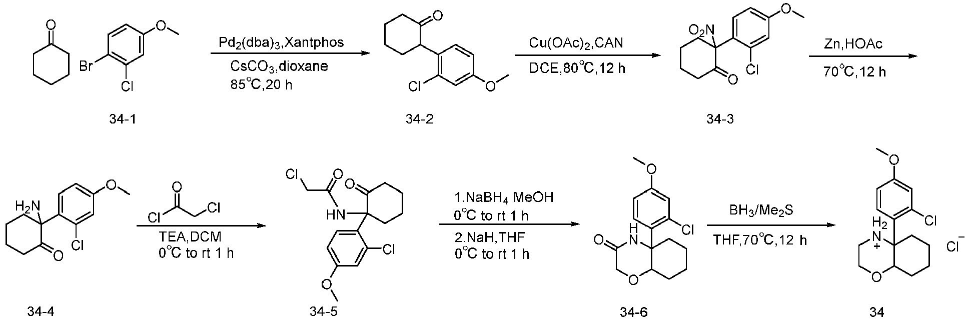

### Step 1: Synthesis of 2-(2-chloro-4-methoxyphenyl)cyclohexanone (34-2)

Under nitrogen protection, Pd₂(dba)₃ (201 mg, 0.22 mmol), Xantphos (260 mg, 0.45 mmol), cesium carbonate (16.2 g, 49.72 mmol) were dissolved in ultra-dry dioxane 30 mL, added with **34-1** (5 g, 22.60 mmol), cyclohexanone (4.4 g, 45.20 mmol) and stirred at 85°C for 20 hours. After the reaction liquid was cooled to room temperature, it was filtered with diatomite, extracted with EA, dried with anhydrous sodium sulfate, separated by flash silica gel chromatography to obtain 2.13 g yellow white solid of 2-(2-chloro-4-methoxyphenyl)cyclohexanone **(34-2)**, yield 30.7%. LCMS: m/z=239.1(M+H)⁺.

### Step 2: Synthesis of 2-(2-chloro-4-methoxyphenyl)-2-nitrocyclohexanone (34-3)

Under nitrogen protection, cerium ammonium nitrate (11.2 g, 20.40 mmol), copper acetate (1.2 g, 6.80 mmol), **34-2**(1.6 g, 6.80 mmol) were dissolved in 1,2-dichloroethane (20 mL), stirred at 80°C for 12 hours and diluted with DCM after being cooled to room temperature. The reaction liquid was filtered with diatomite, washed with DCM, separated and purified by flash silica chromatography after concentration to obtain 819 mg yellow liquid of 2-(2-chloro-4-methoxyphenyl)-2-nitrocyclohexanone **(34-3)**, yield 42.2%.

### Step 3: Synthesis of 2-(2-chloro-4-methoxyphenyl)-2-aminocyclohexanone (34-4)

Under nitrogen protection, **34-3** (819 mg, 2.90 mmol) was dissolved in 8 mL methanol and added with 8 mL icy acetic acid. 940 mg zinc powder was added slowly and stirred at 80°C for 12 hours. The reaction liquid was adjusted to pH of 10 by adding 2 M NaOH and extracted with EA. The organic phase was dried with anhydrous sodium sulfate, separated and purified by flash silica gel column chromatography to obtain 477 mg yellow liquid of 2-(2-chloro-4-methoxyphenyl)-2-aminocyclohexanone **(34-4)**, yield 65.1%. LCMS: m/z=254.1 (M+H)⁺.

### Step 4: Synthesis of 2-chloro-N-(1-(2-chloro-4-methoxyphenyl)-2-oxocyclohexyl)acetamide (34-5)

Under nitrogen protection, **34-4** (470 mg, 1.86 mmol) was dissolved in 8 mL ultra-dry DCM, added with triethylamine (226 mg, 2.23 mmol), cooled to 0°C, added dropwise with chloroacetyl chloride (210 mg, 1.86 mmol), stirred at room temperature for 1 hour and extracted with EA. The organic phase was dried with anhydrous sodium sulfate, separated and purified by flash silica gel column chromatography after concentration to obtain 129 mg yellow white solid of 2-chloro-N-(1-(2-chloro-4-methoxyphenyl)-2-oxocyclohexyl)acetamide **(34-5)**, yield 21.0%. LCMS: m/z=330.0(M+H)⁺.

### Step 5: Synthesis of 4α-(2-chloro-4-methoxyphenyl)hexahydro-2H-benzo[b][1,4]oxazin-3(4H)-one (34-6)

Under nitrogen protection, **34-5** (120 mg, 0.36 mmol) was dissolved in 5 mL ultra-dry methanol, cooled to 0°C, added with sodium borohydride (14 mg, 0.36 mmol), warmed to room temperature and stirred for 1 hour with rotary evaporating solvents under reduced pressure. 5 mL ultra-dry THF was added for dissolving, cooled to 0°C, added with 60% sodium hydride (18 mg, 0.43 mmol), heated to room temperature and stirred for 12 hours. The reaction was quenched by adding 1 N HCl and extracted with DCM. The organic phase was dried with anhydrous sodium sulfate, separated and purified by flash silica gel column chromatography after concentration to obtain 48 mg white solid of 4*α*-(2-chloro-4-methoxyphenyl)hexahydro-2*H*-benzo[*b*][1,4]oxazin-3(4*H*)-one **(34-6)**, yield 45.3%. LCMS: m/z=296.1(M+H)⁺.

### Step 6: Synthesis of 4α-(2-chloro-4-methoxyphenyl)octahydro-2H-benzo[b][1,4]oxazine hydrochloride (34)

Under nitrogen protection, **34-6** (48 mg, 0.16 mmol) was dissolved in 3 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution and extracted with EA. The organic phase was dried with anhydrous sodium sulfate, then dried with a rotary evaporator under reduced pressure. The product was dissolved in a small amount of MeOH, separated and purified by HPLC, then added with 1 N HCl and lyophilized to obtain 34 mg white powder of 4*α*-(2-chloro-4-methoxyphenyl)octahydro-2*H*-benzo[*b*][1,4]oxazine hydrochloride **(34)**, yield 66.9%. LCMS: m/z=282.1(M+H)⁺.

¹H NMR (400 MHz, chloroform-d) δ 11.56 (s, 1H), 8.15 (d, *J* = 9.1 Hz, 1H), 7.79 (s, 1H), 7.00 (d, *J* = 2.3 Hz, 1H), 6.86 (dd, *J* = 9.1, 2.3 Hz, 1H), 4.62 - 4.45 (m, 2H), 3.97 (d, *J* = 10.7 Hz, 1H), 3.84 (s, 3H), 3.47 - 3.33 (m, 2H), 2.93 (q, *J* = 10.9, 10.1 Hz, 1H), 2.25 (t, *J* = 13.8 Hz, 1H), 2.08 (d, *J* = 17.0 Hz, 2H), 1.74 (d, *J* = 10.9 Hz, 1H), 1.60 (d, *J* = 13.8 Hz, 2H), 0.94 (q, *J* = 13.7 Hz, 1H).

### Synthesis of Compound 35

### Synthesis of (4aR,8aS)-4α-(2-thiophenyl)octahydro-2H-benzo[b][1,4]oxazine hydrochloride (Compound 35)

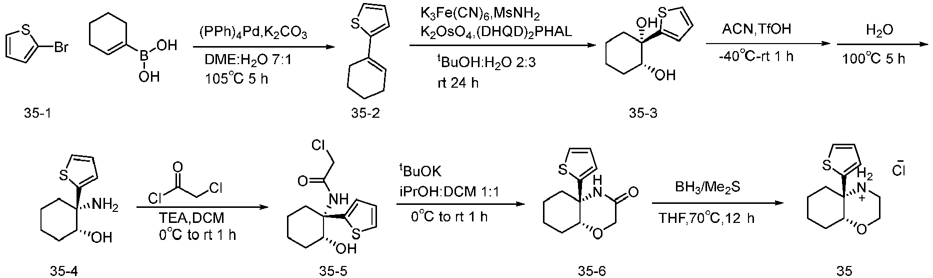

### Step 1: Synthesis of 2-(cyclohexen-1-yl)thiophene (35-2)

Under nitrogen protection, potassium carbonate (3.4 g, 24.54 mmol) was dissolved in a mixed solution of glycol dimethyl ether: water (35: 5 mL), added with **35-1** (2 g, 12.27 mmol), 1-cyclohexenyl boronic acid (1.6 g, 12.27 mmol), tetrakis(triphenylphosphine)palladium (570 mg, 0.49 mmol), and refluxed at 105°C for 5 hours. After being cooled to room temperature, the reaction liquid was extracted by adding water and EA, dried with anhydrous sodium sulfate, separated by flash silica gel chromatography to obtain 1.1 g colorless liquid of 2-(cyclohexen-1-yl)thiophene **(35-2)**, yield 54.7%. LCMS: m/z=165.1(M+H)⁺.

### Step 2: Synthesis of (1S,2R)-1-(2-thiophenyl)hexane-1,2-diol (35-3)

Water (9.37 mL) was added in the flask. Potassium ferricyanide (6.6 g, 20.07 mmol), anhydrous potassium carbonate (2.8 g, 20.07 mmol), methanesulfonamide (636 mg, 6.69 mmol), potassium osmate dihydrate (1.2 mg, 0.003 mmol), (DHQD)₂PHAL(13 mg, 0.017 mmol), **35-2** (1.1 g, 6.69 mmol) and tert-butanol (6.25 mL) were added in sequence and stirred vigorously for 2 days. The product was dissolved by adding EA and filtered. The aqueous layer was separated from the filtrate. The organic layer was added with 2 M NaOH, vigorously shaken to remove methanesulfonamide, dried with anhydrous sodium sulfate, separated by flash silica gel chromatography to obtain 1.05 g yellow white solid of (1S,2R)-1-(2-thiophenyl)hexane-1,2-diol **(35-3)**, yield 79.1%. LCMS: m/z=181.1(M-OH)⁺.

### Step 3: Synthesis of (1R,2S)-2-amino-2-(2-thiophenyl)hexane-1-ol (35-4)

Under nitrogen protection, **35-3** (1.0 g, 10.24 mmol) was dissolved in 8 mL acetonitrile and cooled to -40°C. Trifluoromethanesulfonic acid (1.5 g, 10.24 mmol) was added slowly, warmed to room temperature and stirred for 1 hour, then added with 8 mL water and stirred for 10 mins. After being heated to 100°C, the remaining aqueous solution was refluxed at 100°C for 5 h. After being cooled to room temperature, the layer was separated by adding DCM. The organic layer was discarded. The aqueous phase was adjusted to pH of 13 with 50% NaOH under ice bath, extracted with EA, dried with anhydrous sodium sulfate, and concentrated to obtain 220 mg yellow white solid of (1R,2S)-2-amino-2-(2-thiophenyl)hexane-1-ol **(35-4)**, yield 21.8%. LCMS: m/z=181.1⁺, 183.0⁺.

### Step 4: Synthesis of 2-chloro-N-((1S,2R)-2-hydroxyl-1-(2-thiophenyl)hexyl)acetamide (35-5)

Under nitrogen protection, **35-4** (220 mg, 1.12 mmol) was dissolved in 5 mL ultra-dry DCM, added with triethylamine (136 mg, 1.34 mmol), cooled to 0°C, added dropwise chloroacetyl chloride (126 mg, 1.12 mmol), stirred at room temperature for 1 hour and extracted with EA. The organic phase was dried with anhydrous sodium sulfate, separated and purified by flash silica gel column chromatography after concentration to obtain 173 mg light yellow liquid of 2-chloro-N-((1S,2R)-2-hydroxyl-1-(2-thiophenyl)hexyl)acetamide **(35-5)**, yield 56.7%. LCMS: m/z=274.0(M+H)⁺.

### Step 5: Synthesis of (4aS,8aR)-4α-(2-thiophenyl)hexahydro-2H-benzo[b][1,4]oxazin-3(4H)-one (35-6)

Under nitrogen protection, **35-5** (173 mg, 0.63 mmol) was dissolved in 1 mL ultra-dry DCM, cooled to 0°C, added with isopropyl alcohol (1 mL), potassium tert-butoxide (233 mg, 2.08 mmol), warmed to room temperature and stirred for 1 hour. The reaction liquid was adjusted to pH of 7 by adding 2 M HCl and extracted with EA. The organic phase was dried with anhydrous sodium sulfate, concentrated, then separated by flash silica gel chromatography to obtain 117 mg white solid of (4aS,8aR)-4*α*-(2-thiophenyl)hexahydro-2*H*-benzo[*b*][1,4]oxazin-3(4*H*)-one **(35-6)**, yield 78.5%. LCMS: m/z=238.1(M+H)⁺.

### Step 6: Synthesis of (4aR,8aS)-4α-(2-thiophenyl)octahydro-2H-benzo[b][1,4]oxazine hydrochloride (35)

Under nitrogen protection, **35-6** (117 mg, 0.49 mmol) was dissolved in 4 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then added with 1 N HCl and lyophilized to obtain 58 mg white powder of (4aR,8aS)-4*α*-(2-thiophenyl)octahydro-2*H*-benzo[*b*][1,4]oxazine hydrochloride **(35)**, yield 46.0%. LCMS: m/z=224.1(M+H)⁺.

¹H NMR (400 MHz, chloroform-d) δ 9.96 (s, 1H), 9.57 (s, 1H), 7.46 (d, *J* = 3.3 Hz, 1H), 7.38 (d, *J* = 5.0 Hz, 1H), 7.08 - 7.01 (m, 1H), 4.31 (s, 1H), 4.09 - 3.86 (m, 2H), 3.20 - 3.00 (m, 1H), 2.58 - 2.36 (m, 3H), 1.92 - 1.40 (m, 5H), 1.39 - 1.30 (m, 1H).

### Synthesis of Compound 36

### Synthesis of 4a-(2-chloro-6-fluorophenyl)octahydro-2H-benzo[b][1,4]oxazine hydrochloride (Compound 36)

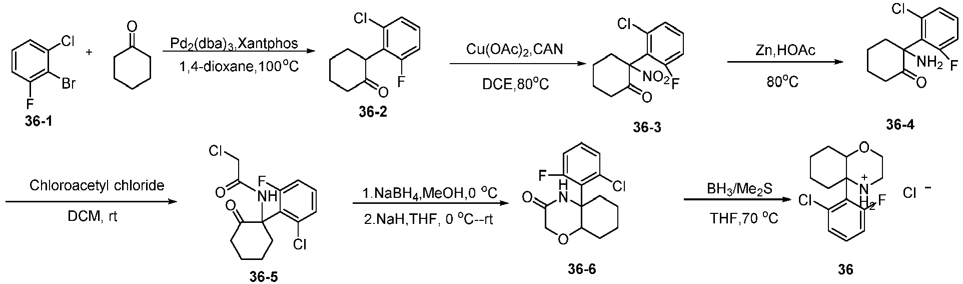

### Step 1: Synthesis of 2-(2-chloro-5-fluorophenyl)cyclohexanone (36-2)

Under nitrogen protection, Pd₂(dba)₃ (437 mg, 0.478 mmol), Xantphos (553 mg, 0.955 mmol) and cesium carbonate (34 g, 105 mmol) were dissolved in ultra-dry 1,4-dioxane (50 mL), added with 2-chloro-6-fluorobromobenzene (10 g, 47.75 mmol) and cyclohexanone (9.4 g, 95.5 mmol), heated at 100°C for 20 hours. The reaction liquid was extracted with EA and water after being cooled. The organic layer was dried with anhydrous sodium sulfate, concentrated, separated and purified by silica gel column chromatography to obtain 4.57 g yellow oil of 2-(2-chloro-6-fluorophenyl)cyclohexanone **(36-2)**, yield 42 %.

### Step 2: Synthesis of 2-(2-chloro-6-fluorophenyl)-2-nitrocyclohexanone (36-3)

**36-2** (4.57 g, 20.2 mmol) was dissolved in DCE (40 mL), added with copper acetate (1.83 g, 10.1 mmol) and cerium ammonium nitrate (27.7 g, 50.5 mmol), and heated at 80°C for 12 hours. The reaction liquid was filtered, washed with EA, concentrated, separated and purified by silica gel column chromatography to obtain 2.3 g yellow oil of 2-(2-chloro-6-fluorophenyl)-2-nitrocyclohexanone **(36-3)**, yield 42 %.

### Step 3: Synthesis of 2-(2-chloro-6-fluorophenyl)-2-aminocyclohexanone (36-4)

Under nitrogen protection, **36-3** (2.3 g, 8.5 mmol) was dissolved in acetic acid (20 mL), added with zinc powder (2.7 g, 42.3 mmol), and heated at 80°C for 12 hours. After being cooled, the reaction liquid was adjusted to pH> 10 with a 2 M solution of sodium hydroxide, extracted with ethyl acetate. Then the organic phase was dried with anhydrous sodium sulfate, concentrated, separated and purified by silica gel column chromatography to obtain a colorless oil of 2-(2-chloro-6-fluorophenyl)-2-aminocyclohexanone **(36-4)** 471 mg, yield 23 %. LCMS: m/z= 242.1 (M+H)⁺.

### Step 4: Synthesis of 2-chloro-N-(1-(2-chloro-6-fluorophenyl)-2-oxocyclohexyl)acetamide (36-5)

Under nitrogen protection, **36-4** (471 mg, 1.95 mmol) was dissolved in ultra-dry DCM (10 mL), added with ultra-dry triethylamine (0.3 mL, 1.95 mmol). Chloroacetyl chloride (155 µL, 1.95 mmol) was added dropwise at 0 °C, stirred at room temperature for 1 hour until the raw material disappeared by analysis of TLC. The reaction liquid was concentrated directly, separated and purified by silica gel column chromatography to obtain a white solid of 2-chloro-N-(1-(2-chloro-6-fluorophenyl)-2-oxocyclohexyl)acetamide **(36-5)** 542 mg, yield 87.4 %. LCMS: m/z= 318.0 (M+H)⁺.

### Step 5: Synthesis of 4a-(2-chloro-6-fluorophenyl)hexahydro-2H-benzo[b][1,4]oxazine-3(4H)-one (36-6)

**36-5** (542 mg, 1.7mmol) was put in a 25 mL round bottom flask. After 5 mL methanol was added for dissolving, sodium borohydride (129 mg, 3.41 mmol) was added slowly at 0°C. The reaction liquid was dried by a rotary evaporator after stirring for 30 mins. After 5 mL THF was added for dissolving, 60 % sodium hydride (136 mg, 3.41 mmol) was added under ice bath and reacted for 10 hours. The reaction liquid was quenched with saturated saline, extracted with ethyl acetate. The organic phase was concentrated, separated and purified by silica gel column chromatography to obtain a white solid of 4*a*-(2-chloro-6-fluorophenyl)hexahydro-2*H-*benzo[*b*][1,4]oxazine-3(4*H*)-one **(36-6)** 326 mg, yield 67.6 %. LCMS: m/z= 284.1 (M+H)⁺.

### Step 6: Synthesis of 4a-(2-chloro-6-fluorophenyl)octahydro-2H-benzo[b][1,4]oxazine hydrochloride (Compound 36)

Under nitrogen protection, **36-6** (326 mg, 1.15 mmol) was dissolved in ultra-dry THF (10 mL), added with a solution of borane-methyl sulfide in THF (2.9 mL, 5.74 mmol, 2.0 M), and refluxed at 70°C for 12 hours. The reaction was quenched by adding dropwise a small amount of methanol. After being cooled, the reaction liquid was added with 2 M hydrochloric acid and stirred for 30 minutes at room temperature. After the reaction liquid was neutralized with a 2 M solution of sodium hydroxide, it was extracted with EA. The organic layer was dried with anhydrous sodium sulfate to obtain a crude product of Compound **36**.

The crude product of Compound **36** was separated and purified by HPLC. The purified product was concentrated, then added with a small amount of hydrochloric acid and lyophilized to obtain a white solid of 4*a*-(2-chloro-6-fluorophenyl)octahydro-2*H*-benzo[*b*][1,4]oxazine hydrochloride 225 mg, yield 72.6 %. LCMS: m/z= 270.1 (M+H)⁺.

¹H NMR (400 MHz, chloroform-d) δ 11.58 (s, 1H), 8.03 (s, 1H), 7.43 - 7.33 (m, 2H), 7.15 (dd, J = 12.3, 8.4 Hz, 1H), 4.54 (t, J = 12.3 Hz, 2H), 3.95 (d, J = 10.9 Hz, 1H), 3.67 (d, J = 12.1 Hz, 1H), 3.35 (d, J = 13.4 Hz, 1H), 2.93 - 2.75 (m, 1H), 2.38 (t, J = 12.8 Hz, 1H), 2.22 - 2.08 (m, 1H), 2.07 - 1.97 (m, 1H), 1.77 - 1.51 (m, 3H), 0.85 (q, J = 13.1 Hz, 2H).

### Synthesis of Compound 37

### Synthesis of (4aS,8aS)-4α-(2-chloro-3-thiophenyl)octahydro-2H-benzo[b][1,4]oxazine hydrochloride (Compound 37)

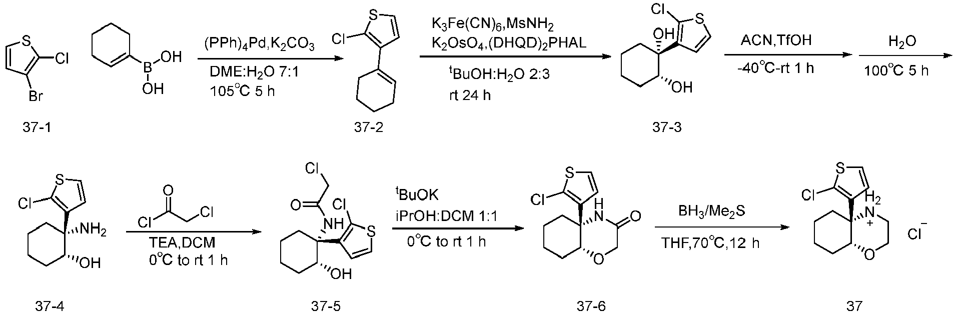

### Step 1: Synthesis of 2-chloro-3-(cyclohexen-1-yl)thiophene (37-2)

Under nitrogen protection, potassium carbonate (4.2 g, 30.30 mmol) was dissolved in a mixed solution of glycol dimethyl ether: water (35: 7 mL), added with **37-1** (2 g, 10.10 mmol), 1-cyclohexenyl boronic acid (1.4 g, 11.2 mmol), tetrakis(triphenylphosphine)palladium (583 mg, 0.50 mmol), and refluxed at 105°C for 5 hours. After being cooled to room temperature, the reaction liquid was extracted by adding water and EA, dried with anhydrous sodium sulfate, separated by flash silica gel chromatography to obtain 1.78 g colorless liquid of 2-chloro-3-(cyclohexen-1-yl)thiophene **(37-2),** yield 89.0%.

### Step 2: Synthesis of (1R,2R)-1-(2-chloro-3-thiophenyl)hexane-1,2-diol (37-3)

Water (12.5 mL) was added in the flask. Potassium ferricyanide (8.9 g, 26.97 mmol), anhydrous potassium carbonate (3.7 g, 26.97 mmol), methanesulfonamide (855 mg, 8.99 mmol), potassium osmate dihydrate (1.7 mg, 0.005 mmol), (DHQD)₂PHAL (18 mg, 0.023 mmol), **37-2** (1.7 g, 8.99mmol) and tert-butanol (8.3 mL) were added in sequence, stirred vigorously for 2 days. The product was dissolved by adding EA and filtered. The aqueous layer was separated from the filtrate. The organic layer was added with 2 M NaOH, vigorously shaken to remove methanesulfonamide, dried with anhydrous sodium sulfate, separated by flash silica gel chromatography to obtain 910 mg white solid of (1R,2R)-1-(2-chloro-3-thiophenyl)hexane-1,2-diol **(37-3),** yield 43.5%. LCMS: m/z=215.0 (M-OH)⁺.

### Step 3: Synthesis of (1R,2R)-2-amino-2-(2-chloro-3-thiophenyl)hexane-1-ol (37-4)

Under nitrogen protection, **37-3** (910 mg, 3.92 mmol) was dissolved in 6 mL acetonitrile and cooled to -40°C. Trifluoromethanesulfonic acid (1.2 g, 7.84 mmol) was added slowly, warmed to room temperature and stirred for 1 hour, then added with 6 mL water and stirred for 10 mins. After being heated to 100°C, the remaining aqueous solution was refluxed at 100°C for 5 h. After being cooled to room temperature, the layer was separated by adding DCM. The organic layer was discarded. The aqueous phase was adjusted to pH of 13 with 50% NaOH under ice bath, extracted with EA, dried with anhydrous sodium sulfate and concentrated to obtain 649 mg white viscous liquid (1R,2R)-2-amino-2-(2-chloro-3-thiophenyl)hexane-1-ol **(37-4),** yield 71.4%. LCMS: m/z=232.0(M+H)⁺.

### Step 4: Synthesis of 2-chloro-N-((1R,2R)-1-(2-chloro-3-thiophenyl)-2-hydroxylhexyl)acetamide (37-5)

Under nitrogen protection, **37-4** (649 mg, 2.80 mmol) was dissolved in 12 mL ultra-dry DCM, added with triethylamine (340 mg, 3.36 mmol), cooled to 0°C, added dropwise with chloroacetyl chloride (317 mg, 2.80 mmol), stirred at room temperature for 1 hour, extracted with EA. The organic phase was dried with anhydrous sodium sulfate, separated and purified by flash silica gel column chromatography after concentration to obtain 534 mg light yellow liquid of 2-chloro-N-((1R,2R)-1-(2-chloro-3-thiophenyl)-2-hydroxylhexyl)acetamide **(37-5),** yield 61.9%. LCMS: m/z=308.0(M+H)⁺.

### Step 5: Synthesis of (4aR,8aR)-4α-(2-chloro-3-thiophenyl)hexahydro-2H-benzo[b][1,4]oxazin-3(4H)-one (37-6)

Under nitrogen protection, **37-5** (534 mg, 1.73 mmol) was dissolved in 4 mL ultra-dry DCM, cooled to 0°C, added with isopropyl alcohol (4 mL), potassium tert-butoxide (776 mg, 6.92 mmol), heated to room temperature and stirred for 1 hour. The reaction liquid was adjusted to pH of 7 by adding 2 M HCl and extracted with EA. The organic phase was dried with anhydrous sodium sulfate, concentrated, then separated by flash silica gel chromatography to obtain 286 mg white solid of (4aR,8aR)-4*α*-(2-chloro-3-thiophenyl)hexahydro-2*H*-benzo[*b*][1,4]oxazin-3(4*H*)-one **(37-6),** yield 61.1%. LCMS: m/z=274.0(M+H)⁺.

### Step 6: Synthesis of (4aS,8aS)-4α-(2-chloro-3-thiophenyl)octahydro-2H-benzo[b][1,4]oxazine hydrochloride (37)

Under nitrogen protection, **37-6** (286 mg, 1.05 mmol) was dissolved in 5 mL ultra-dry THF, added with borane/dimethyl sulfide, and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then added with 1 N HCl and lyophilized to obtain 198 mg white powder of (4aS,8aS)-4*α*-(2-chloro-3-thiophenyl)octahydro-2*H*-benzo[*b*][1,4]oxazine hydrochloride **(37),** yield 73.3%. LCMS: m/z=258.0(M+H)⁺.

¹H NMR (400 MHz, chloroform-d) δ 9.83 (s, 1H), 9.60 (s, 1H), 7.29 (d, *J* = 5.9 Hz, 1H), 7.15 (d, *J* = 6.0 Hz, 1H), 4.80 (s, 1H), 3.96 (d, *J* = 6.9 Hz, 2H), 3.19 (dq, *J* = 18.6, 9.5 Hz, 1H), 2.80 (d, *J* = 12.9 Hz, 1H), 2.64 (s, 1H), 2.43 (td, *J* = 12.9, 3.5 Hz, 1H), 1.77 - 1.68 (m, 1H), 1.65 - 1.50 (m, 2H), 1.42 - 1.19 (m, 2H).

### Synthesis of Compound 38

### Synthesis of (4aS,8aR)-4α-(2-thiophenyl)octahydro-2H-benzo[b][1,4]oxazine hydrochloride (Compound 38)

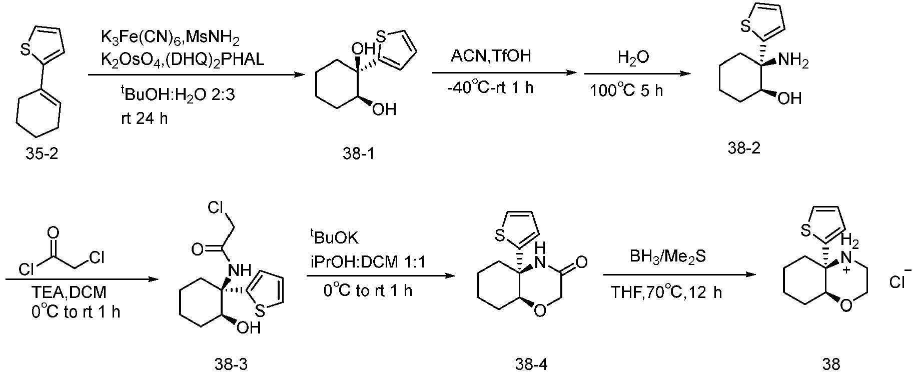

### Step 1: Synthesis of (1R,2S)-1-(2-thiophenyl)hexane-1,2-diol (38-1)

Water was added in the flask (9.37 mL). Potassium ferricyanide (6.6 g, 20.07 mmol), anhydrous potassium carbonate (2.8 g, 20.07 mmol), methanesulfonamide (636 mg, 6.69 mmol), potassium osmate dihydrate (1.2 mg, 0.003 mmol), (DHQ)₂PHAL (13 mg, 0.017 mmol), **35-2**(1.1 g, 6.69 mmol) and tert-butanol (6.25 mL) were added in sequence and stirred vigorously for 1 day. The product was dissolved by adding EA and filtered. The aqueous layer was separated from the filtrate. The organic layer was added with 2 M NaOH, vigorously shaken to remove methanesulfonamide, dried with anhydrous sodium sulfate, separated by flash silica gel chromatography to obtain 1.16 g white solid of (1R,2S)-1-(2-thiophenyl)hexane-1,2-diol **(38-1),** yield 87.8%. LCMS: m/z=181.1(M-OH)⁺.

### Step 2: Synthesis of (1S,2R)-2-amino-2-(2-thiophenyl)hexane-1-ol (38-2)

Under nitrogen protection, **38-1** (1.2 g, 5.85 mmol) was dissolved in 9 mL acetonitrile and cooled to -40°C. Trifluoromethanesulfonic acid (1.7 g, 11.70 mmol) was added slowly, heated to room temperature and stirred for 1 hour, then added with 9 mL water and stirred for 10 mins. After being heated to 100°C, the remaining aqueous solution was refluxed at 100°C for 5 h. After being cooled to room temperature, the layer was separated by adding DCM. The organic layer was discarded. The aqueous phase was adjusted to pH of 13 with 50% NaOH under ice bath, extracted with EA, dried with anhydrous sodium sulfate and concentrated to obtain 300 mg light yellow powder of (1S,2R)-2-amino-2-(2-thiophenyl)hexane-1-ol **(38-2),** yield 26.0%. LCMS: m/z=181.1⁺.

### Step 3: Synthesis of 2-chloro-N-((1R,2S)-2-hydroxyl-1-(2-thiophenyl)hexyl)acetamide (38-3)

Under nitrogen protection, **38-2** (300 mg, 1.52 mmol) was dissolved in 5 mL ultra-dry DCM, added with triethylamine (184 mg, 1.82 mmol), cooled to 0°C, added dropwise with chloroacetyl chloride (172 mg, 1.52 mmol), stirred at room temperature for 1 hour and extracted with EA. The organic phase was dried with anhydrous sodium sulfate, separated and purified by flash silica gel column chromatography after concentration to obtain 101 mg yellow liquid of 2-chloro-N-((1R,2S)-2-hydroxyl-1-(2-thiophenyl)hexyl)acetamide **(38-3),** yield 24.4%. LCMS: m/z=274.0(M+H)⁺.

### Step 4: Synthesis of (4aR,8aS)-4α-(2-thiophenyl)hexahydro-2H-benzo[b][1,4]oxazin-3(4H)-one (38-4)

Under nitrogen protection, **38-3** (101 mg, 0.37 mmol) was dissolved in 1 mL ultra-dry DCM, cooled to 0°C, added with isopropyl alcohol (1 mL), potassium tert-butoxide (166 mg, 1.48 mmol), warmed to room temperature and stirred for 1 hour. The reaction liquid was adjusted to pH of 7 by adding 2 M HCl and extracted with EA. The organic phase was dried with anhydrous sodium sulfate, concentrated, then separated by flash silica gel chromatography to obtain 46 mg white solid of (4aR,8aS)-4*α*-(2-thiophenyl)hexahydro-2*H*-benzo[*b*][1,4]oxazin-3(4*H*)-one **(38-4),** yield 52.9%. LCMS: m/z=238.1(M+H)⁺.

### Step 5: Synthesis of (4aS,8aR)-4α-(2-thiophenyl)octahydro-2H-benzo[b][1,4]oxazine hydrochloride (38)

Under nitrogen protection, **38-4** (48 mg, 0.20 mmol) was dissolved in 1 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then added with 1 N HCl and lyophilized to obtain 23 mg white powder of (4aR,8aS)-4*α*-(2-thiophenyl)octahydro-2*H*-benzo[*b*][1,4]oxazine hydrochloride **(38),** yield 52.9%. LCMS: m/z=224.1(M+H)⁺.

¹H NMR (400 MHz, chloroform-*d*) δ 9.95 (s, 1H), 9.55 (s, 1H), 7.41 (dd, *J* = 31.2, 4.3 Hz, 2H), 7.04 (t, *J* = 4.2 Hz, 1H), 4.29 (s, 1H), 3.95 (dd, *J* = 37.8, 12.1 Hz, 2H), 3.09 (s, 1H), 2.60 - 2.33 (m, 3H), 1.90 - 1.30 (m, 6H).

### Synthesis of Compound 39

### Synthesis of (4aR,8aS)-4α-(3-methyl-2-thiophenyl)octahydro-2H-benzo[b][1,4]oxazine hydrochloride (Compound 39)

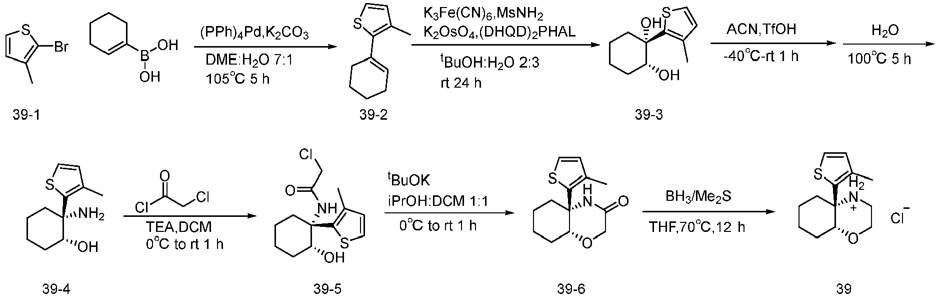

### Step 1: Synthesis of 2-(cyclohexen-1-yl)-3-methyl thiophene (39-2)

Under nitrogen protection, potassium carbonate (3.1 g, 22.60 mmol) was dissolved in a mixed solution of glycol dimethyl ether: water (35: 5 mL), added with **39-1** (2 g, 11.30 mmol), 1-cyclohexenyl boronic acid (1.4 g, 11.30 mmol), tetrakis(triphenylphosphine)palladium (519 mg, 0.45 mmol), refluxed at 105°C for 5 hours. After being cooled to room temperature, the reaction liquid was extracted by adding water and EA, dried with anhydrous sodium sulfate, separated by flash silica gel chromatography to obtain 2.2 g colorless liquid of 2-(cyclohexen-1-yl)-3-methyl thiophene **(39-2),** yield 100%.

### Step 2: Synthesis of (1S,2R)-1-(3-methyl-2-thiophenyl)hexane-1,2-diol (39-3)

Water (18.0 mL) was added in the flask. Potassium ferricyanide (12.6 g, 38.19 mmol), anhydrous potassium carbonate (5.3 g, 38.19 mmol), methanesulfonamide (1.2 g, 12.73 mmol), potassium osmate dihydrate (23 mg, 0.063 mmol), (DHQD)₂PHAL (247 mg, 0.32 mmol), **39-2**(2.2 g, 12.73 mmol) and tert-butanol (12.0 mL) were added in sequence, stirred vigorously overnight. The product was dissolved by adding EA and filtered. The aqueous layer was separated from the filtrate. The organic layer was added with 2 M NaOH, vigorously shaken to remove methanesulfonamide, dried with anhydrous sodium sulfate, separated by flash silica gel chromatography to obtain 2.6 g white solid of (1S,2R)-1-(3-methyl-2-thiophenyl)hexane-1,2-diol **(39-3),** yield 95.9%. LCMS: m/z=195.1(M-OH)⁺.

### Step 3: Synthesis of (1R,2S)-2-amino-2-(3-methyl-2-thiophenyl)hexane-1-ol (39-4)

Under nitrogen protection, **39-3** (1.0 g, 4.71 mmol) was dissolved in 8 mL acetonitrile and cooled to -40°C. Trifluoromethanesulfonic acid (1.4 g, 9.43 mmol) was added slowly, warmed to room temperature and stirred for 1 hour, then added with 8 mL water and stirred for 10 mins. After being heated to 100°C, the remaining aqueous solution was refluxed at 100°C for 5 h. After being cooled to room temperature, the layer was separated by adding DCM. The organic layer was discarded. The aqueous phase was adjusted to pH of 13 with 50% NaOH under ice bath, extracted with EA, dried with anhydrous sodium sulfate, and concentrated to obtain 190 mg light yellow solid of (1R,2S)-2-amino-2-(3-methyl-2-thiophenyl)hexane-1-ol **(39-4),** yield 19.1%. LCMS: m/z=195.0⁺.

### Step 4: Synthesis of 2-chloro-N-((1S,2R)-2-hydroxyl-1-(3-methyl-2-thiophenyl)hexyl)acetamide (39-5)

Under nitrogen protection, **39-4** (190 mg, 0.90 mmol) was dissolved in 4 mL ultra-dry DCM, added with triethylamine (109 mg, 1.08 mmol), cooled to 0°C, added dropwise with chloroacetyl chloride (101 mg, 0.90 mmol), stirred at room temperature for 1 hour, extracted with EA. The organic phase was dried with anhydrous sodium sulfate, separated and purified by flash silica gel column chromatography after concentration to obtain 116 mg light yellow oily compound of 2-chloro-N-((1S,2R)-2-hydroxyl-1-(3-methyl-2-thiophenyl)hexyl)acetamide **(39-5),** yield 45.0%. LCMS: m/z=310.0(M+Na)⁺.

### Step 5: Synthesis of (4aS,8aR)-4α-(3-methyl-2-thiophenyl)hexahydro-2H-benzo[b][1,4]oxazin-3(4H)-one (39-6)

Under nitrogen protection, **39-5** (116 mg, 0.40 mmol) was dissolved in 1 mL ultra-dry DCM, cooled to 0°C, added with isopropyl alcohol (1 mL), potassium tert-butoxide (134 mg, 1.20 mmol), heated to room temperature and stirred for 1 hour. The reaction liquid was adjusted to pH of 7 by adding 2 M HCl and extracted with EA. The organic phase was dried with anhydrous sodium sulfate, concentrated, then separated by flash silica gel chromatography to obtain 52 mg light yellow solid of (4aS,8aR)-4*α*-(3-methyl-2-thiophenyl)hexahydro-2*H*-benzo[*b*][1,4]oxazin-3(4*H*)-one **(39-6),** yield 52.0%. LCMS: m/z=252.0(M+H)⁺.

### Step 6: Synthesis of (4aR,8aS)-4α-(3-methyl-2-thiophenyl)octahydro-2H-benzo[b][1,4]oxazine hydrochloride (39)

Under nitrogen protection, **39-6** (52 mg, 0.20 mmol) was dissolved in 2 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then added with 1 N HCl and lyophilized to obtain 28 mg white powder of (4aR,8aS)-4*α*-(3-methyl-2-thiophenyl)octahydro-2*H*-benzo[*b*][1,4]oxazine hydrochloride **(39),** yield 59.5%. LCMS: m/z=238.1(M+H)⁺.

¹H NMR (400 MHz, chloroform-d) δ 9.85 (s, 1H), 9.40 (s, 1H), 7.27 (s, 1H), 6.77 (d, *J* = 4.9 Hz, 1H), 4.04 - 3.87 (m, 2H), 3.16 (d, *J* = 12.4 Hz, 1H), 2.62 - 2.44 (m, 6H), 1.87 - 1.52 (m, 5H), 1.50 - 1.34 (m, 2H).

### Synthesis of Compound 40

Synthesis of (4aR,8aR)-4a-(4-methyl-3-thiophenyl)octahydro-2H-benzo[b][1,4]oxazine hydrochloride (Compound **40**)

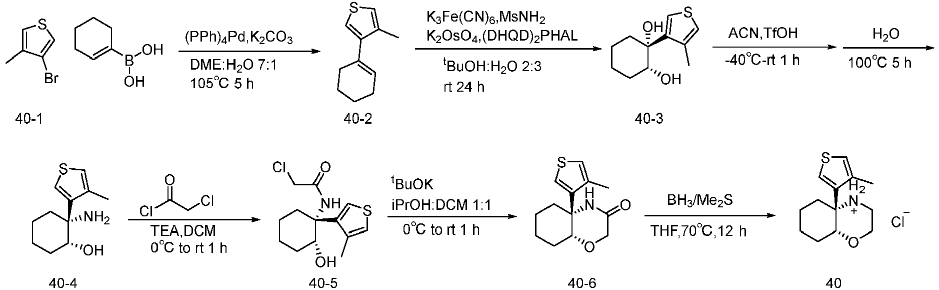

### Step 1: Synthesis of 3-(cyclohexen-1-yl)-4-methyl thiophene (40-2)

Under nitrogen protection, potassium carbonate (3.1 g, 22.60 mmol) was dissolved in a mixed solution of glycol dimethyl ether: water (35: 5 mL), added with **40-1** (2 g, 11.30 mmol), 1-cyclohexenyl boronic acid (1.4 g, 11.30 mmol), tetrakis(triphenylphosphine)palladium (519 mg, 0.45 mmol) and refluxed at 105°C for 5 hours. After being cooled to room temperature, the reaction liquid was extracted by adding water and EA, dried with anhydrous sodium sulfate, separated by flash silica gel chromatography to obtain 1.9 g colorless liquid of 3-(cyclohexen-1-yl)-4-methyl thiophene **(40-2),** yield 93.0%.

### Step 2: Synthesis of (1R,2R)-1-(4-methyl-3-thiophenyl)hexane-1,2-diol (40-3)

Water (15.0 mL) was added in the flask. Potassium ferricyanide (10.4 g, 31.46 mmol), anhydrous potassium carbonate (4.4 g, 31.46 mmol), methanesulfonamide (997 mg, 10.48 mmol), potassium osmate dihydrate (19 mg, 0.052 mmol), (DHQD)₂PHAL (204 mg, 0.26 mmol), **40-2**(1.9 g, 10.48 mmol) and tert-butanol (10.0 mL) were added in sequence and stirred vigorously overnight. The product was dissolved by adding EA and filtered. The aqueous layer was separated from the filtrate. The organic layer was added with 2 M NaOH, vigorously shaken to remove methanesulfonamide, dried with anhydrous sodium sulfate, separated by flash silica gel chromatography to obtain 2.0 g colorless oily compound of (1R,2R)-1-(4-methyl-3-thiophenyl)hexane-1,2-diol **(40-3),** yield 87.4%. LCMS: m/z=195.1(M-OH)⁺.

### Step 3: Synthesis of (1R,2R)-2-amino-2-(4-methyl-3-thiophenyl)hexane-1-ol (40-4)

Under nitrogen protection, **40-3** (1.0 g, 4.71 mmol) was dissolved in 8 mL acetonitrile and cooled to -40°C. Trifluoromethanesulfonic acid (1.4 g, 9.43 mmol) was added slowly, heated to room temperature and stirred for 1 hour, then added with 8 mL water and stirred for 10 mins. After being heated to100°C, the remaining aqueous solution was refluxed at 100°C for 5 h. After being cooled to room temperature, the layer was separated by adding DCM. The organic layer was discarded. The aqueous phase was adjusted to pH of 13 with 50% NaOH under ice bath, extracted with EA, dried with anhydrous sodium sulfate and concentrated to obtain 214 mg colorless viscous liquid of (1R,2R)-2-amino-2-(4-methyl-3-thiophenyl)hexane-1-ol **(40-4),** yield 21.5%. LCMS: m/z=195.1⁺.

### Step 4: Synthesis of 2-chloro-N-((1R,2R)-2-hydroxyl-1-(4-methyl-3-thiophenyl)hexyl)acetamide (40-5)

Under nitrogen protection, **40-4** (214 mg, 1.01 mmol) was dissolved in 5 mL ultra-dry DCM, added with triethylamine (122 mg, 1.21 mmol), cooled to 0°C, added dropwise chloroacetyl chloride (114 mg, 1.01 mmol), stirred at room temperature for 1 hour and extracted with EA. The organic phase was dried with anhydrous sodium sulfate, separated and purified by flash silica gel column chromatography after concentration to obtain 177 mg colorless oily compound of 2-chloro-N-((1R,2R)-2-hydroxyl-1-(4-methyl-3-thiophenyl)hexyl)acetamide **(40-5),** yield 61.2%. LCMS: m/z=288.0(M+H)⁺.

### Step 5: Synthesis of (4aR,8aR)-4α-(4-methyl-3-thiophenyl)hexahydro-2H-benzo[b][1,4]oxazin-3(4H)-one (40-6)

Under nitrogen protection, **40-5** (177 mg, 0.61 mmol) was dissolved in 2 mL ultra-dry DCM, cooled to 0°C, added with isopropyl alcohol (2 mL), potassium tert-butoxide (206 mg, 1.83 mmol), warmed to room temperature and stirred for 1 hour. The reaction liquid was adjusted to pH of 7 by adding 2 M HCl and extracted with EA. The organic phase was dried with anhydrous sodium sulfate, concentrated, then separated by flash silica gel chromatography to obtain 81 mg white solid of (4aR,8aR)-4*α*-(4-methyl-3-thiophenyl)hexahydro-2*H*-benzo[*b*][1,4]oxazin-3(4*H*)-one **(40-6),** yield 52.9%. LCMS: m/z=252.1(M+H)⁺.

### Step 6: Synthesis of (4aR,8aR)-4α-(4-methyl-3-thiophenyl)octahydro-2H-benzo[b][1,4]oxazine hydrochloride (40)

Under nitrogen protection, **40-6** (81 mg, 0.32 mmol) was dissolved in 2 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then added with 1 N HCl and lyophilized to obtain 46 mg white powder of (4aR,8aR)-4*α*-(4-methyl-3-thiophenyl)octahydro-2*H*-benzo[*b*][1,4]oxazine hydrochloride **(40),** yield 61.3%. LCMS: m/z=238.1(M+H)⁺.

¹H NMR (400 MHz, chloroform-d) δ 9.59 (s, 1H), 9.29 (s, 1H), 7.63 (d, *J* = 3.4 Hz, 1H), 6.94 (d, *J* = 3.3 Hz, 1H), 4.55 (s, 1H), 3.93 (td, *J* = 13.4, 12.8, 9.6 Hz, 2H), 3.18 - 3.04 (m, 1H), 2.52 (d, *J* = 30.7 Hz, 6H), 2.00 (s, 1H), 1.70 (d, *J* = 11.6 Hz, 1H), 1.63 - 1.49 (m, 2H), 1.41 - 1.24 (m, 2H).

### Synthesis of Compound 41

Compound 41 and Compound 42 cannot be obtained separately by passing racemic Compound 9 through chiral column or resolving racemic Compound 9 by SFC. It can be obtained using the following chiral synthesis method.

### Synthesis of (4aR,8aR)-4α-(3-chlorophenyl)octahydro-2H-benzo[b][1,4]oxazine hydrochloride (Compound 41)

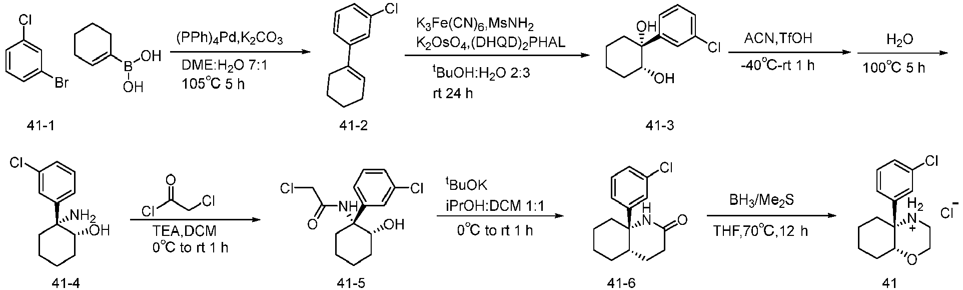

### Step 1: Synthesis of 3'-chloro-2,3,4,5-tetrahydro-1,1'-biphenyl (41-2)

Under nitrogen protection, potassium carbonate (5.8 g, 41.80 mmol) was dissolved in a mixed solution of glycol dimethyl ether: water (70: 10 mL), added with **41-1** (4 g, 20.90 mmol), 1-cyclohexenyl boronic acid (2.6 g, 20.90 mmol), tetrakis(triphenylphosphine)palladium (965 mg, 0.83 mmol), refluxed at 105°C for 5 hours. After being cooled to room temperature, the reaction liquid was extracted by adding water and EA, dried with anhydrous sodium sulfate, separated by flash silica gel chromatography to obtain 3.0 g colorless liquid of 3'-chloro-2,3,4,5-tetrahydro-1,1'-biphenyl **(41-2),** yield 74.7%.

### Step 2: Synthesis of (1R,2R)-1-(3-chlorophenyl)hexane-1,2-diol (41-3)

Water (11.0 mL) was added in the flask. Potassium ferricyanide (7.7 g, 23.43 mmol), anhydrous potassium carbonate (3.2 g, 23.43 mmol), methanesulfonamide (743 mg, 7.81 mmol), potassium osmate dihydrate (15 mg, 0.039 mmol), (DHQD)₂PHAL (152 mg, 0.20 mmol), **41-2** (1.5 g, 10.48 mmol) and tert-butanol (7.3 mL) were added in sequence, stirred vigorously overnight. The product was dissolved by adding EA and filtered. The aqueous layer was separated from the filtrate. The organic layer was added with 2 M NaOH, vigorously shaken to remove methanesulfonamide, dried with anhydrous sodium sulfate, separated by flash silica gel chromatography to obtain 756 mg colorless oily compound of (1R,2R)-1-(3-chlorophenyl)hexane-1,2-diol **(41-3),** yield 42.7%. LCMS: m/z=209.0(M-OH)⁺.

### Step 3: Synthesis of (1R,2R)-2-amino-2-(3-chlorophenyl)hexane-1-ol (41-4)

Under nitrogen protection, **41-3** (3.5 g, 15.43 mmol) was dissolved in 26 mL acetonitrile and cooled to -40°C. Trifluoromethanesulfonic acid (4.6 g, 30.86 mmol) was added slowly, warmed to room temperature and stirred for 1 hour, then added with 26 mL water and stirred for 10 mins. After being heated to100°C, the remaining aqueous solution was refluxed at 100°C for 5 h. After being cooled to room temperature, the layer was separated by adding DCM. The organic layer was discarded. The aqueous phase was adjusted to pH of 13 with 50% NaOH under ice bath, extracted with EA, dried with anhydrous sodium sulfate and concentrated to obtain 434 mg white solid of (1R,2R)-2-amino-2-(3-chlorophenyl)hexane-1-ol **(41-4),** yield 12.5%. LCMS: m/z=226.1(M+H)⁺.

### Step 4: Synthesis of 2-chloro-N-((1R,2R)-2-hydroxyl-1-(3-chlorophenyl)hexyl)acetamide (41-5)

Under nitrogen protection, **41-4** (434 mg, 1.93 mmol) was dissolved in 8 mL ultra-dry DCM, added with triethylamine (390 mg, 3.86 mmol), cooled to 0°C, added dropwise with chloroacetyl chloride (239 mg, 2.12 mmol), stirred at room temperature for 1 hour and extracted with DCM. The organic phase was dried with anhydrous sodium sulfate, separated and purified by flash silica gel column chromatography after concentration to obtain 125 mg light yellow oily compound of 2-chloro-N-((1R,2R)-2-hydroxyl-1-(3-chlorophenyl)hexyl)acetamide **(41-5),** yield 21.5%. LCMS: m/z=302.0(M+H)⁺.

### Step 5: Synthesis of (4aR,8aR)-4α-(3-chlorophenyl)hexahydro-2H-benzo[b][1,4]oxazin-3(4H)-one (41-6)

Under nitrogen protection, **41-5** (125 mg, 0.41 mmol) was dissolved in 2 mL ultra-dry DCM, cooled to 0°C, added with isopropyl alcohol (2 mL), potassium tert-butoxide (139 mg, 1.24 mmol), warmed to room temperature and stirred for 1 hour. The reaction liquid was adjusted to pH of 7 by adding 2 M HCl and extracted with EA. The organic phase was dried with anhydrous sodium sulfate, concentrated, then separated by flash silica gel chromatography to obtain 93 mg white solid of (4aR,8aR)-4*α*-(3-chlorophenyl)hexahydro-2*H*-benzo[*b*][1,4]oxazin-3(4*H*)-one **(41-6),** yield 86.1%. LCMS: m/z=266.0(M+H)⁺.

### Step 6: Synthesis of (4aR,8aR)-4α-(3-chlorophenyl)octahydro-2H-benzo[b][1,4]oxazine hydrochloride (41)

Under nitrogen protection, **41-6** (93 mg, 0.35 mmol) was dissolved in 4 mL ultra-dry THF, added with borane/dimethyl sulfide and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution and extracted with EA. The organic phase was dried with anhydrous sodium sulfate, then dried with a rotary evaporator under reduced pressure. The product was dissolved in a small amount of MeOH, separated and purified by HPLC, then added with 1 N HCl and lyophilized to obtain 40 mg white powder of (4aR,8aR)-4*α*-(3-chlorophenyl)octahydro-2*H*-benzo[*b*][1,4]oxazine hydrochloride **(41),** yield 46.0%. LCMS: m/z=252.1(M+H)⁺.

¹H NMR (400 MHz, chloroform-d) δ 9.81 (s, 1H), 9.53 (s, 1H), 7.75 (s, 1H), 7.65 (d, *J* = 7.1 Hz, 1H), 7.36 (d, *J* = 8.9 Hz, 2H), 4.38 (s, 0H), 3.92 (s, 2H), 2.50 (dd, *J* = 49.6, 12.2 Hz, 2H), 1.88 (s, 3H), 1.79 - 1.58 (m, 1H), 1.50 (d, *J* = 13.4 Hz, 1H), 1.42 - 1.17 (m, 2H).

### Synthesis of Compound 42

### Synthesis of (4aS,8aS)-4α-(3-chlorophenyl)octahydro-2H-benzo[b][1,4]oxazine hydrochloride (Compound 42)

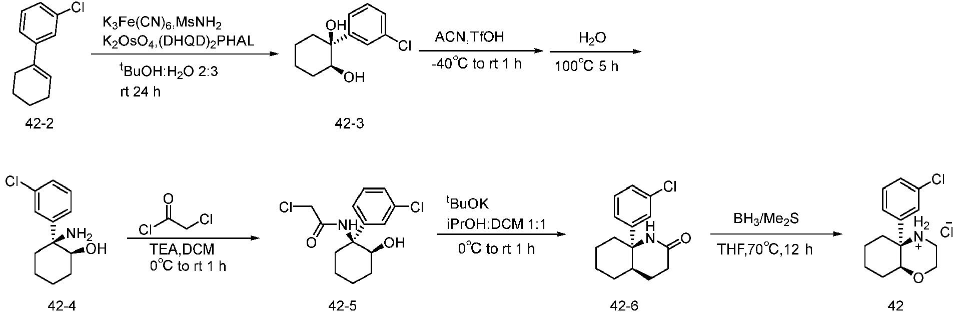

### Step 1: Synthesis of (1S,2S)-1-(3-chlorophenyl)hexane-1,2-diol (42-3)

Water (11.0 mL) was added in the flask. Potassium ferricyanide (7.7 g, 23.43 mmol), anhydrous potassium carbonate (3.2 g, 23.43 mmol), methanesulfonamide (743 mg, 7.81 mmol), potassium osmate dihydrate (15 mg, 0.039 mmol), (DHQ)₂PHAL(152 mg, 0.20 mmol), **42-2** (1.5 g, 10.48 mmol) and tert-butanol (7.3 mL) were added in sequence, stirred vigorously overnight. The product was dissolved by adding EA and filtered. The aqueous layer was separated from the filtrate. The organic layer was added with 2 M NaOH, vigorously shaken to remove methanesulfonamide, dried with anhydrous sodium sulfate, separated by flash silica gel chromatography to obtain 788 mg colorless oily compound of (1S,2S)-1-(3-chlorophenyl)hexane-1,2-diol **(42-3),** yield 44.5%. LCMS: m/z=209.0(M-OH)⁺.

### Step 2: Synthesis of (1S,2S)-2-amino-2-(3-chlorophenyl)hexane-1-ol (42-4)

Under nitrogen protection, **42-3** (3.5 g, 15.43 mmol) was dissolved in 26 mLacetonitrile and cooled to -40°C. Trifluoromethanesulfonic acid (4.6 g, 30.86 mmol) was added slowly, warmed to room temperature and stirred for 1 hour, then added with 26 mL water and stirred for 10 mins. After being heated to 100°C, the remaining aqueous solution was refluxed at 100°C for 5 h. After being cooled to room temperature, the layer was separated by adding DCM. The organic layer was discarded. The aqueous phase was adjusted to pH of 13 with 50% NaOH under ice bath, extracted with EA, dried with anhydrous sodium sulfate and concentrated to obtain 641 mg white solid of (1S,2S)-2-amino-2-(3-chlorophenyl)hexane-1-ol **(42-4),** yield 18.4%. LCMS: m/z=226.1(M+H)⁺.

### Step 3: Synthesis of 2-chloro-N-((1S,2S)-2-hydroxyl-1-(3-chlorophenyl)hexyl)acetamide (42-5)

Under nitrogen protection, **42-4** (641 mg, 2.83 mmol) was dissolved in 12 mL ultra-dry DCM, added with triethylamine (572 mg, 5.66 mmol), cooled to 0°C, added dropwise with chloroacetyl chloride (353 mg, 3.12 mmol), stirred at room temperature for 1 hour, extracted with DCM. The organic phase was dried with anhydrous sodium sulfate, separated and purified by flash silica gel column chromatography after concentration to obtain 116 mg light yellow oily compound of 2-chloro-N-((1S,2S)-2-hydroxyl-1-(3-chlorophenyl)hexyl)acetamide **(42-5),** yield 13.6%. LCMS: m/z=302.0(M+H)⁺.

### Step 4: Synthesis of (4aS,8aS)-4α-(3-chlorophenyl)hexahydro-2H-benzo[b][1,4]oxazin-3(4H)-one (42-6)

Under nitrogen protection, **42-5** (116 mg, 0.38 mmol) was dissolved in 2 mL ultra-dry DCM, cooled to 0°C, added with isopropyl alcohol (2 mL), potassium tert-butoxide (129 mg, 1.24 mmol), heated to room temperature and stirred for 1 hour. The reaction liquid was adjusted to pH of 7 by adding 2 M HCl and extracted with EA. The organic phase was dried with anhydrous sodium sulfate, concentrated, then separated by flash silica gel chromatography to obtain 90 mg white solid of (4aS,8aS)-4*α*-(3-chlorophenyl)hexahydro-2*H*-benzo[*b*][1,4]oxazin-3(4*H*)-one **(42-6),** yield 89.1%. LCMS: m/z=266.0(M+H)⁺.

### Step 5: Synthesis of (4aS,8aS)-4α-(3-chlorophenyl)octahydro-2H-benzo[b][1,4]oxazine hydrochloride (42)

Under nitrogen protection, **42-6** (110 mg, 0.42 mmol) was dissolved in 4 mL ultra-dry THF, added with borane/dimethyl sulfide, and stirred at 70°C for 12 hours. After being cooled to room temperature, the reaction was quenched by adding dropwise MeOH, added with 1 N HCl and stirred for 1 hour. The reaction liquid was adjusted to pH of 8-9 with saturated NaHCO₃ solution, extracted with EA. The organic phase was dried with anhydrous sodium sulfate, then dried with a rotary evaporator under reduced pressure. The product was dissolved in a small amount of MeOH, separated and purified by HPLC, then added with 1 N HCl and lyophilized to obtain 30 mg white powder of (4aS,8aS)-4*α*-(3-chlorophenyl)octahydro-2*H*-benzo[*b*][1,4]oxazine hydrochloride **(42),** yield 36.6%. LCMS: m/z=252.1(M+H)⁺.

¹H NMR (400 MHz, chloroform-d) δ 9.83 (s, 1H), 9.56 (s, 1H), 7.76 (s, 1H), 7.66 (d, *J* = 7.1 Hz, 1H), 7.44 - 7.32 (m, 2H), 4.39 (s, 0H), 3.94 (s, 2H), 3.04 (s, 1H), 2.51 (dd, *J* = 49.1, 11.3 Hz, 2H), 1.94 (s, 2H), 1.78 - 1.58 (m, 1H), 1.51 (d, *J* = 13.3 Hz, 1H), 1.41 - 1.20 (m, 2H).

### Synthesis of Compound 43

Compound 43 and Compound 44 cannot be obtained separately by passing racemic Compound 11 through chiral column or resolving racemic Compound 11 by SFC. It can be obtained using the following chiral synthesis method.

### Synthesis of (4aR,8aR)-4α-(2-chlorophenyl)octahydro-2H-benzo[b][1,4]oxazine hydrochloride (Compound 43)

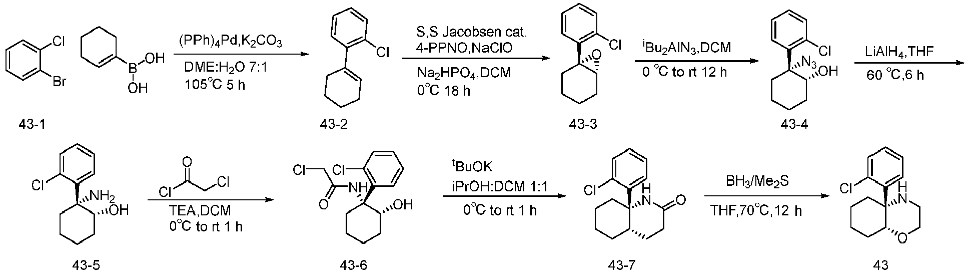

### Step 1: Synthesis of 2'-chloro-2,3,4,5-tetrahydro-1,1'-biphenyl (43-2)

Under nitrogen protection, potassium carbonate (5.8 g, 41.80 mmol) was dissolved in a mixed solution of glycol dimethyl ether: water (70: 10 mL), added with **43-1** (4 g, 20.90 mmol), 1-cyclohexenyl boronic acid (2.6 g, 20.90 mmol), tetrakis(triphenylphosphine)palladium (965 mg, 0.83 mmol), and refluxed at 105°C for 5 hours. After being cooled to room temperature, the reaction liquid was extracted by adding water and EA, dried with anhydrous sodium sulfate, separated by flash silica gel chromatography to obtain 3.3 g colorless liquid of 2'-chloro-2,3,4,5-tetrahydro-1,1'-biphenyl **(43-2),** yield 83.1%.

### Step 2: Synthesis of (1R,6R)-1-(2-chlorophenyl)-7-oxabicyclo[4.1.0]heptane (43-3)

**43-2**(2 g, 10.38 mmol), S,S Jacobsen cat (330 mg, 0.52 mmol) and 4-phenylpyridine-N-oxide (355 mg, 2.08 mmol) were dissolved in 14 mL DCM, cooled to 0 °C, and added with 42 mL precooled bleach buffer (8 mL 11-15% sodium hypochlorite solution and 34 mL 0.05M dibasic sodium phosphate). The reactant was stirred vigorously for 18 hours at 0 °C. After the completion of the reaction, two phases were separated. The aqueous phase was extracted with DCM. The organic phase was combined, washed with saturated saline, dried with anhydrous sodium sulfate, separated by flash silica gel chromatography to obtain 370 mg light yellow liquid of (1R,6R)-1-(2-chlorophenyl)-7-oxabicyclo[4.1.0]heptane **(43-3),** yield 17.1%. LCMS: m/z=209(M+H)⁺.

### Step 3: Synthesis of (1R,2R)-2-azido-2-(2-chlorophenyl)hexane-1-ol (43-4)

Under nitrogen protection, sodium azide (347 mg, 5.34 mmol) was dissolved in DCM (10 mL), cooled to 0 °C, added slowly with diisobutylaluminum chloride (3.3 mL, 2.67 mmol), heated to room temperature and stirred for 12 h. The reaction liquid was cooled to 0°C, added with a solution of **43-3** (370 mg, 1.78 mmol) in DCM (30 mL), warmed to room temperature and continued to react for 3h. The reaction liquid was added with 2 mL saturated sodium bicarbonate solution to quench the reaction and filtered. The filtered cake was washed with DCM. The aqueous phase was extracted with DCM twice. The organic phase was combined, washed with saturated sodium chloride solution, dried with anhydrous sodium sulfate, then dried directly with a rotary evaporator to obtain 400 mg light yellow oily compound of (1R,2R)-2-azido-2-(2-chlorophenyl)hexane-1-ol **(43-4),** yield 89.7%.

### Step 4: Synthesis of (1R,2R)-2-amino-2-(2-chlorophenyl)hexyl-1-ol (43-5)

Under nitrogen protection, **43-4** (575 mg, 2.29 mmol) was dissolved in 15 mL THF, cooled to 0°C, added with lithium aluminum hydride (348 mg, 9.16 mmol), heated to 60°C and refluxed for 12 h. After being cooled to room temperature, the reaction liquid was added dropwise with 2 mL H₂O, 6 mL 15% NaOH solution, 2 mL H₂O in sequence to quench the reaction and filtered with diatomite. The filtered cake was washed with DCM. The filtrate was separated. The aqueous phase was extracted with DCM. The organic phase was combined, dried with anhydrous sodium sulfate, separated by flash silica gel chromatography to obtain 43-5. LCMS: m/z=227(M+H)⁺.

### Step 5: Synthesis of 2-chloro-N-((1R,2R)-1-(2-chlorophenyl)-2-hydroxylhexyl)acetamide (43-6)

Under nitrogen protection, **43-5** (225 mg, 1.00 mmol) was dissolved in 4 mL DCM, added with triethylamine (202 mg, 2.00 mmol), cooled to 0°C and added dropwise with chloroacetyl chloride (124 mg, 1.10 mmol). After the completion of adding dropwise, the reaction was performed at room temperature for 1 h. The reaction liquid was extracted with DCM and water. The organic phase was dried with anhydrous sodium sulfate, separated by flash silica chromatography to obtain 2-chloro-N-((1R,2R)-1-(2-chlorophenyl)-2-hydroxylhexyl)acetamide **(43-6).** LCMS: m/z=303(M+H)⁺.

### Step 6: Synthesis of (4aS,8aS)-8a-(2-chlorophenyl)octahydroquinolin-2(1H)-one(43-7)

Under nitrogen protection, **43-6** (179 mg, 0.59 mmol) was dissolved in 2 mL DCM, cooled to 0°C and added with isopropyl alcohol (2 mL), potassium tert-butoxide (200 mg, 1.77 mmol). After the completion of addition, the reaction liquid was warmed to room temperature, stirred for 1 h, neutralized to pH=7 by adding dropwise 2 M HCl and extracted with EA. The organic phase was washed with saturated saline, dried with anhydrous sodium sulfate, separated by flash silica chromatography to obtain (4aS,8aS)-8a-(2-chlorophenyl)octahydroquinolin-2(1*H*)-one **(43-7).** LCMS: m/z=266(M+H)⁺.

### Step 7: Synthesis of (4aR,8aR)-4a-(2-chlorophenyl)octahydro-2H-benzo[b][1,4]oxazine hydrochloride (43)

Under nitrogen protection, **43-7** (121 mg, 0.46 mmol) was dissolved in 2 mL THF, added dropwise with borane/dimethyl sulfide (2.3mL, 4.6 mmol), and stirred at 70°C for 12 h. After being cooled to room temperature, the reaction was quenched by adding dropwise MeOH, added with 1 N HCl and stirred for 1 h. The reaction liquid was adjusted to pH of 8-9 with saturated sodium bicarbonate solution and extracted with EA. The organic phase was dried with anhydrous sodium sulfate, then dried with a rotary evaporator under reduced pressure, dissolved with a small amount of methanol, then separated and purified by HPLC, added with 1 N HCl and lyophilized to obtain (4aR,8aR)-4a-(2-chlorophenyl)octahydro-2*H*-benzo[*b*][1,4]oxazine hydrochloride **(43).** LCMS: m/z = 252 (M+H)⁺.

### Synthesis of Compound 44

With reference to Compound 43, Compound 44 was obtained by five-step reaction starting with 43-2 using the same route.

### Synthesis of (4aS,8aS)-4α-(2-chlorophenyl)octahydro-2H-benzo[b][1,4]oxazine hydrochloride (Compound 44)

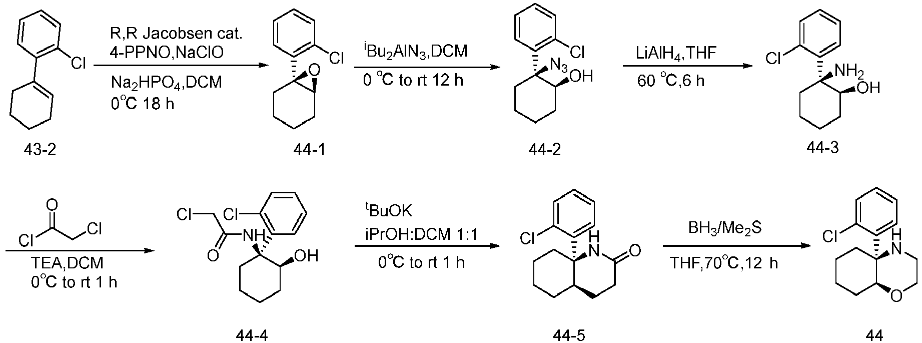

### Step 1: Synthesis of (1S,6S)-1-(2-chlorophenyl)-7-oxabicyclo[4.1.0]heptane (44-1)

**43-2** (2 g, 10.38 mmol), R,R Jacobsen cat (330 mg, 0.52 mmol) and 4-phenylpyridine-N-oxide (355 mg, 2.08 mmol) were dissolved in 14 mL DCM, cooled to 0 °C, added with 42 mL precooled bleach buffer (8 mL 11-15% sodium hypochlorite solution and 34 mL 0.05M dibasic sodium phosphate). The reactant was stirred vigorously for 18 hours at 0 °C. After the completion of the reaction, two phases were separated. The aqueous phase was extracted with DCM. The organic phase was combined, washed with saturated saline, dried with anhydrous sodium sulfate, separated by flash silica gel chromatography to obtain 500 mg light yellow liquid of (1S,6S)-1-(2-chlorophenyl)-7-oxabicyclo[4.1.0]heptane **(44-1),** yield 23.2%. LCMS: m/z=209(M+H)⁺.

### Step 2: Synthesis of (1S,2S)-2-azido-2-(2-chlorophenyl)hexane-1-ol (44-2)

Under nitrogen protection, sodium azide (469 mg, 7.21 mmol) was dissolved in DCM (10 mL), cooled to 0 °C, added slowly with diisobutylaluminum chloride (4.5 mL, 3.60 mmol), warmed to room temperature and stirred for 12 h. The reaction liquid was cooled to 0°C, added with a solution of **44-1** (500 mg, 2.40 mmol) in DCM (30 mL), warmed to room temperature and continued to react for 3h. The reaction liquid was added with 4 mL saturated sodium bicarbonate solution to quench the reaction and filtered. The filtered cake was washed with DCM. The aqueous phase was extracted with DCM twice. The organic phase was combined, washed with saturated sodium chloride solution, dried with anhydrous sodium sulfate, then dried directly with a rotary evaporator to obtain 511 mg dark yellow liquid of (1S,2S)-2-azido-2-(2-chlorophenyl)hexane-1-ol **(44-2),** yield 84.9%.

### Step 3: Synthesis of (1S,2S)-2-amino-2-(2-chlorophenyl)hexyl-1-ol (44-3)

Under nitrogen protection, **44-2** (511 mg, 2.03 mmol) was dissolved in 14 mL THF, cooled to 0°C, added with lithium aluminum hydride (309 mg, 8.14 mmol), heated to 60°C and refluxed overnight. After being cooled to room temperature, the reaction liquid was added dropwise with 2 mL H₂O, 6 mL 15% NaOH solution, 2 mL H₂O in sequence to quench the reaction and filtered with diatomite. The filtered cake was washed with DCM. The filtrate was separated. The aqueous phase was extracted with DCM. The organic phase was combined, dried with anhydrous sodium sulfate, separated by flash silica chromatography to obtain 157 mg light yellow solid of (1S,2S)-2-amino-2-(2-chlorophenyl)hexyl-1-ol **(44-3),** yield 34.4%. LCMS: m/z=227(M+H)⁺.

### Step 4: Synthesis of 2-chloro-N-((1S,2S)-1-(2-chlorophenyl)-2-hydroxylhexyl)acetamide (44-4)

Under nitrogen protection, **44-3** (157 mg, 0.70 mmol) was dissolved in 4 mL DCM, added with triethylamine (142 mg, 1.40 mmol), cooled to 0°C and added dropwise with chloroacetyl chloride (87 mg, 0.77 mmol). After the completion of adding dropwise, the reaction was performed at room temperature for 1 h. The reaction liquid was extracted with DCM and water. The organic phase was dried with anhydrous sodium sulfate, separated by flash silica chromatography to obtain 179 mg light yellow viscous liquid of 2-chloro-N-((1S,2S)-1-(2-chlorophenyl)-2-hydroxylhexyl)acetamide **(44-4),** yield 84.8%. LCMS: m/z=303(M+H)⁺.

### Step 5: Synthesis of (4aR,8aR)-8a-(2-chlorophenyl)octahydroquinolin-2(1H)-one (44-5)

Under nitrogen protection, **44-4** (179 mg, 0.59 mmol) was dissolved in 2 mL DCM, cooled to 0°C, added with isopropyl alcohol (2 mL), potassium tert-butoxide (200 mg, 1.77 mmol). After the completion of addition, the reaction liquid was warned to room temperature, stirred for 1 h, neutralized to pH=7 by adding dropwise 2 M HCl and extracted with EA. The organic phase was washed with saturated saline, dried with anhydrous sodium sulfate, separated by flash silica chromatography to obtain 121 mg white solid of (4aR,8aR)-8a-(2-chlorophenyl)octahydroquinolin-2(1*H*)-one **(44-5),** yield 91.6%. LCMS: m/z=266 (M+H)⁺.

### Step 6: Synthesis of (4aS,8aS)-4a-(2-chlorophenyl)octahydro-2H-benzo[b][1,4]oxazine hydrochloride (44)

Under nitrogen protection, **44-5** (121 mg, 0.46 mmol) was dissolved in 2 mL THF, added dropwise with borane/dimethyl sulfide (2.3mL, 4.6 mmol), and stirred at 70°C for 12 h. After being cooled to room temperature, the reaction was quenched by adding dropwise MeOH, added with 1 N HCl and stirred for 1 h. The reaction liquid was adjusted to pH of 8-9 with saturated sodium bicarbonate solution and extracted with EA. The organic phase was dried with anhydrous sodium sulfate, then dried with a rotary evaporator under reduced pressure, dissolved with a small amount of methanol, separated and purified by HPLC, added with 1 N HCl and lyophilized to obtain (4aS,8aS)-4a-(2-chlorophenyl)octahydro-2*H*-benzo[*b*][1,4]oxazine hydrochloride **(44).** LCMS: m/z = 252 (M+H)⁺.

### Biological experimental methods

### Test Example 1: detection of NMDA receptor-mediated electrical currents on HEK293 cells

### 1. Expression of human NMDA receptors GluN1 and GluN2A subunits in HEK293 cells

On the day before transfection, HEK293 cells were seeded in a 24-well plate with a 10 mm glass sheet, which was coated with polylysine in advance to increase the adhesion capacity of the cells. The plate was added with DMEM medium containing 10% FBS and incubated at 37 °C in 5% CO₂ incubator. When the cell fusion reached 60%-70% after 18-24 hours, the plasmid cDNAs containing TRE-GluN1-GluN2A of human GluN1 and GluN2A, tTA, and pCAG-EGFP were transfected into HEK293 by liposome transfection (TRE-GluN1-GluN2A: tTA: pCAG-EGFP is 0.2 µg : 0.2 µg : 0.02 µg). Before transfection, a new DMEM medium was changed and added with 1 µg/mL Dox (doxycycline) to induce stable expression in the medium, and added with 100 µM D-APV and 10 mM MgCl₂ to avoid cell excitatory toxicity caused by NMDAR overexpression. Cells with green fluorescence were selected for electrophysiological recording after transfection for about 24 h.

### 2. Whole-cell patch-clamp recording of HEK293 cells

A glass plate seeded with HEK293 cells was placed in a recording bath and perfused with the extracellular fluid at a rate of 4 mL/min (NaCl 140 mM, KCl 2.8 mM, HEPES 10 mM, CaCl₂ 1 mM, glycine 0.1 mM, pH 7.2). The channels of the ALA-VC³ eight-channel pressure perfusion system were filled with extracellular fluid for washing, extracellular fluid containing glutamic acid (100 µM), and extracellular fluid containing glutamic acid and compound to be tested, respectively, with a drop rate of about 1 drop/sec and 10 µL/drop. HEK293 cells with moderate green fluorescence intensity and good adhesion were selected under a fluorescence microscope for recording. The microglass tube of the perfusion system was placed about ten cells away from the cells to be recorded, and the drug was sprayed to the cell surface under the pressure of air (0.02 MPa). The glass electrode was prepared by drawing with a microelectrode drawing instrument with a resistance of 4-6 MS2 and filled with electrode liquid (CsCl 125 mM, HEPES 10 mM, EGTA 11 mM). The microelectrode manipulator was used to slowly push the glass electrode towards the cells. The electrode was attached to the cell membranes and applied with negative pressure, so that a high-resistance seal of more than 1 GS2 was formed between the electrode tip and the cell membrane, and then negative pressure was given to suck through the cell membrane to form a whole-cell recording mode. After the formation of whole-cell recording mode, the cell membrane potential was clamped at -70 mV. The extracellular fluid containing 100 µM glutamic acid, the extracellular fluid containing 100 µM glutamic acid and the compound to be tested, and the extracellular fluid were sprayed to the cell membrane surface sequentially through the perfusion system for 10 s, 20 s, and 10 s to record the NMDA receptor-mediated current and evaluate the inhibitory effect of the compound on GluN2A-containing NMDAR. The experimental process was controlled by pCLAMP 10.6, and the Digidata 1440A digital-to-analog converter was used to complete the generation of stimulus signal and the acquisition of feedback signal.

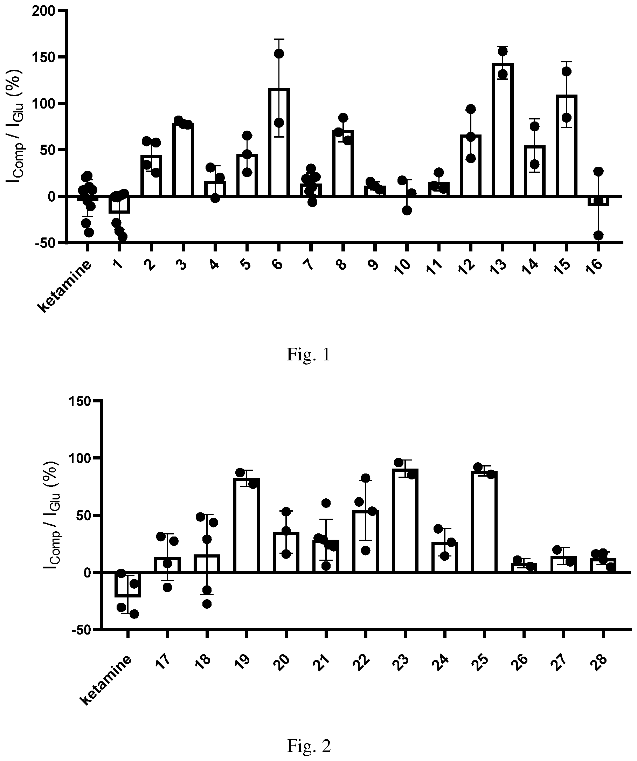

The test results of some compounds are shown in Figures 1 and 2. The ordinate in the figure is the normalized current ratio, which represents the ratio of the current mediated by NMDAR after administration of ketamine and the compound at a concentration of 10 µM to the current mediated by NMDAR before administration. The stronger the NMDAR-mediated current inhibitory effect of the compound, the smaller the ratio. Ketamine was used as a positive control. The results showed that Compounds 1 and 16 had similar inhibitory effects to ketamine, Compounds 4, 7, 9-11, 17 and 26-28 had stronger inhibitory effects, and Compounds 2, 3, 5, 8, 12, 14, 18, 20-22 and 24 had different degrees of inhibitory effects on NMDAR-mediated currents.

The inhibitory percentages of the compound according to the present disclosure on NMDAR-mediated current = (NMDAR mediated-current after administration)/ NMDAR mediated-current before administration *100%. The results showed that Compounds 1, 10, 16, 26, 32, 35, 37, 41 and 42 had similar inhibitory effects to ketamine.

The inhibitory percentages of each compound on NMDAR-mediated current are shown in Table 1.

**Table 1: Inhibitory percentages of each compound on NMDAR-mediated current**

| Compound No. | current inhibitory percentages | Compound No. | current inhibitory percentages |
|---|---|---|---|
| Ketamine | 101.8% | 23 | 9.25% |
| 1 | 115.35% | 24 | 73.72% |
| 2 | 55.97% | 25 | 11.21% |
| 3 | 21.2% | 26 | 91.96% |
| 4 | 83.69% | 27 | 85.6% |
| 5 | 54.53% | 28 | 87.76% |
| 7 | 86.39% | 29 | 85.65% |
| 8 | 28.90% | 30 | 86.62% |
| 9 | 88.76% | 31 | 71.58% |
| 10 | 98.32% | 32 | 101.54% |
| 11 | 85.0% | 33 | 76.44% |
| 12 | 33.73% | 34 | 48.61% |
| 14 | 45.21% | 35 | 118.33% |
| 16 | 106.94% | 36 | 42.53% |
| 17 | 86.6% | 37 | 100.09% |
| 18 | 84.42 | 38 | 88.60% |
| 19 | 17.79% | 39 | 81.89% |
| 20 | 64.86% | 40 | 89.28% |
| 21 | 71.47% | 41 | 92.93% |
| 22 | 45.82% | 42 | 104.88% |

### Test example 2: High dose toxicity test

1. Experiments were performed in a quiet environment where animals needed to be weighed and acclimatized in the laboratory room for at least 30 min before the experiment.
2. The compound to be tested was dissolved with 0.9% physiological saline at a concentration of 10mg/ml. According to the body weight of mice, a corresponding volume of the compound was intraperitoneal injected (i.p.), so as to provide the target dose.
3. The mice after administration were placed in transparent observation boxes, and the behavioral changes of the mice were recorded by video and observed for about one hour. (Note: During the observation period, if a mouse was lying still on the stomach, the mouse was turned over with your hand to check whether the righting reflex (RR) of the mouse disappeared. If it disappeared, the mouse was in the state of anesthesia, the time of anesthesia in the mouse was recorded).
4. After one hour of observation, the mice were returned to the feeding cage and the observation boxes were wiped with 75% alcohol so that the information left over from the previous round (e.g. animal stool, urine and other odors) would not affect the next test results. Animals were replaced for the next round to continue the experiment.

High-dose (150 mg/kg bw) toxicity test results are summarized in Table 2. Mice died after ketamine administration. After administration of Compound 9, it was observed that the state of anesthesia of mice returned to normal after half an hour. After administration of Compound 11, the activity reduction in mice lasted for half an hour before returning to normal. The results show that the compound of the present disclosure has a better safety profile than ketamine.

**Table 2**

| Compound | behavior during observation |
|---|---|
| Compound 9 | the state of anesthesia lasted for half an hour |
| Compound 11 | the activity reduction lasted for half an hour |
| Ketamine | dead |

The test results for the medium dose (100 mg/kg body weight) are shown in Table 3. Compound 9 caused behaviors such as intermittent shaky walking, lying still on the stomach, and tail stiffness (seizure for about 10 seconds) in mice. According to Rodent Epileptiform Behavior Scale score, Compound 9 induced epileptiform behavior in mice with a maximum score of 3. However, there were no obvious abnormalities after administration of its chiral isomers Compound 41 and 42, and the mice only showed slightly shaky walking. According to the Rodent Epileptiform Behavior Scale score, Compound 41 and 42 induced epileptiform behavior in mice with a maximum score of 1. Compound 11 caused decreased activity in mice, and Compound 11 caused epileptiform behavior in mice with a maximum score of 1. However, there were no obvious abnormalities after administration of its chiral isomers Compound 43 and 44. It showed that the chiral isomer of the compound according to the present disclosure had a better safety profile than the racemate. The epileptiform behavior was scored according to the Rodent Epileptiform Behavior Scale score.

**Table 3: Summary of the behavior of mice during observation period after administration**

| Compound | behavior during observation | Compound No. | behavior during observation |
|---|---|---|---|
| Compound 9 | Moderate epilepsy (score of 3) | 11 | Mild epilepsy (score of 1) |
| Compound 41 | Slight shaking, score between 0∼1 | 43 | No abnormal |
| Compound 42 | Slight shaking, score between 0∼1 | 44 | No abnormal |

| Rodent Epileptiform Behavior Scale score | | | |
|---|---|---|---|
| score | Behavioral phenotype | | |
| 0 | No abnormal behavior | | |
| 1 | Unsteady walking/reduced exercise ability/immobility | | |
| 2 | Generalized clonus/trembling/writhing | | |
| 3 | The tail is stiff/swinging | | |
| 4 | Forelimb contractures | | |
| 5 | Generalized spasms/convulsions | | |
| 6 | Startle or running convulsive seizures | | |
| 7 | Generalized tonic-clonic seizures | | |
| 8 | dead | | |

## Claims

1. A compound of Formula I, or a pharmaceutically acceptable salt thereof, or an enantiomer, a diastereomer, a tautomer, a solvate, an isotope substitute, a polymorph, a prodrug or a metabolite thereof: 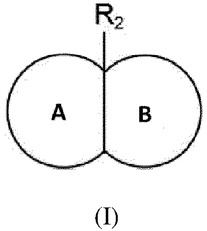 wherein:
ring A is a substituted or unsubstituted 4-10 membered heterocyclic group containing 1-3 heteroatoms selected from the group consisting of N, O and S;
ring B is a substituted or unsubstituted, saturated or unsaturated 3-10 membered carbon ring;
R₂ is a substituted or unsubstituted 3-8 membered cycloalkyl, substituted or unsubstituted 6-14 membered aryl, substituted or unsubstituted 5-10 membered heteroaryl, or substituted or unsubstituted 4-10 membered heterocyclic group.

2. The compound according to claim 1, or a pharmaceutically acceptable salt thereof, or an enantiomer, a diastereomer, a tautomer, a solvate, an isotope substitute, a polymorph, a prodrug or a metabolite thereof, wherein
ring A contains 1 or 2 heteroatoms selected from the group consisting of N and O; preferably, the ring atom number of ring A is 5-8; preferably, ring A is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl; and/or
ring B is a 5-8 membered saturated carbon ring; preferably, ring B is a 6 membered saturated carbon ring; preferably, ring B is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl; and/or
R₂ is 3-8 membered cycloalkyl , 6-14 membered aryl or 5-10 membered heteroaryl, preferably phenyl, naphthyl, thiophenyl or furanyl; preferably, R₂ is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl.

3. The compound according to claim 2, or a pharmaceutically acceptable salt thereof, or an enantiomer, a diastereomer, a tautomer, a solvate, an isotope substitute, a polymorph, a prodrug or a metabolite thereof, wherein
ring A contains one N atom and one O atom, the number of ring atom in ring A is 5-8 and ring A is optionally substituted with 1-3 C1-C4 alkyl;
ring B is a 5-8 membered saturated carbon ring, and ring B is optionally substituted with 1-3 C1-C4 alkyl;
R₂is 6-14 membered aryl or 5-10 membered heteroaryl, preferably phenyl or thiophenyl, and R₂is optionally substituted by 1-3 substituents selected from the group consisting of C1-C4 alkoxy, halogen, C1-C4 alkyl, halogenated C1-C4 alkyl and halogenated C1-C4 alkoxy.

4. The compound according to claim 1, or a pharmaceutically acceptable salt thereof, or an enantiomer, a diastereomer, a tautomer, a solvate, an isotope substitute, a polymorph, a prodrug or a metabolite thereof, wherein the compound of Formula I has the structure represented by the following Formula II: 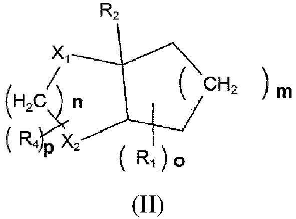 wherein:
X₁ is NH or O;
X₂ is NH or O;
each R₁ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
R₂ is as defined according to any one of claims 1-3;
each R₄ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
n and m are each independently 1, 2 or 3;
o and p are each independently 0, 1, 2 or 3.

5. The compound according to claim 4, or a pharmaceutically acceptable salt thereof, or an enantiomer, a diastereomer, a tautomer, a solvate, an isotope substitute, a polymorph, a prodrug or a metabolite thereof, wherein,
R₁ is selected from the group consisting of hydroxyl, halogen, C1-C4 alkyl, C1-C4 alkoxy, C2-C4 alkenyl and C2-C4 alkynyl; preferably, R₁ is C1-C4 alkyl; and/or
o is 0, 1 or 2; and/or
R₂ is 3-8 membered cycloalkyl , 6-14 membered aryl or 5-10 membered heteroaryl, preferably phenyl, naphthyl, thiophenyl or furanyl; preferably, R₂ is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl; preferably, R₂ is optionally substituted by 1-3 substituents selected from the group consisting of C1-C4 alkoxy, halogen, C1-C4 alkyl, halogenated C1-C4 alkyl and halogenated C1-C4 alkoxy; and/or
each R₄ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl; preferably, each R₄ is independently C1-C4 alkyl; and/or
p is 0, 1 or 2.

6. The compound according to claim 4, or a pharmaceutically acceptable salt thereof, or an enantiomer, a diastereomer, a tautomer, a solvate, an isotope substitute, a polymorph, a prodrug or a metabolite thereof, wherein the compound of Formula II has the structure represented by the following Formula III: 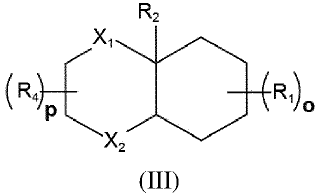 wherein:
X₁ is NH or O;
X₂ is NH or O;
each R₁ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
R₂ is as defined according to claim 4;
each R₄ is as defined according to claim 4;
o and p are each independently 0, 1, 2 or 3.

7. The compound according to claim 6, or a pharmaceutically acceptable salt thereof, or an enantiomer, a diastereomer, a tautomer, a solvate, an isotope substitute, a polymorph, a prodrug or a metabolite thereof, wherein,
each R₁ is independently selected from the group consisting of hydroxyl, halogen, C1-C4 alkyl, C1-C4 alkoxy, C2-C4 alkenyl and C2-C4 alkynyl; preferably, R₁ is C1-C4 alkyl; and/or
o is 0, 1 or 2; and/or
R₂ is 3-8 membered cycloalkyl, 6-14 membered aryl or 5-10 membered heteroaryl, preferably phenyl, naphthyl, thiophenyl or furanyl; preferably, R₂ is optionally substituted by 1-3 substituents selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl; preferably, R₂ is optionally substituted by 1-3 substituents selected from the group consisting of C1-C4 alkoxy, halogen, C1-C4 alkyl, halogenated C1-C4 alkyl and halogenated C1-C4 alkoxy; and/or
each R₄ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl; preferably, each R₄ is independently C1-C4 alkyl; and/or
p is 0, 1 or 2.

8. The compound according to any one of claims 1-7, or a pharmaceutically acceptable salt thereof, or an enantiomer, a diastereomer, a tautomer, a solvate, an isotope substitute, a polymorph, a prodrug or a metabolite thereof, wherein R₂ is phenyl or thiophenyl.

9. The compound according to any one of claims 1-8, or a pharmaceutically acceptable salt thereof, or an enantiomer, a diastereomer, a tautomer, a solvate, an isotope substitute, a polymorph, a prodrug or a metabolite thereof, wherein R₂ is phenyl.

10. The compound according to any one of claims 1-9, or a pharmaceutically acceptable salt thereof, or an enantiomer, a diastereomer, a tautomer, a solvate, an isotope substitute, a polymorph, a prodrug or a metabolite thereof, wherein R₂ is optionally substituted with halogen.

11. The compound according to any one of claims 1-10, or a pharmaceutically acceptable salt thereof, or an enantiomer, a diastereomer, a tautomer, a solvate, an isotope substitute, a polymorph, a prodrug or a metabolite thereof, wherein R₂ is optionally substituted with 1, 2 or 3 Cl.

12. The compound according to claim 6, or a pharmaceutically acceptable salt thereof, or an enantiomer, a diastereomer, a tautomer, a solvate, an isotope substitute, a polymorph, a prodrug or a metabolite thereof, wherein the compound of Formula III has the structure represented by Formula IIIa or IIIIb: 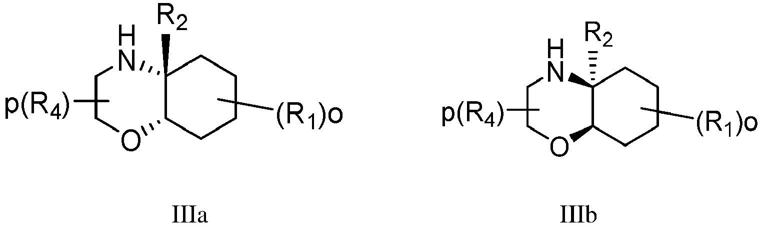 wherein R₁, R₂, R₄, o and p are as defined according to Example 6 or 7.

13. The compound according to claim 8, or a pharmaceutically acceptable salt thereof, or an enantiomer, a diastereomer, a tautomer, a solvate, an isotope substitute, a polymorph, a prodrug or a metabolite thereof, wherein o is 0, i.e., R₁ is not present, or o is 1, R₁ is methyl, such as 6-methyl; R₂ is 6-14 membered aryl or 5-10 membered heteroaryl that is optionally substituted with 1 or 2 substituents selected from the group consisting of F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position, preferably, the 6-14 membered aryl is phenyl or naphthyl, the 5-10 membered heteroaryl is thiophenyl or furanyl; p is 0, i.e., R₄ is not present.

14. The compound according to claim 12 or 13, or a pharmaceutically acceptable salt thereof, or an enantiomer, a diastereomer, a tautomer, a solvate, an isotope substitute, a polymorph, a prodrug or a metabolite thereof, wherein R₂ is phenyl or thiophenyl optionally substituted with Cl in ortho position and/or Cl in meta position.

15. The compound according to claim 6, or a pharmaceutically acceptable salt thereof, or an enantiomer, a diastereomer, a tautomer, a solvate, an isotope substitute, a polymorph, a prodrug or a metabolite thereof, wherein the compound of Formula III has the structure represented by the following Formula IV, IVa or IVb: 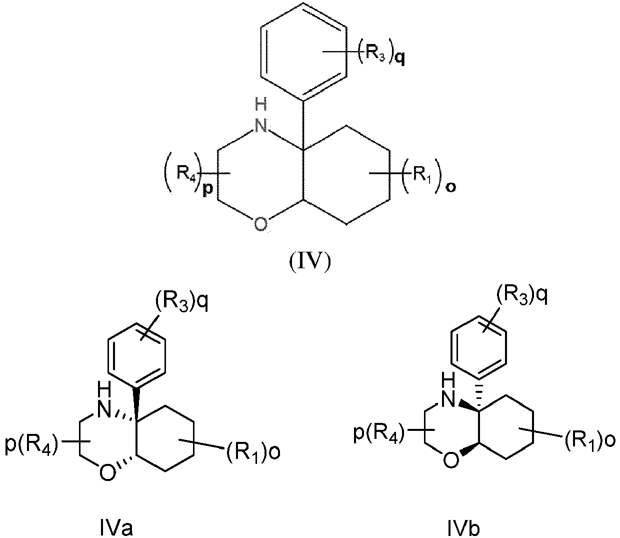 wherein:
R₁ is as defined according to claim 6;
each R₃ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
each R₄ is independently selected from the group consisting of hydroxyl, halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl, C2-C4 alkynyl, -NRₐR_{b}, carboxyl, cyano, 6-14 membered aryl, 5-10 membered heteroaryl, 4-10 membered heterocyclic group and C1-C6 acyl, wherein Rₐ and R_{b} are each independently selected from the group consisting of H, C1-C4 alkyl, halogenated C1-C4 alkyl and hydroxyl-substituted C1-C4 alkyl;
o, p and q are each independently 0, 1, 2 or 3.

16. The compound according to claim 15, or a pharmaceutically acceptable salt thereof, or an enantiomer, a diastereomer, a tautomer, a solvate, an isotope substitute, a polymorph, a prodrug or a metabolite thereof, wherein,
each R₁ is independently selected from the group consisting of hydroxyl, halogen, C1-C4 alkyl, C1-C4 alkoxy, C2-C4 alkenyl and C2-C4 alkynyl; preferably, R₁ is C1-C4 alkyl; and/or
o is 0, 1 or 2; and/or
each R₃ is independently selected from the group consisting of halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl and C2-C4 alkynyl; preferably, each R₃ is independently selected from the group consisting of C1-C4 alkoxy, halogen, C1-C4 alkyl, halogenated C1-C4 alkyl and halogenated C1-C4 alkoxy; and/or
q is 0, 1 or 2; and/or
each R₄ is independently halogen, C1-C4 alkyl, halogenated C1-C4 alkyl, hydroxyl-substituted C1-C4 alkyl, C1-C4 alkoxy, halogenated C1-C4 alkoxy, C2-C4 alkenyl or C2-C4 alkynyl; preferably, each R₄ is independently C1-C4 alkyl; and/or
p is 0, 1 or 2.

17. The compound according to claim 15, or a pharmaceutically acceptable salt thereof, or an enantiomer, a diastereomer, a tautomer, a solvate, an isotope substitute, a polymorph, a prodrug or a metabolite thereof, wherein, o is 0, i.e., R₁ is not present, or o is 1, R₁ is methyl, such as 6-methyl; q is 0, i.e., R₃ is not present, or q is 1 or 2, each R₃ is independently selected from the group consisting of halogen (such as F, Cl), C1-C4 alkyl, C1-C3 alkoxy, halogenated C1-C3 alkyl and halogenated C1-C3 alkoxy, preferably 1 or 2 substituents selected from the group consisting of F in ortho position, F in meta position, F in para position, Cl in ortho position, Cl in meta position, C1-C3 alkoxy in ortho position, C1-C3 alkoxy in meta position, C1-C3 alkoxy in para position, C1-C4 alkyl in ortho position, C1-C4 alkyl in meta position, C1-C4 alkyl in para position, halogenated C1-C3 alkyl in para position and halogenated C1-C3 alkoxy in meta position; p is 0, i.e., R₄ is not present.

18. The compound according to claim 1, or a pharmaceutically acceptable salt thereof, or an enantiomer, a diastereomer, a tautomer, a solvate, an isotope substitute, a polymorph, a prodrug or a metabolite thereof:
4*a*-phenyloctahydro-2*H*-benzo[*b*][1,4]oxazine;
4*a*-(2-methoxyphenyl)octahydro-2*H*-benzo[*b*][1,4]oxazine;
4*a*-(3-fluorophenyl)octahydro-2*H*-benzo[*b*][1,4]oxazine;
4*a*-(4-fluorophenyl)octahydro-2*H*-benzo[b][1,4]oxazine;
4*a*-(2,3-difluorophenyl)octahydro-2*H*-benzo[*b*] [1,4]oxazine;
4*a*-(2-fluorophenyl)octahydro-2*H*-benzo[*b*][1,4]oxazine;
6-methyl-4a-phenyloctahydro-2H-benzo[b] [1,4]oxazine;
6-ethyl-4*a*-phenyloctahydro-2*H*-benzo[*b*][1,4]oxazine;
4*a*-(3-chlorophenyl)octahydro-2*H*-benzo[b][1,4]oxazine;
4*a*-(3-methylphenyl)octahydro-2*H*-benzo[*b*][1,4]oxazine;
4*a*-(2-chlorophenyl)octahydro-2*H*-benzo[*b*][1,4]oxazine;
4*a*-(2-methylphenyl)octahydro-2*H*-benzo[b][1,4]oxazine;
4*a*-(4-chlorophenyl)octahydro-2*H*-benzo[*b*][1,4]oxazine;
4*a*-(4-methylphenyl)octahydro-2*H*-benzo[*b*][1,4]oxazine;
4*a*-(3-(trifluoromethyl)phenyl)octahydro-2*H*-benzo[*b*][1,4]oxazine;
8-methyl-4*a*-phenyloctahydro-2*H*-benzo[*b*][1,4]oxazine;
5-methyl-4*a*-phenyloctahydro-2*H*-benzo[*b*][1,4]oxazine;
7-methyl-4*a*-phenyloctahydro-2*H*-benzo[*b*][1,4]oxazine;
4-methyl-4*a*-phenyloctahydro-2*H*-benzo[b][1,4]oxazine;
3,3-dimethyl-5*a*-phenyldecahydrobenzo[*b*][1,4]olanzapine;
4*a*-(3-methoxyphenyl)octahydro-2*H*-benzo[*b*][1,4]oxazine;
4*a*-(3-(trifluoromethoxy)phenyl)octahydro-2*H*-benzo[*b*][1,4]oxazine;
4*a*-(4-(trifluoromethyl)phenyl)octahydro-2*H*-benzo[*b*][1,4]oxazine;
4*a*-(2,6-dimethylphenyl)octahydro-2*H*-benzo[*b*][1,4]oxazine;
4*a*-(4-(tertbutyl)phenyl)octahydro-2*H*-benzo[*b*][1,4]oxazine;
4*a*-(2,3-dichlorophenyl)octahydro-2*H*-benzo[*b*][1,4]oxazine;
4*a*-(2-isopropylphenyl)octahydro-2*H*-benzo[*b*][1,4]oxazine;
4*a*-(2,5-dimethylphenyl)octahydro-2*H*-benzo[*b*][1,4]oxazine;
4*a*-(2-chloro-3-fluorophenyl)octahydro-2*H*-benzo[*b*][1,4]oxazine;
4*a*-(3,4-difluorophenyl)octahydro-2*H*-benzo[*b*][1,4]oxazine;
6,6-dimethyl-4*a*-phenyloctahydro-2*H*-benzo[*b*][1,4]oxazine;
4*a*-(2-chloro-5-fluorophenyl)octahydro-2*H*-benzo[*b*][1,4]oxazine;
4*a*-(3-ethoxyphenyl)octahydro-2*H*-benzo[*b*][1,4]oxazine;
4*a*-(2-chloro-4-methoxyphenyl)octahydro-2*H*-benzo[*b*][1,4]oxazine;
(4aR,8aS)-4*α*-(2-thiophenyl)octahydro-2*H*-benzo*[*b][1,4]oxazine;
4*a*-(2-chloro-6-fluorophenyl)octahydro-2*H*-benzo[*b*][1,4]oxazine;
(4aS,8aS)-4*α*-(2-chloro-3-thiophenyl)octahydro-2*H*-benzo[*b*][1,4]oxazine;
(4aS,8aR)-4*α*-(2-thiophenyl)octahydro-2*H*-benzo[*b*][1,4]oxazine;
(4aR,8aS)-4*α*-(3-methyl-2-thiophenyl)octahydro-2*H*-benzo[*b*][1,4]oxazine;
(4aR,8aR)-4*α*-(4-methyl-3-thiophenyl)octahydro-2*H*-benzo[*b*][1,4]oxazine;
(4aR,8aR)-4*α*-(3-chlorophenyl)octahydro-2*H*-benzo[*b*][1,4]oxazine;
(4aS,8aS)-4*α*-(3-chlorophenyl)octahydro-2*H*-benzo[*b*][1,4]oxazine;
(4aR,8aR)-4*α*-(2-chlorophenyl)octahydro-2*H*-benzo[*b*][1,4]oxazine; and
(4aS,8aS)-4*α*-(2-chlorophenyl)octahydro-2*H*-benzo[*b*][1,4]oxazine;
preferably, the pharmaceutically acceptable salt thereof includes:
4*a*-phenyloctahydro-2*H*-benzo[b][1,4]oxazine hydrochloride;
4*a*-(2-methoxyphenyl)octahydro-2*H*-benzo[*b*][1,4]oxazinehydrochloride;
4*a*-(3-fluorophenyl)octahydro-2*H*-benzo[*b*][1,4]oxazine hydrochloride;
4*a*-(4-fluorophenyl)octahydro-2*H*-benzo[*b*][1,4]oxazine formate;
4*a*-(2,3-difluorophenyl)octahydro-2*H*-benzo[*b*] [1,4]oxazine formate;
4*a*-(2-fluorophenyl)octahydro-2*H*-benzo[*b*][1,4]oxazine formate;
6-methyl-4*a*-phenyloctahydro-2*H*-benzo[*b*][1,4]oxazine formate;
6-ethyl-4*a*-phenyloctahydro-2*H*-benzo[*b*][1,4]oxazine formate;
4*a*-(3-chlorophenyl)octahydro-2*H*-benzo[*b*][1,4]oxazine formate;
4*a*-(3-methylphenyl)octahydro-2*H*-benzo[*b*][1,4]oxazine formate;
4*a*-(2-chlorophenyl)octahydro-2*H*-benzo[*b*][1,4]oxazine formate;
4*a*-(2-methylphenyl)octahydro-2*H*-benzo[*b*][1,4]oxazine formate;
4*a*-(4-chlorophenyl)octahydro-2*H*-benzo[*b*][1,4]oxazine formate;
4*a*-(4-methylphenyl)octahydro-2*H*-benzo[*b*][1,4]oxazine formate;
4*a*-(3-(trifluoromethyl)phenyl)octahydro-2*H*-benzo[*b*][1,4]oxazine hydrochloride;
8-methyl-4*a*-phenyloctahydro-2*H*-benzo[*b*][1,4]oxazine formate;
5-methyl-4*a*-phenyloctahydro-2*H*-benzo[*b*][1,4]oxazine formate;
7-methyl-4*a*-phenyloctahydro-2*H*-benzo[*b*][1,4]oxazine formate;
4-methyl-4*a*-phenyloctahydro-2*H*-benzo[*b*][1,4]oxazine hydrochloride;
3,3-dimethyl-5*a*-phenyldecahydrobenzo[*b*][1,4]olanzapine hydrochloride;
4*a*-(3-methoxyphenyl)octahydro-2*H*-benzo[*b*][1,4]oxazine hydrochloride;
4*a*-(3-(trifluoromethoxy)phenyl)octahydro-2*H*-benzo[*b*][1,4]oxazine hydrochloride;
4*a*-(4-(trifluoromethyl)phenyl)octahydro-2*H*-benzo[*b*][1,4]oxazine hydrochloride;
4*a*-(2,6-dimethylphenyl)octahydro-2*H*-benzo[*b*][1,4]oxazine hydrochloride;
4*a*-(4-(tertbutyl)phenyl)octahydro-2*H*-benzo[*b*][1,4]oxazine hydrochloride;
4*a*-(2,3-dichlorophenyl)octahydro-2*H*-benzo[*b*][1,4]oxazine hydrochloride;
4*a*-(2-isopropylphenyl)octahydro-2*H*-benzo[*b*][1,4]oxazine hydrochloride;
4*a*-(2,5-dimethylphenyl)octahydro-2*H*-benzo[*b*][1,4]oxazine hydrochloride;
4*a*-(2-chloro-3-fluorophenyl)octahydro-2*H*-benzo[*b*] [1,4]oxazine hydrochloride;
4*a*-(3,4-difluorophenyl)octahydro-2*H*-benzo[*b*][1,4]oxazine hydrochloride;
6,6-dimethyl-4*a*-phenyloctahydro-2*H*-benzo[*b*][1,4]oxazine hydrochloride;
4*a*-(2-chloro-5-fluorophenyl)octahydro-2*H*-benzo[*b*][1,4]oxazine hydrochloride;
4*α*-(3-ethoxyphenyl)octahydro-2*H*-benzo[*b*][1,4]oxazine hydrochloride;
4*α*-(2-chloro-4-methoxyphenyl)octahydro-2*H*-benzo[*b*][1,4]oxazine hydrochloride;
(4aR,8aS)-4*α*-(2-thiophenyl)octahydro-2*H*-benzo[*b*][1,4]oxazine hydrochloride;
4*α*-(2-chloro-6-fluorophenyl)octahydro-2*H*-benzo[*b*][1,4]oxazine hydrochloride;
(4aS,8aS)-4*α*-(2-chloro-3-thiophenyl)octahydro-2*H*-benzo[*b*][1,4]oxazine hydrochloride;
(4aS,8aR)-4*α*-(2-thiophenyl)octahydro-2*H*-benzo[*b*][1,4]oxazine hydrochloride;
(4aR,8aS)-4*α*-(3-methyl-2-thiophenyl)octahydro-2*H*-benzo[*b*][1,4]oxazine hydrochloride;
(4aR,8aR)-4*α*-(4-methyl-3-thiophenyl)octahydro-2*H*-benzo[b][1,4]oxazine hydrochloride;
(4aR,8aR)-4*α*-(3-chlorophenyl)octahydro-2*H*-benzo[*b*][1,4]oxazine hydrochloride;
(4aS,8aS)-4*α*-(3-chlorophenyl)octahydro-2*H*-benzo[*b*][1,4]oxazine hydrochloride;
(4aR,8aR)-4*α*-(2-chlorophenyl)octahydro-2*H*-benzo[*b*][1,4]oxazine hydrochloride; and
(4aS,8aS)-4*α*-(2-chlorophenyl)octahydro-2*H*-benzo[*b*][1,4]oxazine hydrochloride.

19. A pharmaceutical composition comprising the compound according to any one of claims 1-18, or a pharmaceutically acceptable salt thereof, or an enantiomer, a diastereomer, a tautomer, a solvate, an isotope substitute, a polymorph, a prodrug or a metabolite thereof, and a pharmaceutically acceptable carrier.

20. The pharmaceutical composition according to claim 19, wherein the pharmaceutical composition is an anesthetic or analgesic.

21. Use of the compound according to any one of claims 1-18, or a pharmaceutically acceptable salt thereof, or an enantiomer, a diastereomer, a tautomer, a solvate, an isotope substitute, a polymorph, a prodrug or a metabolite thereof in the manufacture of drugs for treating or preventing NMDA receptor mediated diseases, or in the manufacture of anesthetics or analgesics.

22. Use according to claim 21, wherein the NMDA receptor mediated disease is selected from the group consisting of cerebral ischemia, traumatic brain injury, infarction, stroke, Alzheimer's disease, Parkinson's disease, Huntington's disease, depression, anxiety, bipolar disorder, schizophrenia, autism, epilepsy, anti-NMDA receptor encephalitis, neuropathic pain and other neurological events or neurodegeneration caused by NMDA receptor activation; preferably, the NMDA receptor-mediated disorders are depression, schizophrenia, or epilepsy.
